(19) Europäisches Patentamt
European Patent Office
Office européen des brevets

(11) EP 4 212 525 A1

(12) EUROPEAN PATENT APPLICATION
published in accordance with Art. 153(4) EPC

(43) Date of publication:
**19.07.2023 Bulletin 2023/29**

(21) Application number: **21865915.9**

(22) Date of filing: **02.09.2021**

(51) International Patent Classification (IPC):
***C07D 403/06*** *(2006.01)* ***A61K 31/404*** *(2006.01)*
***A61P 29/00*** *(2006.01)*

(52) Cooperative Patent Classification (CPC):
**A61K 31/404; A61K 31/4155; A61K 31/416; A61K 31/428; A61K 31/437; A61K 31/4439; A61K 31/444; A61K 31/454; A61K 31/4709; A61K 31/496; A61K 31/5025; A61K 31/506; A61K 31/517; A61K 31/519; A61K 31/53;** (Cont.)

(86) International application number:
**PCT/CN2021/116256**

(87) International publication number:
**WO 2022/052861 (17.03.2022 Gazette 2022/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.09.2020 CN 202010943478**
**20.04.2021 CN 202110426935**

(71) Applicant: **Origiant Pharmaceutical Co., Ltd**
**Chengdu, Sichuan 610000 (CN)**

(72) Inventors:
- **YANG, Shengyong**
**Chengdu, Sichuan 610000 (CN)**
- **LI, Linli**
**Chengdu, Sichuan 610000 (CN)**

(74) Representative: **Elzaburu S.L.P.**
**Edificio Torre de Cristal**
**Paseo de la Castellana 259 C, planta 28**
**28046 Madrid (ES)**

# (54) 5-SUBSTITUTED INDOLE 3-AMIDE DERIVATIVE, PREPARATION METHOD AND USE THEREOF

(57) Provided are a 5-substituted indole 3-amide derivative, a preparation method and a use thereof, belonging to the field of medicine. A compound represented by formula I or a pharmaceutically acceptable salt thereof is provided. This type of compound can significantly inhibit the activity of RIPK1 kinase, has high selectivity and excellent safety, serves as a RIPK1 kinase inhibitor, and can be used as a potential therapeutic drug for inflammation, immune diseases, tumors and neurodegenerative diseases. TNFα-induced SIRS model experiments proved that the compound can inhibit a RIPK1 kinase in vivo. Pharmacokinetic results showed that this series of compounds has excellent pharmacokinetic properties, thus providing a novel strategy and means for disease treatments targeting RIPK1.

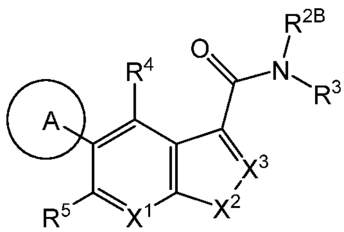

I

EP 4 212 525 A1

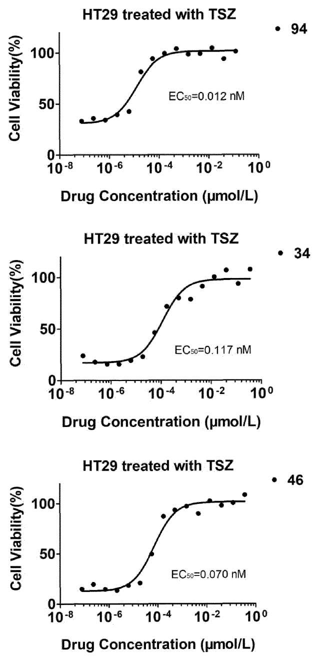


FIG. 3

(52) Cooperative Patent Classification (CPC): (Cont.)
**A61K 31/5377; A61P 1/00; A61P 25/28; A61P 29/00; A61P 35/00; A61P 37/06; C07D 209/42; C07D 231/56; C07D 401/04; C07D 403/04; C07D 403/06; C07D 405/14; C07D 413/04; C07D 417/04; C07D 417/14; C07D 471/04; C07D 487/04; C07D 519/00**

## Description

### Technical Field

The present invention belongs to the field of medicines, and in particular, relates to 5-substituted indole 3-amide derivatives and a preparation method therefor and a use thereof.

### Background Art

Programmed necrosis ( or Necroptosis) is a new caspase-independent mode of programmed cell death discovered in recent years, which is different from apoptosis. It is regulated by death signals and exhibits necrosis-like structural features. Compared with apoptosis, there are no apoptotic bodies and no chromatin condensation during programmed necrosis; in contrast to necrosis, programmed necrosis is a controlled mode of cell death regulated by a variety of genes. After the addition of the caspase inhibitor Z-VAD-FMK into the in vitro culture system, programmed necrosis is induced by TNF, with morphological features of necrosis exhibited by cells including cell swelling, rupture, and release of cellular contents, which in turn cause inflammation and immune responses. In addition to TNF, ligands of TLR3 and TLR4, certain bacteria, viral infections, etc. can cause programmed necrosis.

Receptor-interacting protein kinase 1 (RIPK1) is a protein with specific serine/threonine kinase activity, which has an N-terminal kinase domain like other protein kinases but a different binding domain. Studies have shown that RIPK1 is a key regulator for programmed necrosis, which regulates programmed necrosis through the RIPK1/RIPK3/MLKL signal transduction axis. Various death receptors, such as TNFR, FAS, TRAILR, and Toll-like receptors, can trigger upstream signals of programmed necrosis upon stimulation by inflammatory factors or exogenous infection. In cases of limited cIAPs and caspase activity, RIPK1 and RIPK3 form necrosomes. RIPK3 further recruits MLKL, and the phosphorylated MLKL will self-oligomerize and migrate to the cell membrane for "perforating" the cell membrane, leading to leakage of cell contents and disruption of the ionization equilibrium, eventually leading to cell necrosis.

Programmed necrosis is closely related to the occurrence and development of inflammation, autoimmune diseases, neurodegenerative diseases, tumor and other related diseases. For example, studies have shown that programmed necrosis is a significant cause of systemic inflammatory response syndrome (SIRS). SIRS is a systemic inflammatory response that is caused by the fact that infection or non-infection factors act on the body to cause the body to be out of control and self-destructed. Critical patients are prone to SIRS due to the decreased ability of compensatory anti-inflammatory response and metabolic dysfunction. The TNF $\alpha$-induced SIRS disease model was shown to be highly correlated with RIPK1-dependent programmed necrosis in long-term studies. Inflammatory bowel disease (IBD) refers to abnormal immune-mediated intestinal inflammation caused by environmental, genetic, infection, immune, and other reasons, which is a chronic, non-specific inflammatory bowel disease. The pathogenesis of IBD is not clear yet, but excessive apoptosis of intestinal epithelial cells, impaired intestinal mucosal barrier, and increased permeability of intestinal epithelial cells are considered to be one of the causes of IBD. Some studies have shown that programmed necrosis plays an important role in the pathogenesis of IBD. Furthermore, programmed necrosis plays an important role in the pathogenesis of various autoimmune diseases such as rheumatoid arthritis (RA), psoriasis, and multiple sclerosis (MS). Activated microglia play a key role in the development of Alzheimer's disease (AD), wherein RIPK1 is highly expressed in microglia, and RIPK1 inhibitors can effectively protect A$\beta$-induced neuronal apoptosis in vitro. In addition to Alzheimer's disease, programmed necrosis has also been implicated in the development of numerous neurodegenerative diseases such as amyotrophic lateral sclerosis (ALS), frontotemporal dementia (FTD), and Parkinson's disease (PD). Strilic et al. first revealed in 2016 that tumor cells can induce programmed necrosis of vascular endothelial cells, and then the tumor cells pass through the vascular wall to achieve distant metastasis via blood circulation, which is an important cause of tumor metastasis, and experiments showed that RIPK1 inhibitors can effectively inhibit tumor metastasis. In addition, studies have shown that RIPK1 kinase can promote the differentiation of resistant macrophages in the tumor microenvironment of pancreatic cancer, while RIPK1 inhibition can differentiate immunogenic macrophages in the tumor microenvironment of pancreatic cancer, resulting in adaptive immune activation and tumor protection.

In conclusion, RIPK1 is an important therapeutic target for programmed necrosis-related diseases such as inflammation, autoimmune diseases, neurodegenerative diseases, and tumors, etc. , and RIPK1 inhibitors are expected to become potential therapeutic drugs for these diseases.

### Summary of the Invention

In view of the need to develop new drugs against diseases related to programmed necrosis, the present invention provides 5-substituted indole 3-amide derivatives, preparation method and use thereof; the compounds of the present invention are able to exert an inhibitory effect on RIPK1 kinase in vivo, and the pharmacokinetic results show that this

series of compounds have good pharmacokinetic properties. The present invention provides a new strategy and means for targeting RIPK1 to treat inflammation, autoimmune diseases, neurodegenerative diseases, tumors and other related diseases.

A compound represented by Formula I, or a stereisomer, or a pharmaceutically acceptable salt thereof:

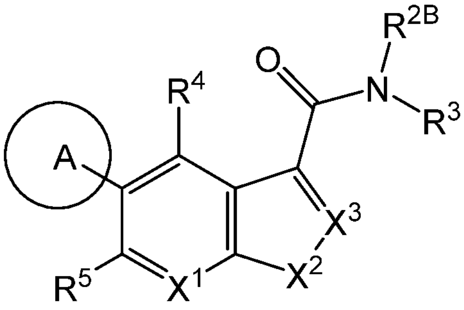

Formula I,

wherein,

$X^1$ and $X^3$ are independently selected from $-CR^6-$ or N;

$X^2$ is selected from $-NR^1-$ or $-CH=CH-$;

wherein $R^1$ is selected from hydrogen, substituted or unsubstituted $C_1$-$C_{10}$ alkyl, substituted or unsubstituted $C_1$-$C_{10}$ ether group, substituted or unsubstituted $C_3$-$C_{10}$ cycloalkyl, substituted or unsubstituted 3-10-membered heterocycloalkyl, substituted or unsubstituted $C_6$-$C_{20}$ aryl, substituted or unsubstituted 3-20-membered heteroaryl; wherein the substituent is deuterium, $C_1$-$C_{10}$ alkyl, cyano, hydroxyl, carboxyl, halogen, or nitro;

$R^{2B}$ is selected from

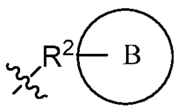

;

wherein $R^2$ is selected from $C_0$-$C_6$ alkylene substituted by one or two $R^{21}$ or unsubstituted;

wherein $R^{21}$ is selected from substituted or unsubstituted $C_1$-$C_{10}$ alkyl, substituted or unsubstituted $C_3$-$C_{10}$ cycloalkyl, cyano, hydroxyl, carboxyl, halogen, or nitro; wherein the substituent is cyano, hydroxyl, carboxyl, halogen, or nitro;

ring B is selected from $C_4$-$C_{10}$ aryl substituted by one, two, or three $R^{22}$ or unsubstituted, or 4-10-membered heteroaryl substituted by one, two, or three $R^{22}$ or unsubstituted; wherein each $R^{22}$ is independently selected from substituted or unsubstituted $C_1$-$C_{10}$ alkyl, substituted or unsubstituted $C_1$-$C_6$ alkoxy, substituted or unsubstituted 4-10-membered heterocycloalkyl, cyano, hydroxy, carboxyl, halogen, or nitro; or two $R^{22}$ are joined to form a substituted or unsubstituted 4-10-membered heterocycloalkyl; wherein the substituent is $C_1$-$C_{10}$ alkyl, cyano, hydroxyl, carboxyl, halogen, or nitro;

$R^3$ is selected from hydrogen, substituted or unsubstituted $C_1$-$C_{10}$ alkyl; wherein the substituent is cyano, hydroxyl, carboxyl, halogen, or nitro;

or $R^{21}$ and $R^3$ are joined to form substituted or unsubstituted 3-10-membered heterocycloalkyl;

wherein the substituent is $C_1$-$C_{10}$ alkyl, $C_4$-$C_{10}$ aryl, $C_4$-$C_{10}$ aryl substituted by one or two $R^{31}$, cyano, hydroxyl, carboxyl, halogen, or nitro; wherein the $R^{31}$ is selected from $C_1$-$C_{10}$ alkyl or halogen;

$R^4$, $R^5$, and $R^6$ are each independently selected from hydrogen, $C_1$-$C_{10}$ alkyl, cyano, hydroxyl, carboxyl, halogen, or nitro;

ring A is selected from $C_4$-$C_{10}$ aryl substituted by one or two $R^A$ or unsubstituted, 4-10-membered heteroaryl substituted by one or two $R^A$ or unsubstituted; wherein $R^A$ is selected from substituted or unsubstituted amino,

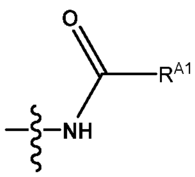

,

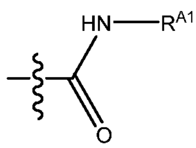

,

substituted or unsubstituted $C_1$-$C_{10}$ alkyl, substituted or unsubstituted $C_4$-$C_{10}$ aryl, substituted or unsubstituted 4-10-membered heteroaryl, substituted or unsubstituted 3-10-membered heterocycloalkyl; wherein the substituent is -$R^{A2}$-$R^{A3}$, $R^{A4}$, $C_1$-$C_{10}$ alkyl, halogen-substituted $C_1$-$C_{10}$ alkyl, cyano, hydroxyl, carboxyl, halogen, or nitro; wherein $R^{A4}$ is selected from 3-10-membered heterocycloalkyl substituted by -$R^{A2}$-$R^{A3}$ or unsubstituted;

$R^{A1}$ is selected from substituted or unsubstituted $C_1$-$C_{10}$ alkyl, substituted or unsubstituted $C_1$-$C_{10}$ alkenyl, substituted or unsubstituted $C_3$-$C_{10}$ cycloalkyl, substituted or unsubstituted 3-10-membered heterocycloalkyl, substituted or unsubstituted $C_1$-$C_6$ alkoxy, substituted or unsubstituted $C_2$-$C_6$ ether group, or substituted or unsubstituted $C_2$-$C_6$ amine; wherein the substituent is $C_1$-$C_{10}$ alkyl, hydroxyl-substituted $C_1$-$C_{10}$ alkyl, a $C_1$-$C_{10}$ ester group, 3-10-membered heterocycloalkyl, amino, amine, cyano, hydroxyl, carboxyl, halogen, or nitro; $R^{A2}$ is selected from substituted or unsubstituted $C_0$-$C_6$ alkylene or carbonyl; wherein the substituent is $C_1$-$C_{10}$ alkyl, cyano, hydroxyl, carboxyl, halogen, or nitro;

$R^{A3}$ is selected from substituted or unsubstituted 3-10-membered heterocycloalkyl; wherein the substituent is $C_1$-$C_{10}$ alkyl, cyano, hydroxyl, carboxyl, halogen, or nitro.

Preferably, the compound of Formula I is represented by Formula II:

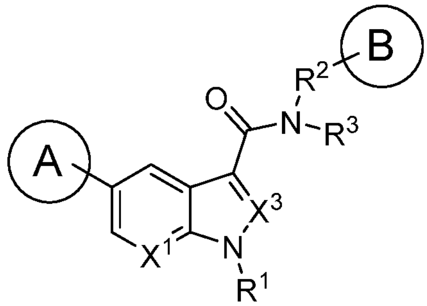

Formula II

wherein,

$R^1$ is selected from hydrogen, substituted or unsubstituted $C_1$-$C_{10}$ alkyl, substituted or unsubstituted $C_3$-$C_{10}$ cycloalkyl, substituted or unsubstituted 3-10-membered heterocycloalkyl, substituted or unsubstituted $C_6$-$C_{20}$ aryl, substituted or unsubstituted 3-20-membered heteroaryl; wherein the substituent is, $C_1$-$C_{10}$ alkyl, cyano, hydroxyl, carboxyl, halogen, or nitro;

$R^2$ is selected from $C_0$-$C_6$ alkylene substituted by one $R^{21}$ or unsubstituted; wherein $R^{21}$ is selected from substituted or unsubstituted $C_1$-$C_{10}$ alkyl, cyano, hydroxy, carboxyl, halogen, or nitro; wherein the substituent is cyano, hydroxyl, carboxyl, halogen, or nitro;

ring B is selected from $C_4$-$C_{10}$ aryl substituted by one, two, or three $R^{22}$ or unsubstituted, or 4-10-membered heteroaryl substituted by one, two, or three $R^{22}$ or unsubstituted; wherein each $R^{22}$ is independently selected from substituted or unsubstituted $C_1$-$C_{10}$ alkyl, substituted or unsubstituted $C_1$-$C_6$ alkoxy, substituted or unsubstituted 4-10-membered heterocycloalkyl, cyano, hydroxy, carboxyl, halogen, or nitro; or two $R^{22}$ are joined to form a substituted or unsubstituted 4-10-membered heterocycloalkyl; wherein the substituent is $C_1$-$C_{10}$ alkyl, cyano, hydroxyl, carboxyl, halogen, or nitro;

$R^3$ is selected from hydrogen, substituted or unsubstituted $C_1$-$C_{10}$ alkyl; wherein the substituent is cyano, hydroxyl, carboxyl, halogen, or nitro;

or $R^2$ and $R^3$ are joined to form a substituted or unsubstituted 3-10 membered heterocycloalkyl; wherein the substituent is $C_1$-$C_{10}$ alkyl, cyano, hydroxyl, carboxyl, halogen, or nitro;

$X^1$ and $X^3$ are independently selected from CH or N;

ring A is selected from $C_4$-$C_{10}$ aryl substituted by one or two $R^A$ or unsubstituted, 4-10-membered heteroaryl substituted by one or two $R^A$ or unsubstituted; wherein $R^A$ is selected from amino,

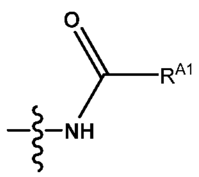

,

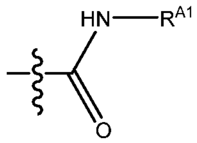

,

substituted or unsubstituted $C_1$-$C_{10}$ alkyl, substituted or unsubstituted $C_4$-$C_{10}$ aryl, substituted or unsubstituted 4-10-membered heteroaryl, substituted or unsubstituted 3-10-membered heterocycloalkyl; wherein the substituent is -$R^{A2}$-$R^{A3}$, $R^{A4}$, $C_1$-$C_{10}$ alkyl, cyano, hydroxyl, carboxyl, halogen, or nitro; wherein $R^{A4}$ is selected from 3-10-membered heterocycloalkyl substituted by -$R^{A2}$-$R^{A3}$ or unsubstituted;

$R^{A1}$ is selected from substituted or unsubstituted $C_1$-$C_{10}$ alkyl, substituted or unsubstituted $C_1$-$C_{10}$ alkenyl, substituted or unsubstituted $C_3$-$C_{10}$ cycloalkyl, substituted or unsubstituted 3-10-membered heterocycloalkyl, substituted or unsubstituted $C_1$-$C_6$ alkoxy, or substituted or unsubstituted $C_2$-$C_6$ ether group; wherein, the substituent is $C_1$-$C_{10}$ alkyl, 3-10-membered heterocycloalkyl, amino, amine, cyano, hydroxyl, carboxyl, halogen, or nitro;

$R^{A2}$ is selected from substituted or unsubstituted $C_0$-$C_6$ alkylene or carbonyl; wherein the substituent is $C_1$-$C_{10}$ alkyl, cyano, hydroxyl, carboxyl, halogen, or nitro;

$R^{A3}$ is selected from substituted or unsubstituted 3-10-membered heterocycloalkyl; wherein the substituent is $C_1$-$C_{10}$ alkyl, cyano, hydroxyl, carboxyl, halogen, or nitro.

Preferably, the compound of Formula I is represented by Formula III or Formula IV:

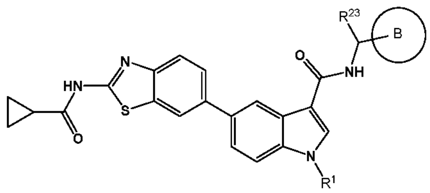

Formula III

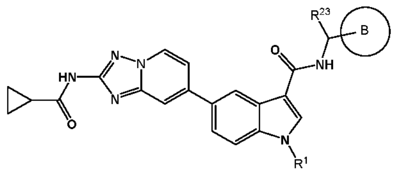

Formula IV

wherein $R^1$ is selected from hydrogen or $C_1$-$C_4$ alkyl; $R^{23}$ is selected from hydrogen or methyl; ring B is selected from phenyl substituted by one or two $R^{22}$ or unsubstituted; wherein each $R^{22}$ is independently selected from F, Cl, cyano, methyl, trifluoromethyl, methoxy, or trifluoromethoxy.

Preferably, the compound of Formula I is represented by Formula V:

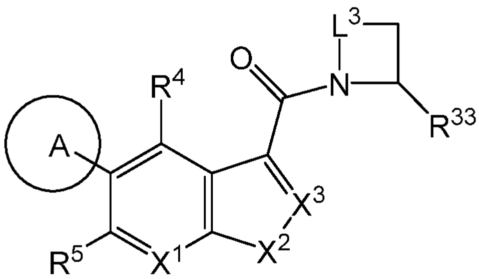

Formula V

wherein,

$X^1$ and $X^3$ are independently selected from -$CR^6$- or N;

$X^2$ is selected from -$NR^1$- or -CH = CH-;

the $R^1$ is selected from hydrogen, substituted or unsubstituted $C_1$-$C_{10}$ alkyl, substituted or unsubstituted $C_3$-$C_{10}$ cycloalkyl, substituted or unsubstituted 3-10-membered heterocycloalkyl, substituted or unsubstituted $C_6$-$C_{20}$ aryl, substituted or unsubstituted 3-20-membered heteroaryl; wherein the substituent is $C_1$-$C_{10}$ alkyl, cyano, hydroxyl, carboxyl, halogen, or nitro; $L^3$ is selected from substituted or unsubstituted $C_1$-$C_4$ alkylene; wherein the substituent is $C_1$-$C_{10}$ alkyl, cyano, hydroxyl, carboxyl, halogen, or nitro;

$R^{33}$ is selected from hydrogen, substituted or unsubstituted $C_1$-$C_{10}$ alkyl, substituted or unsubstituted $C_3$-$C_{10}$ cycloalkyl, substituted or unsubstituted 3-10-membered heterocycloalkyl, substituted or unsubstituted $C_6$-$C_{10}$ aryl; wherein the substituent is $C_1$-$C_{10}$ alkyl, cyano, hydroxyl, carboxyl, halogen, or nitro;

$R^4$, $R^5$, and $R^6$ are each independently selected from hydrogen, $C_1$-$C_{10}$ alkyl, cyano, hydroxyl, carboxyl, halogen, or nitro;

ring A is selected from $C_4$-$C_{10}$ aryl substituted by one or two $R^A$ or unsubstituted, 4-10-membered heteroaryl substituted by one or two $R^A$ or unsubstituted; wherein RA is selected from amino,

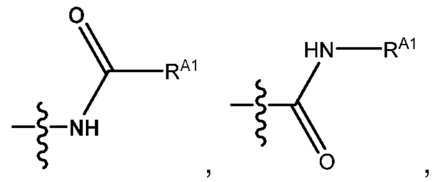

substituted or unsubstituted $C_1$-$C_{10}$ alkyl, substituted or unsubstituted $C_4$-$C_{10}$ aryl, substituted or unsubstituted 4-10-membered heteroaryl, substituted or unsubstituted 3-10-membered heterocycloalkyl; wherein the substituent is -$R^{A2}$-$R^{A3}$, $R^{A4}$, $C_1$-$C_{10}$ alkyl, cyano, hydroxyl, carboxyl, halogen, or nitro; wherein $R^{A4}$ is selected from 3-10-membered heterocycloalkyl substituted by -$R^{A2}$-$R^{A3}$ or unsubstituted;

$R^{A1}$ is selected from substituted or unsubstituted $C_1$-$C_{10}$ alkyl, substituted or unsubstituted $C_1$-$C_{10}$ alkenyl, substituted or unsubstituted $C_3$-$C_{10}$ cycloalkyl, substituted or unsubstituted 3-10-membered heterocycloalkyl, substituted or unsubstituted $C_1$-$C_6$ alkoxy, or substituted or unsubstituted $C_2$-$C_6$ ether group; wherein the substituent is $C_1$-$C_{10}$ alkyl, a $C_1$-$C_{10}$ ester group, 3-10-membered heterocycloalkyl, amino, amine, cyano, hydroxyl, carboxyl, halogen, or nitro;

$R^{A2}$ is selected from substituted or unsubstituted $C_0$-$C_6$ alkylene or carbonyl; wherein the substituent is $C_1$-$C_{10}$ alkyl, cyano, hydroxyl, carboxyl, halogen, or nitro;

$R^{A3}$ is selected from substituted or unsubstituted 3-10-membered heterocycloalkyl; wherein the substituent is $C_1$-$C_{10}$ alkyl, cyano, hydroxyl, carboxyl, halogen, or nitro.

Preferably, $R^{33}$ is selected from phenyl, phenyl substituted by one or two substituents; wherein the substituents are selected from F, Cl, or methyl.

Preferably, the compound of Formula I is represented by Formula VI:

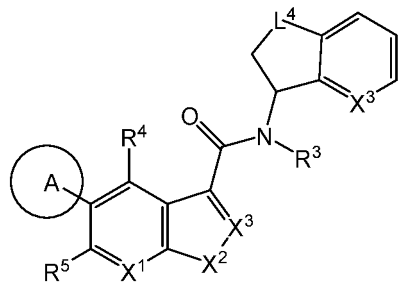

Formula VI

wherein,

$X^1$ and $X^3$ are independently selected from -$CR^6$- or N;

$X^2$ is selected from -$NR^1$- or -CH = CH-;

$X^3$ is selected from CH or N;

the $R^1$ is selected from hydrogen, substituted or unsubstituted $C_1$-$C_{10}$ alkyl, substituted or unsubstituted $C_3$-$C_{10}$ cycloalkyl, substituted or unsubstituted 3-10-membered heterocycloalkyl, substituted or unsubstituted $C_6$-$C_{20}$ aryl, substituted or unsubstituted 3-20-membered heteroaryl; wherein the substituent is $C_1$-$C_{10}$ alkyl, cyano, hydroxyl, carboxyl, halogen, or nitro; $L^4$ is selected from substituted or unsubstituted $C_1$-$C_3$ alkylene; wherein the substituent is $C_1$-$C_{10}$ alkyl, cyano, hydroxyl, carboxyl, halogen, or nitro;

$R^3$ is selected from hydrogen, substituted or unsubstituted $C_1$-$C_{10}$ alkyl; wherein the substituent is cyano, hydroxyl, carboxyl, halogen, or nitro;

$R^4$, $R^5$, and $R^6$ are each independently selected from hydrogen, $C_1$-$C_{10}$ alkyl, cyano, hydroxyl, carboxyl, halogen, or nitro;

ring A is selected from $C_4$-$C_{10}$ aryl substituted by one or two $R^A$ or unsubstituted, 4-10-membered heteroaryl substituted by one or two $R^A$ or unsubstituted; wherein $R^A$ is selected from amino,

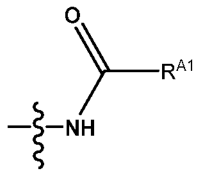, 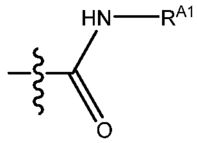,

substituted or unsubstituted $C_1$-$C_{10}$ alkyl, substituted or unsubstituted $C_4$-$C_{10}$ aryl, substituted or unsubstituted 4-10-membered heteroaryl, substituted or unsubstituted 3-10-membered heterocycloalkyl; wherein the substituent is -$R^{A2}$-$R^{A3}$, $R^{A4}$, $C_1$-$C_{10}$ alkyl, cyano, hydroxyl, carboxyl, halogen, or nitro; wherein $R^{A4}$ is selected from 3-10-membered heterocycloalkyl substituted by -$R^{A2}$-$R^{A3}$ or unsubstituted;

$R^{A1}$ is selected from substituted or unsubstituted $C_1$-$C_{10}$ alkyl, substituted or unsubstituted $C_1$-$C_{10}$ alkenyl, substituted or unsubstituted $C_3$-$C_{10}$ cycloalkyl, substituted or unsubstituted 3-10-membered heterocycloalkyl, substituted or unsubstituted $C_1$-$C_6$ alkoxy, or substituted or unsubstituted $C_2$-$C_6$ ether group; wherein the substituent is $C_1$-$C_{10}$ alkyl, 3-10-membered heterocycloalkyl, amino, amine, cyano, hydroxyl, carboxyl, halogen, or nitro;

$R^{A2}$ is selected from substituted or unsubstituted $C_0$-$C_6$ alkylene or carbonyl; wherein the substituent is $C_1$-$C_{10}$ alkyl, cyano, hydroxyl, carboxyl, halogen, or nitro;

$R^{A3}$ is selected from substituted or unsubstituted 3-10 membered heterocycloalkyl; wherein the substituent is $C_1$-$C_{10}$ alkyl, cyano, hydroxyl, carboxyl, halogen, or nitro.

Preferably, L4 is selected from $C_1$- $C_2$ alkylene.

Preferably, $X^3$ is selected from CH or N;

Preferably, $R^1$ is selected from hydrogen, $C_1$-$C_4$ alkyl, or $C_3$ ether group;

Preferably, $R^4$, $R^5$, and $R^6$ are each independently selected from hydrogen or F.

Preferably, ring A is selected from 5-9-membered heteroaryl substituted by one or two $R^A$ or unsubstituted, 6-membered aryl substituted by one or two $R^A$ or unsubstituted; wherein $R^A$ is selected from amino, $C_1$-$C_4$ alkyl-substituted amino, -$CF_3$-substituted amino, -$CHF_2$-substituted amino,

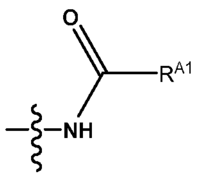, 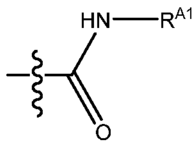,

methyl, 5-6-membered heteroaryl substituted by -$R^{A2}$-$R^{A3}$ or unsubstituted, $R^{A4}$-substituted methyl, phenyl substituted by -$R^{A2}$-$R^{A3}$ or unsubstituted; wherein $R^{A4}$ is selected from 6-membered heterocycloalkyl substituted by -$R^{A2}$-$R^{A3}$ or unsubstituted; $R^{A1}$ is selected from $C_2$-$C_5$ alkyl, chlorine-substituted $C_4$ alkyl, 5-membered heterocycloalkyl-substituted methyl, vinyl, N,N-dimethylamino-substituted vinyl, $C_3$-$C_6$ cycloalkyl, $C_3$-$C_6$ cycloalkyl substituted by one or two fluorine, 4-6-membered heterocycloalkyl, hydroxyl-substituted 4-6-membered heterocycloalkyl, -$CH_2OH$-substituted 4-5-membered heterocycloalkyl, methyl-substituted 6-membered heterocycloalkyl, methoxy, fluorine-substituted methoxy, $C_2$-$C_3$ ether group, or hydroxyl-substituted propylamine;

$R^{A2}$ is selected from $C_0$-$C_1$ alkylene or carbonyl;

$R^{A3}$ is selected from methyl-substituted or unsubstituted 6-membered heterocycloalkyl.

Preferably, ring A is selected from the group consisting of:

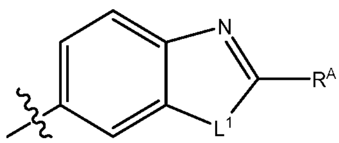,

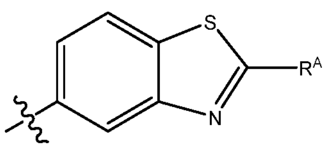, 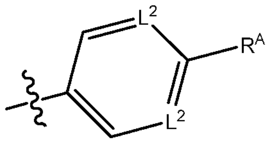, 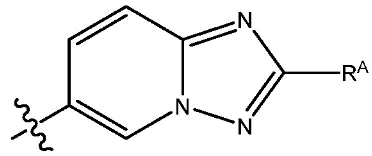,

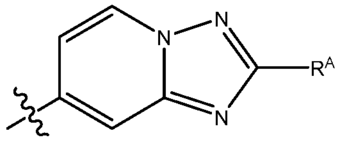, 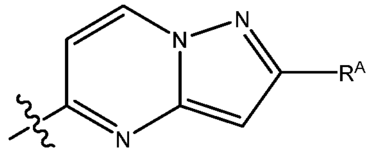, 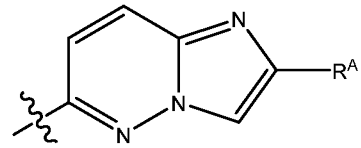,

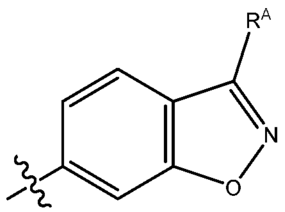, 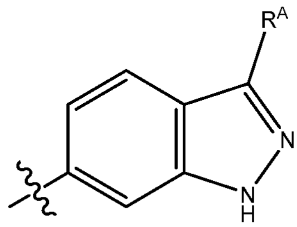, 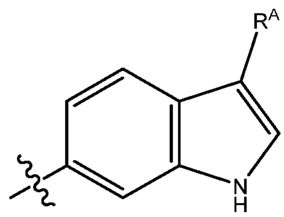, 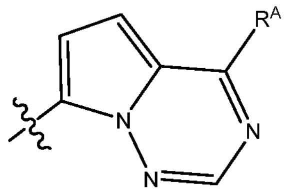,

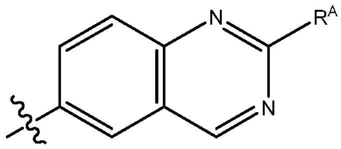, 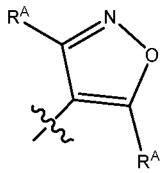, 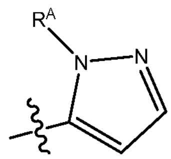, 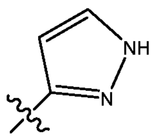;

wherein $L^1$ is selected from S or NH; and each $L^2$ is independently selected from CH or N;

Preferably, ring A is selected from:

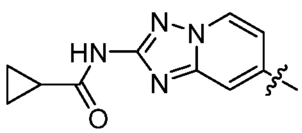, 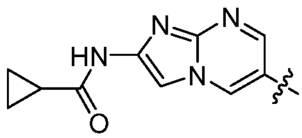,

or

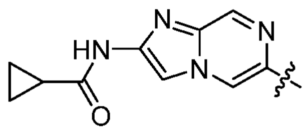.

Preferably, $R^2$ is selected from $C_0$-$C_2$ alkylene substituted by one or two $R^{21}$ or unsubstituted; wherein $R^{21}$ is selected from methyl;
ring B is selected from $C_6$-$C_{10}$ aryl substituted by one, two, or three $R^{22}$ or unsubstituted, 5-9-membered heteroaryl substituted by one, two, or three $R^{22}$ or unsubstituted, or $C_9$-$C_{10}$ cycloalkyl substituted by one, two, or three $R^{22}$ or unsubstituted; wherein each $R^{22}$ is independently selected from $C_1$-$C_4$ alkyl, methoxy, halogen, halogen-substituted methyl, halogen-substituted methoxy, cyano, nitro, or

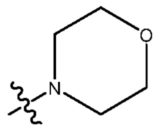;

halogen is selected from F, Cl, or Br.

Preferably, $R^3$ is selected from hydrogen.

Preferably, the compound of Formula I is represented by Formula VII:

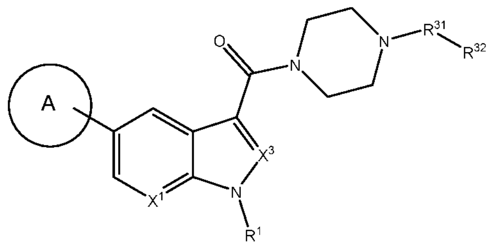

Formula VII

wherein,

wherein $R^1$ is selected from hydrogen, substituted or unsubstituted $C_1$-$C_{10}$ alkyl, substituted or unsubstituted $C_3$-$C_{10}$ cycloalkyl, substituted or unsubstituted 3-10-membered heterocycloalkyl, substituted or unsubstituted $C_6$-$C_{20}$ aryl, substituted or unsubstituted 3-20-membered heteroaryl; wherein the substituent is, $C_1$-$C_{10}$ alkyl, cyano, hydroxyl, carboxyl, halogen, or nitro;

$R^{31}$ is selected from $C_1$-$C_6$ alkylene;

$R^{32}$ is selected from $C_4$-$C_{10}$ aryl substituted by one, two, or three $R^{311}$ or unsubstituted, wherein each $R^{311}$ is independently selected from $C_1$-$C_6$ alkoxy, or two $R^{311}$ are joined to form a 4-10-membered heterocycloalkyl;

$X^1$ and $X^3$ are independently selected from CH or N;

ring A is selected from $C_4$-$C_{10}$ aryl substituted by one or two $R^A$ or unsubstituted, 4-10-membered heteroaryl substituted by one or two $R^A$ or unsubstituted; wherein $R^A$ is selected from amino,

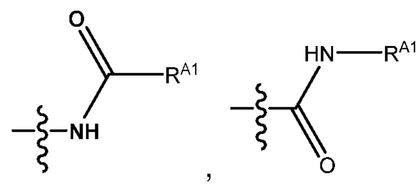 , ,

substituted or unsubstituted $C_1$-$C_{10}$ alkyl, substituted or unsubstituted $C_4$-$C_{10}$ aryl, substituted or unsubstituted 4-10-membered heteroaryl, substituted or unsubstituted 3-10-membered heterocycloalkyl; wherein the substituent is $-R^{A2}$-$R^{A3}$, $R^{A4}$, $C_1$-$C_{10}$ alkyl, cyano, hydroxyl, carboxyl, halogen, or nitro; wherein $R^{A4}$ is selected from 3-10-membered heterocycloalkyl substituted by $-R^{A2}$-$R^{A3}$ or unsubstituted;

$R^{A1}$ is selected from substituted or unsubstituted $C_1$-$C_{10}$ alkyl, substituted or unsubstituted $C_1$-$C_{10}$ alkenyl, substituted or unsubstituted $C_3$-$C_{10}$ cycloalkyl, substituted or unsubstituted 3-10-membered heterocycloalkyl, substituted or unsubstituted $C_1$-$C_6$ alkoxy, or substituted or unsubstituted $C_2$-$C_6$ ether group; wherein the substituent is $C_1$-$C_{10}$ alkyl, 3-10-membered heterocycloalkyl, amino, amine, cyano, hydroxyl, carboxyl, halogen, or nitro;

$R^{A2}$ is selected from substituted or unsubstituted $C_0$-$C_6$ alkylene or carbonyl; wherein the substituent is $C_1$-$C_{10}$ alkyl, cyano, hydroxyl, carboxyl, halogen, or nitro;

$R^{A3}$ is selected from substituted or unsubstituted 3-10-membered heterocycloalkyl; wherein the substituent is $C_1$-$C_{10}$ alkyl, cyano, hydroxyl, carboxyl, halogen, or nitro.

Preferably, $-R^{31}$-$R^{32}$ is selected from

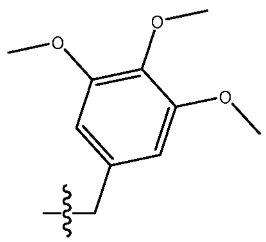 or 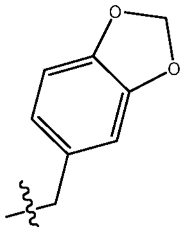 ;

Preferably, the compounds of Formulas I-VII are as follows:

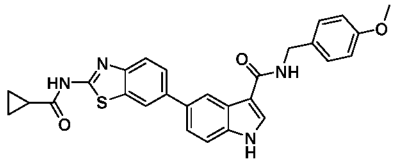 ,

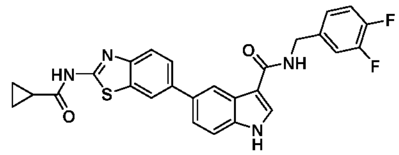

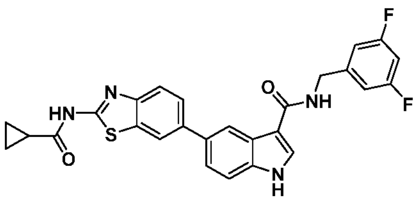 ,

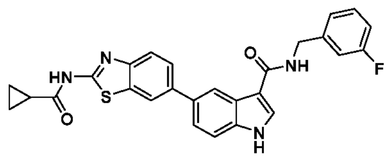

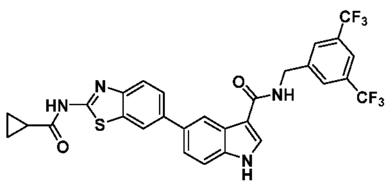 ,

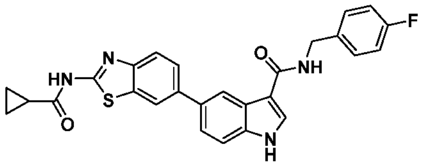

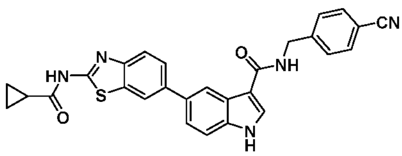 ,

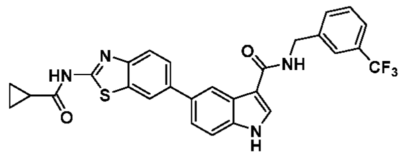

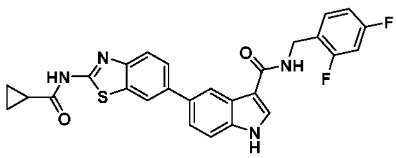 ,

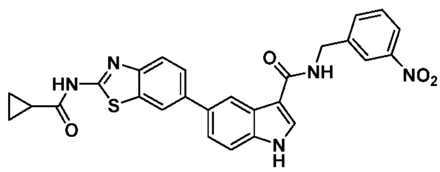 ,

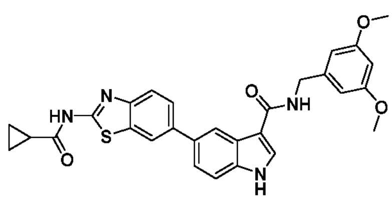 ,

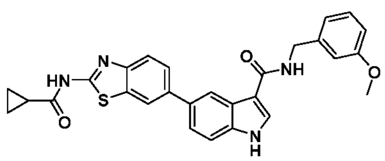

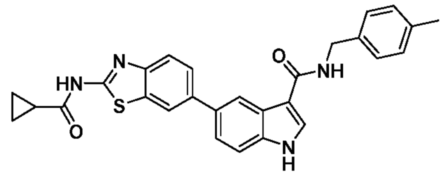 , 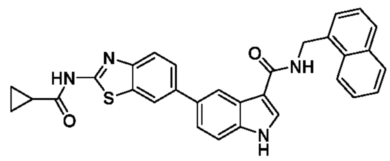 ,

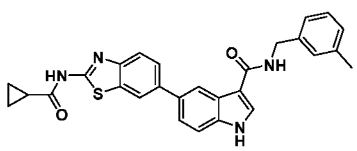 , 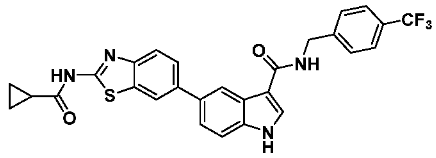

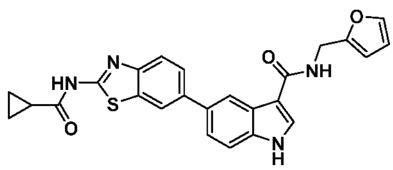 , 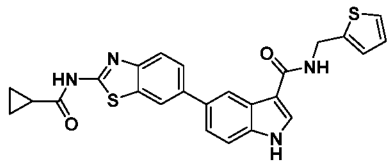 ,

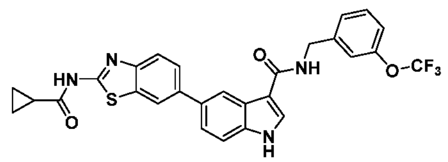 , 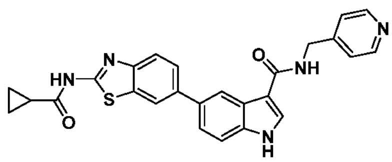 ,

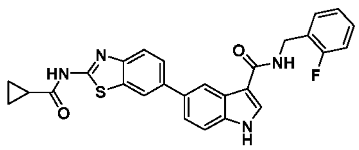 , 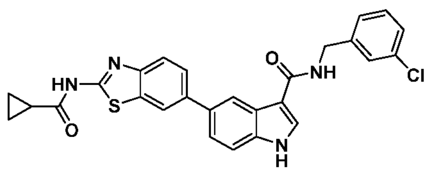

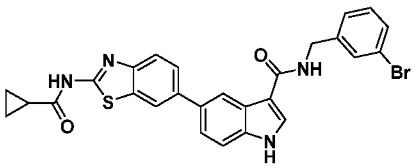 , 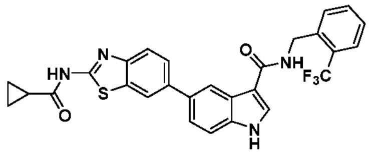 ,

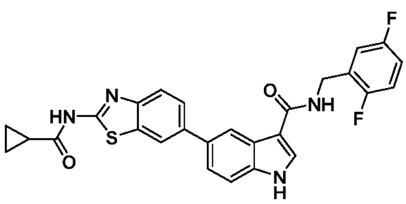 , 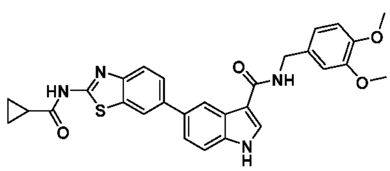

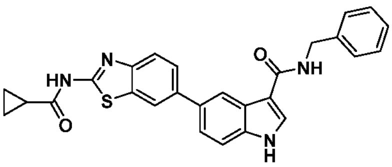 , 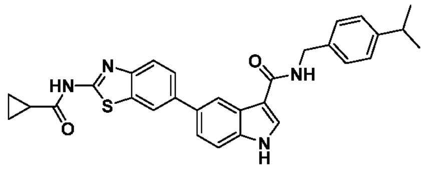

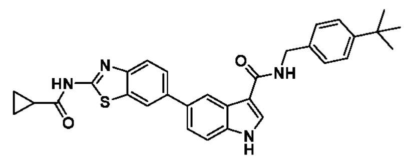,
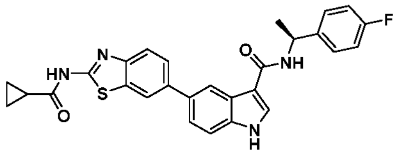,

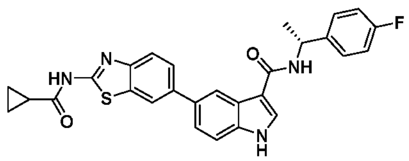,
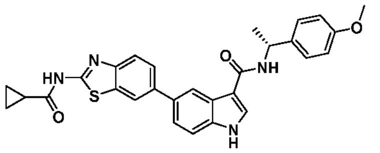

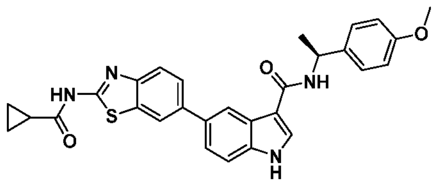,
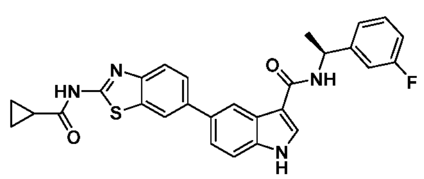,

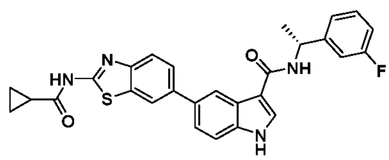,
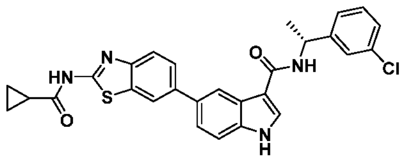,

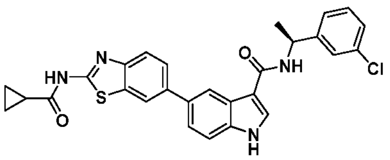,
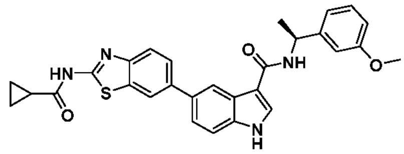

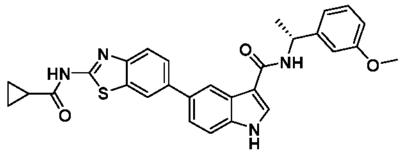,
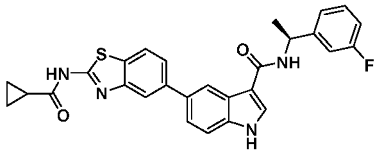

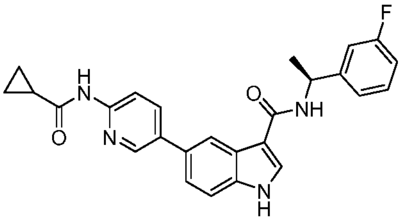,
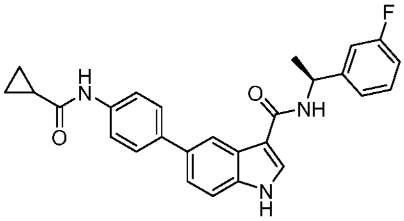,

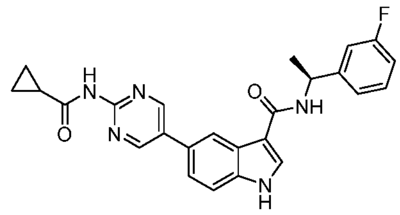

,

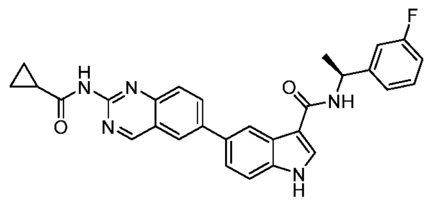

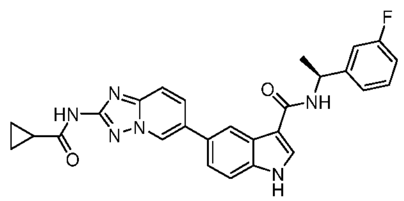

,

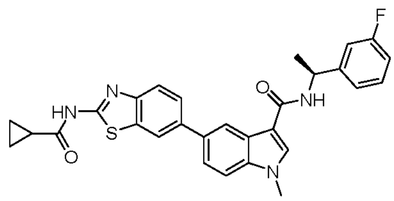

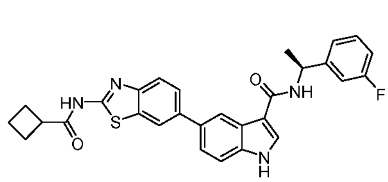

,

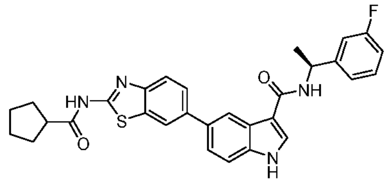

,

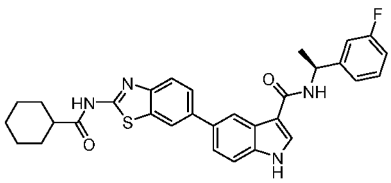

,

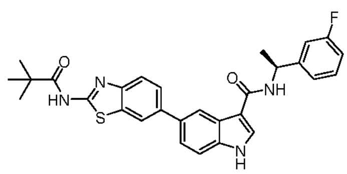

,

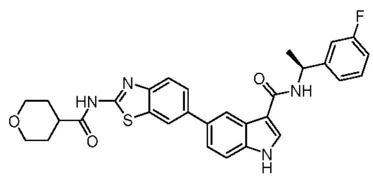

,

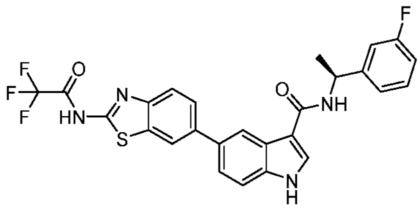

,

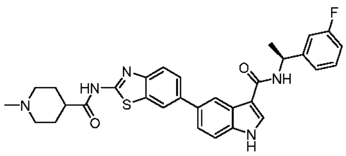

,

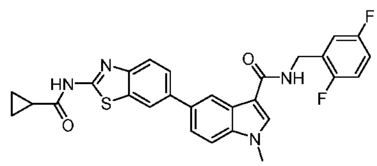

,

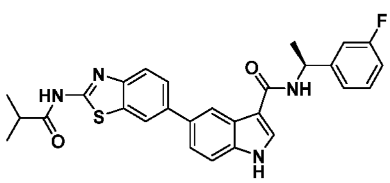

,

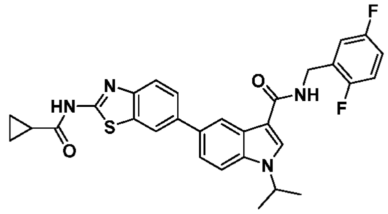

,

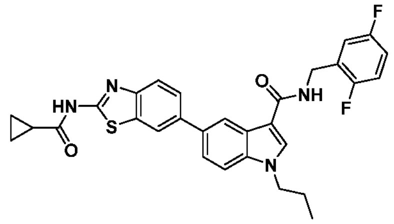

,

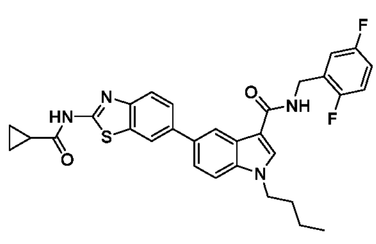

,

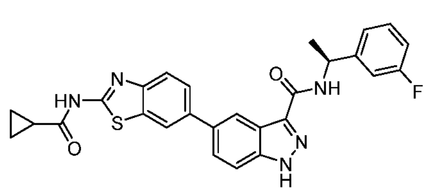

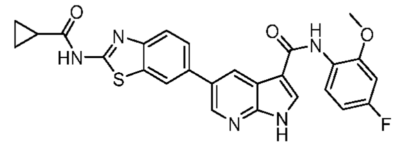,

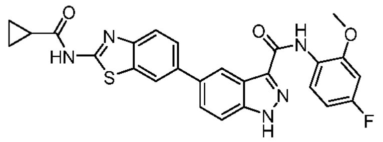, 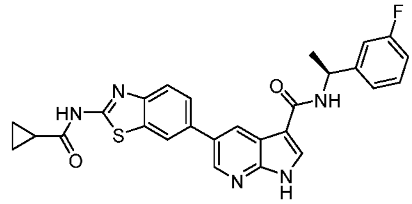, 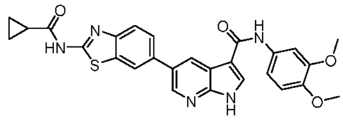,

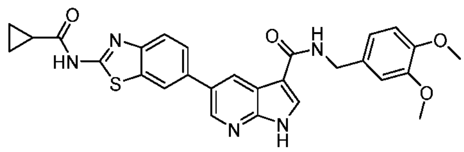, 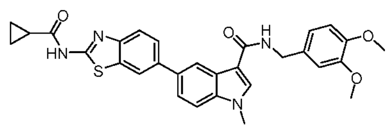,

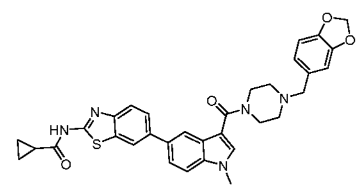,

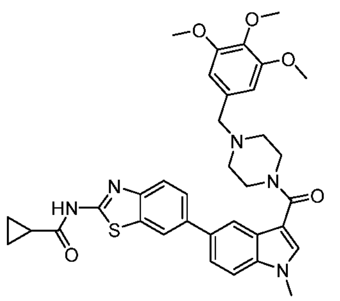, 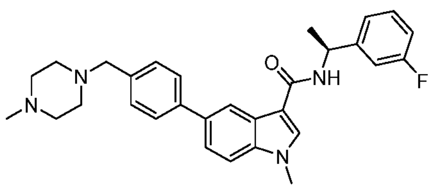, 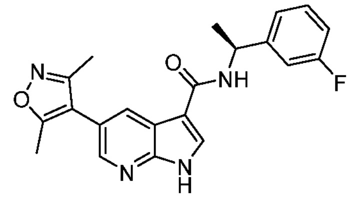,

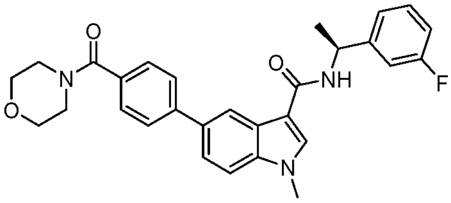, 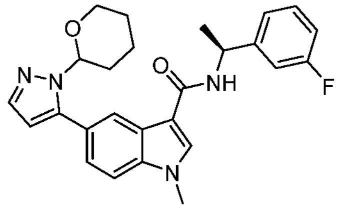, 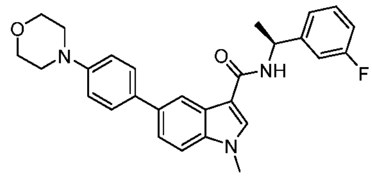,

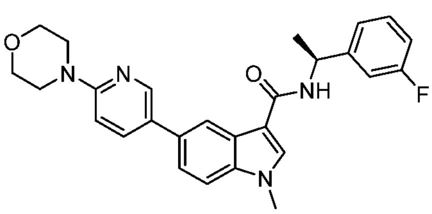, 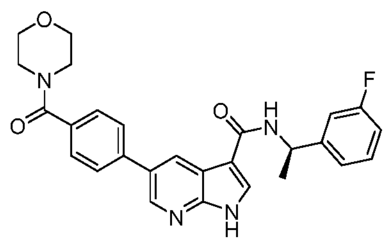, 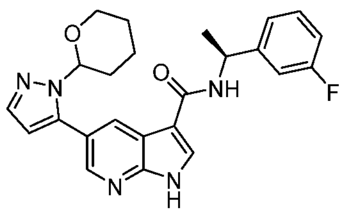,

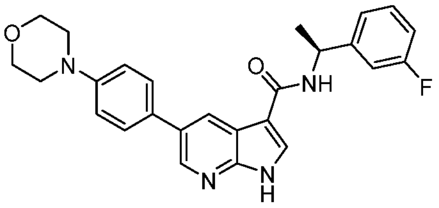
,
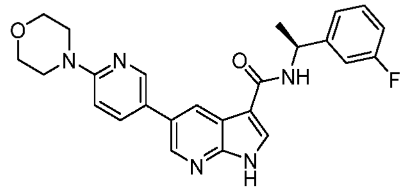
,

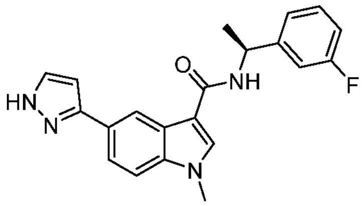
,

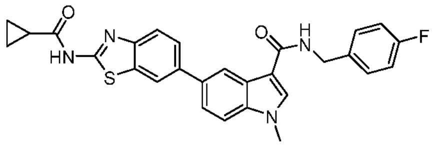
,
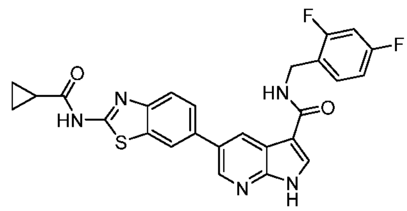
,

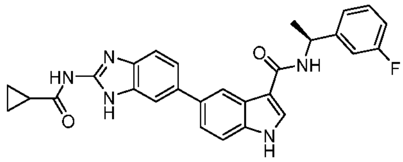
,
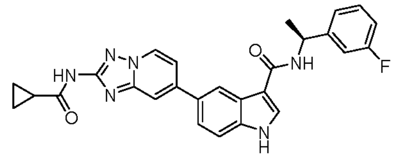

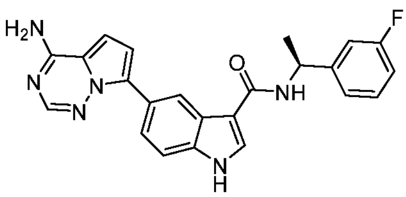
,
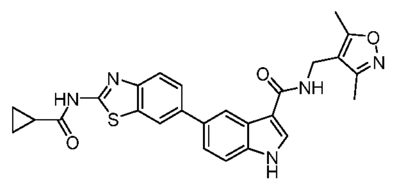

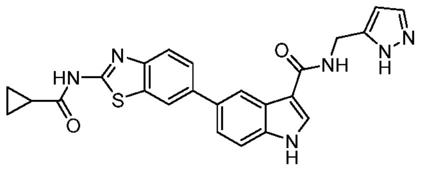
,
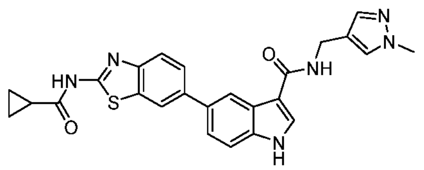
,

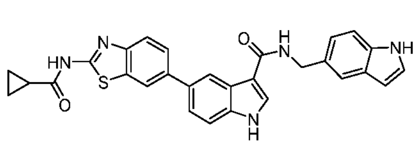
,
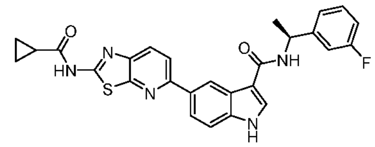

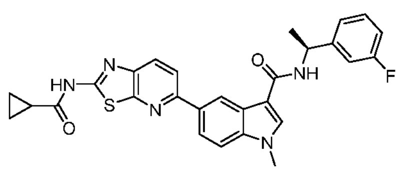
,
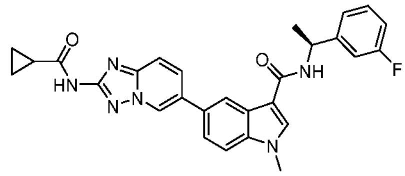

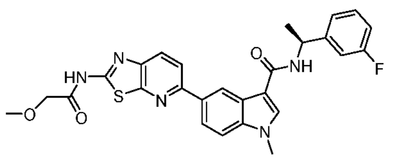

,

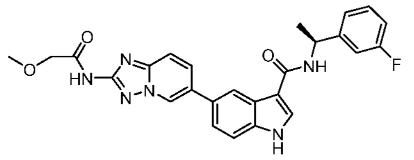

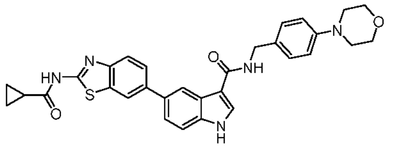

,

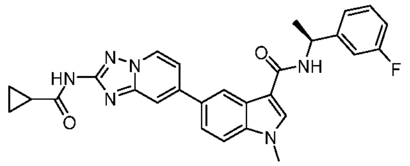

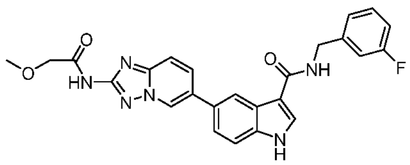

,

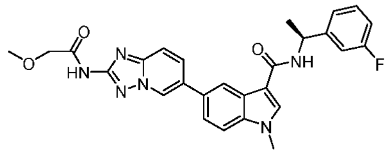

,

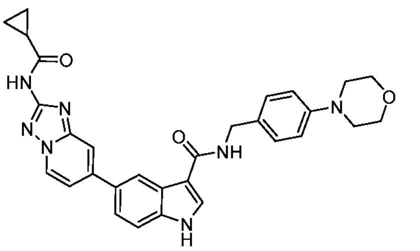

,

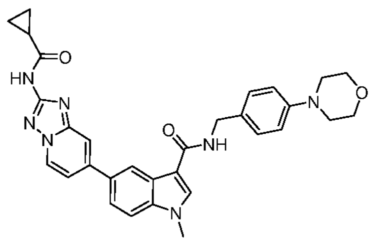

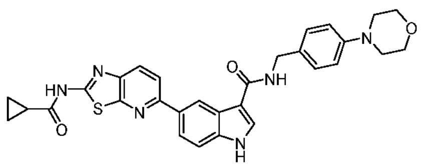

,

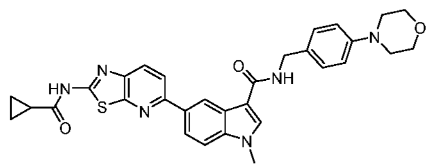

,

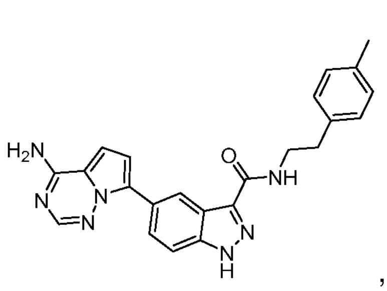

,

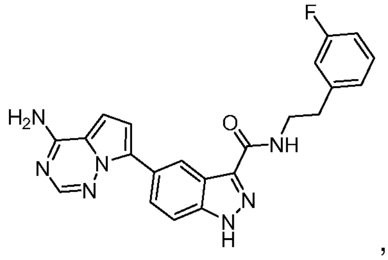

,

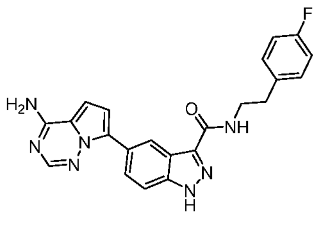

,

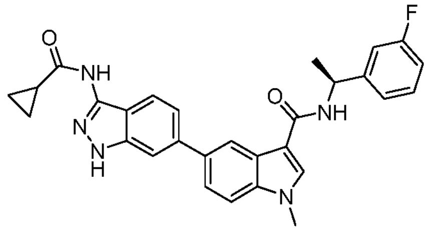

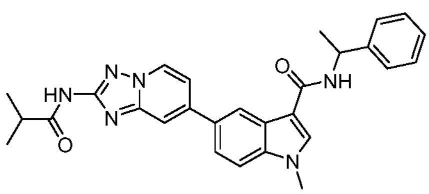,

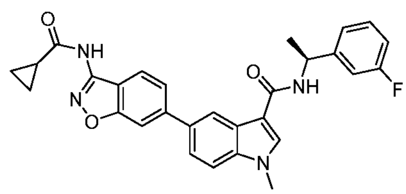,

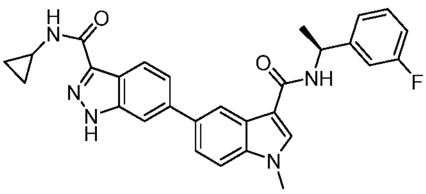,

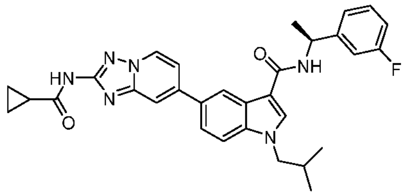,

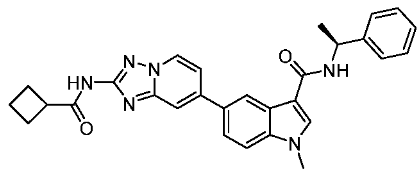

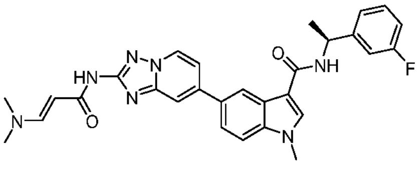,

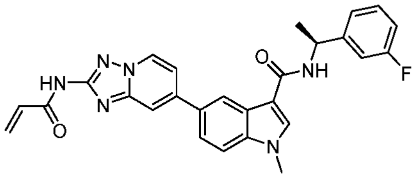

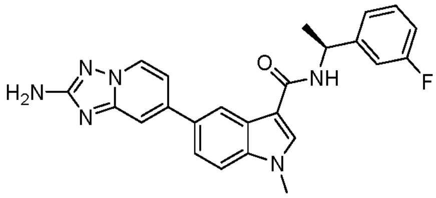,

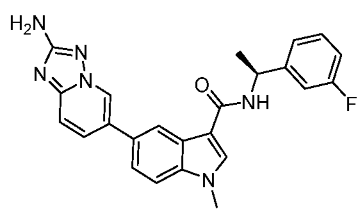

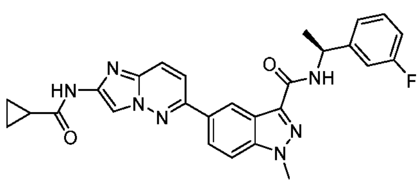,

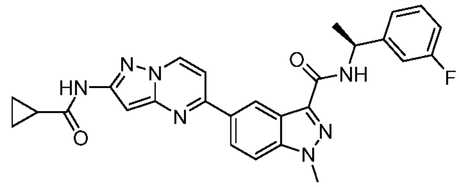,

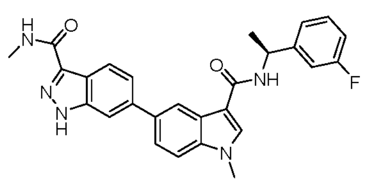,

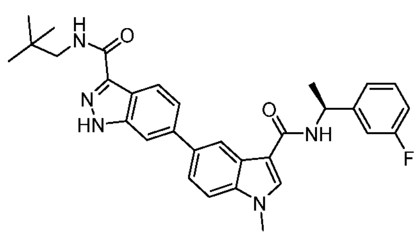

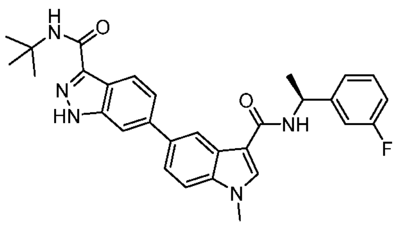

,

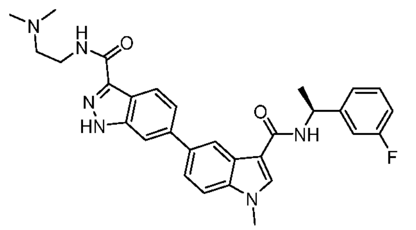

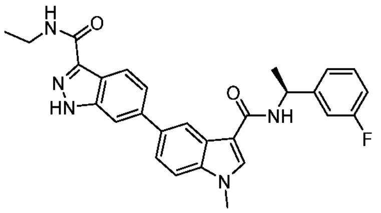

,

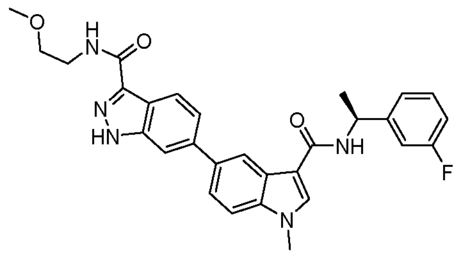

,

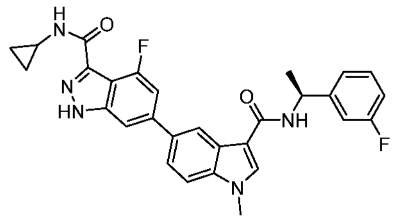

,

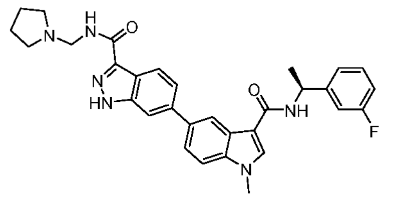

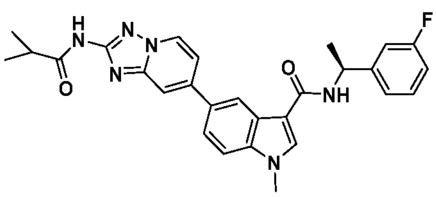

,

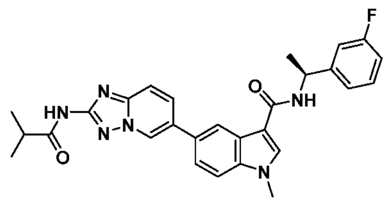

,

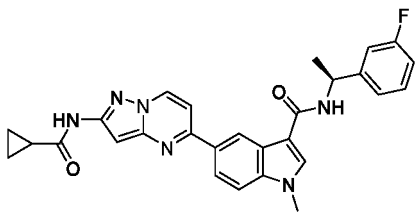

,

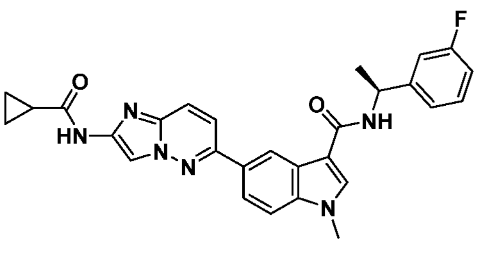

,

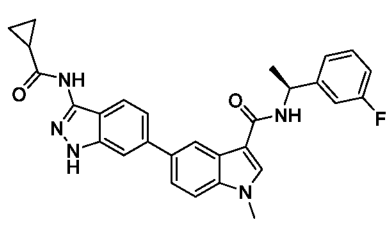

,

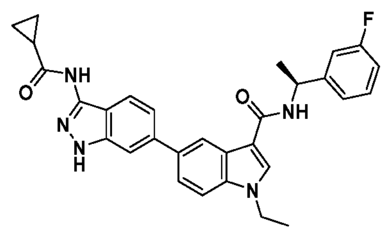,
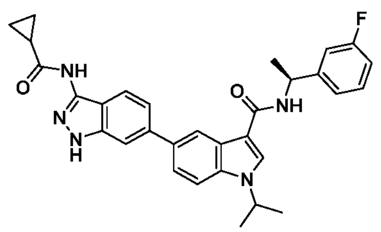,
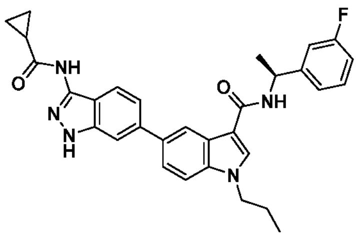,

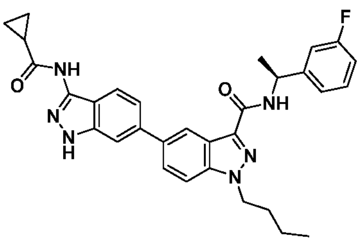,

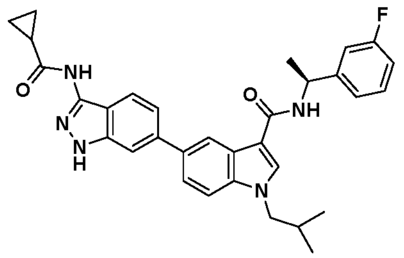,
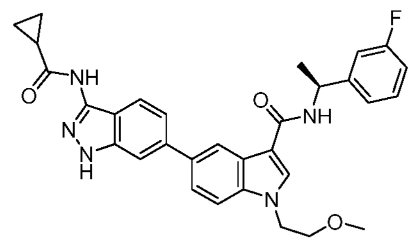

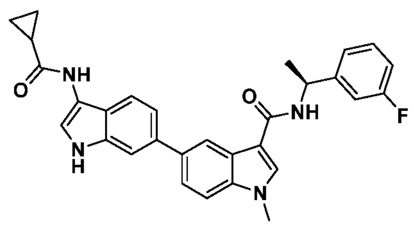,
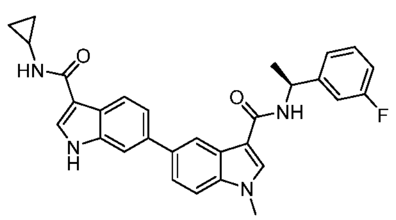

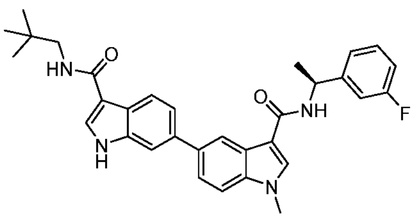,
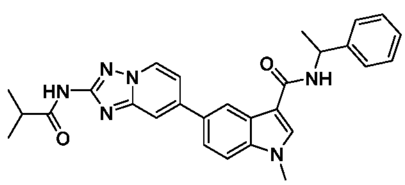,

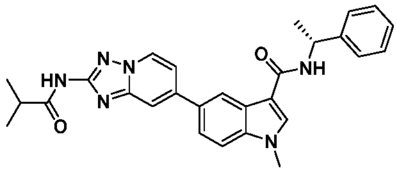,
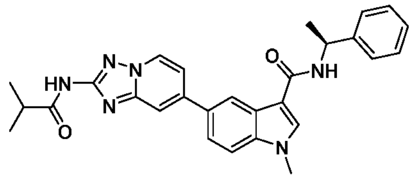

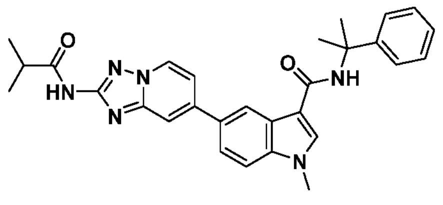

,

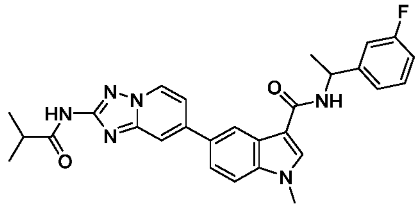

,

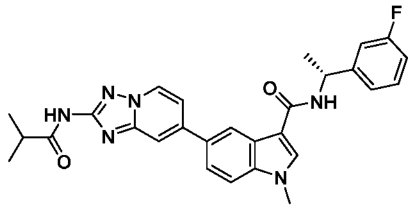

,

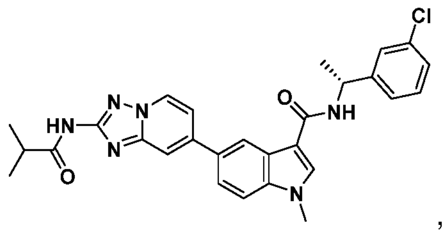

,

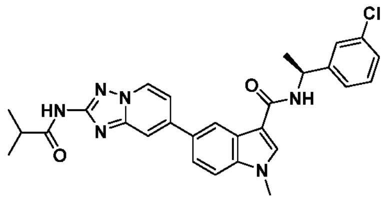

,

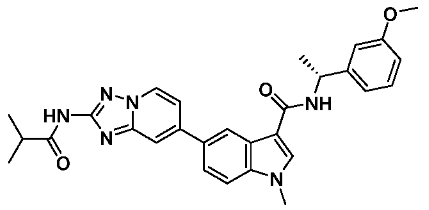

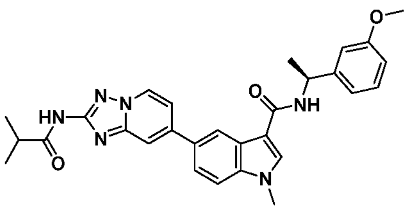

,

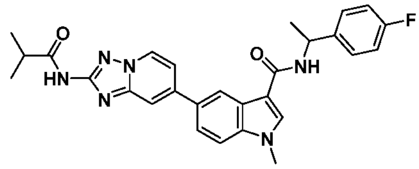

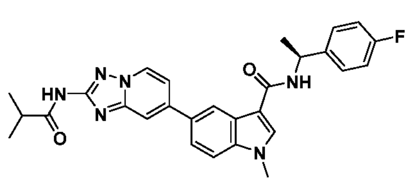

,

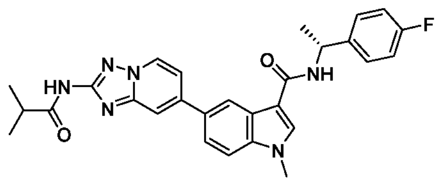

,

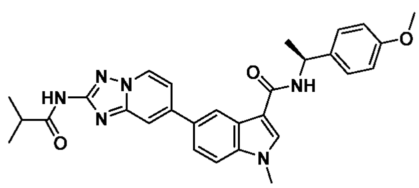

,

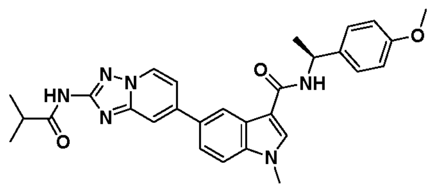

,

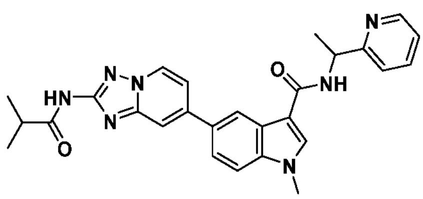

,

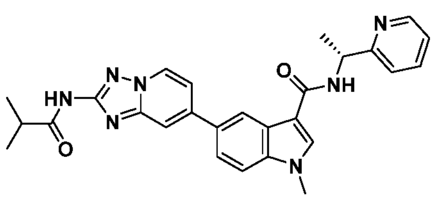

,

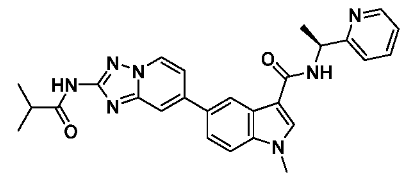,

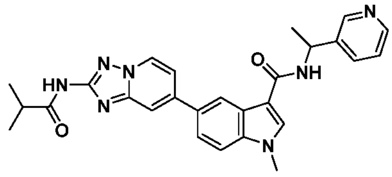,

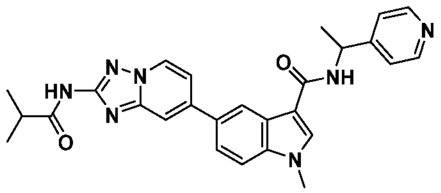,

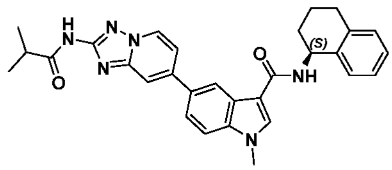,

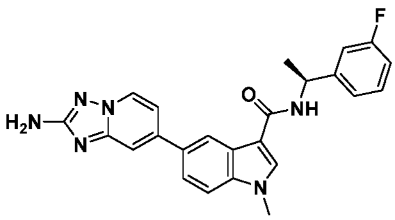,

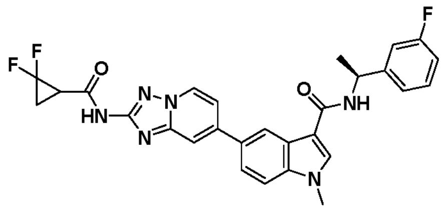,

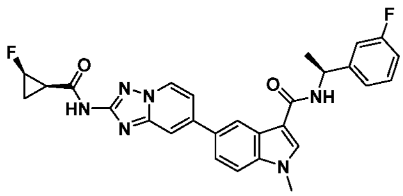,

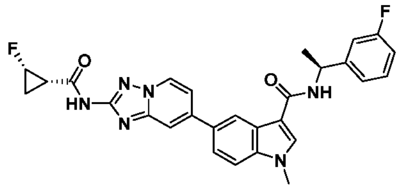,

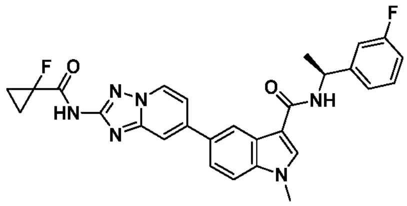,

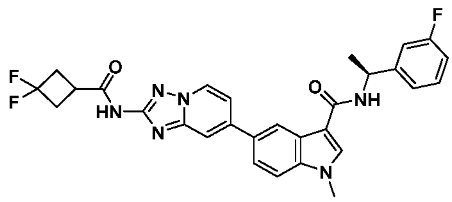,

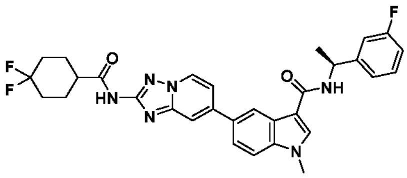,

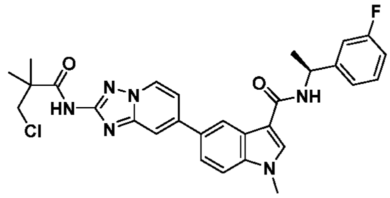

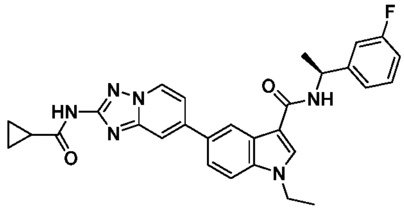,

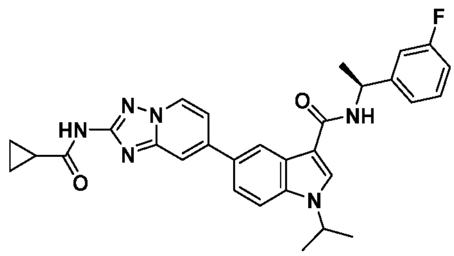,

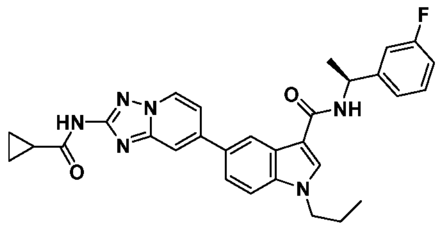 ,

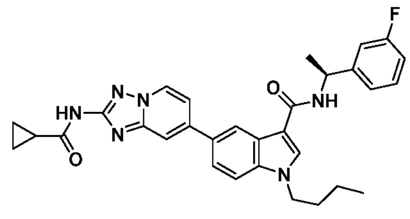 ,

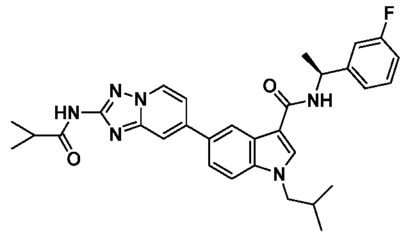 ,

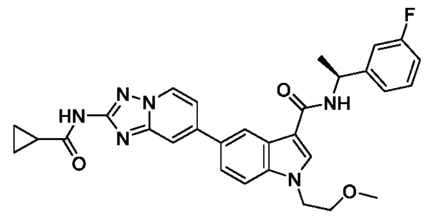

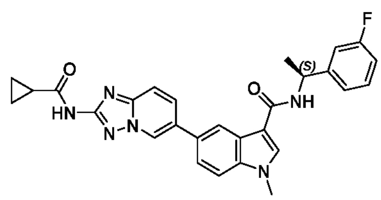 ,

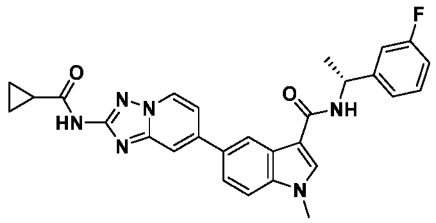 ,

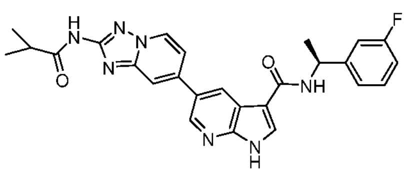 ,

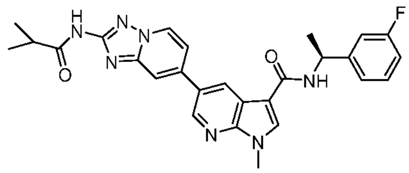 ,

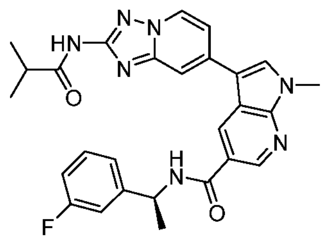 ,

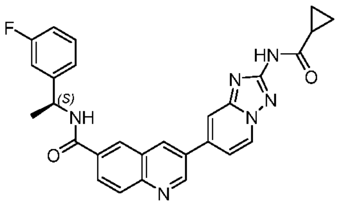 ,

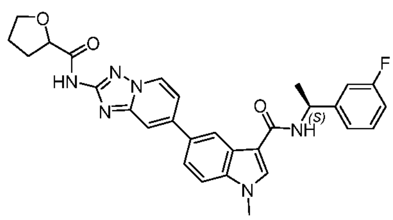

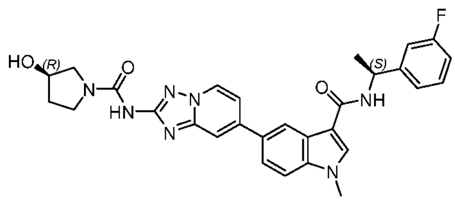 ,

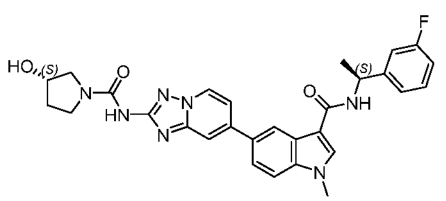
,
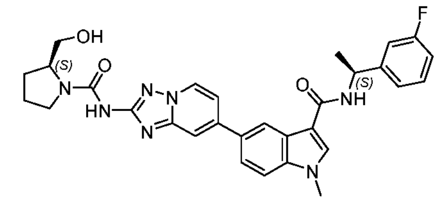
,
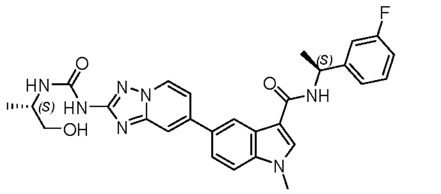
,
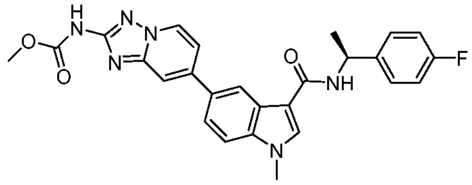
,
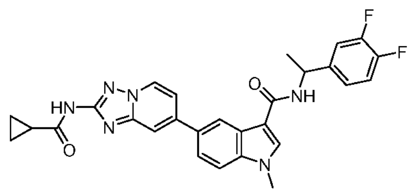
,
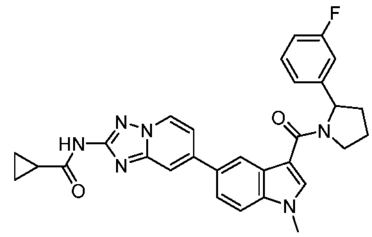
,
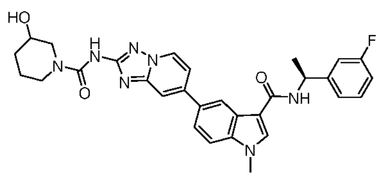
,
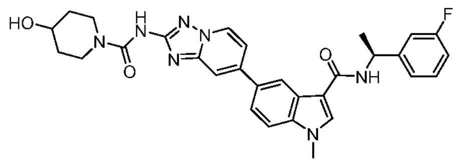
,
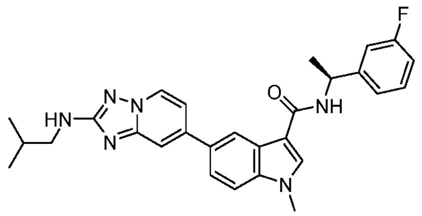
,
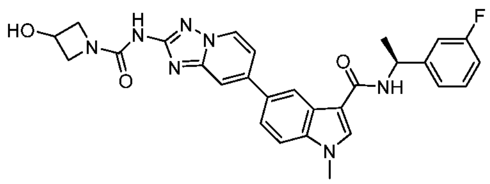
,
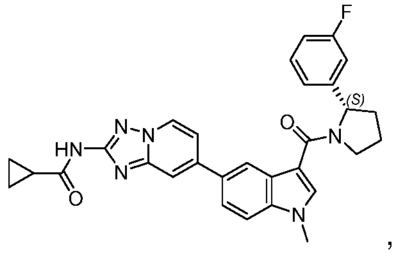
,
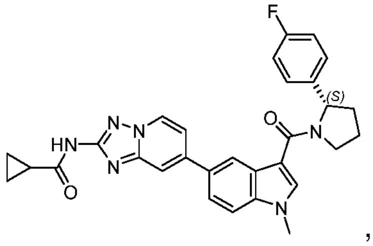
,
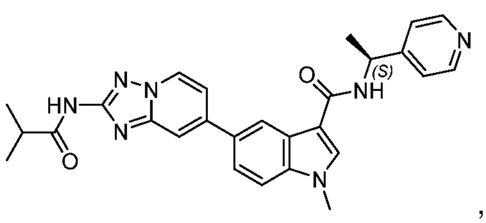
,
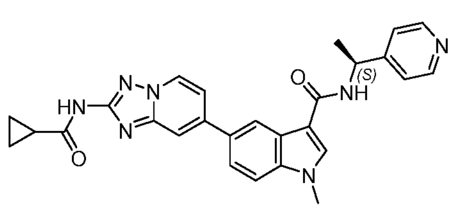
,

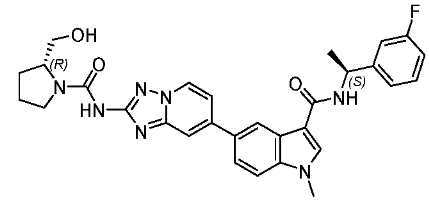,

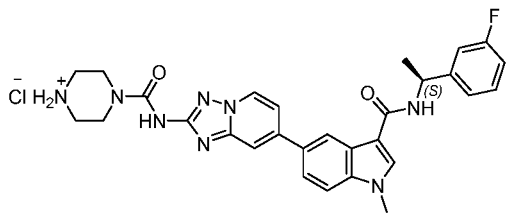,

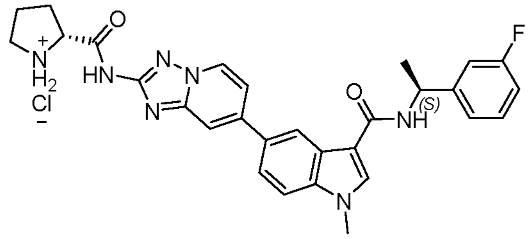,

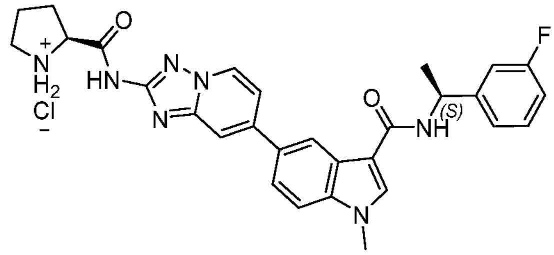,

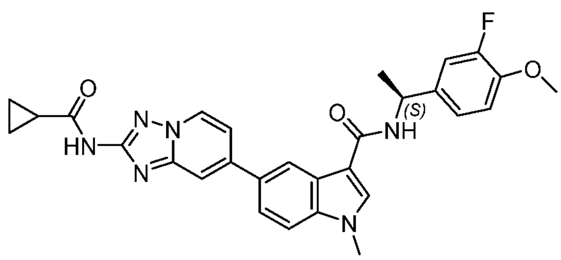,

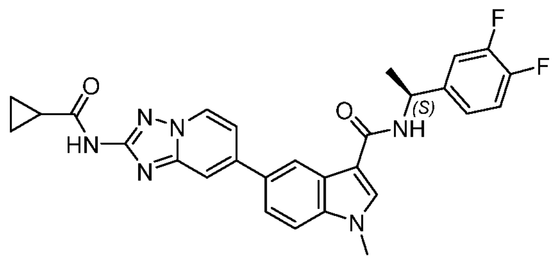,

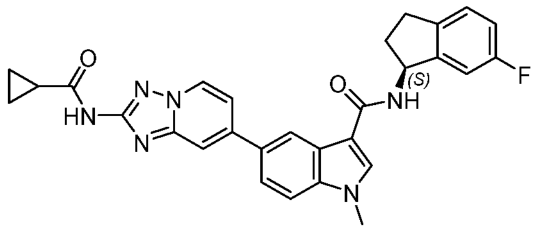,

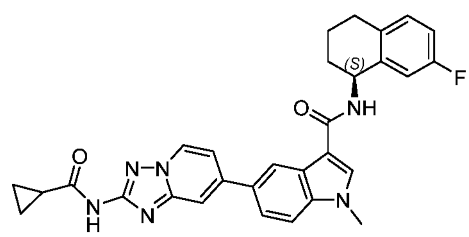,

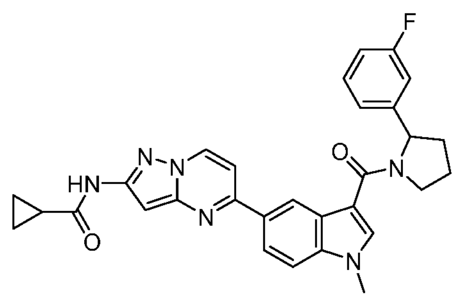,

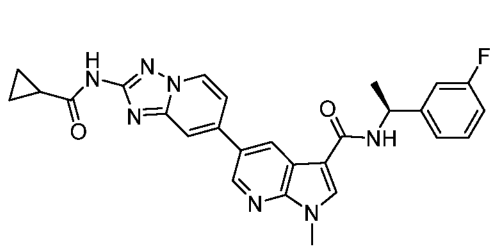,

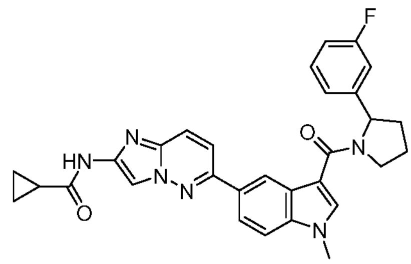,

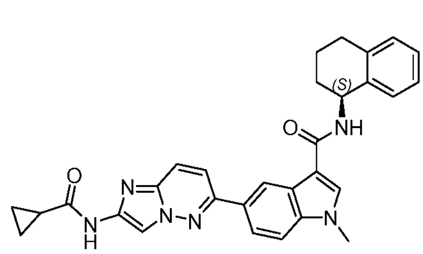,

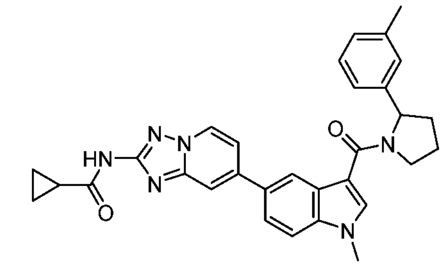,

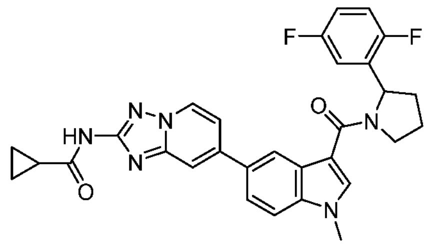,

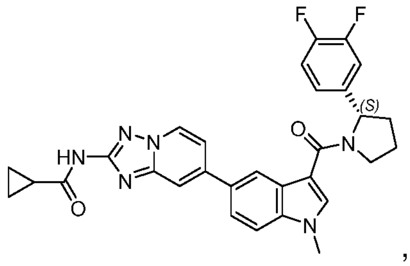,

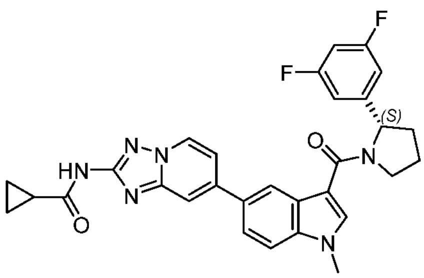,

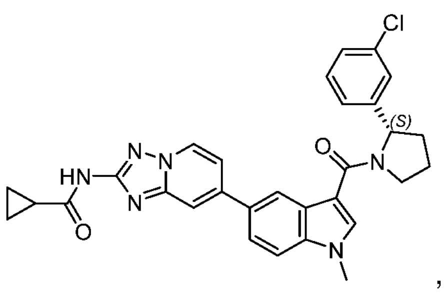,

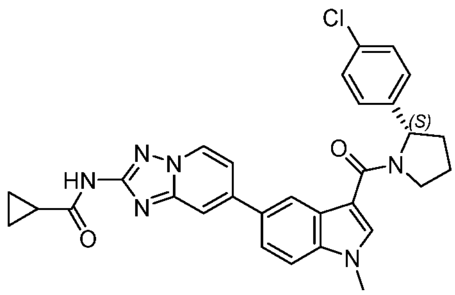,

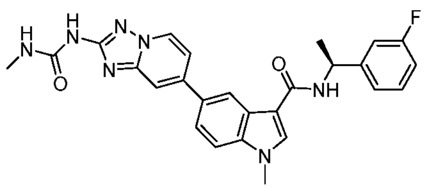,

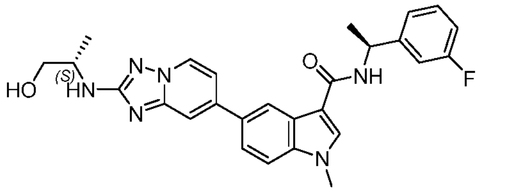,

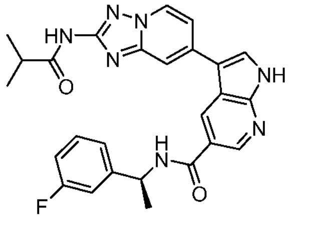,

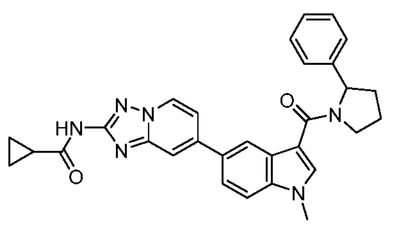,

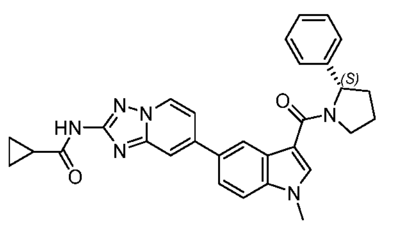,

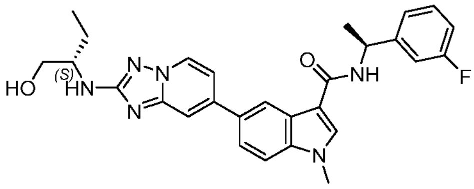,

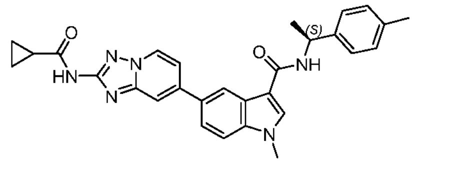,

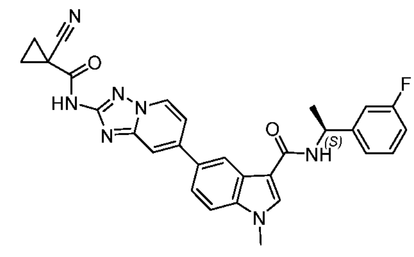,
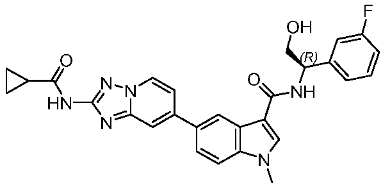,
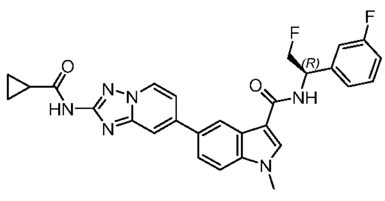,

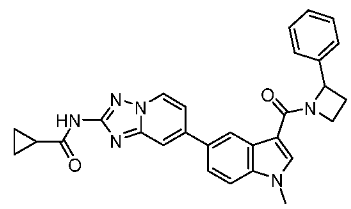,
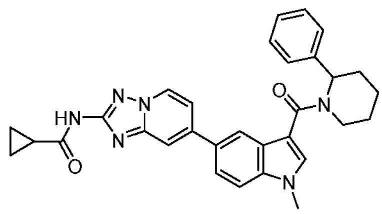,
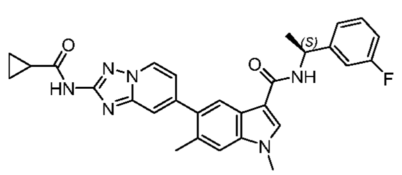,

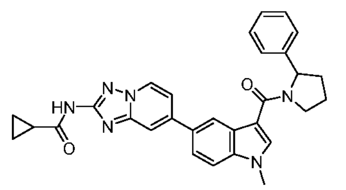,
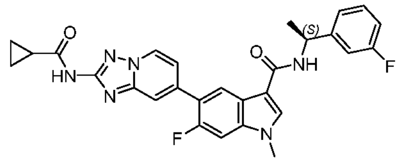,
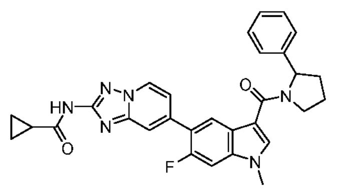,

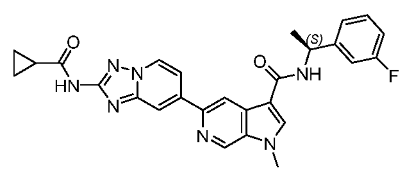,
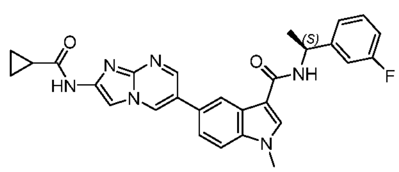,
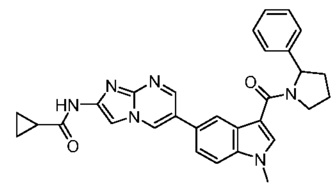,

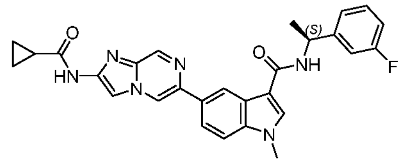,
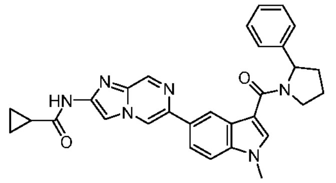,
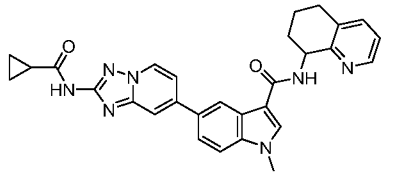,

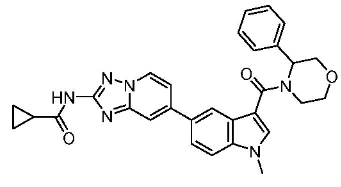,
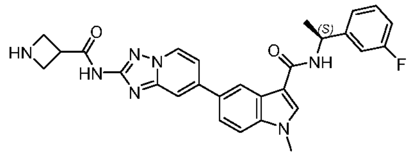,
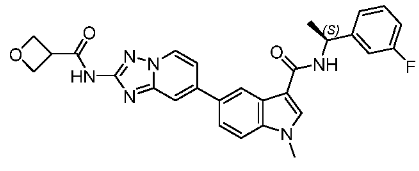,

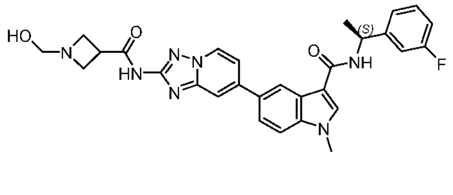,
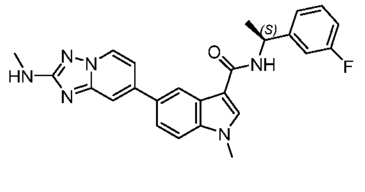

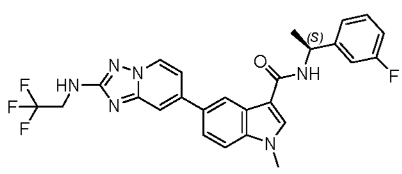,
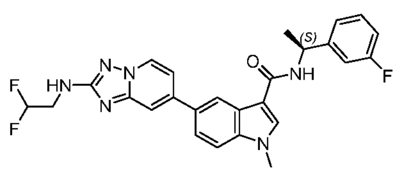,
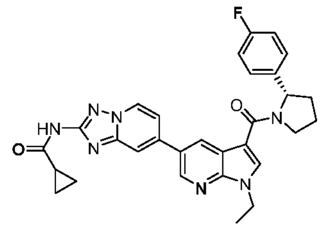,

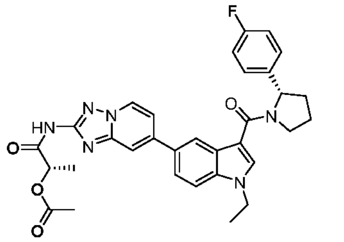,
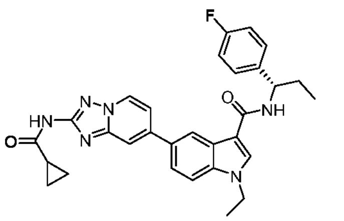,
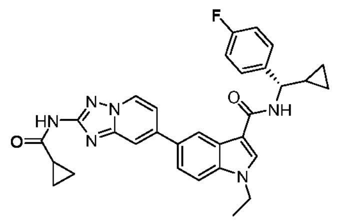,

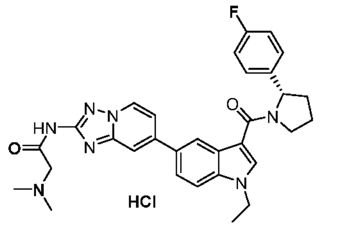,
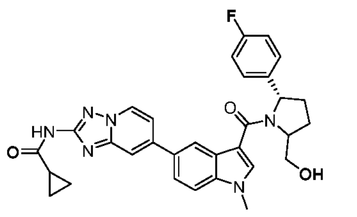,
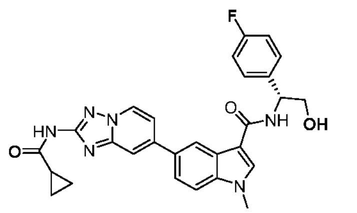,

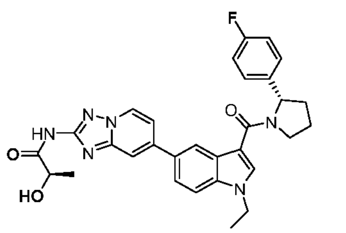,
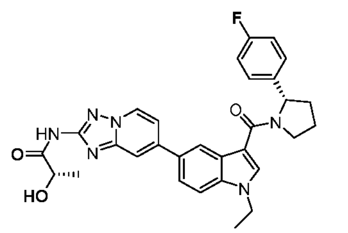,
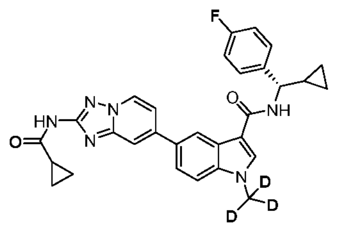,

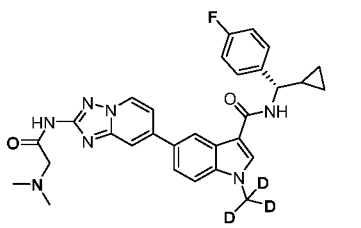,
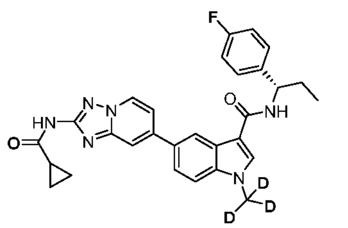,
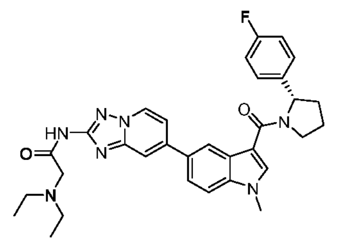,

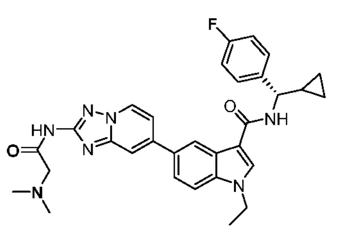,
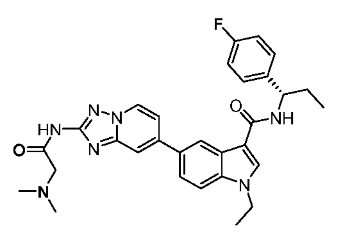,
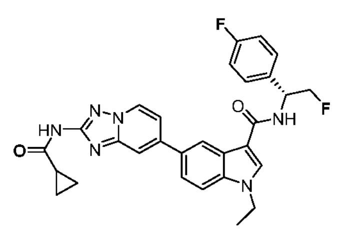,

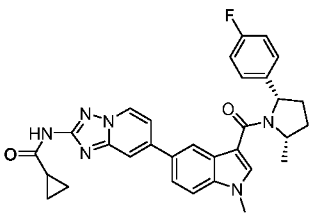
,
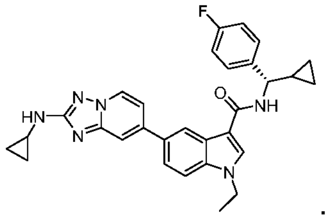
.

The present invention also provides a preparation method of the compound described above, the method is conducted by the following reaction steps:

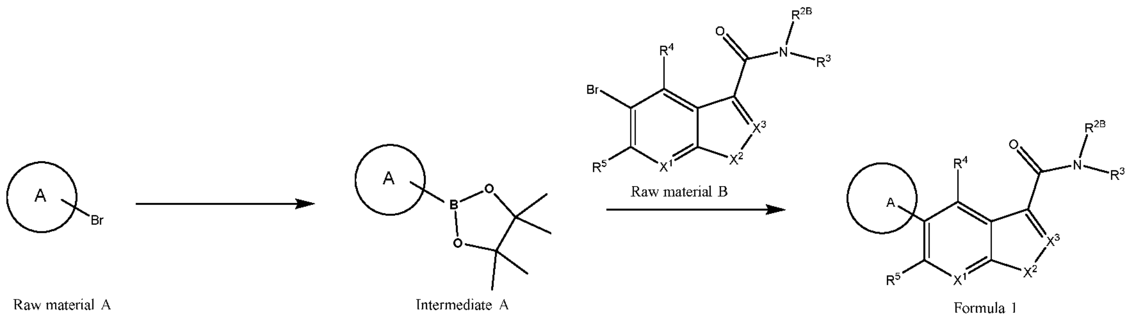


or

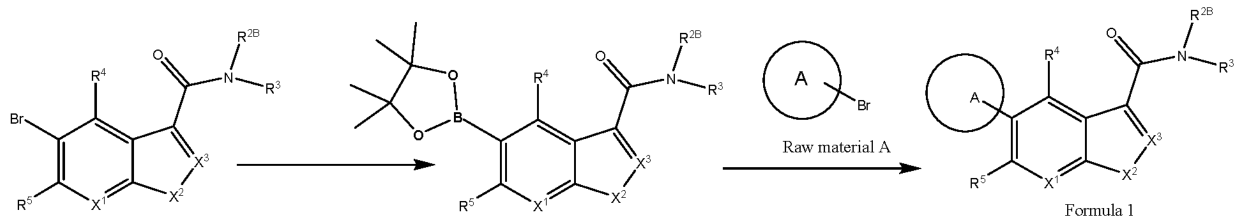


,

wherein $R^{2B}$, $R^4$, $R^5$, $X^1$, $X^2$, $X^3$, and ring A are as described above.

Preferably, a process for the synthesis of Intermediate A is as follows:

dissolving raw material A, potassium acetate, bis(pinacolato)diboron, and [1,1'-bis(diphenylphosphino)ferrocene]palladium(II) dichloride dichloromethane complex in anhydrous dioxane, replacing the reaction system with inert gas, then conducting the reaction, after the reaction is finished, concentrating the reaction solution, mixing the sample, and conducting column chromatography to obtain a product;

a process for the synthesis of the compound of Formula I from Intermediate A and raw material B is as follows:

dissolving Intermediate A, raw material B, [1,1'-bis(diphenylphosphino)ferrocene]palladium(II) dichloride dichloromethane complex, tricyclohexylphosphane, and cesium carbonate in a dioxane/water mixture, replacing the reaction system with inert gas, then conducting the reaction, after the reaction is finished, concentrating the reaction solution, mixing the sample, and conducting column chromatography to obtain a product;

a process for the synthesis of Intermediate B is as follows:

dissolving raw material B, potassium acetate, bis(pinacolato)diboron, and [1,1'-bis(diphenylphosphino)ferrocene]palladium(II) dichloride dichloromethane complex in anhydrous dioxane, replacing the reaction system with inert gas, then conducting the reaction, after the reaction is finished, concentrating and stirring the reaction solution after the reaction is finished, mixing the sample, and conducting column chromatography to obtain a product;

a process for the synthesis of the compound of Formula I from Intermediate B and raw material A is as follows:

dissolving Intermediate B, raw material A, [1,1'-bis(diphenylphosphino)ferrocene]palladium(II) dichloride dichloromethane complex, tricyclohexylphosphane, and cesium carbonate in a dioxane/water mixture, replacing the reaction system with inert gas, then conducting the reaction, after the reaction is finished, concentrating the reaction solution, mixing the sample, and conducting column chromatography to obtain a product.

The present invention also provides a use of the compound described above, or a stereoisomer, or a pharmaceutically acceptable salt thereof in the preparation of a RIPK1 inhibitor.

The present invention also provides a use of a compound described above, or a stereoisomer, or a pharmaceutically acceptable salt thereof, in the preparation of a medicament for treating inflammation, immunological diseases, neurodegenerative diseases, or tumors.

Preferably, the medicament is for treating inflammatory responses associated with programmed necrosis, immunological diseases, neurodegenerative diseases, or tumors. Preferably, the inflammation is colitis.

The present invention also provides a pharmaceutical composition, which is a preparation prepared by the compound described above, or a stereoisomer, or a pharmaceutically acceptable salt thereof, and a pharmaceutically acceptable excipient. Programmed necrosis, as defined herein, is an actively ordered pattern of cell death determined by genes. It refers specifically to the suicide protection measures initiated by gene regulation when cells are stimulated by internal and external environmental factors, including the induced activation of some molecular mechanisms and gene programming, in this way, unnecessary cells or cells to be specialized in the body are removed. Ligands for TNF, TLR3, and TLR4, certain bacteria, viral infections, etc. can cause programmed necrosis.

The compounds and derivatives provided herein can be named according to the IUPAC (International Union of Pure and Applied Chemistry) or CAS (Chemical Abstracts Service, Columbus, OH) nomenclature system.

Definitions of terms used in the present invention: unless otherwise indicated, the initial definition provided for a group or term herein applies to that group or term throughout the specification; for terms not specifically defined herein, the meanings that would be afforded them by a person skilled in the art, in light of the disclosure and context, should be given.

"Substituted" refers to the replacement of a hydrogen atom in a molecule with another different atom or molecule.

The minimum and maximum amounts of carbon atoms in a hydrocarbon group are indicated by a prefix, e.g., the prefix $C_a$-$C_b$ alkyl indicates any alkyl group containing "a" to "b" carbon atoms. Therefore, for example, "$C_1$-$C_4$ alkyl" denotes an alkyl group having 1 to 4 carbon atoms. In particular, alkylene is defined by "$C_0$-$C_b$", it is meant that the site may be free of alkylene.

"Alkyl" refers to a saturated hydrocarbon chain having a specified number of member atoms. For example, $C_1$-$C_6$ alkyl refers to alkyl groups having 1 to 6 member atoms, for example, alkyl groups having 1 to 4 member atoms. Alkyl groups may be linear or branched. Representative branched alkyl groups have one, two, or three branched chains. An alkyl group may be optionally substituted by one or more substituents as defined herein. Alkyl groups include methyl, ethyl, propyl (n-propyl and isopropyl), butyl (n-butyl, isobutyl and tert-butyl), pentyl (n-pentyl, isopentyl and neopentyl), and hexyl. Alkyl groups can also be part of other groups such as $C_1$-$C_6$ alkoxy.

"Cycloalkyl" refers to a saturated or partially saturated cyclic group having 3 to 14 carbon atoms and no heteroatoms and having a single ring or multiple rings, including fused, bridged, and spiro ring systems. When the point of attachment is at a non-aromatic carbon atom, polycyclic ring systems having aromatic and non-aromatic rings containing no heteroatoms are referred to as the term "cycloalkyl" (e.g., 5, 6, 7, 8, -tetrahydronaphthalen-5-yl). The term "cycloalkyl" includes cycloalkenyl groups, such as cyclohexenyl. Examples of cycloalkyl groups include, for example, adamantyl, cyclopropyl, cyclobutyl, cyclohexyl, cyclopentyl, cyclooctyl, cyclopentenyl, and cyclohexenyl.

"Alkenyl" refers to a linear or branched chain hydrocarbyl group having 2 to 10 carbon atoms and in some embodiments 2 to 6 carbon atoms or 2 to 4 carbon atoms and having at least 1 site of vinyl unsaturation site (>C=C<). For example, ($C_a$-$C_b$) alkenyl refers to an alkenyl group having a to b carbon atoms and is intended to include, for example, ethenyl, propenyl, isopropenyl, 1, 3-butadienyl, and the like.

"Alkynyl" refers to a linear monovalent hydrocarbon group or a branched monovalent hydrocarbon group containing at least one triple bond. The term "alkynyl" is also intended to include those hydrocarbyl groups having one triple bond and one double bond. For example, ($C_2$-$C_6$) alkynyl is intended to include ethynyl, propynyl, and the like.

"Halogen" includes fluorine, chlorine, bromine, or iodine.

"Heterocycle" and "heterocycloalkyl" refer to a saturated ring or a non-aromatic unsaturated ring containing at least one heteroatom; wherein the heteroatom refers to nitrogen atom, oxygen atom, or sulfur atom;

"Heteroaryl" refers to an aromatic unsaturated ring group containing at least one heteroatom; wherein heteroatom refers to nitrogen atom, oxygen atom, or sulfur atom;

An "ether group" refers to a group formed by removing an H from a carbon atom of ether compounds. A $C_2$-$C_6$

ether group refers to a total number of carbon atoms of the hydrocarbon group attached to both ends of the oxygen atom in the ether compounds is 2 to 6.

"Amine" refers to a group formed by the removal of one H from an amine compound. The amine compound refers to a compound formed when hydrogen atoms in an ammonia molecule are partially or fully substituted by hydrocarbon groups, and a $C_2$-$C_6$ amine group refers to a compound in which a total number of carbon atoms of the hydrocarbon groups attached to both ends of an oxygen atom is 2 to 6.

An "ester group" refers to a group formed by removing one H from a carbon atom of an ester compound. Ester compounds refer to compounds formed upon dehydration of an acid with an alcohol, which have a "-COO-" functional group. A $C_1$-$C_{10}$ ester group refers to a total number of carbon atoms in the amine compound is 1 to 10.

"$R_a$ and $R_b$ are joined to form a heterocyclic ring" refers to at least one atom of each of $R_a$ and $R_b$ is linked by a chemical bond, such that an atom or a chain of atoms to which $R_a$ and $R_b$ are commonly linked in the general structure together with $R_a$ and $R_b$ form a heterocyclic ring as part of the backbone of the ring structure.

"Stereoisomers" include enantiomers and diastereomers.

In the present invention, a horizontal line attached to a symbol representing a substituent represents a covalent bond. For example, "-R" means that R is linked to other groups by a single covalent bond; "-R-" means that R is linked to other groups by two single covalent bonds; "-$R^{A2}$-$R^{A3}$" means that $R^{A3}$ is linked to $R^{A2}$ by one covalent single bond, and $R^{A2}$ is linked to $R^{A3}$ and one other group, respectively, by two covalent single bonds.

The term "pharmaceutically acceptable" refers to a carrier, vehicle, diluent, adjuvant, and/or salt that is formed generally chemically or physically compatible with the other ingredients that make up the pharmaceutical dosage, and is physiologically compatible with the recipient.

The terms "salt" and "pharmaceutically acceptable salt" refer to acidic and/or basic salts of the above compounds or stereoisomers thereof with inorganic and/or organic acids and bases, including zwitterionic salts (inner salts), as well as quaternary ammonium salts, such as alkylammonium salts. These salts may be obtained directly in the final isolation and purification of the compounds. These salts may also be obtained by mixing the compounds described above, or stereoisomers thereof with an appropriate amount (e.g., an equivalent amount) of an acid or base. These salts may precipitate in solution and be collected by filtration or recovered after evaporation of the vehicle or prepared by reaction in an aqueous medium followed by lyophilization. The salt in the present invention may be hydrochlorides, sulfates, citrates, benzenesulfonates, hydrobromides, hydrofluorides, phosphates, acetates, propionates, succinates, oxalates, malates, succinates, fumarates, maleates, tartrates or trifluoroacetates of the compound.

In some embodiments, one or more compounds of the present invention may be used in combination with each other. The compounds of the present invention may also optionally be used in combination with any other active agent for the manufacture of a medicament or pharmaceutical composition for regulating cell function or treating a disease. If a group of compounds is used, the compounds may be administered to a subject simultaneously, separately, or sequentially.

It will be apparent that various other modifications, substitutions, and alterations can be made in the present invention without departing from the basic technical concept of the invention as described above, according to the common technical knowledge and customary means in the field.

The present invention will be described in further detail with reference to the following specific examples. However, it should not be construed that the subject of the present invention is limited to the following examples. All the technologies implemented based on the above contents of the present invention belong to the scope of the present invention.

**Brief Description of the Drawings**

FIG. 1 is a kinase selectivity profile of compound 34 of the present invention;
FIG. 2 is a kinase selectivity profile of Compound 94 of the present invention;
FIG. 3 is a graph showing the survival rate of cells in Example 5;
FIG. 4 shows the effect of compounds 46 and 94 in Example 6 on the necrotic signaling pathway;
FIG. 5 shows the results of protection of TNF $\alpha$-induced mouse SIRS model by compounds 46 and 94 in Example 7;
FIG. 6 shows a concentration-response curve for compound terfenadine (left) and compound 94 (right) in Example 8.
FIG. 7 shows the results of a contrast experiment on the length of the mouse colon in Example 8; and
FIG. 8 shows a concentration-response curve for the inhibition against hERG current by compound terfenadine (left) and compound 94 (right) in Example 10.

## Detailed Description of the Invention

The present disclosure is further illustrated below with reference to Examples. The foregoing descriptions of specific examples of the present disclosure are presented for purposes of illustration and description. These descriptions are not intended to limit the present disclosure to the precise forms disclosed, and obviously many modifications and variations are possible considering the above teaching. The exemplary embodiments are chosen and described to explain certain principles of the present disclosure and its practical application to enable the person skilled in the art to make and use various exemplary embodiments of the present disclosure as well as various alternatives and modifications.

The experimental methods applied in the following examples are conventional methods, unless otherwise specified.

The materials, reagents, etc. used in the following examples are all commercially available, unless otherwise specified.

NMR was measured using BRUKER 400MR DD2 nucleus magnetic resonance instrument with deuterated dimethyl sulfoxide (DMSO-$d_6$), deuterated methanol ($CD_3OD$), and deuterated chloroform ($CDCl_3$) as vehicles, and tetramethylsilane (TMS) as an internal standard. LC-MS was conducted on an Agilent 1200 Infinity II - Infinity Lab LC/MSD mass spectrometer. HPLC was determined using an Agilent 1200 Infinity II high-pressure liquid chromatograph (Sunfire C18 5um 150 x 4.6 mm column;). HSGF254 silica gel plates with the specification of 0.9-1 mm from Yantai Jiangyou Silica Gel Development Co., Ltd. were used for thin layer chromatography. GF254 silica gel plates with the specification of 0.2-0.25 mm from Yucheng Chemical (Shanghai) Co., Ltd. were used for TLC. Silica gel of 300-400 meshes from Qingdao Hailang Silica Desiccant Co., Ltd. was used as the vehicle for column chromatography. The Flash column was an Claricep Flash amorphous silica gel purification column from Agela & Phenomenex. In the examples of the present invention, the reagents 1-propylphosphonic anhydride, methylmagnesium bromide were purchased from Shanghai Macklin Biochemical Co., Ltd.; 4N hydrochloric dioxane solution was purchased from Panjin Infinity Scientific Co., Ltd.; 1M borane in tetrahydrofuran, N,N-diisopropylethylamine, and (S)-tert-butylsulfinamide were purchased from Shanghai Adamas Reagent Co., Ltd.; other reagents and starting materials were purchased from Shanghai Haohong Scientific Co., Ltd. or may be synthesized by methods known in the art. Unless otherwise specified, all reactions of the present invention are carried out under continuous magnetic stirring, under dry nitrogen or argon, with a vehicle being a dry vehicle and a reaction temperature being degrees Celsius.

### Example 1 Preparation of Compound 34 of the present invention (Method I)

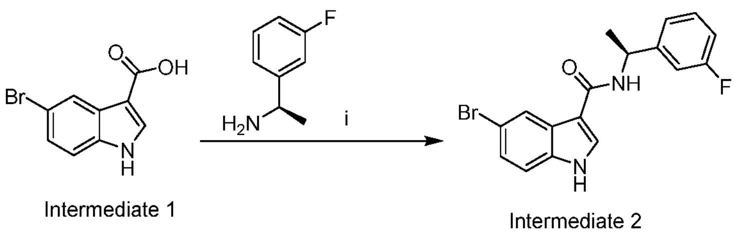


Intermediate 1

Intermediate 2

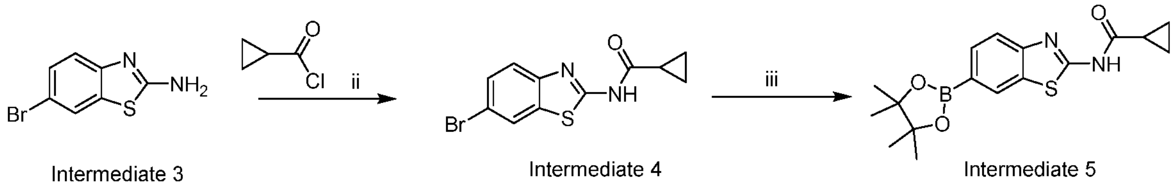


Intermediate 3

Intermediate 4

Intermediate 5

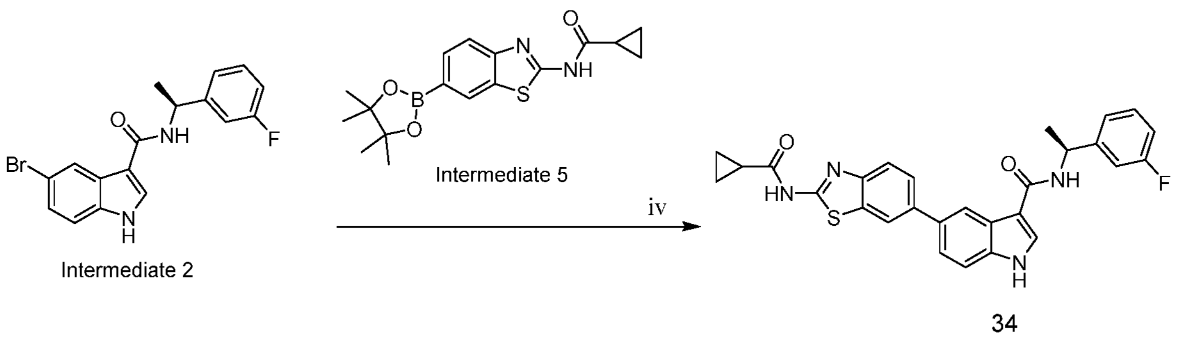


**ii** N,N-dimethylformamide, (S)-1-(3-fluorophenyl)ethanamine, 1-hydroxybenzotriazole, 1-(3-dimethylaminopropyl)-3-ethylcarbodiimide hydrochloride, N,N-diisopropylethylamine, 65°C, 8 h, 85% yield.
ii pyridine, cyclopropylformyl chloride, triethylamine, 0°C to ambient temperature, 6 h, 90% yield;
iii anhydrous dioxane, [1,1'-bis(diphenylphosphino)ferrocene]palladium(II) dichloride dichloromethane complex, potassium acetate, bis(pinacolato)diboron, 95°C, 24 h, 60% yield;
iv 1,4-dioxane/water = 10:1, [1,1'-bis(diphenylphosphino)ferrocene]palladium(II) dichloride dichloromethane complex, tricyclohexylphosphane, cesium carbonate, 100°C, 16 h, 75% yield;
the specific procedures are as follows:

**Preparation of (S)-5-bromo-N-(1-(3-fluorophenyl)ethyl)-1H-indole-3-carboxamide (Intermediate 2):**

5-bromoindole-3-formic acid (4.5 g, 18.8 mmol), 1-hydroxybenzotriazole (HOBT) (3 g, 21.6 mmol), 1-(3-dimethylaminopropyl)-3-ethylcarbodiimide hydrochloride (EDCI) (4.3 g, 23.4 mmol) were dissolved in 60 mL of N,N-dimethylformamide, then N,N-diisopropylethylamine (3.6 mL, 23.4 mmol) was added, the carboxylic acid activated was at room temperature for 0.5 h, (S)-1-(3-fluorophenyl)ethanamine (2.8 g, 19.8 mmol) was added, and the reaction was heated to 65°C for 8 h; after the reaction was finished as monitored by TLC, the reaction solution was concentrated and extracted with dichloromethane and sodium thiosulfate solution; the organic phase was washed with water and saturated sodium chloride solution, and dried over sodium sulfate; after suction filtration and column chromatography, Intermediate 2 (5.7 g, white solid) was obtained with a yield of 85%.

$^1$H NMR (400 MHz, DMSO- $d_6$) δ 11.46 (s, 1H), 8.35 (s, 1H), 8.19 (d, *J* = 5.9 Hz, 1H), 7.96 (d, *J* = 2.5 Hz, 1H), 7.25 (d, *J* = 7.7 Hz, 1H), 7.11 - 7.02 (m, 2H), 7.00 (s, 1H), 6.97 (d, *J* = 7.0 Hz, 1H), 5.21 - 5.11 (m, 1H), 1.47 (d, *J* = 7.1 Hz, 3H)

**Preparation of *N*-(6-bromobenzo[d]thiazol-2-yl)cyclopropanecarboxamide (Intermediate 4):**

1 g of Intermediate 3 (1.0 g, 4.4 mmol) was dissolved in 15 mL of dry pyridine, 1.4 mL of triethylamine (575 mg, 5.7 mmol) was added, and 370 μL of cyclopropylformyl chloride (499 mg, 4.8 mmol) was slowly added dropwise at 0°C. The temperature was gradually raised to room temperature for 6 h; the reaction solution was directly concentrated, extracted with dichloromethane and saturated sodium carbonate solution; the organic phase was washed with water and saturated sodium chloride solution, dried over sodium sulfate, and purified by column chromatography to afford Intermediate 4 (1.2 g, pale yellow solid) with a yield of 90%. $^1$H NMR (400 MHz, $CDCl_3$) δ 9.01 (s, 1H), 8.11 (d, *J* = 6.9 Hz, 1H), 7.55 (s, 1H), 7.02 (d, *J* = 5.4 Hz, 1H), 1.23 (d, *J* = 11.0 Hz, 1H), 1.16 (dt, *J* = 7.4 3.9 Hz, 2H), 0.93 (td, *J* = 7.0, 3.9 Hz, 2H).

**Preparation** of **N-(6-(pinacolato diboron)benzo[d]thiazol-2-yl)cyclopropanecarboxamide (Intermediate 5):**

Intermediate 4 (10 g, 33.8 mmol), potassium acetate (6.6 g, 67.6 mmol), 12 g of bis (pinacolato) diboron (12 g, 47.2 mmol), and 2.8 g of [1,1'-bis (diphenylphosphino)ferrocene]palladium(II) dichloride dichloromethane complex (2.8 g, 3.4 mmol) were dissolved in 200 ml of anhydrous dioxane; after replacing the reaction system with nitrogen for three times, the mixture was left to react at 95°C for 24 h; a developing solvent petroleum ether/ethyl acetate (20:1) was used to monitor the reaction; after the reaction was finished, the reaction solution was concentrated, mixed with the sample, and subjected to column chromatography using a petroleum ether/ethyl acetate ( 80:1) eluent to afford Intermediate 5 (6.9 g, white solid) with a yield of 60%. $^1$H NMR (400 MHz, DMSO-$d_6$) δ 12.01 (s, 1H), 8.45 (d, *J* = 6.7 Hz, 1H), 8.03 (s, 1H), 7.87 (d, *J* = 5.7 Hz, 1H), 1.21 (d, *J* = 11.2 Hz, 1H), 1.33 (s, 12H), 1.11 (dt, *J* = 7.4 3.9 Hz, 2H), 0.99 (td, *J* = 7.0, 3.9 Hz, 2H).

**Preparation of (S)-5-(2-(cyclopropanecarboxamido)benzo[d]thiazol -6-yl)-N-(1-(3-fluorophenyl)ethyl)-1*H*-indole-3-carboxamide (Compound 34):**

Intermediate 2 (400 mg, 1.1 mmol), Intermediate 5 (580 mg, 1.7 mmol), 133 mg of [1,1'-bis (diphenylphosphino) ferrocene]palladium(II) dichloride dichloromethane complex (133 mg, 0.16 mmol), 41 mg of tricyclohexylphosphane (45 mg, 0.16 mmol), and 780 mg of cesium carbonate (780 mg, 2.4 mmol) were dissolved in 100 ml of a mixture of 1, 4-dioxane/water (10:1); after replacing the reaction system with nitrogen for three times, the mixture was left to react at 100°C for 16 h; a developing solvent ethyl acetate/petroleum ether (1:1) was used to monitor the reaction; after the reaction was finished as monitored by TLC, the reaction solution was concentrated, mixed with the sample, and subjected to column chromatography using ethyl acetate/petroleum ether (5:1) eluent to afford compound 34 (318 mg, off-white solid) with a yield of 58%

$^1$H NMR (400 MHz, DMSO) δ 12.63 (s, 1H), 11.66 (s, 1H), 8.43 (s, 1H), 8.31 (d, *J* = 7.9 Hz, 1H), 8.21 (d, *J* = 2.4 Hz, 1H), 7.81 - 7.75 (m, 2H), 7.70 (dd, *J* = 8.5, 1.6 Hz, 2H), 7.53 (d, *J* = 8.1 Hz, 2H), 7.37 (dd, *J*= 14.1, 8.1 Hz, 2H), 7.25 (t, *J*= 9.4 Hz, 2H), 7.09 - 7.00 (m, 1H), 5.75 (s, 1H), 5.27 - 5.15 (m, 1H), 2.06 (d, *J*= 11.0 Hz, 1H), 0.96 (d, *J* = 5.1 Hz, 4H).

**Example 2 Preparation of Compound 94 of the present invention ( Method II)**

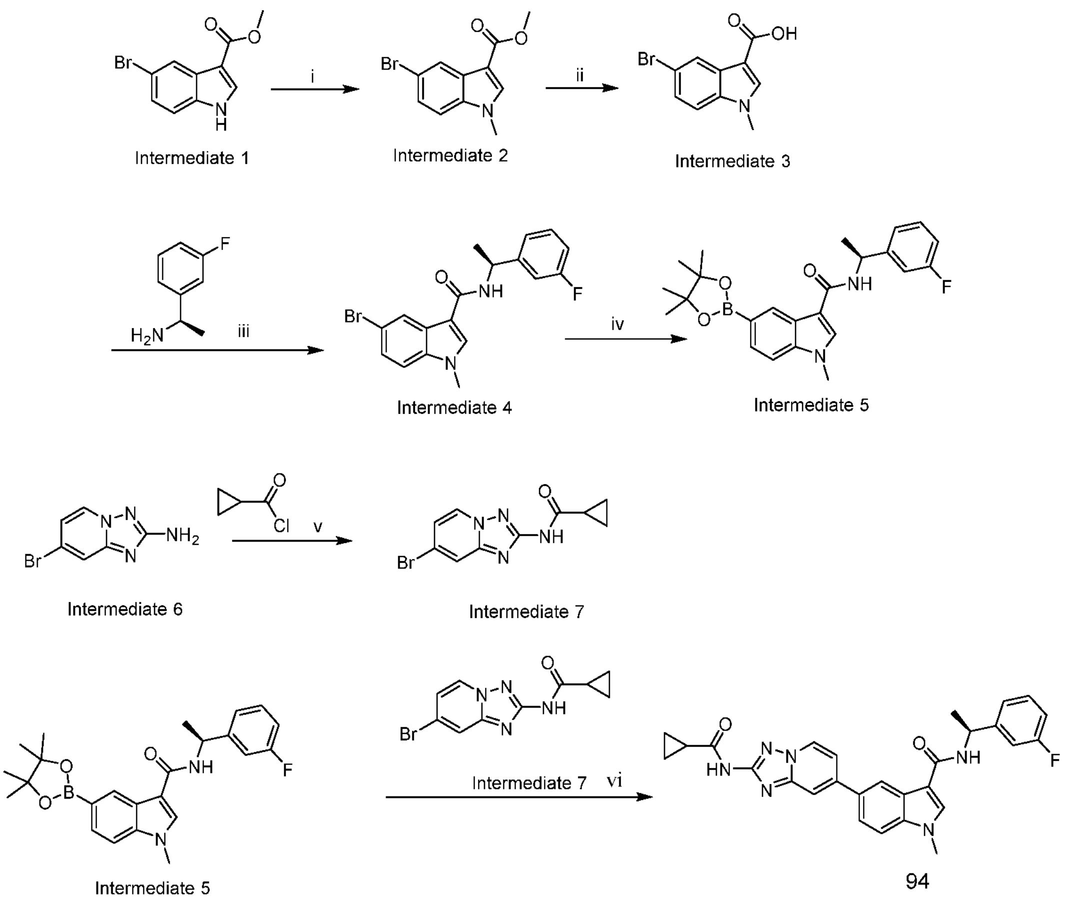


Intermediate 1, Intermediate 2, Intermediate 3, Intermediate 4, Intermediate 5, Intermediate 6, Intermediate 7, Intermediate 5, 94

i N,N-dimethylformamide, iodomethane, cesium carbonate, 80°C, 8 h, 90%.
ii methanol, water, sodium hydroxide, 60°C, 3 h, 95%;
iii N,N-dimethylformamide, (S)-1-(3-fluorophenyl)ethylamine, 1-hydroxybenzotriazole, 1-(3-dimethylaminopropyl)-3-ethylcarbodiimide hydrochloride, N,N-diisopropylethyl amine, 65°C, 8 h, 85%.
iv 1,4-dioxane/water = 5:1, [1,1'-bis(diphenylphosphino)ferrocene]palladium(II) dichloride dichloromethane complex, tricyclohexyl phosphorus, cesium carbonate, 95°C, 12 h, 75%;
v pyridine, Cyclopropanecarbonyl chloride, triethylamine, 0°C to ambient temperature, 6 h, 90%;
vi 1,4-dioxane/water = 10:1, [1,1'-bis(diphenylphosphino)ferrocene]palladium(II) dichloride dichloromethane complex, tricyclohexylphosphane, cesium carbonate, 95°C, 12 h, 75%; the specific procedures are as follows:

**Preparation of methyl 5-bromo-1-methyl-1H-indole-3-carboxylate (Intermediate 2):**

Intermediate 1 (1.0 g, 3.9 mmol), iodomethane (700 mg, 4.9 mmol), and cesium carbonate (1.8 g, 5.5 mmol) were dissolved in 30 mL of N,N-dimethylformamide and reacted at 80°C for 8 h under nitrogen; a developing solvent ethyl acetate/petroleum ether (1:3) was used to monitor the reaction; after the reaction was finished, the reaction solution was directly concentrated, mixed with the sample, and subjected to column chromatography. Purification was conducted with ethyl acetate:petroleum ether (1:1) eluent to afford Intermediate 2 (937 mg, white solid) with a yield of 90%.

$^1$H NMR (400 MHz, DMSO-$d_6$) δ 8.18 (s, 1H), 8.11 (d, *J* = 1.9 Hz, 1H), 7.55 (d, *J* = 8.7 Hz, 1H), 7.41 (dd, *J* = 8.7, 1.9 Hz, 1H), 3.86 (s, 3H), 3.81 (s, 3H).

**Preparation of 5-bromo-1-methyl-1H-indole-3-carboxylic acid (Intermediate 3):**

Intermediate 2 (507 mg, 1.9 mmol) was dissolved in 20 ml of methanol, 10 ml of 2 mol/L sodium hydroxide solution was added, the reaction was stirred at 60°C for 3 h, a developing solvent ethyl acetate/petroleum ether (1:1) was used to monitor the reaction; after the reaction was finished, the reaction solution was directly concentrated, diluted with water to adjust pH = 4, a solid was precipitated, and the solid was suction filtered and dried to obtain Intermediate 3 (432 mg, white solid) with a yield of 90%, which was directly subjected to the next reaction without further purification.

**Preparation of (S)-5-bromo-N-(1-(3-fluorophenyl)ethyl)-1-methyl-1H-indole-3-carboxamide (Intermediate 4):**

Intermediate 3 (987 mg, 3.9 mmol), 1-hydroxybenzotriazole (HOBT) (806 mg, 5.8 mmol), 1-(3-dimethylaminopropyl)-3-ethylcarbodiimide hydrochloride (EDCI) (1.0 g, 5.8 mmol) were dissolved in 20 mL of N,N-dimethylformamide, then N,N-diisopropylethylamine (1.0 g, 7.8 mmol) was added, the carboxylic acid activated was at room temperature for 0.5 h, (S)-1-(3-fluorophenyl)ethanamine (650 mg, 4.6 mmol) was added, and the reaction was heated to 65°C for 8 h; after the reaction was finished as monitored by TLC, the reaction solution was concentrated and extracted with dichloromethane and sodium thiosulfate solution; the organic phase was washed with water and saturated sodium chloride solution, and dried over sodium sulfate; after suction filtration and column chromatography, Intermediate 4 (1.2 g, white solid) was obtained with a yield of 84%.

$^1$H NMR (400 MHz, DMSO) δ 8.34 (d, *J* = 8.3 Hz, 1H), 8.26 (d, *J* = 1.9 Hz, 1H), 8.19 (s, 1H), 7.49 (d, *J* = 8.8 Hz, 1H), 7.37 - 7.30 (m, 2H), 7.22 (t, *J* = 9.0 Hz, 2H), 7.05 (d, *J* = 9.3 Hz, 1H), 5.17 (d, *J* = 7.5 Hz, 1H), 3.84 (s, 3H), 1.46 (d, *J* = 7.1 Hz, 3H).

**Preparation** of **(S)-N-(1-(3-fluorophenyl)ethyl)-1-methyl-5-(pinacolato diboron)-1H-indole-3-carboxamide (Intermediate 5):**

Intermediate 4 (2.0 g, 5.3 mmol), potassium acetate (1.1 g, 11.2 mmol), bis (pinacolato) diboron (1.7 g, 6.9 mmol), and [1,1'-bis (diphenylphosphino) ferrocene] palladium(II) dichloride dichloromethane complex (410 mg, 0.5 mmol) were dissolved in 50 ml of anhydrous dioxane; after replacing the reaction system with nitrogen for three times, the mixture was left to react at 95°C for 12 h; after the reaction was finished, the reaction solution was filtered with diatomite, then concentrated, mixed with the sample, and subjected to column chromatography using a petroleum ether/ethyl acetate (1:1) eluent to afford Intermediate 5 (1.3 g, pale yellow solid) with a yield of 60%.

$^1$H NMR (400 MHz, DMSO-$d_6$) δ 8.55 (s, 1H), 8.33 (d, *J* = 7.9 Hz, 1H), 8.14 (s, 1H), 7.48 (t, *J* = 6.8 Hz, 2H), 7.36 (dd, *J* = 14.1, 8.0 Hz, 1H), 7.22 (t, *J* = 9.5 Hz, 2H), 7.06 - 6.99 (m, 1H), 5.19 - 5.11 (m, 1H), 3.84 (s, 3H), 1.47 (d, *J* = 7.1 Hz, 3H), 1.30 (s, 12H).

**Preparation of N-(7-bromo-[1,2,4]triazolo[1,5-a]pyridin-2-yl)cyclopropanecarboxamide (Intermediate 7):**

Intermediate 6 (1.0 g, 4.7 mmol) was dissolved in 15 mL of dry pyridine, 1.4 mL of triethylamine (949 mg, 9.4 mmol) was added, and cyclopropylformyl chloride (541 mg, 5.2 mmol) was slowly added dropwise at 0°C. The temperature was gradually raised to room temperature for 6 h; the reaction solution was directly concentrated, extracted with dichloromethane and saturated sodium carbonate solution; the organic phase was washed with water and saturated sodium chloride solution, dried over sodium sulfate, and purified by column chromatography to afford Intermediate 7 (1.2 g, yellow solid) with a yield of 90%.

$^1$H NMR (400 MHz, CDCl3) δ 9.25 (s, 1H), 8.40 (d, *J* = 7.1 Hz, 1H), 7.82 (s, 1H), 7.09 (d, J = 5.9 Hz, 1H), 1.27 (d, J = 11.5 Hz, 1H), 1.21 (dt, J = 7.6, 3.9 Hz, 2H), 0.96 (td, J = 7.0, 3.9 Hz, 2H).

**Preparation** of **(S)-5-(2-(cyclopropanecarboxamido)-[1,2,4]triazolo[1,5-a]pyridin-7-yl)-N-(1-(3-fluorophenyl)ethyl)-1-methyl-1H-indole-3-carboxamide (Compound 94):**

Intermediate 7 (280 mg, 1.0 mmol), Intermediate 5 (422 mg, 1.0 mmol), [1,1'-bis (diphenylphosphino) ferrocene] palladium(II) dichloride dichloromethane complex (85 mg, 0.1 mmol), tricyclohexylphosphane (28 mg, 0.1 mmol), and cesium carbonate (390 mg, 1.2 mmol) were dissolved in 100 ml of a mixture of 1,4-dioxane/water (10:1); after replacing the reaction system with nitrogen for three times, the mixture was left to react at 100°C for 12 h; a developing solvent ethyl acetate/petroleum ether (3:1) was used to monitor the reaction; compared with the polarity of the raw material, the product has increased polarity; after the reaction was finished as monitored by TLC, the reaction solution was concentrated, mixed with the sample, and subjected to column chromatography using ethyl acetate/petroleum ether (10:1) eluent to afford compound 94 (372 mg, white solid) with a yield of 75%

$^1$H NMR (400 MHz, DMSO) δ 11.04 (s, 1H), 8.83 (d, *J* = 7.1 Hz, 1H), 8.53 (s, 1H), 8.37 (d, *J* = 8.0 Hz, 2H), 8.22 (s, 1H), 7.86 (s, 1H), 7.72 (d, *J* = 8.6 Hz, 1H), 7.66 (d, *J* = 8.7 Hz, 1H), 7.46 - 7.40 (m, 1H), 7.37 (dd, *J* = 14.2, 7.8 Hz, 2H), 7.25 (t, *J* = 9.7 Hz, 2H), 7.05 (t, *J* = 8.6 Hz, 1H), 5.22 (dd, *J* = 14.3, 6.9 Hz, 1H), 3.90 (s, 3H), 2.07 (s, 1H), 0.96 (d, *J* = 5.3 Hz, 4H).

**Example 3 Preparation of Compound 142 of the present invention (Method III)**

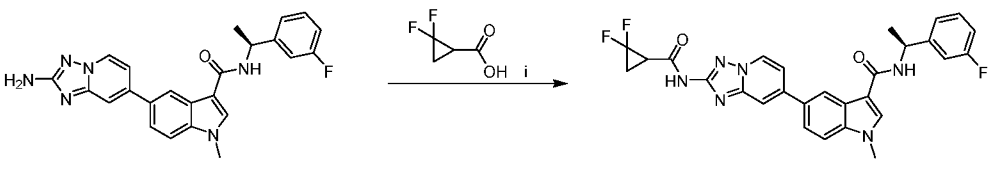


141

143

**i** 2,4,6-tripropyl-1,3,5,2,4,6-trioxatriphosphinane-2,4,6-trioxide, pyridine, 80°C, 12 h, 80% yield.

the specific procedures are as follows:

**Preparation of (*S*)-5-(2-amino-[1,2,4]triazolo[1,5-a]pyridin-7-yl)-N-(1-(3-fluorophenyl)ethyl)-1-methyl-1H -indole-3-carboxamide (141):**

Compound 141 may be prepared according to the procedures shown in Method II.

$^1$H NMR (400 MHz, DMSO-$d_6$) δ 8.55 (d, *J* = 7.0 Hz, 1H), 8.48 (d, *J* = 1.8 Hz, 1H), 8.37 (d, *J* = 8.0 Hz, 1H), 8.21 (s, 1H), 7.73 - 7.60 (m, 2H), 7.55 (d, *J* = 2.0 Hz, 1H), 7.37 (q, *J* = 7.4 Hz, 1H), 7.32 - 7.12 (m, 3H), 7.04 (td, *J* = 8.7, 2.6 Hz, 1H), 5.99 (s, 2H), 5.20 (q, *J* = 7.3 Hz, 1H), 3.89 (s, 3H), 1.49 (d, *J* = 7.1 Hz, 3H).

**Preparation of 5-(2-(2,2-difluorocyclopropane-1-carboxamido)-[1,2,4]triazolo[1,5-a]pyridin-7-yl)-N-((S)-1-(3-fluorophenyl)ethyl)-1-methyl-1H-indole-3-carboxamide (142):**

Compound 141 (150 mg, 0.35 mmol) and 2,2-difluorocyclopropyl carboxylic acid (85 mg, 0.70 mmol) were dissolved in 15 mL of dry pyridine, 2,4,6-tripropyl-1,3,5,2,4,6-trioxatriphosphinane-2,4,6-trioxide (229 mg, 50% in ethyl acetate) was added at room temperature, the temperature was gradually raised to 80°C for 6 h; the reaction solution was directly concentrated, mixed with dichloromethane, and purified by column chromatography to afford 142 (149 mg, pale yellow solid) with a yield of 80%.

$^1$H NMR (400 MHz, DMSO-$d_6$) δ 11.41 (s, 1H), 8.93 (d, *J* = 7.0 Hz, 1H), 8.60 (d, *J* = 1.8 Hz, 1H), 8.45 (d, *J* = 8.1 Hz, 1H), 8.29 (s, 1H), 7.96 (d, *J* = 1.9 Hz, 1H), 7.80 (dd, *J* = 8.6, 1.9 Hz, 1H), 7.72 (d, *J* = 8.7 Hz, 1H), 7.53 (dd, *J* = 7.2, 2.0 Hz, 1H), 7.46 - 7.39 (m, 1H), 7.35 - 7.28 (m, 2H), 7.15 - 7.05 (m, 1H), 5.27 (t, *J* = 7.4 Hz, 1H), 3.95 (d, *J* = 13.0 Hz, 3H), 2.10 (d, *J* = 7.1 Hz, 1H), 1.55 (d, *J* = 7.0 Hz, 3H), 1.31 - 1.18 (m, 2H).

**Example 4 Preparation of Compound 162 of the present invention** ( **Method IV)**

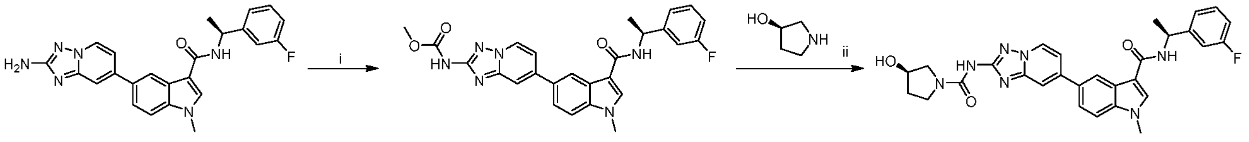


141 166 162

i Methyl chloroformate, N,N-diisopropylethyl amine, dichloromethane, room temperature, 6 h, 90%.
**ii** (R)-3-pyrrolidinol, pyridine, 100°C, 16 h, 80%;

**Preparation of (*S*)-5-(2-amino-[1,2,4]triazolo[1,5-a]pyridin-7-yl)-N-(1-(3-fluorophenyl)ethyl)-1-methyl-1H -indole-3-carboxamide (141):**

Compound 141 may be prepared according to the procedures shown in Method II.

$^1$H NMR (400 MHz, DMSO-$d_6$) δ 8.55 (d, J = 7.0 Hz, 1H), 8.48 (d, J = 1.8 Hz, 1H), 8.37 (d, J = 8.0 Hz, 1H), 8.21 (s, 1H), 7.73-7.60 (m, 2H), 7.55 (d, J = 2.0 Hz, 1H), 7.37 (q, J = 7.4 Hz, 1H), 7.32-7.12 (m, 3H), 7.04 (td, J = 8.7, 2.6 Hz, 1H), 5.99 (s, 2H), 5.20 (q, J = 7.3 Hz, 1H), 3.89 (s, 3H), 1.49 (d, J = 7.1 Hz, 3H).

**Preparation of methyl (S)-(7-(3-((1-(3-fluorophenyl)ethyl)carbamoyl)-1-methyl-1H-indol-5-yl)-[1,2,4]triazolo[1,5 -a]pyridin-2-yl)carbamate (166):**

To a flask was added Intermediate 141 (513 mg, 1.2 mmol), followed by the addition of 10 ml of dichloromethane, 1 ml of N,N-diisopropylethyl amine (310 mg, 2.4 mmol), and methyl chloroformate (131 mg, 1.4 mmol), and the reaction was stirred at room temperature for 6 h; a developing solvent dichloromethane/methanol (20:1) was used to monitor the reaction; after the reaction was finished, the reaction solution was directly concentrated, mixed with the sample, and purified by silica gel column to afford 166 (525 mg, pale yellow solid) with a yield of 90%.

$^1$H NMR (400 MHz, DMSO-$d_6$) δ 10.55 (s, 1H), 8.89 - 8.80 (m, 1H), 8.53 (s, 1H), 8.39 (d, *J* = 8.0 Hz, 1H), 8.23 (s, 1H), 7.86 (s, 1H), 7.78 - 7.63 (m, 2H), 7.50 - 7.35 (m, 2H), 7.25 (t, *J* = 9.7 Hz, 2H), 7.04 (d, *J* = 10.0 Hz, 1H), 5.22 (s, 1H), 3.91 (s, 3H), 3.70 (s, 3H), 1.50 (d, *J* = 7.1 Hz, 3H).

**Preparation of N-((S)-1-(3-fluorophenyl)ethyl)-5-(2-((R)-3-hydroxypyrrolidine-1-carboxamido)-[1,2,4]tria zolo[1,5-a]pyridin-7-yl)-1-methyl-1H-indole-3-carboxamide (162):**

100 mg of Intermediate 166 (100 mg, 0.2 mmol) was dissolved in pyridine, 50 mg of (R)-3-pyrrolidinol (52 mg, 0.6 mmol) was added, and the reaction was heated to 100°C for 16 h, a developing solvent dichloromethane/methanol (20:1) was used to monitor the reaction; after the reaction was finished, the reaction solution was concentrated and purified by reverse phase chromatography to afford 162 (86 mg, white solid) with a yield of 80%.

$^1$H NMR (400 MHz, DMSO-$d_6$) δ 9.23 (s, 1H), 8.80 (d, J = 7.1 Hz, 1H), 8.53 (s, 1H), 8.41 (d, J = 8.0 Hz, 1H), 8.24 (s, 1H), 7.82 (s, 1H), 7.72 (dd, J = 8.6, 1.9 Hz, 1H), 7.66 (d, J = 8.6 Hz, 1H), 7.45 - 7.33 (m, 2H), 7.29 - 7.20 (m, 2H), 7.05 (ddd, J = 10.3, 8.2, 2.7 Hz, 1H), 5.22 (q, J = 7.3 Hz, 1H), 4.98 (d, J = 3.4 Hz, 1H), 4.30 (s, 1H), 3.90 (s, 3H), 3.49 (s, 3H), 3.31 (s, 1H), 1.96 - 1.88 (m, 1H), 1.81 (s, 1H), 1.49 (d, J = 7.0 Hz, 3H).

Other compounds of the present invention may be synthesized in a similar manner to Examples 1-4 using the synthetic methods and structural characterizations shown in the following table:

Table 1 Synthesis method and structural characterization of compounds of the present invention

| **Compound No.** | **Structure** | **Structural characterization** | **ESI-MS** | **Synthetic raw material** | **Synthetic method** |
|---|---|---|---|---|---|
| 1 | 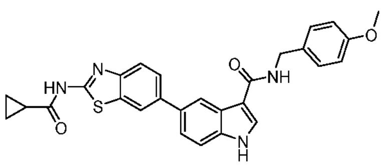 | $^1$H NMR (400 MHz, DMSO-$d_6$) δ 12.64 (s, 1H), 11.63 (s, 1H), 8.48 (s, 1H), 8.44 (s, 1H), 8.23 (s, 1H), 8.09 (d, *J* = 2.7 | 497.59 | Replacing i (S)-1-(3-fluoroph enyl) ethanamine with 4-methoxybenzy lamine | I |

(continued)

| Compound No. | Structure | Structural characterization | ESI-MS | Synthetic raw material | Synthetic method |
|---|---|---|---|---|---|
| | | Hz, 1H), 7.80 (d, *J* = 8.6 Hz, 1H), 7.73 (d, *J* = 9.6 Hz, 2H), 7.52 (s, 1H), 7.28 (d, *J* = 8.5 Hz, 2H), 6.89 (d, *J* = 8.5 Hz, 2H), 4.42 (s, 2H), 3.72 (s, 3H), 1.94 (m, 1H), 0.93 (d, *J* = 6.2 Hz, 4H). | | | |
| 2 | 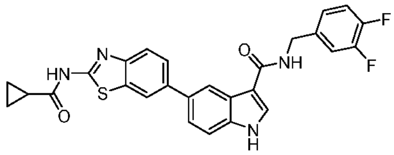 | $^1$H NMR (400 MHz, DMSO-$d_6$) δ 12.63 (s, 1H), 11.66 (s, 1H), 8.55 (t, *J* = 5.9 Hz, 1H), 8.46 (s, 1H), 8.23 (s, 1H), 8.10 (d, *J* = 2.6 Hz, 1H), 7.76 (dd, *J* = 28.5, 8.4 Hz, 2H), 7.53 (s, 2H), 7.39 (dd, *J* = 19.3, 8.4 Hz, 2H), 7.21 (s, 1H), 4.48 (d, *J* = 6.0 Hz, 2H), 2.06 - 1.93 (m, 1H), 0.96 (d, *J* = 5.0 Hz, 4H). | 503.40 | Replacing i (S)-1-(3-fluoroph enyl) ethanamine with 3,4-difluorobenz ylamine | I |
| 3 | 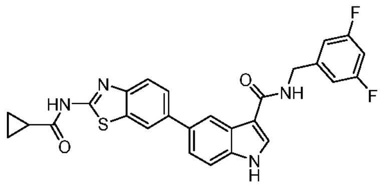 | $^1$H NMR (400 MHz, DMSO-$d_6$) δ 12.61 (s, 1H), 11.70 (s, 1H), 8.60 (t, *J* = 5.7 Hz, 1H), 8.46 (s, 1H), 8.23 (s, 1H), 8.13 (s, 1H), 7.95 (s, 1H), 7.76 (dd, *J* = 26.4, 8.4 Hz, 2H), 7.53 (s, 2H), 7.08 (dd, *J* = 16.0, 9.2 Hz, 2H), 4.51 (d, *J* = 5.8 Hz, 2H), 2.06 (m, 1H), 0.96 (d, *J* = 5.6 Hz, 4H). | 503.34 | Replacing i (S)-1-(3-fluoroph enyl) ethanamine with 3,5-difluorobenz ylamine | I |
| 4 | 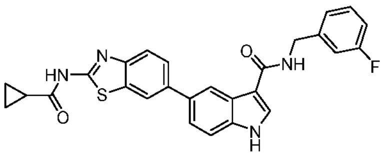 | $^1$H NMR (400 MHz, DMSO-$d_6$) δ 12.61 (s, 1H), 11.29 (s, 1H), 8.78 (s, 1H), 8.35 (t, | 485.53 | Replacing i (S)-1-(3-fluoroph enyl) ethanamine with | I |

(continued)

| Compound No. | Structure | Structural characterization | ESI-MS | Synthetic raw material | Synthetic method |
|---|---|---|---|---|---|
| | | *J* = 5.8 Hz, 1H), 7.95 (d, *J* = 2.7 Hz, 1H), 7.52 (d, *J* = 2.0 Hz, 2H), 7.36 (dd, *J* = 14.1, 7.8 Hz, 1H), 7.19 (dd, *J* = 12.4, 8.2 Hz, 1H), 7.12 (d, *J* = 10.2 Hz, 2H), 7.05 (t, *J* = 8.6 Hz, 1H), 6.64 (dd, *J* = 8.6, 2.3 Hz, 1H), 6.52 (s, 1H), 4.47 (d, *J* = 6.0 Hz, 2H), 1.96 (m, 1H), 0.98 (d, *J* = 5.4 Hz, 4H). | | 3-fluorobenzylamine | |
| 5 | 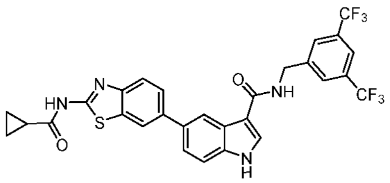 | $^1$H NMR (400 MHz, DMSO-$d_6$) δ 12.62 (s, 1H), 11.71 (s, 1H), 8.69 (s, 1H), 8.43 (s, 1H), 8.22 (s, 1H), 8.11 (d, *J* = 2.7 Hz, 1H), 8.05 (s, 2H), 8.00 (s, 1H), 7.79 (d, *J* = 8.6 Hz, 1H), 7.72 (d, *J* = 8.3 Hz, 1H), 7.54 (s, 2H), 4.67 (d, *J* = 5.7 Hz, 2H), 2.02 (d, *J* = 6.6 Hz, 1H), 0.96 (d, *J* = 4.7 Hz, 4H). | 603.43 | Replacing i(S)-1-(3-fluorop henyl) ethanamin e with 3,5-bis(trifluoro methyl)benzylamine | I |
| 6 | 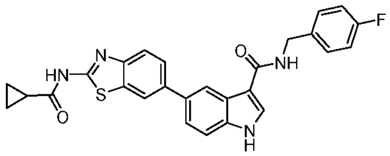 | $^1$H NMR (400 MHz, DMSO-$d_6$) δ 12.64 (s, 1H), 11.65 (s, 1H), 8.58 - 8.44 (m, 2H), 8.13 (d, *J* = 5.3 Hz, 2H), 7.83 - 7.67 (m, 1H), 7.53 (s, 1H), 7.45 - 7.36 (m, 1H), 7.34 - 7.26 (m, 2H), 7.15 (dd, *J* = 12.1, 5.2 Hz, 3H), 4.29 (d, *J* = 5.9 Hz, 2H), 2.10 (m, 1H), 0.97 (d, *J* = 5.8 Hz, 4H). | 485.41 | Replacing i(S)-1-(3-fluorop henyl) ethanamin e with 4-fluorobenzylamine | I |
| 7 | 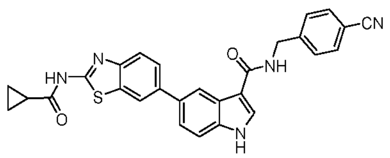 | $^1$H NMR (400 MHz, DMSO-$d_6$) δ 12.63 (s, 1H), 11.68 (s, 1H), 8.59 (s, 1H), 8.46 (s, 1H), 8.23 (s, 1H), 8.12 (d, *J* = 2.7 Hz, 1H), 7.79 (d, *J* = 8.5 Hz, 2H), 7.75 - 7.67 (m, 3H), 7.59 - 7.49 (m, 3H), 4.55 (d, *J* = 5.8 Hz, 2H), 2.08 (s, 1H), 0.96 (d, *J* = 5.0 Hz, 4H). | 492.40 | Replacing i(S)-1-(3-fluorop henyl) ethanamin e with 4-cyanobenzylamine | I |

(continued)

| Compound No. | Structure | Structural characterization | ESI-MS | Synthetic raw material | Synthetic method |
|---|---|---|---|---|---|
| 8 | 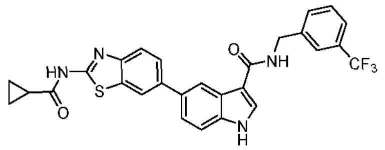 | $^1$H NMR (400 MHz, DMSO-$d_6$) δ 12.65 (s, 1H), 11.69 (s, 1H), 8.57 (d, *J* = 60.0 Hz, 2H), 8.20 (d, *J* = 42.9 Hz, 2H), 7.81 (d, *J* = 7.8 Hz, 1H), 7.77 - 7.66 (m, 3H), 7.64 - 7.47 (m, 4H), 4.61 (s, 2H), 2.04 (s, 1H), 0.96 (d, *J* = 5.1 Hz, 4H). | 535.52 | Replacing i(S)-1-(3-fluorop henyl) ethanamin e with 3-trifluoromethyl benzylamine | I |
| 9 | 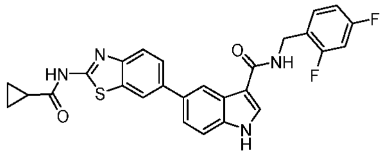 | $^1$H NMR (400 MHz, DMSO-$d_6$) δ 12.63 (s, 1H), 11.66 (s, 1H), 8.48 (d, *J* = 14.0 Hz, 2H), 8.23 (s, 1H), 8.12 (s, 1H), 7.79 (d, *J* = 8.5 Hz, 1H), 7.72 (d, *J* = 8.9 Hz, 1H), 7.52 (s, 2H), 7.47 (d, *J* = 7.4 Hz, 1H), 7.23 (d, *J* = 10.0 Hz, 2H), 4.50 (d, *J* = 5.3 Hz, 2H), 2.02 (m, 1H), 0.96 (d, *J* = 5.3 Hz, 4H). | 503.46 | Replacing i(S)-1-(3-fluorop henyl) ethanamin e with 2,4-difluorobenz ylamine | I |
| 10 | 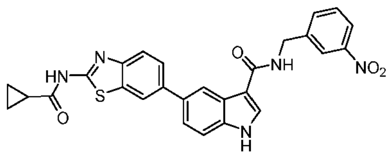 | $^1$H NMR (400 MHz, DMSO-$d_6$) δ 12.63 (s, 1H), 11.69 (s, 1H), 8.68 (d, *J* = 5.8 | 512.37 | Replacing i(S)-1-(3-fluorop henyl) ethanamin e with | I |
| | | Hz, 1H), 8.46 (s, 1H), 8.23 (s, 2H), 8.12 (d, *J* = 3.0 Hz, 2H), 7.81 (dd, *J* = 17.8, 8.1 Hz, 2H), 7.72 (dd, *J* = 8.5, 1.7 Hz, 1H), 7.65 (t, *J* = 7.9 Hz, 1H), 7.53 (s, 2H), 4.62 (d, *J* = 5.9 Hz, 2H), 2.06 - 1.97 (m, 1H), 0.96 (d, *J* = 4.9 Hz, 4H). | | 3-nitrobenzylami ne | |
| 11 | 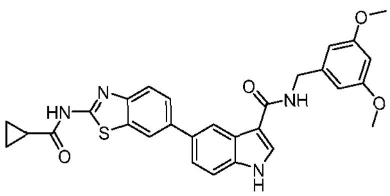 | $^1$H NMR (400 MHz, DMSO-$d_6$) δ 12.63 (s, 1H), 11.64 (s, 1H), 8.50 - 8.41 (m, 2H), 8.23 (s, 1H), 8.11 (d, *J* = 2.8 Hz, 1H), 7.79 (d, *J* = 8.4 Hz, 1H), 7.73 (d, *J* = 8.4 Hz, 1H), 7.52 (s, 2H), 6.52 (d, *J* = 2.1 Hz, 2H), 6.37 (s, 1H), 4.44 (d, *J* = 5.5 Hz, 2H), 3.72 (s, 6H), 2.04 - 1.98 (m, 1H), 0.96 (d, *J* = 5.1 Hz, 4H). | 527.46 | Replacing i(S)-1-(3-fluorop henyl) ethanamin e with 3,5-dimethoxybe nzylamine | I |

(continued)

| Compound No. | Structure | Structural characterization | ESI-MS | Synthetic raw material | Synthetic method |
|---|---|---|---|---|---|
| 12 | 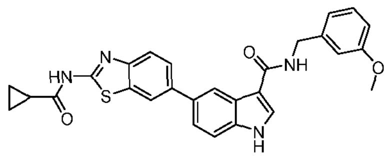 | $^1H$ NMR (400 MHz, DMSO-$d_6$) δ 12.64 (s, 1H), 11.66 (s, 1H), 8.48 (d, *J* = 6.7 Hz, 1H), 8.23 (s, 1H), 8.12 (s, 1H), 7.95 (s, 1H), 7.79 (d, *J* = 8.3 Hz, 1H), 7.72 (d, *J* = 9.6 Hz, 1H), 7.52 (s, 1H), 7.28 - 7.22 (m, 1H), 6.94 (d, *J* = 7.3 Hz, 2H), 6.81 (d, *J* = 8.6 Hz, 2H), 4.47 (d, *J* = 6.1 Hz, 2H), 3.73 (s, 3H), 2.02 (m, 1H), | 497.47 | Replacing i(S)-1-(3-fluorop henyl) ethanamin e with 3-methoxybenzy lamine | I |
| | | 0.96 (d, *J* = 5.2 Hz, 4H). | | | |
| 13 | 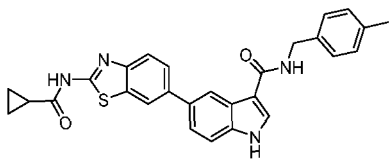 | $^1H$ NMR (400 MHz, DMSO-$d_6$) δ 12.63 (s, 1H), 11.62 (s, 1H), 8.46 (d, *J* = 9.8 Hz, 2H), 8.23 (s, 2H), 8.10 (s, 1H), 7.79 (d, *J* = 8.4 Hz, 1H), 7.72 (d, *J* = 9.4 Hz, 1H), 7.52 (s, 1H), 7.23 (t, *J* = 10.2 Hz, 2H), 7.13 (d, *J* = 7.6 Hz, 2H), 4.45 (d, *J* = 5.4 Hz, 2H), 2.27 (s, 3H), 2.02 (m, 1H), 0.96 (d, *J* = 5.3 Hz, 4H). | 481.57 | Replacing i(S)-1-(3-fluorop henyl) ethanamin e with 4-methylbenzyla mine | I |
| 14 | 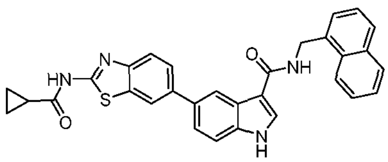 | $^1H$ NMR (400 MHz, DMSO-$d_6$) δ 12.64 (s, 1H), 11.65 (s, 1H), 8.51 (d, *J* = 11.2 Hz, 2H), 8.28 - 8.19 (m, 2H), 8.18 - 8.10 (m, 1H), 8.07 (d, *J* = 6.5 Hz, 1H), 7.96 (d, *J* = 7.7 Hz, 1H), 7.85 (d, *J* = 7.7 Hz, 1H), 7.80 (d, *J* = 8.3 Hz, 1H), 7.72 (d, *J* = 8.7 Hz, 1H), 7.61 - 7.42 (m, 5H), 4.98 (d, *J* = 5.5 Hz, 2H), 2.05 - 1.98 (m, 1H), 0.95 (t, *J* = 11.4 Hz, 4H). | 517.53 | Replacing i(S)-1-(3-fluorop henyl) ethanamin e with 1-naphthalenem ethylamine | I |
| 15 | 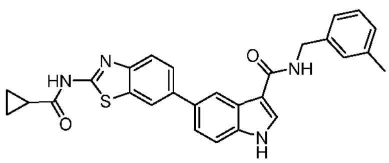 | $^1H$ NMR (400 MHz, DMSO-$d_6$) δ 12.63 (s, 1H), 11.63 (s, 1H), 8.47 (d, *J* = 5.7 Hz, 2H), 8.23 (s, 1H), 8.11 (d, *J* = 2.8 Hz, 1H), 7.79 (d, *J* = 8.4 Hz, 1H), 7.73 (d, *J* = 10.1 Hz, 1H), | 481.53 | Replacing i(S)-1-(3-fluorop henyl) ethanamin e with 3-methylbenzyla mine | I |

(continued)

| Compound No. | Structure | Structural characterization | ESI-MS | Synthetic raw material | Synthetic method |
|---|---|---|---|---|---|
| | | 7.24 - 7.18 (m, 2H), 7.15 (d, *J* = 11.8 Hz, 2H), 7.05 (d, *J* = 6.8 Hz, 2H), 4.47 (d, *J* = 6.0 Hz, 2H), 2.29 (s, 3H), 2.05 - 1.98 (m, 1H), 0.96 (d, *J* = 5.1 Hz, 4H). | | | |
| 16 | 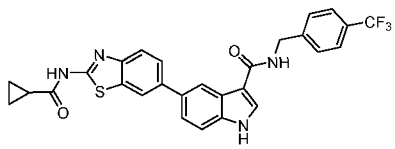 | $^1$H NMR (400 MHz, DMSO-$d_6$) δ 12.65 (s, 1H), 11.12 (s, 1H), 8.67 (t, *J* = 5.5 Hz, 1H), 8.61 (s, 1H), 8.48 (s, 1H), 8.23 (s, 1H), 8.18 - 8.09 (m, 1H), 7.79 (d, *J* = 8.4 Hz, 1H), 7.76 - 7.66 (m, 3H), 7.56 (dd, *J* = 15.0, 7.9 Hz, 2H), 7.44 (dd, *J* = 17.8, 8.2 Hz, 1H), 4.57 (d, *J* = 5.6 Hz, 2H), 2.07 - 1.95 (m, 1H), 0.97 (d, *J* = 5.8 Hz, 4H). | 535.43 | Replacing i(S)-1-(3-fluorop henyl) ethanamin e with p-(trifluoromethy l) benzylamine | I |
| 17 | 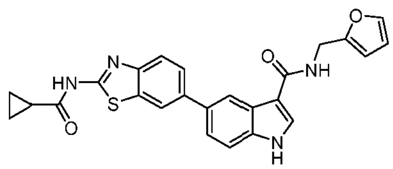 | $^1$H NMR (400 MHz, DMSO-$d_6$) δ 12.64 (s, 1H), 11.63 (s, 1H), 8.48 (s, 1H), 8.41 (t, *J* = 5.6 Hz, 1H), 8.23 (s, 1H), 8.10 (d, *J* = 2.7 Hz, 1H), 7.80 (d, *J* = 8.4 Hz, 1H), 7.73 (dd, *J* = 8.4, 1.5 Hz, 1H), 7.55 (d, *J* = 24.1 Hz, 3H), 6.40 (d, *J* = 1.9 Hz, 1H), 6.29 (d, *J* = 3.0 Hz, 1H), 4.49 (d, *J* = 5.6 Hz, 2H), 2.07 - 1.97 (m, 1H), 0.97 (d, *J* = 5.5 Hz, 4H). | 457.47 | Replacing i(S)-1-(3-fluorop henyl) ethanamin e with 2-furfurylamine | I |
| 18 | 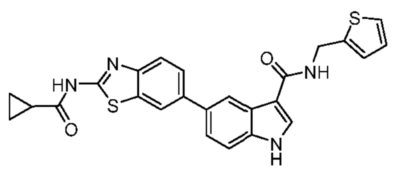 | $^1$H NMR (400 MHz, DMSO-$d_6$) δ 12.64 (s, 1H), 11.66 (s, 1H), 8.54 (d, *J* = 43.6 Hz, 2H), 8.17 (d, *J* = 60.4 Hz, 2H), 7.77 (dd, *J* = 26.9, 8.0 Hz, 2H), 7.61 - 7.31 (m, 3H), 7.00 (d, *J* = 28.4 Hz, 2H), 4.66 (d, *J* = 4.9 Hz, 2H), 2.06 - 1.86 (m, 1H), 0.97 (d, *J* = 5.2 Hz, 4H). | 473.46 | Replacing i (S)-1-(3-fluoroph enyl) ethanamine with 2-thienylmethylam ine | I |

(continued)

| Compound No. | Structure | Structural characterization | ESI-MS | Synthetic raw material | Synthetic method |
|---|---|---|---|---|---|
| 19 | 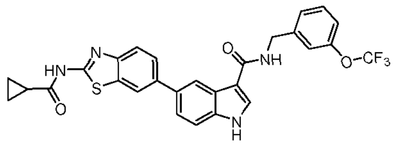 | $^1$H NMR (400 MHz, DMSO-$d_6$) δ 12.63 (s, 1H), 11.67 (s, 1H), 8.60 (s, 1H), 8.46 (s, 1H), 8.17 (d, *J* = 44.2 Hz, 2H), 7.76 (d, *J* = 26.5 Hz, 2H), 7.54 (t, *J* = 25.7 Hz, 3H), 7.45 - 7.15 (m, 3H), 4.55 (d, *J* = 5.7 Hz, 2H), 2.03 (m, 1H), 0.97 (d, *J* = 5.1 Hz, 4H). | 551.51 | Replacing i (S)-1-(3-fluoroph enyl) ethanamine with 3-trifluorometho xybenzylamine | I |
| 20 | 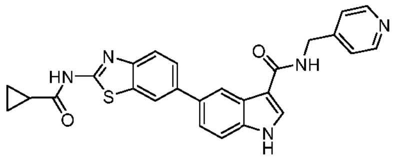 | $^1$H NMR (400 MHz, DMSO-$d_6$) δ 12.63 (s, 1H), 11.66 (s, 1H), 8.57 (d, *J* = 10.4 Hz, 2H), 8.46 (d, *J* = 4.2 Hz, 2H), 8.23 (s, 1H), 8.10 (d, *J* = 2.8 Hz, 1H), 7.76 (dt, *J* = 14.6, 8.5 Hz, 3H), 7.52 (s, 2H), 7.36 (dd, *J* = 7.8, 4.6 Hz, 1H), 4.52 (d, *J* = 5.7 Hz, 2H), 2.01 (s, 1H), 0.96 (d, *J* = 5.1 Hz, 4H). | 468.38 | Replacing i(S)-1-(3-fluorop henyl) ethanamin e with 4-methylaminopyri din | I |
| 21 | 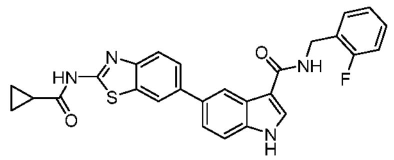 | $^1$H NMR (400 MHz, DMSO-$d_6$) δ 12.63 (s, 1H), 11.66 (s, 1H), 8.55 - 8.38 (m, 2H), 8.27 - 8.09 (m, 2H), 7.76 (dd, *J* = 27.1, 8.3 Hz, 2H), 7.53 (s, 2H), 7.44 (s, 1H), 7.31 (d, *J* = 7.3 Hz, 1H), 7.18 (dd, *J* = 12.6, 6.0 Hz, 2H), 4.55 (d, *J* = 5.5 Hz, 2H), 2.06 - 1.94 (m, 1H), 0.97 (d, *J* = 5.7 Hz, 4H). | 485.39 | Replacing i(S)-1-(3-fluorop henyl) ethanamin e with 2-fluorobenzyla mine | I |
| 22 | 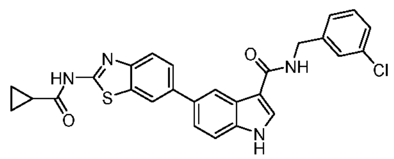 | $^1$H NMR (400 MHz, DMSO-$d_6$) δ 12.63 (s, 1H), 11.67 (s, 1H), 8.56 (s, 1H), 8.46 (s, 1H), 8.23 (s, 1H), 8.11 (d, *J* = 2.8 Hz, 1H), 7.83 - 7.69 (m, 2H), 7.53 (s, 2H), 7.43 - 7.28 (m, 4H), 4.50 (d, *J* = 5.8 Hz, 2H), 2.06 - 1.98 (m, 1H), 0.96 (d, *J* = 5.1 Hz, 4H). | 501.87 | Replacing i(S)-1-(3-fluorop henyl) ethanamin e with 3-chlorobenzylami ne | I |

(continued)

| Compound No. | Structure | Structural characterization | ESI-MS | Synthetic raw material | Synthetic method |
|---|---|---|---|---|---|
| 23 | 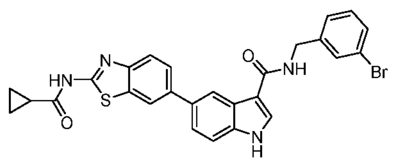 | $^1$H NMR (400 MHz, DMSO-$d_6$) δ 12.60 (s, 1H), 11.72 (s, 1H), 8.48 (d, *J* = 41.9 Hz, 2H), 8.33 - 7.96 (m, 3H), 7.85 - 7.57 (m, 2H), 7.37 (ddd, *J* = 58.5, 26.3, 7.2 Hz, 5H), 4.52 (d, *J* = 5.6 Hz, 2H), 1.96 (m, 1H), 0.91 (d, *J* = 5.2 Hz, 4H). | 546.41 | Replacing i(S)-1-(3-fluorop henyl) ethanamin e with 3-bromobenzyla mine | I |
| 24 | 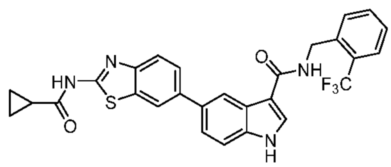 | $^1$H NMR (400 MHz, DMSO-$d_6$) δ 12.63 (s, 1H), 11.71 (s, 1H), 8.60 (s, 1H), | 535.54 | Replacing i(S)-1-(3-fluorop henyl) ethanamin e with | I |
| | | 8.47 (s, 1H), 8.21 (d, *J* = 20.0 Hz, 2H), 7.84 - 7.69 (m, 2H), 7.64 (d, *J* = 15.4 Hz, 2H), 7.58 (s, 2H), 7.51 (d, *J* = 29.4 Hz, 2H), 4.70 (d, *J* = 5.1 Hz, 2H), 2.01 (m, 1H), 0.96 (d, *J* = 5.6 Hz, 4H). | | 2-trifluoromethyl benzylamine | |
| 25 | 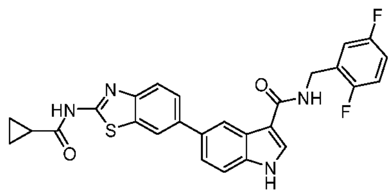 | $^1$H NMR (400 MHz, DMSO-$d_6$) δ 12.63 (s, 1H), 11.68 (s, 1H), 8.53 (s, 1H), 8.46 (s, 1H), 8.23 (s, 1H), 7.79 (d, *J* = 8.5 Hz, 2H), 7.72 (d, *J* = 8.1 Hz, 2H), 7.53 (s, 2H), 7.34 - 7.19 (m, 2H), 4.52 (d, *J* = 5.0 Hz, 2H), 2.02 (m, 1H), 0.96 (d, *J* = 5.6 Hz, 4H). | 503.41 | Replacing i(S)-1-(3-fluorop henyl) ethanamin e with 2,5-difluorobenz ylamine | I |
| 26 | 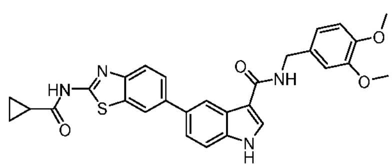 | $^1$H NMR (400 MHz, DMSO-$d_6$) δ 12.63 (s, 1H), 11.68 (s, 1H), 8.62 (s, 1H), 8.43 (s, 2H), 8.23 (s, 1H), 8.09 (d, *J* = 17.1 Hz, 2H), 7.76 (dd, *J* = 27.2, 8.3 Hz, 1H), 7.52 (s, 2H), 7.43 (dd, *J* = 20.3, 8.1 Hz, 2H), 4.42 (d, *J* = 5.9 Hz, 2H), 3.73 (s, 6H), 2.10 - 1.96 (m, 1H), 0.96 (d, *J* = 5.8 Hz, 4H). | 527.55 | Replacing i(S)-1-(3-fluorop henyl) ethanamin e with 3,4-dimethoxybe nzylamine | I |

(continued)

| Compound No. | Structure | Structural characterization | ESI-MS | Synthetic raw material | Synthetic method |
|---|---|---|---|---|---|
| 27 | 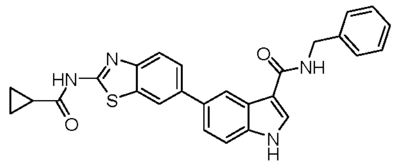 | $^1$H NMR (400 MHz, DMSO-$d_6$) δ 12.66 (s, 1H), 11.71 (s, 1H), 8.56 (s, 1H), 8.49 (s, 1H), 8.23 (s, 1H), 8.15 (d, *J* = 2.1 | 467.42 | Replacing i(S)-1-(3-fluorop henyl) ethanamin e with benzylamine | I |
| | | Hz, 1H), 7.76 (dd, *J* = 26.1, 8.4 Hz, 2H), 7.53 (s, 2H), 7.35 (dt, *J* = 14.7, 7.4 Hz, 3H), 7.30 - 7.14 (m, 2H), 4.51 (d, *J* = 5.8 Hz, 2H), 2.08 - 1.98 (m, 1H), 0.97 (d, *J* = 5.9 Hz, 4H). | | | |
| 28 | 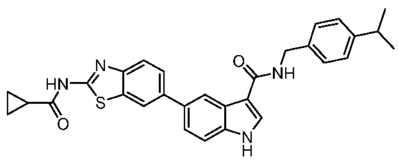 | $^1$H NMR (400 MHz, DMSO-$d_6$) δ 12.61 (s, 1H), 11.67 (s, 1H), 8.56 - 8.43 (m, 2H), 8.23 (d, *J* = 1.5 Hz, 1H), 8.12 (s, 1H), 7.85 - 7.69 (m, 2H), 7.52 (s, 2H), 7.24 (dd, *J* = 34.4, 8.1 Hz, 4H), 4.46 (d, *J* = 5.8 Hz, 2H), 2.02 (s, 1H), 1.23 (s, 1H), 1.18 (d, *J* = 6.9 Hz, 6H), 0.96 (d, *J* = 6.0 Hz, 4H). | 509.18 | Replacing i(S)-1-(3-fluorop henyl) ethanamin e with 4-isopropylbenz ylamine | I |
| 29 | 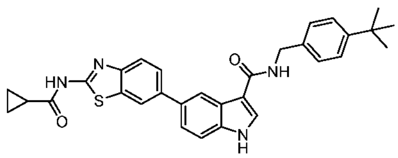 | $^1$H NMR (400 MHz, DMSO-$d_6$) δ 12.64 (s, 1H), 11.66 (s, 1H), 8.54 - 8.39 (m, 2H), 8.23 (d, *J* = 1.3 Hz, 1H), 8.12 (s, 1H), 7.80 (d, *J* = 8.4 Hz, 1H), 7.73 (dd, *J* = 8.5, 1.5 Hz, 1H), 7.52 (s, 2H), 7.32 (dd, *J* = 23.7, 8.3 Hz, 4H), 4.46 (d, *J* = 5.7 Hz, 2H), 2.08 - 1.98 (m, 1H), 1.26 (s, 9H), 0.97 (d, *J* = 5.9 Hz, 4H). | 523.23 | Replacing i(S)-1-(3-fluorop henyl) ethanamin e with 4-tert-butylbenz ylamine | I |
| 30 | 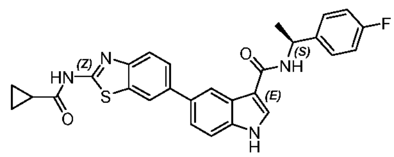 | $^1$H NMR (400 MHz, DMSO-$d_6$) δ 12.67 (s, 1H), 11.75 (s, 1H), 8.44 (s, 1H), | 499.13 | Replacing i(S)-1-(3-fluorop henyl) ethanamin e with | I |

(continued)

| Compound No. | Structure | Structural characterization | ESI-MS | Synthetic raw material | Synthetic method |
|---|---|---|---|---|---|
| | | 8.34 (t, *J* = 6.3 Hz, 1H), 8.25 (d, *J* = 6.6 Hz, 1H), 8.21 (d, *J* = 1.5 Hz, 1H), 7.79 (d, *J* = 8.4 Hz, 1H), 7.71 (dd, *J* = 8.5, 1.7 Hz, 1H), 7.52 (s, 2H), 7.47 (dt, *J* = 8.5, 4.4 Hz, 2H), 7.15 (t, *J* = 8.9 Hz, 2H), 5.21 (p, *J* = 7.0 Hz, 1H), 2.09 - 1.99 (m, 1H), 1.49 (d, *J* = 7.0 Hz, 3H), 0.97 (d, *J* = 5.9 Hz, 4H). | | (S)-(-)-1-(4-fluorophenyl)ethanamine | |
| 31 | 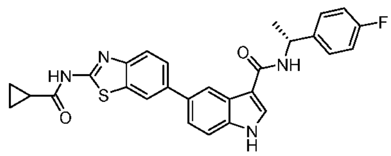 | $^1$H NMR (400 MHz, DMSO-$d_6$) δ 12.65 (s, 1H), 11.73 (s, 1H), 8.43 (s, 1H), 8.32 (t, *J* = 6.3 Hz, 1H), 8.23 (d, *J* = 6.5 Hz, 1H), 8.21 (d, *J* = 1.4 Hz, 1H), 7.79 (d, *J* = 8.2 Hz, 1H), 7.70 (d, *J* = 8.5, Hz, 1H), 7.55 (s, 2H), 7.44 (dt, *J* = 8.2, 4.4 Hz, 2H), 7.11 (t, *J* = 8.7 Hz, 2H), 5.21 (p, *J* = 7.0 Hz, 1H), 2.09 - 1.99 (m, 1H), 1.48 (d, *J* = 7.0 Hz, 3H), 0.96 (d, *J* = 5.7 Hz, 4H). | 499.14 | Replacing i(S)-1-(3-fluorop henyl) ethanamin e with (R)-(-)-1-(4-fluorophenyl)ethanamine | I |
| 32 | 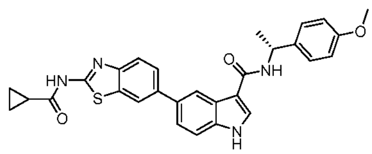 | $^1$H NMR (400 MHz, DMSO-$d_6$) δ 12.63 (s, 1H), 11.62 (s, 1H), 8.44 (s, 2H), 8.19 (d, *J* = 9.7 Hz, 2H), 7.78 (d, *J* = 8.5 Hz, 1H), 7.70 (d, *J* = 8.5 Hz, 1H), 7.50 (s, 2H), 7.34 (d, *J* = 8.6 Hz, 2H), 6.89 (d, *J* = | 511.33 | Replacing i(S)-1-(3-fluorop henyl) ethanamin e with (R)-(-)-1-(4-methoxyphenyl)ethyl amine | I |
| | | 8.7 Hz, 2H), 5.22 - 5.09 (m, 1H), 3.72 (s, 3H), 2.05 - 1.99 (m, 1H), 1.47 (d, *J* = 7.0 Hz, 3H), 0.96 (d, *J* = 5.0 Hz, 4H). | | | |

(continued)

| Compound No. | Structure | Structural characterization | ESI-MS | Synthetic raw material | Synthetic method |
|---|---|---|---|---|---|
| 33 | 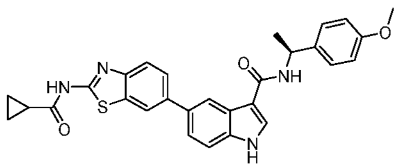 | $^1$H NMR (400 MHz, DMSO-$d_6$) δ 12.63 (s, 1H), 11.62 (s, 1H), 8.44 (s, 2H), 8.19 (d, *J* = 11.2 Hz, 2H), 7.78 (d, *J* = 8.3 Hz, 1H), 7.70 (d, *J* = 8.1 Hz, 1H), 7.50 (s, 2H), 7.34 (d, *J* = 8.6 Hz, 2H), 6.89 (d, *J* = 8.6 Hz, 2H), 5.21 - 5.10 (m, 1H), 3.72 (s, 3H), 2.00 (d, *J* = 11.0 Hz, 1H), 1.47 (d, *J* = 6.9 Hz, 3H), 0.96 (d, *J* = 5.1 Hz, 4H). | 511.26 | Replacing i(S)-1-(3-fluorop henyl) ethanamin e with (S)-(-)-1-(4-meth oxyphenyl)ethyl amine | I |
| 34 | 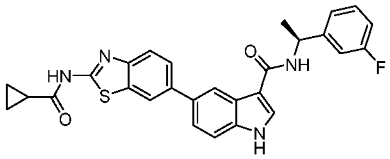 | $^1$H NMR (400 MHz, DMSO-$d_6$) δ 12.63 (s, 1H), 11.66 (s, 1H), 8.43 (s, 1H), 8.31 (d, *J* = 7.9 Hz, 1H), 8.21 (d, *J* = 2.4 Hz, 2H), 7.81 - 7.75 (m, 1H), 7.70 (dd, *J* = 8.5, 1.6 Hz, 1H), 7.53 (d, *J* = 8.1 Hz, 2H), 7.37 (dd, *J* = 14.1, 8.1 Hz, 1H), 7.25 (t, *J* = 9.4 Hz, 2H), 7.09 - 7.00 (m, 1H), 5.27 - 5.15 (m, 1H), 2.06 (d, *J* = 11.0 Hz, 1H), 1.50 (d, *J* = 7.0 Hz, 3H), 0.96 (d, *J* = 5.1 Hz, 4H). | 499.39 | | I |
| 35 | 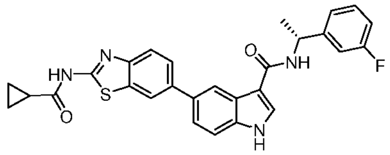 | $^1$H NMR (400 MHz, DMSO-$d_6$) δ 12.64 (s, 1H), 11.68 (s, 1H), 8.45 (s, 1H), 8.33 (d, *J* = 8.1 Hz, 1H), 8.22 (s, 2H), 7.79 (d, *J* = 8.4 Hz, 1H), 7.72 (d, *J* = 7.4 Hz, 1H), 7.53 (s, 2H), 7.38 (dd, *J* = 14.1, 7.8 Hz, 1H), 7.26 (t, *J* = 8.6 Hz, 2H), 7.05 (t, *J* = 7.3 Hz, 1H), 5.26 - 5.13 (m, 1H), 2.02 (dd, *J* = 12.9, 7.0 Hz, 1H), 1.50 (d, *J* = 7.0 Hz, 3H), 0.97 (d, *J* = 5.8 Hz, 4H). | 499.48 | Replacing i(S)-1-(3-fluorop henyl) ethanamin e with (R)-(-)-1-(3-fluor ophenyl)ethana mine | I |

(continued)

| Compound No. | Structure | Structural characterization | ESI-MS | Synthetic raw material | Synthetic method |
|---|---|---|---|---|---|
| 36 | 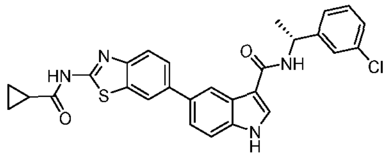 | $^1$H NMR (400 MHz, DMSO-$d_6$) δ 12.63 (s, 1H), 11.68 (s, 1H), 8.42 (s, 1H), 8.33 (d, *J* = 7.7 Hz, 1H), 8.21 (s, 2H), 7.74 (dd, *J* = 30.9, 8.4 Hz, 2H), 7.49 (d, *J* = 17.6 Hz, 3H), 7.41 - 7.20 (m, 3H), 5.23 - 5.11 (m, 1H), 2.00 (d, *J* = 11.6 Hz, 1H), 1.49 (d, *J* = 6.9 Hz, 3H), 0.96 (d, *J* = 4.9 Hz, 4H). | 515.89 | Replacing i(S)-1-(3-fluorop henyl) ethanamin e with (R)-(-)-1-(3-chlor ophenyl)ethylam ine | I |
| 37 | 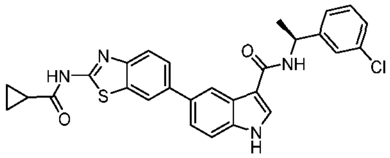 | $^1$H NMR (400 MHz, DMSO-$d_6$) δ 12.63 (s, 1H), 11.67 (d, *J* = 2.1 Hz, 1H), 8.43 (s, 1H), 8.33 (d, *J* = 7.9 Hz, 1H), 8.21 (s, 2H), 7.78 (d, *J* = 8.4 Hz, 1H), 7.71 (dd, *J* = 8.5, 1.7 Hz, 1H), 7.50 (d, *J* = 18.6 Hz, | 515.90 | Replacing i(S)-1-(3-fluorop henyl) ethanamin e with (S)-(-)-1-(3-chlor ophenyl)ethylam ine | I |
| | | 3H), 7.42 - 7.33 (m, 3H), 5.26 - 5.11 (m, 1H), 2.01 (d, *J* = 6.1 Hz, 1H), 1.47 (t, *J* = 11.4 Hz, 3H), 0.97 (d, *J* = 5.9 Hz, 4H). | | | |
| 38 | 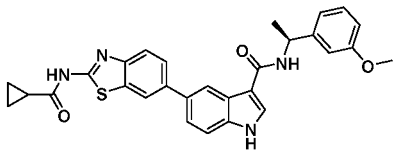 | $^1$H NMR (400 MHz, DMSO-$d_6$) δ 12.64 (s, 1H), 11.65 (d, *J* = 1.9 Hz, 1H), 8.46 (s, 1H), 8.30 - 8.16 (m,2H), 7.81 - 7.68 (m, 2H), 7.52 (s, 2H), 7.24 (td, *J* = 8.1, 3.1 Hz, 1H), 7.00 (d, *J* = 6.0 Hz, 2H), 6.93 - 6.74 (m, 2H), 5.24 - 5.10 (m, 1H), 3.75 (s, 3H), 2.03 (s, 1H), 1.49 (d, *J* = 7.0 Hz, 3H), 0.97 (d, *J* = 6.0 Hz, 4H). | 511.44 | Replacing i(S)-1-(3-fluorop henyl) ethanamin e with (S)-(-)-1-(3-meth oxyphenyl)ethyl amine | I |

(continued)

| Compound No. | Structure | Structural characterization | ESI-MS | Synthetic raw material | Synthetic method |
|---|---|---|---|---|---|
| 39 | 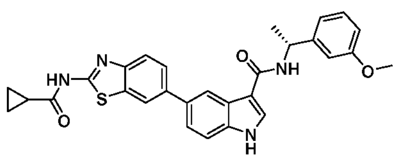 | $^1H$ NMR (400 MHz, DMSO-$d_6$) δ 12.64 (s, 1H), 11.65 (s, 1H), 8.46 (s, 1H), 8.34 - 8.11 (m, 3H), 7.75 (dd, *J* = 29.8, 8.4 Hz, 2H), 7.52 (s, 2H), 7.25 (t, *J* = 8.0 Hz, 1H), 7.00 (s, 2H), 6.80 (d, *J* = 7.7 Hz, 1H), 5.24 - 5.11 (m, 1H), 3.75 (s, 3H), 2.09 - 1.96 (m, 1H), 1.49 (d, *J* = 6.9 Hz, 3H), 0.97 (d, *J* = 5.9 Hz, 4H). | 511.42 | Replacing i(S)-1-(3-fluorop henyl) ethanamin e with (R)-(-)-1-(3-meth oxyphenyl)ethyl amine | I |
| 40 | 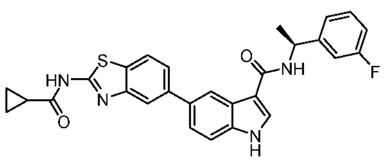 | $^1H$ NMR (400 MHz, DMSO-$d_6$) δ 12.63 (s, 1H), 11.68 (s, 1H), 8.45 (s, 1H), 8.40 (d, *J* = 8.1 Hz, 1H), 8.23 (s, 2H), | 499.47 | | I |
| | | 7.80 (d, *J* = 8.4 Hz, 1H), 7.72 (d, *J* = 7.4 Hz, 1H), 7.52 (s, 2H), 7.40 (dd, *J* = 14.1, 7.8 Hz, 1H), 7.26 (t, *J* = 8.6 Hz, 2H), 7.05 (t, *J* = 7.3 Hz, 1H), 5.26 - 5.13 (m, 1H), 2.02 (d, *J* = 7.0 Hz, 1H), 1.50 (t, *J* = 7.0 Hz, 3H), 0.97 (d, *J* = 5.9 Hz, 4H). | | | |
| 41 | 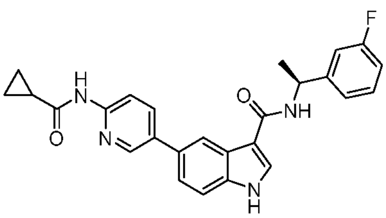 | $^1H$ NMR (400 MHz, DMSO-$d_6$) δ 11.69 (s, 1H), 10.84 (s, 1H), 8.58 (d, *J* = 2.1 Hz, 1H), 8.37 (s, 1H), 8.31 (d, *J* = 8.0 Hz, 1H), 8.22 (d, *J* = 2.8 Hz, 1H), 8.14 (d, *J* = 8.6 Hz, 1H), 8.02 (dd, *J* = 8.7, 2.5 Hz, 1H), 7.49 (dt, *J* = 8.6, 5.1 Hz, 2H), 7.41 - 7.33 (m, 1H), 7.24 (t, *J* = 9.5 Hz, 2H), 7.04 (s, 1H), 5.26 - 5.16 (m, 1H), 2.02 (d, *J* = 4.7 Hz, 1H), 1.49 (t, *J* = 7.1 Hz, 3H), 0.82 (d, *J* = 6.4 Hz, 4H). | 443.30 | Replacing ii Intermediate 3 with 2-amino-5-brom opyridine | I |

(continued)

| Compound No. | Structure | Structural characterization | ESI-MS | Synthetic raw material | Synthetic method |
|---|---|---|---|---|---|
| 42 | 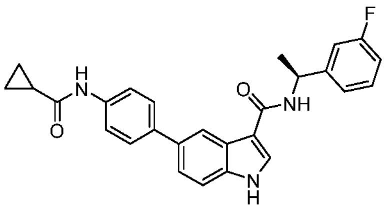 | $^1$H NMR (400 MHz, DMSO-$d_6$) δ 11.56 (s, 1H), 10.16 (s, 1H), 8.23 (dd, *J* = 36.0, 29.0 Hz, 3H), 7.70 - 7.08 (m, 9H), 6.96 (s, 1H), 5.15 (m, 1H), 1.74 (m, 1H), 1.42 (d, *J* = 5.8 Hz, 3H), 0.75 (d, *J* = 6.5 Hz, 4H). | 442.35 | Replacing ii Intermediate 3 with 4-bromoaniline | I |
| 43 | 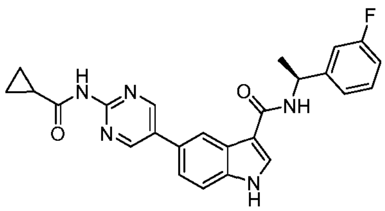 | $^1$H NMR (400 MHz, DMSO-$d_6$) δ 11.74 (s, 1H), 10.90 (s, 1H), 8.91 (s, 2H), 8.38 (s, 1H), 8.32 (d, *J* = 7.6 Hz, 1H), 8.24 (d, *J* = 2.9 Hz, 1H), 7.54 (dd, *J* = 22.4, 8.5 Hz, 2H), 7.37 (dd, *J* = 13.6, 7.5 Hz, 2H), 7.24 (t, *J* = 9.9 Hz, 1H), 7.05 (d, *J* = 9.1 Hz, 1H), 5.25 - 5.17 (m, 1H), 2.16 (m, 1H), 1.49 (d, *J* = 7.0 Hz, 3H), 0.82 (d, *J* = 8.1 Hz, 4H). | 444.38 | Replacing ii Intermediate 3 with 2-amino-5-bromopyrimidine | I |
| 44 | 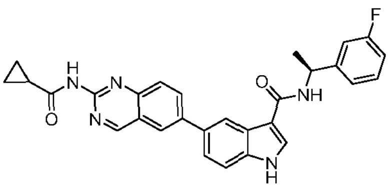 | $^1$H NMR (400 MHz, DMSO-$d_6$) δ 11.78 (s, 1H), 10.99 (s, 1H), 9.56 (s, 1H), 8.56 (s, 1H), 8.39 (d, *J* = 8.0 Hz, 1H), 8.28 (dd, *J* = 11.4, 8.8 Hz, 3H), 7.86 (d, *J* = 8.8 Hz, 1H), 7.64 - 7.55 (m, 2H), 7.38 (dd, *J* = 14.2, 7.9 Hz, 1H), 7.26 (t, *J* = 8.4 Hz, 2H), 7.05 (t, *J* = 8.6 Hz, 1H), 5.27 - 5.17 (m, 1H), 2.27 (s, 1H), 1.50 (d, *J* = 7.0 Hz, 3H), 0.86 (d, *J* = 7.9 Hz, 4H). | 494.45 | Replacing ii Intermediate 3 with 6-bromo-2-quinazolinamine | I |
| 45 | 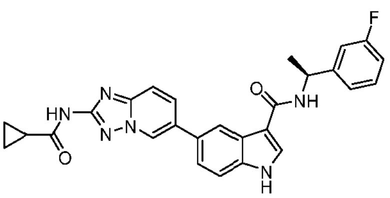 | $^1$H NMR (400 MHz, DMSO-$d_6$) δ 11.76 (s, 1H), 10.89 (s, 1H), 8.65 (d, *J* = 2.0 Hz, 1H), 8.29 (s, 1H), 8.24 (d, *J* = 8.0 Hz, 1H), 8.16 (d, *J* = 2.3 Hz, 1H), 8.08 (d, *J* = 8.2 Hz, 1H), 7.99 | 483.22 | Replacing ii Intermediate 3 with 6-bromo-[1,2,4]triazolo[1,5-a]pyridin- 2-amine | I |

(continued)

| Compound No. | Structure | Structural characterization | ESI-MS | Synthetic raw material | Synthetic method |
|---|---|---|---|---|---|
| | | (dd, *J* = 8.7, 2.5 Hz, 1H), 7.41 (dt, *J* = 8.3, 4.2 Hz, 2H), 7.38 - 7.25 (m, 1H), 7.18 (t, *J* = 9.8 Hz, 2H), 7.00 (s, 1H), 5.21 - 5.10 (m, 1H), 2.01 (d, *J* = 4.5 Hz, 1H), 1.48 (t, *J* = 6.8 Hz, 3H), 0.96 (d, *J* = 6.5 Hz, 4H). | | | |
| 46 | 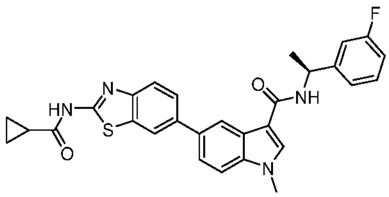 | $^1$H NMR (400 MHz, DMSO-$d_6$) δ 12.63 (s, 1H), 8.43 (s, 1H), 8.32 (d, *J* = 8.0 Hz, 1H), 8.23 (d, *J* = 1.3 Hz, 1H), 8.18 (s, 1H), 7.79 (d, *J* = 8.4 Hz, 1H), 7.72 (dd, *J* = 8.5, 1.6 Hz, 1H), 7.59 (s, 2H), 7.37 (dd, *J* = 14.2, 8.0 Hz, 1H), 7.24 (t, *J* = 8.5 Hz, 2H), 7.08 - 7.00 (m, 1H), 5.25 - 5.15 (m, 1H), 3.88 (s, 3H), 2.09 - 1.97 (m, 1H), 1.48 (d, *J* = 7.1 Hz, 3H), 0.96 (d, *J* = 5.5 Hz, 4H). | 513.45 | Replacing v Intermediate 6 with Intermediate 3 | II |
| 47 | 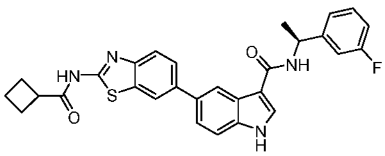 | $^1$H NMR (400 MHz, DMSO-$d_6$) δ 11.80 (s, 1H), 8.45 (s, 1H), 8.39 (d, *J* = 7.9 Hz, 1H), 8.30 - 8.19 (m, 2H), 7.80 - 7.67 (m, 2H), 7.53 (s, 2H), 7.37 (dd, *J* = 8.0, 6.1 Hz, 1H), 7.30 - 7.21 (m, 2H), 7.04 (dd, *J* = 8.4, 6.4 Hz, 1H), 5.27 - 5.16 (m, 1H), 2.34 - 2.23 (m, 2H), 2.18 (dt, *J* = 12.0, 6.6 Hz, 2H), 1.98 (d, | 513.40 | Replacing ii cyclopropylform yl chloride with cyclobutylformyl chloride | I |
| | | *J* = 9.0 Hz, 2H), 1.91 - 1.82 (m, 1H), 1.80 (s, 1H), 1.50 (d, *J* = 7.1 Hz, 3H). | | | |

(continued)

| Compound No. | Structure | Structural characterization | ESI-MS | Synthetic raw material | Synthetic method |
|---|---|---|---|---|---|
| 48 | 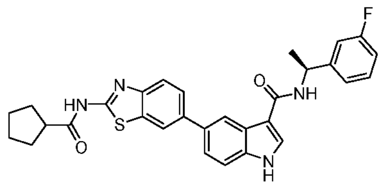 | $^1H$ NMR (400 MHz, DMSO-$d_6$) δ 11.81 (s, 1H), 8.47 (s, 1H), 8.40 (d, *J* = 8.0 Hz, 1H), 8.31 - 8.21 (m, 2H), 7.78 (d, *J* = 8.4 Hz, 1H), 7.72 (dd, *J* = 8.5, 1.7 Hz, 2H), 7.58 - 7.48 (m, 2H), 7.43 - 7.33 (m, 1H), 7.30 - 7.22 (m, 2H), 7.05 (td, *J* = 8.2, 2.2 Hz, 1H), 5.29 - 5.17 (m, 1H), 3.06 - 2.95 (m, 1H), 2.00 - 1.86 (m, 2H), 1.85 - 1.66 (m, 4H), 1.64 - 1.55 (m, 2H), 1.50 (d, *J* = 12.3, Hz, 3H). | 527.31 | Replacing ii cyclopropylform yl chloride with cyclopentylform yl chloride | I |
| 49 | 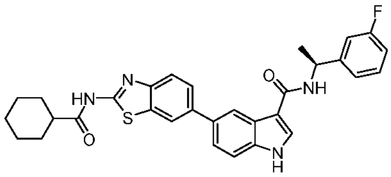 | $^1H$ NMR (400 MHz, DMSO-$d_6$) δ 11.79 (s, 1H), 8.46 (s, 1H), 8.40 (d, *J* = 8.0 Hz, 1H), 8.30 - 8.20 (m, 2H), 7.78 (d, *J* = 8.4 Hz, 1H), 7.71 (dd, *J* = 8.5, 1.7 Hz, 1H), 7.57 - 7.49 (m, 2H), 7.41 - 7.33 (m, 1H), 7.26 (dd, *J* = 10.8, 4.3 Hz, 2H), 7.05 (td, *J* = 8.3, 2.2 Hz, 1H), 5.30 - 5.17 (m, 1H), 2.62 - 2.53 (m, 1H), 1.86 (t, *J* = 11.4 Hz, 2H), 1.77 (d, *J*= 12.1 Hz, 2H), 1.56 - 1.38 (m, 6H), 1.24 (tt, *J* = 23.7, 11.8 Hz, 4H). | 541.14 | Replacing ii cyclopropylform yl chloride with cyclohexylformyl chloride | I |
| 50 | 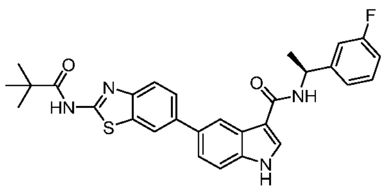 | $^1H$ NMR (400 MHz, DMSO-$d_6$) δ 11.89 (s, 1H), 8.55 - 8.42 (m, 2H), 8.30 (d, *J* = 33.0 Hz, 2H), 7.77 (dt, *J* = 9.8, 4.9 Hz, 2H), 7.57 (q, *J* = 8.5 Hz, 2H), 7.39 (dd, *J* = 14.0, 7.8 Hz, 2H), 7.30 (d, *J* = 8.6 Hz, 2H), 7.06 (t, *J* = 8.0 Hz, 1H), 5.32 - 5.21 (m, 1H), 1.53 (d, *J* = 7.0 Hz, 3H), 1.32 (s, 9H). | 515.51 | Replacing ii cyclopropylform yl chloride with tert-butylformyl chloride | I |

(continued)

| Compound No. | Structure | Structural characterization | ESI-MS | Synthetic raw material | Synthetic method |
|---|---|---|---|---|---|
| 51 | 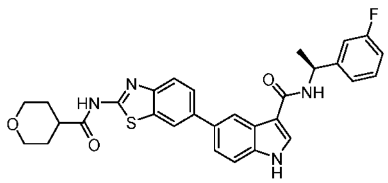 | $^{1}$H NMR (400 MHz, DMSO-$d_6$) δ 12.37 (s, 1H), 11.69 (s, 1H), 8.48 (s, 1H), 8.35 (d, *J* = 7.6 Hz, 1H), 8.25 (s, 2H), 7.76 (dd, *J* = 28.2, 8.2 Hz, 2H), 7.54 (s, 2H), 7.43 - 7.30 (m, 1H), 7.31 - 7.17 (m, 2H), 7.04 (t, *J* = 7.3 Hz, 1H), 5.34 - 5.13 (m, 1H), 3.92 (d, *J* = 9.4 Hz, 2H), 3.35 (d, *J* = 10.6 Hz, 2H), 2.83 (s, 1H), 1.90 - 1.62 (m, 4H), 1.50 (d, *J* = 6.5 Hz, 3H). | 543.28 | Replacing ii cyclopropylform yl chloride with tetrahydropyran-4-formyl chloride | I |
| 52 | 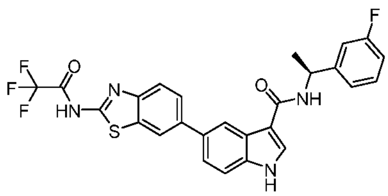 | $^{1}$H NMR (400 MHz, DMSO-$d_6$) δ 12.48 (s, 1H), 11.79 (s, 1H), 8.67 (s, 1H), 8.47 (d, *J* = 7.4 Hz, 1H), 8.35 (s, 2H), 7.88 (d, *J* = 8.2 Hz, 2H), 7.66 (s, 2H), 7.56 - 7.46 (m, 1H), 7.37 - 7.27 (m, 2H), 7.11 (t, *J* = 7.3 Hz, | 527.43 | Replacing ii cyclopropylform yl chloride with trifluoroacetyl chloride | I |
| | | 1H), 5.30 - 5.12 (m, 1H), 1.50 (d, *J* = 6.5 Hz, 3H). | | | |
| 53 | 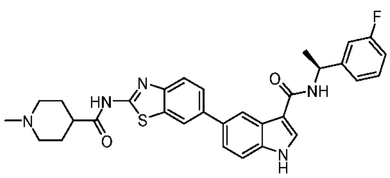 | $^{1}$H NMR (400 MHz, DMSO-$d_6$) δ 12.17 (s, 1H), 11.39 (s, 1H), 8.34 (s, 1H), 8.23 (d, *J* = 7.5 Hz, 1H), 8.23 (s, 2H), 7.68 (dd, *J* = 28.1, 8.1 Hz, 2H), 7.43 (s, 2H), 7.37 - 7.29 (m, 1H), 7.25 - 7.14 (m, 2H), 7.00 (t, *J* = 7.1 Hz, 1H), 5.31 - 5.11 (m, 1H), 3.92 (d, *J* = 9.4 Hz, 2H), 3.88 (s, 3H), 3.37 (d, *J* = 10.5 Hz, 2H), 2.80 (s, 1H), 1.89 - 1.65 (m, 4H), 1.49 (d, *J* = 6.3 Hz, 3H). | 556.51 | Replacing ii cyclopropylform yl chloride with N-methyl-4-pipe ridineformyl chloride | I |

(continued)

| Compound No. | Structure | Structural characterization | ESI-MS | Synthetic raw material | Synthetic method |
|---|---|---|---|---|---|
| 54 | 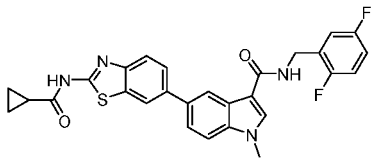 | $^1$H NMR (400 MHz, DMSO-$d_6$) δ 12.65 (s, 1H), 8.49 (d, $J$ = 14.1 Hz, 2H), 8.33 (s, 1H), 8.09 (s, 1H), 7.80 (d, $J$ = 8.4 Hz, 1H), 7.80 (d, $J$ = 8.8 Hz, 1H), 7.65 (s, 2H), 7.50 (d, $J$ = 7.2 Hz, 1H), 7.21 (d, $J$ = 10.1 Hz, 2H), 4.51 (d, $J$ = 5.3 Hz, 2H), 3.88 (s, 3H), 2.02 (m, 1H), 0.97 (d, $J$ = 5.6 Hz, 4H). | 517.35 | Replacing v Intermediate 6 with Intermediate 3, Replacing i(S)-1-(3-fluorop henyl) ethanamin e with 2,5-difluroaniline | II |
| 55 | 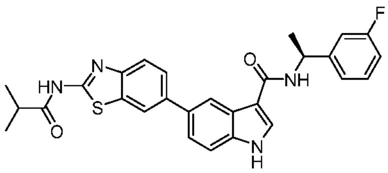 | $^1$H NMR (400 MHz, DMSO-$d_6$) δ 12.13 (s, 1H), 10.73 (s, 1H), 8.83 (d, $J$ = 7.0 Hz, 1H), 8.50 (s, 1H), 8.31 (d, $J$ = 7.9 Hz, 1H), 8.17 (s, | 501.44 | Replacing ii cyclopropylform yl chloride with isopropylformyl chloride | I |
| | | 1H), 7.79 (s, 1H), 7.70 - 7.60 (m, 2H), 7.36 (dd, $J$ = 10.1, 4.6 Hz, 2H), 7.30 (t, $J$ = 7.2 Hz, 2H), 7.19 (t, $J$ = 7.2 Hz, 1H), 5.21 5.13 (m, 1H), 2.01 - 1.95 (m, 1H), 1.51 (d, $J$ = 7.1 Hz, 3H), 1.10 (t, $J$ = 5.7 Hz, 6H). | | | |
| 56 | 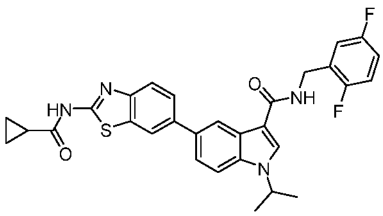 | $^1$H NMR (400 MHz, DMSO-$d_6$) δ 12.39 (s, 1H), 8.50 (d, $J$ = 15.1 Hz, 2H), 8.29 (d, $J$ = 35.5 Hz, 2H), 7.80 (d, $J$ = 8.3 Hz, 1H), 7.71 (dd, $J$ = 18.9, 8.6 Hz, 2H), 7.58 (d, $J$ = 8.1 Hz, 1H), 7.22 (t, $J$ = 24.0 Hz, 3H), 4.52 (d, $J$ = 5.0 Hz, 2H), 2.02 (m, 1H), 1.52 (d, $J$ = 6.5 Hz, 1H), 1.52 (t, $J$ = 6.5 Hz, 6H), 0.95 (t, $J$ = 11.4 Hz, 4H). | 545.12 | Replacing i methyl iodide with 2-iodopropane | II |

(continued)

| Compound No. | Structure | Structural characterization | ESI-MS | Synthetic raw material | Synthetic method |
|---|---|---|---|---|---|
| 57 | 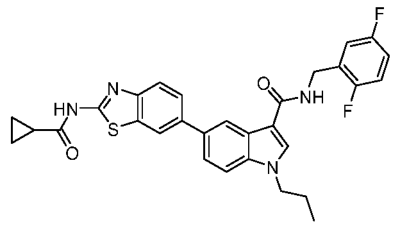 | $^1$H NMR (400 MHz, DMSO-$d_6$) δ 12.64 (s, 1H), 8.51 (dd, *J* = 17.3, 11.4 Hz, 2H), 8.25 (d, *J* = 1.4 Hz, 1H), 8.16 (s, 1H), 7.84 - 7.70 (m, 2H), 7.70 - 7.49 (m, 3H), 7.29 - 7.09 (m, 2H), 4.52 (d, *J* = 5.5 Hz, 2H), 4.21 (dd, *J* = 15.4, 8.6 Hz, 2H), 2.01 (dd, *J* = 12.3, 6.4 Hz, 1H), 1.85 (dd, *J* = 14.2, 7.0 Hz, 2H), 1.08 (t, *J* = 6.8 Hz, 3H), 0.95 (d, *J* = | 545.21 | Replacing i methyl iodide with 1-iodopropane | II |
| | | 6.4 Hz, 4H). | | | |
| 58 | 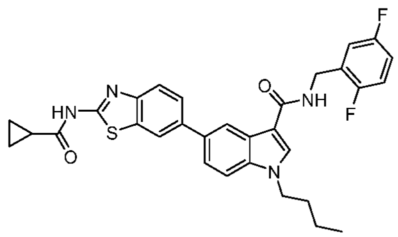 | $^1$H NMR (400 MHz, DMSO-$d_6$) δ 12.61 (s, 1H), 8.59 - 8.40 (m, 2H), 8.20 (d, *J* = 28.9 Hz, 2H), 7.76 (dd, *J* = 24.6, 9.1 Hz, 2H), 7.65 (d, *J* = 8.5 Hz, 1H), 7.58 - 7.47 (m, 1H), 7.31 - 7.08 (m, 3H), 4.51 (d, *J* = 5.7 Hz, 2H), 4.25 (t, *J* = 7.0 Hz, 2H), 2.05 - 1.98 (m, 1H), 1.85 - 1.76 (m, 2H), 1.31 (dd, *J* = 15.0, 7.2 Hz, 2H), 1.01 - 0.87 (m, 7H). | 559.49 | Replacing i methyl iodide with 1-iodobutane | II |
| 59 | 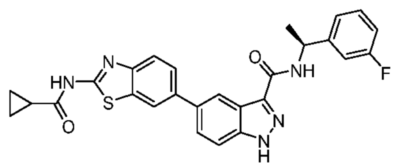 | $^1$H NMR (400 MHz, DMSO-$d_6$) δ 13.72 (s, 1H), 12.66 (s, 1H), 8.87 (d, *J* = 8.6 Hz, 1H), 8.40 (s, 1H), 8.28 (s, 1H), 7.85 - 7.64 (m, 4H), 7.51 (s, 1H), 7.41 - 7.26 (m, 2H), 7.10 - 6.96 (m, 1H), 5.30 - 5.15 (m, 1H), 2.04 - 1.95 (m, 1H), 1.54 (d, *J* = 7.1 Hz, 3H), 0.96 (d, *J* = 5.2 Hz, 4H). | 500.37 | Replacing Intermediate 1 with 5-bromoindazol o-3-formic acid | I |

(continued)

| Compound No. | Structure | Structural characterization | ESI-MS | Synthetic raw material | Synthetic method |
|---|---|---|---|---|---|
| 60 | 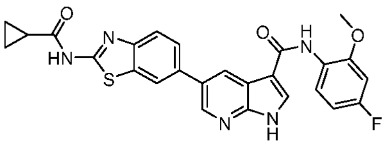 | $^1$H NMR (400 MHz, DMSO-$d_6$) δ 12.39 (s, 1H), 9.13 (s, 1H), 8.69 (dd, *J* = 15.7, 2.2 Hz, 1H), 8.52 (s, 1H), 8.45 (s, 1H), 8.32 (s, 1H), 7.90 - 7.70 (m, 2H), 7.63 - 7.43 (m, 2H), 7.03 (dd, *J* = 10.9, 2.7 Hz, 1H), 6.80 (dd, *J* =10.1, 7.3 Hz, 1H), 3.87 (s, 3H), 2.00 (d, *J* = 5.9 Hz, 1H), 0.95 (d, *J* = 6.0 Hz, 4H). | 502.43 | Replacing Intermediate 1 with 5-bromo-1H-pyrrolo[2,3-b]pyridin - 3-formic acid, Replacing i(S)-1-(3-fluorop henyl) ethanamin e with 2-methoxy-4-fluoroaniline | I |
| 61 | 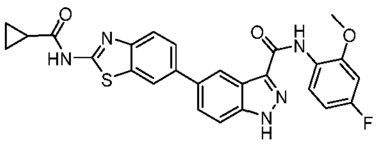 | $^1$H NMR (400 MHz, DMSO-$d_6$) δ 12.59 (s, 1H), 9.33 (s, 1H), 8.86 (d, *J* = 11.7Hz, 1H), 8.72 (s, 1H), 8.65 (s, 1H), 8.39 (s, 1H), 7.89 - 7.73 (m, 2H), 7.63 (s, 2H), 7.09 (dd, *J* = 10.9, 2.8 Hz, 1H), 6.80 (dd, *J* = 10.2, 7.4 Hz, 1H), 3.88 (s, 3H), 2.01 (d, *J* = 5.9 Hz, 1H), 0.96 (d, *J* = 6.1 Hz, 4H). | 502.24 | Replacing Intermediate 1 with 5-bromoindazol o-3-formic acid, Replacing i(S)-1-(3-fluorop henyl) ethanamin e with 2-methoxy-4-fluoroaniline | I |
| 62 | 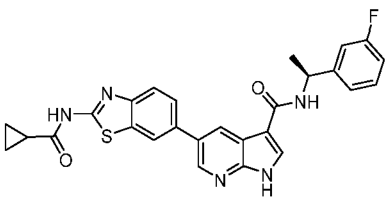 | $^1$H NMR (400 MHz, DMSO-$d_6$) δ 12.22 (s, 1H), 10.64 (s, 1H), 8.64 (dd, *J* = 18.5, 2.1 Hz, 2H), 8.44 (d, *J* = 8.0 Hz, 1H), 8.36 (s, 1H), 8.27 (s, 1H), 7.76 (dd, *J* = 26.8, 8.5 Hz, 2H), 7.38 (dd, *J* = 14.2, 8.2 Hz, 1H), 7.24 (t, *J* = 9.2 Hz, 2H), 7.05 (t, *J* = 8.3 Hz, 1H), 5.25 - 5.16 (m, 1H), 1.99 (d, *J* = 5.8 Hz, 1H), 1.46 (d, J=7.1 Hz, 3H), 0.94 (d, *J* = 5.8 Hz, 4H). | 500.53 | Replacing Intermediate 1 with 5-bromo-1H-pyrrolo[2,3-b]pyridin - 3-formic acid | I |
| 63 | 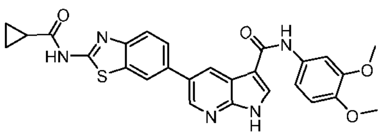 | $^1$H NMR (400 MHz, DMSO-$d_6$) δ 12.23 (s, 1H), 10.64 (s, 1H), 9.71 (s, 1H), 8.50 (dd, *J* = 7.9, 1.5 Hz, 2H), 8.41 (s, 1H), 8.33 - 8.24 (m, | 514.52 | Replacing Intermediate 1 with 5-bromo-1H-pyrrolo[2,3-b]pyridin - 3-formic acid, Replacing | I |

(continued)

| Compound No. | Structure | Structural characterization | ESI-MS | Synthetic raw material | Synthetic method |
|---|---|---|---|---|---|
| | | 1H), 7.48 (s, 1H), 7.32 - 7.13 (m, 2H), 6.92 (d, *J* = 8.7 Hz, 2H), 3.75 (d, *J* = 12.6 Hz, 6H), 1.99 (d, *J* = 5.8 Hz, 1H), 0.96 (d, *J* = 5.9 Hz, 4H). | | i(S)-1-(3-fluorop henyl)ethanamin e with 3,4-dimethoxyan iline | |
| 64 | 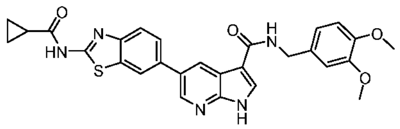 | $^1$H NMR (400 MHz, DMSO-$d_6$) δ 12.64 (s, 1H), 10.64 (s, 1H), 8.44 (d, *J* = 25.8 Hz, 2H), 8.23 (s, 1H), 8.06 (s, 1H), 7.75 (dd, *J* = 21.5, 8.1 Hz, 2H), 7.59 (s, 2H), 7.05 - 6.81 (m, 2H), 4.42 (d, *J* = 5.3 Hz, 2H), 3.86 (s, 3H), 3.73 (s, 3H), 2.01 (m, 1H), 0.95 (d, *J* = 5.7 Hz, 4H). | 528.42 | Replacing Intermediate 1 with 5-bromo-1H-pyrr olo[2,3-b]pyridin - 3-formic acid, Replacing i(S)-1-(3-fluorop henyl) ethanamin e with 3,4-dimethoxybe nzylamine | I |
| 65 | 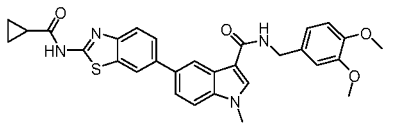 | $^1$H NMR (400 MHz, DMSO-$d_6$) δ 12.64 (s, 1H), 8.44 (d, *J* = 25.8 Hz, 2H), 8.23 (s, 1H), 8.06 (s, 1H), 7.75 (dd, *J* = 21.5, 8.1 Hz, 2H), 7.59 (s, 2H), 7.08 - 6.80 (m, 3H), 4.42 (d, *J* = 5.3 Hz, 2H), 3.86 (s, 3H), 3.73 (d, *J* = 5.6 Hz, 6H), 2.00 (m, 1H), 0.95 (d, *J* = 5.7 Hz, 4H). | 541.50 | Replacing v Intermediate 6 with Intermediate 3, Replacing i(S)-1-(3-fluorop henyl) ethanamin e with 3,4-dimethoxybe nzylamine | II |
| 66 | 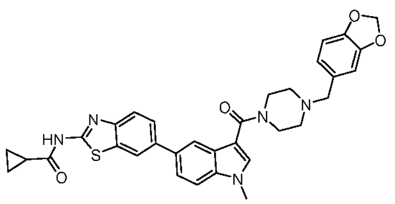 | $^1$H NMR (400 MHz, DMSO-$d_6$) δ 12.64 (s, 1H), 8.26 (d, *J* = 1.5 Hz, 1H), 7.98 (s, 1H), 7.82 - 7.69 (m, 3H), 7.59 (s, 2H), 6.91 - 6.81 (m, 2H), 6.77 (d, *J* = 7.9 Hz, 1H), 5.98 (s, 2H), | 594.32 | Replacing v Intermediate 6 with Intermediate 3, Replacing i(S)-1-(3-fluorop henyl) ethanamin e with 1-piperonylpiperaz | II |
| | | 3.86 (s, 3H), 3.67 (s, 4H), 3.43 (s, 2H), 2.41 (s, 4H), 2.02 (s, 1H), 0.97 (d, *J* = 4.9 Hz, 4H). | | ine | |

(continued)

| Compound No. | Structure | Structural characterization | ESI-MS | Synthetic raw material | Synthetic method |
|---|---|---|---|---|---|
| 67 | 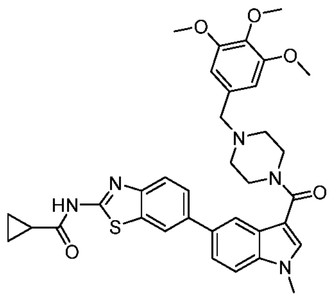 | $^1$H NMR (400 MHz, DMSO-de) δ 12.62 (s, 1H), 8.20 (s, 1H), 8.00 (s, 1H), 7.84 (s, 1H), 7.75 (d, *J* = 8.4 Hz, 1H), 7.70 - 7.55 (m, 3H), 6.76 (d, *J* = 10.1 Hz, 2H), 4.77 (m, 4H), 3.96 - 3.84 (m, 4H), 3.74 (s, 9H), 3.67 (s, 3H), 2.83 (s, 2H), 2.07 - 1.95 (m, 1H), 0.96 (d, *J* = 4.8 Hz, 4H). | 640.67 | Replacing v Intermediate 6 with Intermediate 3, Replacing i(S)-1-(3-fluorop henyl) ethanamin e with 1-(3,4,5-trimethoxybenzy l) piperazine | II |
| 68 | 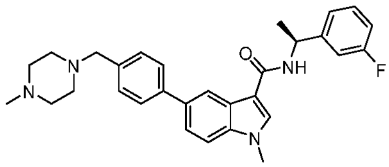 | $^1$H NMR (400 MHz, DMSO-de) δ 9.35 (s, 1H), 8.34 (d, *J* = 8.0 Hz, 1H), 8.26 (d, *J* = 1.9 Hz, 1H), 8.19 (s, 1H), 8.07 (d, *J* = 5.9 Hz, 2H), 7.86 (d, *J* = 6.0 Hz, 2H), 7.49 (d, *J* = 8.7 Hz, 1H), 7.39 - 7.30 (m, 1H), 7.22 (t, *J* = 9.4 Hz, 2H), 7.08 - 6.99 (m, 1H), 5.18 (p, *J* = 7.0 Hz, 1H), 4.66 (d, *J* = 5.1 Hz, 2H). 3.90 (s, 3H), 3.84 (s, 3H), 3.13(s, 4H), 2.48(s, 4H), 1.46 (d, *J* = 7.1 Hz, 3H). | 485.26 | Replacing v Intermediate 7 with 1-(4-bromophen yl)-N-methyl piperazine | II |
| 69 | 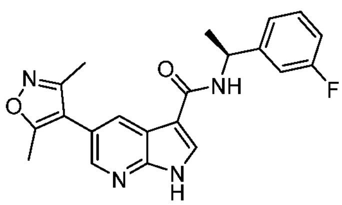 | $^1$H NMR (400 MHz, DMSO-de) δ 12.28 (s, 1H), 8.43 - 8.32 (m, 3H), 8.26 (d, *J* = 2.1 Hz, 1H), 7.40 - 7.29 (m, 1H), 7.23 (t, | 379.38 | Replacing i Intermediate 1 with 5-bromo-1H-pyrr olo[2,3-b]pyridin - 3-formic acid, | I |
| | | *J* = 9.3 Hz, 2H), 7.04 (t, *J* = 8.7 Hz, 1H), 5.25 - 5.13 (m, 1H), 2.39 (s, 3H), 2.20 (s, 3H), 1.48 (d, *J* = 7.1 Hz, 3H). | | Replacing Intermediate 5 with 3,5-dimethylisox azolo-4-boronic acid | |

(continued)

| Compound No. | Structure | Structural characterization | ESI-MS | Synthetic raw material | Synthetic method |
|---|---|---|---|---|---|
| 70 | 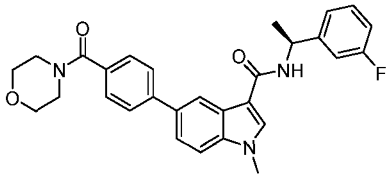 | $^1$H NMR (400 MHz, DMSO-$d_6$) δ 8.42 (s, 1H), 8.33 (d, *J* = 8.0 Hz, 1H), 8.18 (s, 1H), 7.72 (d, *J* = 8.2 Hz, 2H), 7.58 (dd, *J* = 18.3, 8.5 Hz, 2H), 7.49 (d, *J* = 8.2 Hz, 2H), 7.37 (dd, *J* = 14.2, 7.9 Hz, 1H), 7.24 (t, *J* = 8.4 Hz, 2H), 7.05 (dd, *J* = 12.1, 5.1 Hz, 1H), 5.26 - 5.14 (m, 1H), 3.88 (s, 3H), 3.61 (s, 8H), 1.48 (d, *J* = 7.0 Hz, 3H). | 486.53 | Replacing v Intermediate 7 with 4-(morpholine-4 carbonyl)bromo benzene | II |
| 71 | 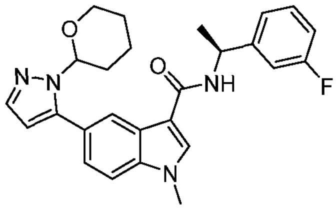 | $^1$H NMR (400 MHz, DMSO-$d_6$) δ 8.43 - 8.10 (m, 3H), 7.74 - 7.48 (m, 2H), 7.43 - 7.16 (m, 3H), 7.03 (s, 1H), 6.39 (s, 1H), 5.19 (d, *J* = 8.1 Hz, 2H), 4.05 - 3.77 (m, 3H), 3.88 (s, 3H), 3.63 - 3.44 (m, 1H), 2.40 (d, *J* = 10.7 Hz, 1H), 2.03 - 1.65 (m, 2H), 1.47 (d, *J* = 6.4 Hz, 5H). | 447.46 | Replacing v Intermediate 7 with 5-bromo-1 -(tetra hydro-2H-pyran-2-yl)-1H-pyrazol e | II |
| 72 | 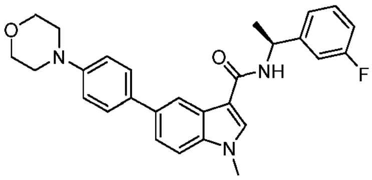 | $^1$H NMR (400 MHz, DMSO-$d_6$) δ 8.36 - 8.22 (m, 2H), 8.14 (s, 1H), 7.49 (dd, *J* = 26.0, 8.7 Hz, 4H), 7.37 (dd, *J* = 14.3, 7.9 Hz, 1H), 7.24 (t, *J* = 8.6 Hz, 2H), 7.03 (t, *J* = 9.7 Hz, 3H), 5.25 - 5.08 (m, 1H), 3.86 (s, 3H), 3.81 - 3.65 (m, 4H), 3.19 - | 458.44 | Replacing v Intermediate 7 with 4-(4-bromophen yl)morpholine | II |
| | | 3.04 (m, 4H), 1.48 (d, *J* = 7.0 Hz, 3H). | | | |

(continued)

| Compound No. | Structure | Structural characterization | ESI-MS | Synthetic raw material | Synthetic method |
|---|---|---|---|---|---|
| 73 | 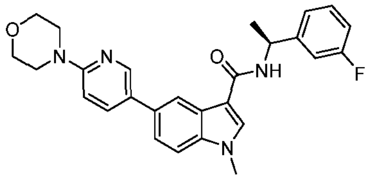 | $^1$H NMR (400 MHz, DMSO-$d_6$) δ 8.43 (d, *J* = 2.3 Hz, 1H), 8.34 - 8.26 (m, 2H), 8.16 (s, 1H), 7.85 (dd, *J* = 8.8, 2.5 Hz, 1H), 7.56 (d, *J* = 8.5 Hz, 1H), 7.47 (dd, J = 8.6, 1.6 Hz, 1H), 7.37 (d, *J* = 6.2 Hz, 1H), 7.24 (t, *J* = 9.0 Hz, 2H), 7.04 (s, 1H), 6.92 (d, *J* = 8.8 Hz, 1H), 5.20 (s, 1H), 3.86 (s, 3H), 3.77 - 3.65 (m, 4H), 3.55 - 3.42 (m, 4H), 1.48 (d, *J* = 7.1 Hz, 3H). | 459.11 | Replacing v Intermediate 7 with 4-N-(5-bromopyridin-2-yl) morpholine | II |
| 74 | 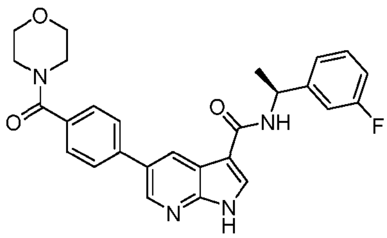 | $^1$H NMR (400 MHz, DMSO-$d_6$) δ 11.36 (s, 1H) 8.42 (s, 1H), 8.32 (d, *J* = 8.0 Hz, 1H), 8.17 (s, 1H), 7.65 (d, *J* = 8.2 Hz, 2H), 7.55 (dd, *J* = 18.4, 8.5 Hz, 2H), 7.52 (d, *J* = 8.2 Hz, 1H), 7.39 (dd, *J* = 14.2, 7.9 Hz, 1H), 7.26 (t, *J* = 8.4 Hz, 2H), 7.04 (dd, *J* = 12.1, 5.1 Hz, 1H), 5.26 - 5.13 (m, 1H), 3.60 (s, 8H), 1.50 (d, *J* = 7.1 Hz, 3H). | 473.14 | Replacing i Intermediate 1 with 5-bromo-1H-pyrrolo[2,3-b]pyridin-3-formic acid, Replacing Intermediate 5 with 3,5-dimethylisoxazole-4-boronic acid | I |
| 75 | 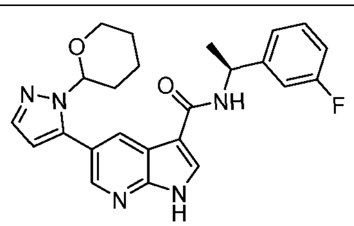 | $^1$H NMR (400 MHz, DMSO-$d_6$) δ 12.16 (s, 1H), 8.73 (d, *J* = 27.8 Hz, 2H), 8.47 - 8.27 (m, 2H), 7.92 (s, 1H), 7.06 (s, 2H), | 434.48 | Replacing i Intermediate 1 with 5-bromo-1H-pyrrolo[2,3-b]pyridin-3-formic acid, | I |
| | | 6.79 (s, 1H), 5.45 (s, 2H), 5.19 (s, 1H), 4.03 - 3.79 (m, 4H), 3.65 - 3.45 (m, 2H), 2.41 (m, 1H), 2.01 - 1.66 (m, 2H), 1.48 (d, *J* = 6.3 Hz, 3H). | | Replacing Intermediate 5 with 5-bromo-1 -(tetrahydro-2H-pyran-2-yl)-1H-pyrazol e | |

(continued)

| Compound No. | Structure | Structural characterization | ESI-MS | Synthetic raw material | Synthetic method |
|---|---|---|---|---|---|
| 76 | 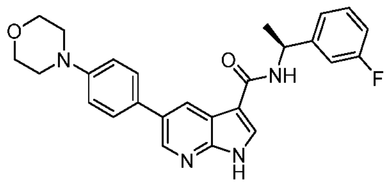 | $^1$H NMR (400 MHz, DMSO-$d_6$) δ 12.39 (s, 1H), 8.36 - 8.22 (m, 2H), 8.14 (s, 1H), 7.49 (dd, *J* = 26.0, 8.7 Hz, 4H), 7.39 (dd, *J* = 14.3, 7.9 Hz, 1H), 7.24 (t, *J* = 8.6 Hz, 1H), 7.03 (t, *J* = 9.6 Hz, 3H), 5.21 - 5.09 (m, 1H), 3.81 - 3.65 (m, 4H), 3.18 - 3.02 (m, 4H), 1.50 (d, *J* = 7.1 Hz, 3H). | 445.40 | Replacing i Intermediate 1 with 5-bromo-1H-pyrrolo[2,3-b]pyridin-3-formic acid, Replacing Intermediate 5 with 4-(4-bromophenyl) morpholine | I |
| 77 | 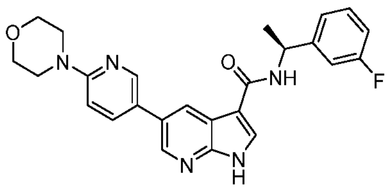 | $^1$H NMR (400 MHz, DMSO-de) δ 12.37 (s, 1H), 9.24 (s, 1H),8.37 - 8.25 (m, 2H), 8.16 (s, 1H), 7.51 (d, *J* = 8.7 Hz, 2H), 7.39 (dd, *J* = 7.9 Hz, 1H), 7.26 (t, *J* = 8.6 Hz, 1H), 7.07 (t, *J* = 9.99 Hz, 3H), 5.20 - 5.04 (m, 1H), 3.80 - 3.65 (m, 4H), 3.17 - 3.00 (m, 4H), 1.48 (d, *J* = 7.1 Hz, 3H). | 446.30 | Replacing i Intermediate 1 with 5-bromo-1H-pyrrolo[2,3-b]pyridin - 3-formic acid, Replacing Intermediate 5 with 4-N-(5-bromopyridin-2-yl) morpholine | I |
| 78 | 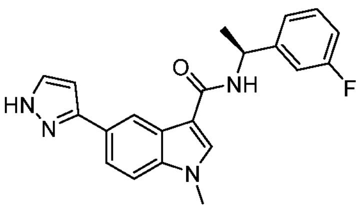 | $^1$H NMR (400 MHz, DMSO-$d_6$) δ 9.65 (s, 1H), 8.43 - 8.10 (m, 3H), 7.74 - 7.48 (m, 2H), 7.43 - 7.16 (m, 2H), 7.03 (s, 1H), 6.39 (s, 1H), 5.19 (d, *J* = 8.1 Hz, 2H), 5.05 | 363.37 | Replacing v Intermediate 7 with 3-bromopyrazol e | II |
| | | - 4.97 (m, 1H), 3.93 (s, 3H), 1.47 (d, *J* = 6.2 Hz, 3H). | | | |
| 79 | 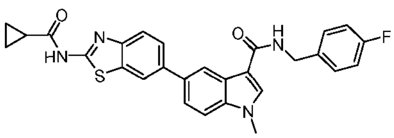 | $^1$H NMR (400 MHz, DMSO-$d_6$) δ 12.64 (s, 1H), 8.59 - 8.46 (m, 2H), 8.11 (d, *J* = 5.1 Hz, 2H), 7.84 - 7.66 (m, 1H), 7.51 (s, 1H), 7.44 - 7.32 (m, 1H), 7.21 - 7.17 (m, 2H), 7.10 (dd, *J* = 12.1, 5.2 Hz, 3H), 4.28 (d, *J* = 5.8 Hz, 2H), 3.90 (s, 3H), 2.11 (s, 1H), 0.96 (d, *J* = 5.9 Hz, 4H). | 499.51 | Replacing v Intermediate 7 with 4-N-(5-bromopyridin-2-yl) morpholine, Replacing i(S)-1-(3-fluorop henyl) ethanamin e with 4-fluorobenzylamine | II |

(continued)

| Compound No. | Structure | Structural characterization | ESI-MS | Synthetic raw material | Synthetic method |
|---|---|---|---|---|---|
| 80 | 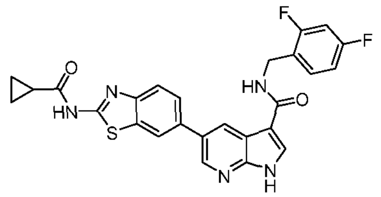 | $^1$H NMR (400 MHz, DMSO-$d_6$) δ 12.65 (s, 1H), 11.68 (s, 1H), 8.48 (s, 1H), 8.33 (s, 1H), 8.17 (s, 1H), 7.79 (d, *J* = 8.6 Hz, 1H), 7.74 (d, *J* = 8.9 Hz, 1H), 7.52 (s, 2H), 7.48 (d, *J* = 7.5 Hz, 1H), 7.21 (d, *J* = 10.2 Hz, 2H), 4.51 (d, *J* = 5.6 Hz, 2H), 2.01 (s, 1H), 0.96 (d, *J* = 5.9 Hz, 4H). | 504.33 | Replacing i Intermediate 1 with 5-bromo-1H-pyrrolo[2,3-b]pyridin-3-formic acid, Replacing i(S)-1-(3-fluorophenyl) ethanamine with 2,4-difluorobenzylamine | I |
| 81 | 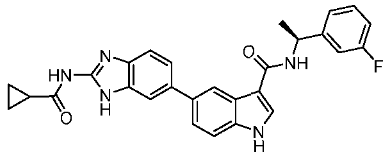 | $^1$H NMR (400 MHz, DMSO-$d_6$) δ 11.97 (s, 1H), 11.87 (s, 1H), 11.61 (s, 1H), 8.37 (s, 1H), 8.28 (d, *J* = 7.7 Hz, 1H), 8.19 (d, *J* = 2.8 Hz, 1H), 7.66 (d, *J* = 38.1 Hz, 2H), 7.52 - 7.31 (m, 4H), 7.29 - 7.17 (m, 2H), 7.07 - 6.99 (m, 1H), 5.24 - 5.16 (m, 1H), 1.99 (m, 1H), | 482.34 | Replacing i Intermediate 1 with 5-bromo-1H-benzoimidazolo-2-amine | I |
| | | 1.49 (d, *J* = 7.0 Hz, 3H), 0.92 (d, *J* = 5.7 Hz, 4H). | | | |
| 82 | 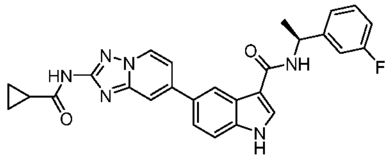 | $^1$H NMR (400 MHz, DMSO-de) δ 11.80 (s, 1H), 11.05 (s, 1H), 8.83 (d, *J* = 7.2 Hz, 1H), 8.53 (s, 1H), 8.36 (d, *J* = 7.9 Hz, 1H), 8.26 (d, *J* = 2.3 Hz, 1H), 7.84 (s, 1H), 7.69 - 7.61 (m, 1H), 7.58 (d, *J* = 8.5 Hz, 1H), 7.40 (ddd, J = 22.0, 10.7, 4.9 Hz, 2H), 7.25 (t, *J* = 10.0 Hz, 2H), 7.05 (dd, *J* = 11.9, 5.4 Hz, 1H), 5.28 - 5.16 (m, 1H), 2.06 (s, 1H), 1.50 (d, *J* = 7.1 Hz, 3H), 0.84 (d, *J* = 5.5 Hz, 4H). | 483.34 | Replacing i Intermediate 1 with 7-bromo-[1,2,4] triazolo[1,5-a]pyridin-2-amine | I |

(continued)

| Compound No. | Structure | Structural characterization | ESI-MS | Synthetic raw material | Synthetic method |
|---|---|---|---|---|---|
| 83 | 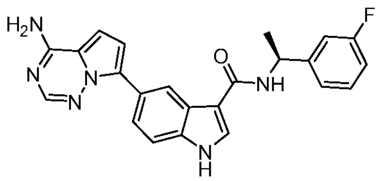 | $^1$H NMR (400 MHz, DMSO-$d_6$) δ 11.66 (s, 1H), 8.72 (s, 1H), 8.26 (d, *J* = 8.1 Hz, 1H), 8.19 (d, *J* = 2.6 Hz, 1H), 7.88 (s, 1H), 7.79 (dd, *J* = 8.6, 1.6 Hz, 1H), 7.66 (d, *J* = 4.5 Hz, 2H), 7.49 (d, *J* = 8.6 Hz, 1H), 7.37 (dd, *J* = 14.1, 8.1 Hz, 1H), 7.24 (t, *J* = 9.3 Hz, 2H), 7.08 - 6.96 (m, 2H), 6.88 (d, *J* = 4.5 Hz, 1H), 5.27 - 5.14 (m, 1H), 1.48 (d, *J* = 7.1 Hz, 3H). | 415.24 | Replacing iii Intermediate 5 with 4-amino-7-bromopyrrolo[2,1 -f][1, 2,4] triazine | I |
| 84 | 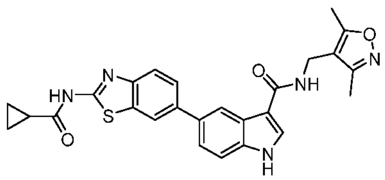 | $^1$H NMR (400 MHz, DMSO-$d_6$) δ 12.45 (s, 1H), 11.64 (s, 1H), 8.44 (s, 1H), 8.23 (dd, *J* = 12.5, 3.5 Hz, 2H), 8.09 - 7.99 (m, 2H), 7.82 - 7.66 (m, 2H), 7.51 (s, 1H), 4.23 (d, *J* = 5.5 Hz, 2H), 2.41 (s, 3H), 2.23 (s, 3H), 2.01 (m, 1H), 0.96 (d, *J* = 5.6 Hz, 4H). | 486.35 | Replacing i(S)-1-(3-fluorop henyl) ethanamin e with 3,5-dimethyl-4-aminomethyl isoxazole | I |
| 85 | 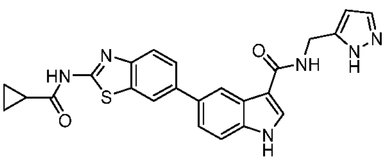 | $^1$H NMR (400 MHz, DMSO-$d_6$) δ 12.66 (s, 1H), 12.38 (s, 1H), 11.41 (s, 1H), 9.48 (s, 1H), 8.53 - 8.43 (m, 2H), 8.33 (s, 1H), 8.15 (d, *J* = 3.7 Hz, 2H), 7.89 - 7.76 (m, 2H), 7.66 - 7.56 (m, 2H), 4.25 (d, *J* = 5.6 Hz, 2H), 2.09 (s, 1H), 0.99 (d, *J* = 5.2 Hz, 4H). | 457.36 | Replacing i(S)-1-(3-fluorop henyl) ethanamin e with 5-methylaminopyrazole | I |
| 86 | 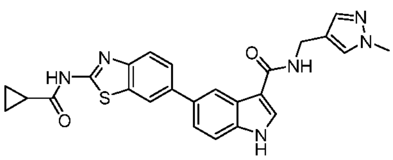 | $^1$H NMR (400 MHz, DMSO-$d_6$) δ 12.34 (s, 1H), 11.62 (s, 1H), 8.46 (s, 1H), 8.24 (s, 1H), 8.15 (s, 1H), 8.05 (s, 1H), 7.70 (d, *J* = 13.6 Hz, 2H), 7.61 (s, 1H), 7.50 (s, 2H), 7.37 (s, 1H), 4.30 (d, *J* = 5.6 Hz, 2H), 3.78 (s, 3H), 1.94 (s, 1H), 0.96 (d, *J* = 5.5 Hz, 4H). | 471.43 | Replacing i(S)-1-(3-fluorop henyl) ethanamin e with 5-methylamino-N-methylpyrazole | I |

(continued)

| Compound No. | Structure | Structural characterization | ESI-MS | Synthetic raw material | Synthetic method |
|---|---|---|---|---|---|
| 87 | 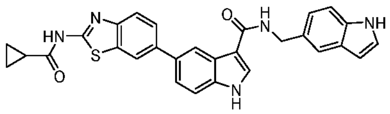 | $^1$H NMR (400 MHz, DMSO-$d_6$) δ 12.67 (s, 1H), 11.60 (s, 1H), 11.09 (s, 1H), | 506.41 | Replacing i(S)-1-(3-fluorop henyl) ethanamin e with 5-(amino | I |
| | | 8.50 (s, 1H), 8.42 (s, 1H), 8.22 (s, 1H), 8.12 (s, 1H), 7.95 (s, 1H), 7.76 (d, *J* = 18.8 Hz, 2H), 7.52 (s, 1H), 7.34 - 7.26 (m, 2H), 7.06 - 6.96 (m, 2H), 6.63 (s, 1H), 4.77 (d, *J* = 5.7 Hz, 2H), 2.01 (m, 1H), 0.96 (d, *J* = 5.6 Hz, 4H). | | methyl)indole | |
| 88 | 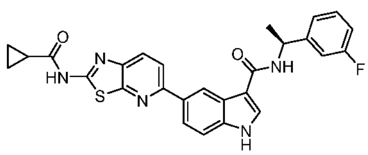 | $^1$H NMR (400 MHz, DMSO-de) δ 12.72 (s, 1H), 11.73 (s, 1H), 8.86 (s, 1H), 8.34 (d, *J* = 8.0 Hz, 1H), 8.23 (d, *J* = 2.7 Hz, 1H), 8.09 (d, *J* = 8.5 Hz, 1H), 7.96 (t, *J* = 8.8 Hz, 2H), 7.52 (d, *J* = 8.6 Hz, 1H), 7.38 (dd, *J* = 14.1, 7.8 Hz, 1H), 7.25 (t, *J* = 10.0 Hz, 2H), 7.05 (t, *J* = 8.6 Hz, 1H), 5.29 - 5.11 (m, 1H), 2.05 - 1.97 (m, 1H), 1.50 (d, *J* = 7.1 Hz, 3H), 0.97 (d, *J* = 5.7 Hz, 4H). | 500.48 | Replacing ii Intermediate 3 with 5-bromo-2-amin othiazolo[5,4-b]p yridine | I |
| 89 | 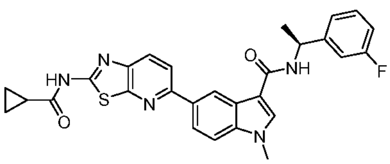 | $^1$H NMR (400 MHz, DMSO-de) δ 11.73 (s, 1H), 8.87 (s, 1H), 8.35 (d, *J* = 8.2 Hz, 1H), 8.21 (d, *J* = 2.8 Hz, 1H), 8.06 (d, *J* = 8.3 Hz, 1H), 7.95 (t, *J* = 8.2 Hz, 2H), 7.50 (d, *J* = 8.3 Hz, 1H), 7.34 (dd, *J* = 14.1, 7.6 Hz, 1H), 7.25 (t, *J* = 10.1 Hz, 2H), 7.01 (t, *J* = 8.8 Hz, | 514.39 | Replacing v Intermediate 6 with 5-bromo-2-amin othiazolo[5,4-b]p yridine | II |
| | | 1H), 5.29 - 5.11 (m, 1H), 3.88 (s, 3H), 2.05 - 1.97 (m, 1H), 1.50 (d, *J* = 7.1 Hz, 3H), 0.97 (d, *J* = 5.7 Hz, 4H). | | | |

(continued)

| Compound No. | Structure | Structural characterization | ESI-MS | Synthetic raw material | Synthetic method |
|---|---|---|---|---|---|
| 90 | 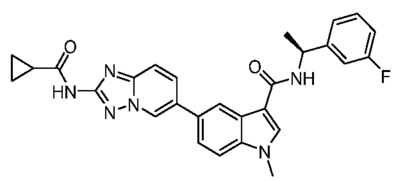 | $^1$H NMR (400 MHz, DMSO-de) δ 11.01 (s, 1H), 8.81 (d, *J* = 6.9 Hz, 1H), 8.50 (s, 1H), 8.28 (d, *J* = 8.1 Hz, 1H), 8.23 (s, 1H), 7.81 (s, 1H), 7.70 (d, *J* = 8.4 Hz, 1H), 7.60 (d, *J* = 8.7 Hz, 1H), 7.45 - 7.42 (m, 1H), 7.35 (dd, *J* = 14.1, 7.5 Hz, 1H), 7.22 (t, *J* = 9.6 Hz, 2H), 7.01 (t, *J* = 8.8 Hz, 1H), 5.22 (dd, *J* = 14.3, 6.9 Hz, 1H), 3.88 (s, 3H), 2.01 (m, 1H), 1.49 (d, *J* = 7.1 Hz, 3H), 0.96 (d, *J* = 5.7 Hz, 4H). | 497.24 | Replacing v Intermediate 6 with 6-bromo-[1,2,4]triazolo[1,5-a]pyridin- 2-amine | II |
| 91 | 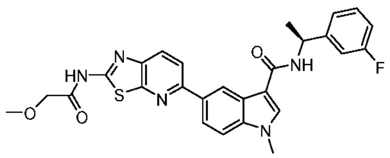 | $^1$H NMR (400 MHz, DMSO-$d_6$) δ 8.78 (s, 1H), 8.32 (d, *J* = 7.9 Hz, 1H), 8.16 (s, 1H), 7.91 (d, *J* = 8.6 Hz, 1H), 7.81 - 7.73 (m, 2H), 7.65 (d, *J* = 8.4 Hz, 1H), 7.55 (d, *J* = 8.7 Hz, 1H), 7.37 (dd, *J* = 14.1, 8.1 Hz, | 518.46 | Replacing v Intermediate 6 with 5-bromo-2-aminothiazolo[5,4-b]pyridine, Replacing cyclopropylform yl chloride with methoxyacetyl | II |
| | | 1H), 7.24 (t, *J* = 9.6 Hz, 2H), 7.04 (t, *J* = 8.4 Hz, 1H), 5.24 - 5.16 (m, 1H), 4.17 (d, *J* = 6.7 Hz,, 2H), 3.87 (s, 3H), 3.80 (s, 3H), 1.49 (d, *J* = 7.1 Hz, 3H). | | chloride | |
| 92 | 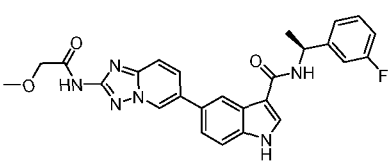 | $^1$H NMR (400 MHz, DMSO-$d_6$) δ 11.71 (s, 1H), 8.73 (s, 1H), 8.37 - 8.27 (m, 2H), 8.23 (d, *J* = 2.7 Hz, 1H), 7.73 (d, *J* = 9.2 Hz, 1H), 7.53 (dd, *J* = 16.7, 8.3 Hz, 2H), 7.37 (dd, *J* = 14.7, 8.3 Hz, 2H), 7.23 (d, *J* = 11.7 Hz, 2H), 5.99 (s, 2H), 5.24 - 5.18 (m, 1H), 3.89 (s, 3H), 1.53 (d, *J* = 7.0 Hz, 3H). | 487.33 | Replacing ii Intermediate 3 with 6-bromo-[1,2,4]triazolo[1,5-a]pyridin-2-amine, Replacing cyclopropylform yl chloride with methoxyacetyl chloride | I |

(continued)

| Compound No. | Structure | Structural characterization | ESI-MS | Synthetic raw material | Synthetic method |
|---|---|---|---|---|---|
| 93 | 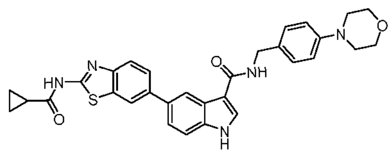 | $^1$H NMR (400 MHz, DMSO-$d_6$) δ 12.63 (s, 1H), 11.61 (s, 1H), 8.48 (s, 1H), 8.38 (d, *J* = 5.9 Hz, 1H), 8.23 (s, 1H), 8.09 (d, *J* = 2.5 Hz, 1H), 7.76 (dd, *J* = 27.6, 8.4 Hz, 3H), 7.51 (s, 1H), 7.22 (t, *J* = 9.0 Hz, 2H), 6.91 (d, *J* = 8.6 Hz, 2H), 4.40 (d, *J* = 5.5 Hz, 2H), 3.76 - 3.66 (m, 4H), 3.08 - 2.99 (m, 4H), 2.01 (d, *J* = 5.7 Hz, 1H), 0.96 (d, *J* = 4.9 Hz, 4H). | 552.54 | Replacing i(S)-1-(3-fluorop henyl) ethanamin e with 4-morpholinobe nzylamine | I |
| 94 | 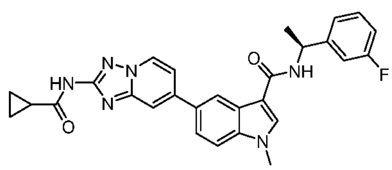 | $^1$H NMR (400 MHz, DMSO-$d_6$) δ 11.04 (s, 1H), 8.83 (d, *J* = 7.1 Hz, 1H), 8.53 (s, 1H), 8.37 (d, *J* = 8.0 Hz, 1H), 8.22 (s, 1H), 7.86 (s, 1H), 7.72 (d, *J* = 8.6 Hz, 1H), 7.66 (d, *J* = 8.7 Hz, 1H), 7.37 (dd, J = 14.2, 7.8 Hz, 1H), 7.25 (t, *J* = 9.7 Hz, 1H), 7.05 (t, *J* = 8.6 Hz, 1H), 5.22 (dd, *J* = 14.3, 6.9 Hz, 1H), 3.90 (s, 3H), 2.07 (m, 1H), 1.56 (d, *J* = 7.3 Hz, 3H), 0.96 (d, *J* = 5.3 Hz, 4H). | 497.21 | | II |
| 95 | 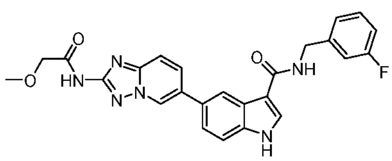 | $^1$H NMR (400 MHz, DMSO-de) δ 11.74 (s, 1H), 10.64 (s, 1H), 9.08 (s, 1H), 8.31 (s, 1H), 8.24 (s, 1H), 7.95 (m, 1H), 7.75 (d, *J* = 9.4 Hz, 2H), 7.56 (s, 1H), 7.37 (d, *J* = 6.0 Hz, 2H), 7.28 - 7.20 (m, 2H), 7.04 (s, 1H), 4.20 (d, *J* = 6.5 Hz,, 2H), 4.16 (d, *J* = 5.7 Hz,, 2H), 3.88 (s, 3H). | 473.32 | Replacing ii Intermediate 3 with 6-bromo-[1,2,4]t riazolo[1,5-a]pyri din- 2-amine, Replacing cyclopropylform yl chloride with methoxyacetyl chloride, Replacing i(S)-1-(3-fluorop henyl) ethanamin e with 3-fluorobenzyla mine | I |

(continued)

| Compound No. | Structure | Structural characterization | ESI-MS | Synthetic raw material | Synthetic method |
|---|---|---|---|---|---|
| 96 | 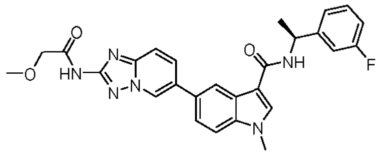 | $^1$H NMR (400 MHz, DMSO-$d_6$) δ 8.73 (s, 1H), 8.42 - 8.35 (m, 2H), 8.25 (d, *J* = 2.6 Hz, 1H), 7.65 (d, *J* = 9.1 Hz, 1H), 7.31 (dd, *J* = 16.5, 8.1 Hz, 3H), 7.24 (dd, *J* = 14.7, 8.3 Hz, 2H), 7.11 (d, *J* = 11.1 Hz, 2H), 7.00 (s, 1H), 5.23 - 5.19 (m, 1H), 4.26 (d, *J* = 6.5 Hz,, 2H), 3.89 (s, 3H), 3.84 (s, 3H), 1.56 (d, *J* = 7.3 Hz, 3H). | 501.36 | Replacing v Intermediate 6 with 6-bromo-[1,2,4]triazolo[1,5-a]pyridin- 2-amine, Replacing cyclopropylform yl chloride with methoxyacetyl chloride | II |
| 97 | 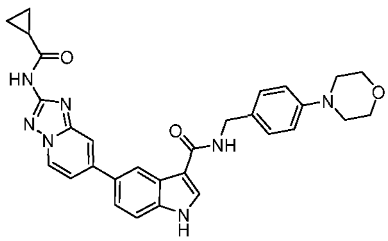 | $^1$H NMR (400 MHz, DMSO-$d_6$) δ 12.69 (s, 1H), 11.71 (s, 1H), 8.51 (s, 1H), 8.44 (d, *J* = 5.5 Hz, 1H), 8.21 (s, 1H), 8.04 (d, *J* = 2.7 Hz, 1H), 7.76 (dd, *J* = 27.5, 8.3 Hz, 2H), 7.52 (s, 2H), 7.29 (t, *J* = 9.3 Hz, 2H), 6.99 (d, *J* = 8.9 Hz, 2H), 4.40 (d, *J* = 5.5 Hz, 2H), 3.79 - 3.68 (m, 4H), 3.12 - 3.03 (m, 4H), 2.00 (d, *J* = 5.7 Hz, 1H), 0.96 (d, *J* = 5.2 Hz, 4H). | 536.41 | Replacing i(S)-1-(3-fluorop henyl) ethanamin e with 4-morpholinobe nzylamine, Replacing Intermediate 3 with Intermediate 6 | I |
| 98 | 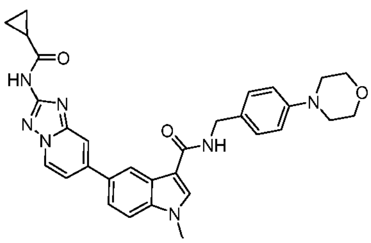 | $^1$H NMR (400 MHz, DMSO-$d_6$) δ 12.63 (s, 1H), 8.62 (s, 1H), 8.36 (d, *J* = 5.6 Hz, 1H), 8.21 (s, 1H), 8.00 (d, *J* = 2.4 Hz, 1H), 7.69 (dd, *J* = 27.2, 8.1 Hz, 2H), 7.43 (s, 2H), 7.21 (t, *J* = 9.3 Hz, 2H), 6.92 | 550.32 | Replacing iii(S)-1-(3-fluoro phenyl) ethanami ne with 4-morpholinobe nzylamine | II |
| | | (d, *J* = 8.9 Hz, 2H), 4.46 (d, *J* = 5.9 Hz, 2H), 3.90 (s, 3H), 3.78 - 3.68 (m, 4H), 3.04 - 2.98 (m, 4H), 2.01 (d, *J* = 5.5 Hz, 1H), 0.96 (d, *J* = 5.2 Hz, 4H). | | | |

(continued)

| Compound No. | Structure | Structural characterization | ESI-MS | Synthetic raw material | Synthetic method |
|---|---|---|---|---|---|
| 99 | 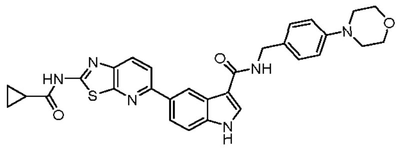 | $^1$H NMR (400 MHz, DMSO-$d_6$) δ 11.80 (s, 1H), 11.16 (s, 1H), 9.18 (s, 1H), 8.52 (d, *J* = 22.4 Hz, 2H), 8.20 (s, 1H), 8.07 (s, 1H), 7.84 (s, 1H), 7.64 (s, 2H), 7.33 (s, 2H), 7.00 (s, 2H), 4.50 (s, 2H), 3.80 (s, 3H), 3.17 (d, *J* = 25.3 Hz, 4H), 2.20 (d, *J* = 38.6 Hz, 1H), 0.93 (d, *J* = 4.9 Hz, 4H). | 553.21 | Replacing i(S)-1-(3-fluorop henyl) ethanamin e with 4-morpholinobe nzylamine, Replacing Intermediate 3 with 5-bromo-2-amin othiazolo[5,4-b]p yridine | I |
| 100 | 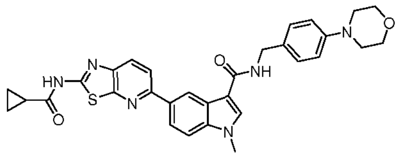 | $^1$H NMR (400 MHz, DMSO-$d_6$) δ 12.65 (s, 1H), 8.34 (d, *J* = 5.3 Hz, 1H), 8.19 (s, 1H), 7.92 (d, *J* = 2.3 Hz, 1H), 7.60 (dd, *J* = 27.1, 8.2 Hz, 2H), 7.45 (s, 2H), 7.13 (t, *J* = 9.0 Hz, 2H), 6.89 (d, *J* = 8.2 Hz, 2H), 4.45 (d, *J* = 5.5 Hz, 2H), 3.90 (s, 3H), 3.77 - 3.65 (m, 4H), 3.01 - 2.99 (m, 4H), 2.01 (d, *J* = 5.8 Hz, 1H), 0.96 (d, *J* = 5.6 Hz, 4H). | 567.49 | Replacing iii(S)-1-(3-fluoro phenyl) ethanami ne with 4-morpholinobe nzylamine, Replacing Intermediate 6 with 5-bromo-2-amin othiazolo[5,4-b]p yridine | II |
| 101 | 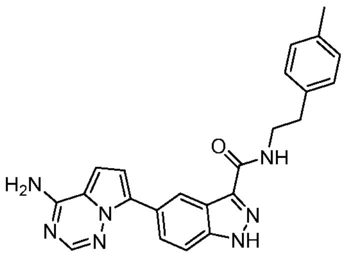 | $^1$H NMR (400 MHz, DMSO-$d_6$) δ 13.63 (s, 1H), 8.88 (s, 1H), 8.45 (s, 1H), 8.00 (d, *J* = 8.7 Hz, 1H), 7.97 (s, 1H), 7.75 (s, 2H), 7.66 (d, *J* = 8.9 Hz, 1H), 7.32 (dd, *J* = 8.3, 5.8 Hz, 2H), 7.17 - 6.99 (m, 4H), 3.55 (d, *J* = 6.7 Hz, 2H), 2.88 (d, *J* = 6.5 Hz, 2H), 2.19 (s, 3H). | 412.29 | Replacing iii Intermediate 5 with 4-amino-7-brom opyrrolo[2,1-f][1, 2,4] triazine, Replacing Intermediate 1 with 5-bromoindazol o-3-formic acid, Replacing i(S)-1-(3-fluorop henyl) ethanamin e with 4-methylphenet hylamine | I |

(continued)

| Compound No. | Structure | Structural characterization | ESI-MS | Synthetic raw material | Synthetic method |
|---|---|---|---|---|---|
| 102 | 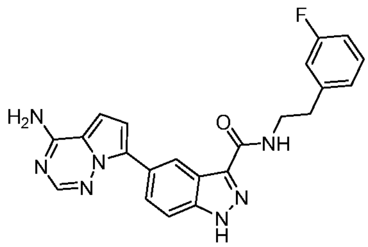 | $^1$H NMR (400 MHz, DMSO-$d_6$) δ 13.62 (s, 1H), 8.88 (s, 1H), 8.45 (s, 1H), 8.02 (d, *J* = 8.4 Hz, 1H), 7.94 (s, 1H), 7.74 (s, H), 7.66 (s, 1H), 7.34 (d, *J* = 6.7 Hz, 2H), 7.12 (d, *J* = 7.8 Hz, 2H), 7.02 (dd, *J* = 13.2, 4.5 Hz, 2H), 3.57 (d, *J* = 6.9 Hz, 3H), 2.98(d, *J* = 6.7 Hz, 2H). | 416.24 | Replacing iii Intermediate 5 with 4-amino-7-bromopyrrolo[2,1-f][1,2,4] triazine, Replacing Intermediate 1 with 5-bromoindazolo-3-formic acid, Replacing i(S)-1-(3-fluorophenyl) ethanamine with 3-fluorophenylethylamine | I |
| 103 | 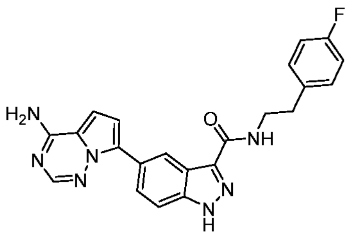 | $^1$H NMR (400 MHz, DMSO-$d_6$) δ 13.61 (s, 1H), 8.88 (s, 1H), 8.43 (s, 1H), 8.03 (d, *J* = 8.9 Hz, 1H), 7.94 (s, 1H), 7.74 (s, 2H), 7.67 (d, *J* = 8.9 Hz, 1H), 7.31 (dd, *J* = 8.3, 5.8 Hz, 2H), 7.15 - 6.98 (m, 4H), | 416.16 | Replacing iii Intermediate 5 with 4-amino-7-bromopyrrolo[2,1-f][1,2,4] triazine, Replacing Intermediate 1 with 5-bromoindazol | I |
| | | 3.54 (d, *J* = 6.7 Hz, 2H), 2.89 (d, *J* = 6.5 Hz, 2H). | | o-3-formic acid, Replacing i(S)-1-(3-fluorophenyl) ethanamine with 4-fluorophenylethylamine | |
| 104 | 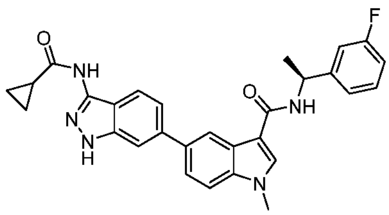 | $^1$H NMR (400 MHz, DMSO-$d_6$) δ 12.60 (s, 1H), 10.67 (s, 1H), 8.44 (s, 1H), 8.34 (d, *J* = 8.0 Hz, 1H), 8.19 (s, 1H), 7.85 (d, *J* = 8.4 Hz, 1H), 7.57 (d, *J* = 17.9 Hz, 3H), 7.37 (dd, *J* = 14.5, 7.9 Hz, 2H), 7.24 (t, *J* = 9.3 Hz, 2H), 7.04 (t, *J* = 7.5 Hz, 1H), 5.24 - 5.14 (m, 1H), 3.88 (s, 3H), 1.95 (s, 1H), 1.48 (d, *J* = 7.0 Hz, 3H), 0.92 (d, *J* = 5.5 Hz, 4H) | 496.21 | Replacing Intermediate 6 with 5-bromoindazolo-3-amine | II |

(continued)

| Compound No. | Structure | Structural characterization | ESI-MS | Synthetic raw material | Synthetic method |
|---|---|---|---|---|---|
| 105 | 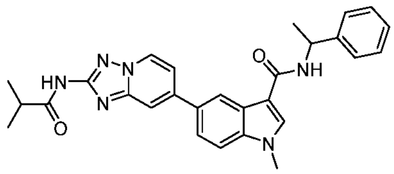 | $^1$H NMR (400 MHz, DMSO-$d_6$) δ 10.73 (s, 1H), 8.84 (d, *J* = 7.1 Hz, 1H), 8.54 (s, 1H), 8.36 (d, *J* = 8.1 Hz, 1H), 8.22 (s, 1H), 7.86 (s, 1H), 7.76 - 7.61 (m, 2H), 7.44 (dd, *J* = 10.3, 4.8 Hz, 3H), 7.33 (t, *J* = 7.6 Hz, 2H), 7.23 (d, *J* = 7.3 Hz, 1H), 5.25 - 5.16 (m, 1H), 3.90 (s, 3H), 2.00 (d, *J* = 8.0 Hz, 1H), 1.49 (d, *J* = 7.0 Hz, 3H), 1.10 (dd, *J* = 11.5, 6.9 Hz, 6H). | 481.23 | Replacing iii(S)-1-(3-fluoro phenyl) ethanami de with α-methylbenzyla mine, Replacing v cyclopropylform yl chloride with isopropylformyl chloride | II |
| 106 | 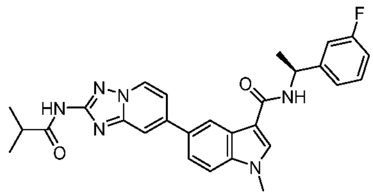 | $^1$H NMR (400 MHz, DMSO-$d_6$) δ 10.73 (s, 1H), 8.84 (d, J = 7.1 Hz, 1H), 8.54 (d, J = 1.9 Hz, 1H), 8.40 (d, J = 8.0 Hz, 1H), 8.23 (s, 1H), 7.95 - 7.78 (m, 1H), 7.78 - 7.59 (m, 2H), 7.50 - 7.31 (m, 2H), 7.33 - 7.18 (m, 2H), 7.05 (td, J = 8.7, 8.1, 2.7 Hz, 1H), 5.22 (q, J = 7.2 Hz, 1H), 3.90 (s, 3H), 2.79 (m, 1H), 1.49 (d, J = 7.1 Hz, 3H), 1.11 (d, J = 6.4 Hz, 6H). | 499.22 | Replacing v cyclopropylform yl chloride with isopropylformyl chloride | II |
| 107 | 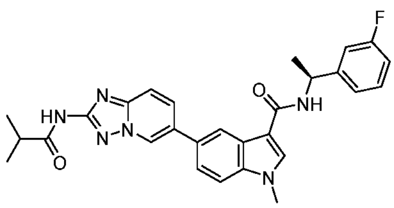 | $^1$H NMR (400 MHz, DMSO-de) δ 11.25 (s, 1H), 9.73 (dd, J = 1.4, 0.6 Hz, 1H), 7.96 (dd, J = 7.5, 1.5 Hz, 1H), 7.88 (t, J = 1.1 Hz, 1H), 7.82 (dd, J = 7.5, 1.5 Hz, 1H), 7.73 (d, J = 7.5 Hz, 1H), 7.63 (d, J = 7.5 Hz, 1H), 7.52 (m, 2H), 7.36 (td, J = 7.5, 5.0 Hz, 1H), 7.15 (ddq, J = 8.3, 4.2, 1.4 Hz, 2H), 7.07 - 7.00 (m, 1H), 5.16 (qt, J = 6.7, 1.0 Hz, 1H), 3.76 (s, 3H), 2.68 (hept, J = 6.7 Hz, 1H), 1.56 (d, J = 6.9 Hz, 3H), 1.09 (dd, J = 20.0, 6.8 Hz, 6H). | 499.22 | Replacing v cyclopropylform yl chloride with isopropylformyl chloride, Replacing Intermediate 6 with 6-bromo-[1,2,4]t riazolo[1,5-a]pyri din- 2-amine | II |

(continued)

| Compound No. | Structure | Structural characterization | ESI-MS | Synthetic raw material | Synthetic method |
|---|---|---|---|---|---|
| 108 | 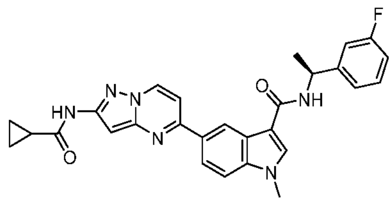 | $^1H$ NMR (400 MHz, DMSO-de) δ 11.17 (s, 1H), 8.94 (dd, J = 6.9, 1.3 Hz, 2H), 8.38 (d, J = 8.0 Hz, 1H), 8.21 (s, 1H), 8.15 - 8.07 (m, 1H), 7.64 (d, J = 8.8 Hz, 1H), 7.53 - 7.42 (m, 3H), 7.22 - 7.10 (m, 2H), 6.86 (s, 1H), 5.21 (t, J = 7.4 Hz, 1H), 3.90 (s, 3H), 1.95 (d, J = 4.7 Hz, 1H), 1.47 (d, J = 7.0 Hz, 3H), 0.90 - 0.80 (m, 4H). | 497.20 | Replacing Intermediate 6 with 2-amino-5-chloropyrazolo[1,5-a] pyrimidine | II |
| 109 | 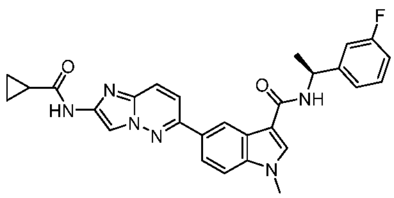 | $^1H$ NMR (400 MHz, DMSO-de) δ 11.16 (s, 1H), 8.76 (d, J = 1.8 Hz, 1H), 8.40 (d, J = 7.9 Hz, 1H), 8.24 (d, J = 9.6 Hz, 2H), 8.01 (d, J = 9.5 Hz, 1H), 7.92 (dd, J = 8.7, 1.9 Hz, 1H), 7.74 (d, J = 9.5 Hz, 1H), 7.66 (d, J = 8.7 Hz, 1H), 7.38 (td, J = 8.1, 6.2 Hz, 1H), 7.31 - 7.20 (m, 2H), 7.05 (td, J = 8.6, 8.0, 2.7 Hz, 1H), 5.21 (q, J = 7.3 Hz, 1H), 3.90 (s, 3H), 1.98 (m, 1H), 1.49 (d, J = 7.1 Hz, 3H), 0.94 - 0.77 (m, 4H). | 497.20 | Replacing Intermediate 6 with 2-amino-6-chloroimidazo[1,2-b]pyridazine | II |
| 110 | 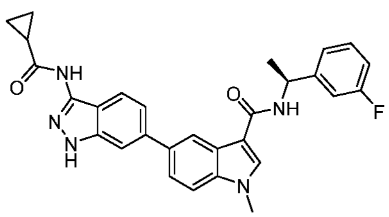 | $^1H$ NMR (400 MHz, DMSO-$d_6$) δ 12.60 (s, 1H), 10.67 (s, 1H), 8.44 (d, J = 1.6 Hz, 1H), 8.34 (d, J = 8.0 Hz, 1H), 8.19 (s, | 496.21 | Replacing Intermediate 6 with 5-bromoindazol o-3-amine | II |
| | | 1H), 7.85 (d, J = 8.6 Hz, 1H), 7.66 - 7.50 (m, 3H), 7.44 - 7.32 (m, 2H), 7.24 (t, J = 9.2 Hz, 2H), 7.09 - 6.99 (m, 1H), 5.21 (t, J = 7.4 Hz, 1H), 3.89 (s, 3H), 2.01 - 1.87 (m, 1H), 1.48 (d, J = 7.1 Hz, 3H), 0.91 - 0.76 (m, 4H)。 | | | |

(continued)

| Compound No. | Structure | Structural characterization | ESI-MS | Synthetic raw material | Synthetic method |
|---|---|---|---|---|---|
| 111 | 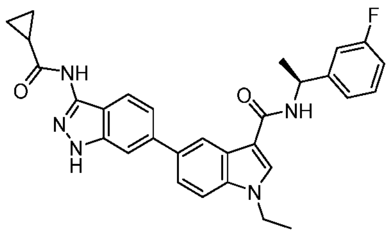 | $^1$H NMR (400 MHz, DMSO-$d_6$) δ 12.60 (s, 1H), 10.67 (s, 1H), 8.45 (d, J = 1.8 Hz, 1H), 8.38 - 8.18 (m, 2H), 7.85 (d, J = 8.5 Hz, 1H), 7.70 - 7.50 (m, 4H), 7.44 - 7.35 (m, 1H), 7.31 - 7.15 (m, 2H), 7.05 (ddd, J = 10.4, 8.3, 2.7 Hz, 1H), 5.21 (q, J = 7.1 Hz, 1H), 4.29 (q, J = 7.2 Hz, 2H), 1.95 (s, 1H), 1.57 - 1.37 (m, 6H), 0.96 - 0.62 (m, 4H). | 510.22 | Replacing Intermediate 6 with 5-bromoindazol o-3-amine, Replacing i methyl iodide with ethyl iodide | II |
| 112 | 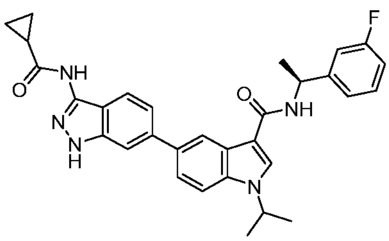 | $^1$H NMR (400 MHz, DMSO-$d_6$) δ 12.60 (s, 1H), 10.67 (s, 1H), 8.48 (s, 1H), 8.40 (s, 1H), 8.31 (d, J = 7.9 Hz, 1H), 7.85 (d, J = 8.6 Hz, 1H), 7.68 (d, J = 8.6 Hz, 1H), 7.57 (d, J = 10.1 Hz, 2H), 7.38 (dt, J = 16.2, 8.1 Hz, 2H), 7.26 (t, J = 10.4 Hz, 2H), 7.11 - 6.97 (m, 1H), 5.29 - 5.13 (m, 1H), 4.86 (m, 1H), 2.10 - 1.86 (m, 1H), | 524.24 | Replacing Intermediate 6 with 5-bromoindazol o-3-amine, Replacing i methyl iodide with 2-iodopropane | II |
| | | 1.52 (dd, J = 14.8, 6.9 Hz, 9H), 0.86 (d, J = 7.3 Hz, 4H). | | | |
| 113 | 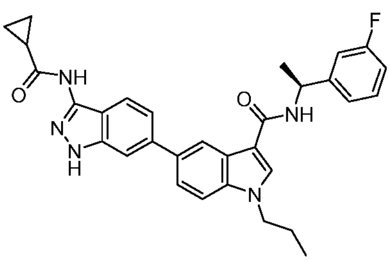 | $^1$H NMR (400 MHz, DMSO-$d_6$) δ 12.60 (s, 1H), 10.66 (s, 1H), 8.45 (d, J = 1.7 Hz, 1H), 8.33 (d, J = 7.9 Hz, 1H), 8.24 (s, 1H), 7.84 (d, J = 8.5 Hz, 1H), 7.65 (d, J = 8.6 Hz, 1H), 7.60 - 7.51 (m, 2H), 7.43 - 7.30 (m, 2H), 7.25 (t, J = 9.5 Hz, 2H), 7.05 (t, J = 8.5 Hz, 1H), 5.30 - 5.03 (m, 1H), 4.23 (t, J = 7.0 Hz, 2H), 2.04 - 1.92 (m, 2H), 1.86 (q, J = 7.1 Hz, 1H), 1.49 (d, J = 7.0 Hz, 3H), 0.95 - 0.83 (m, 7H). | 524.24 | Replacing Intermediate 6 with 5-bromoindazol o-3-amine, Replacing i methyl iodide with 1-iodopropane | II |

(continued)

| Compound No. | Structure | Structural characterization | ESI-MS | Synthetic raw material | Synthetic method |
|---|---|---|---|---|---|
| 114 | 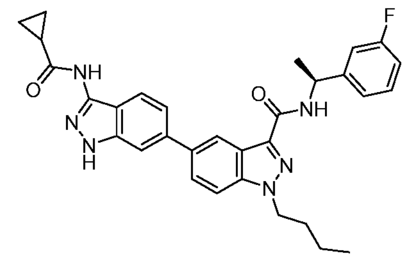 | $^1$H NMR (400 MHz, DMSO-$d_6$) δ 12.60 (s, 1H), 10.67 (s, 1H), 8.45 (s, 1H), 8.33 (d, J = 7.9 Hz, 1H), 8.24 (s, 1H), 7.85 (d, J = 8.6 Hz, 1H), 7.72 - 7.51 (m, 3H), 7.37 (dt, J = 13.3, 7.6 Hz, 2H), 7.25 (t, J = 9.4 Hz, 1H), 7.05 (t, J = 8.4 Hz, 1H), 5.33 - 5.13 (m, 1H), 4.26 (d, J = 7.3 Hz, 2H), 1.95 (m, 1H), 1.81 (q, J = 7.4 Hz, 2H), 1.49 (d, J = 7.1 Hz, 3H), 1.32 (p, J = 7.5 Hz, 2H), 0.93 (t, J = 7.3 Hz, 3H), 0.88 - 0.83 (m, 4H). | 539.25 | Replacing Intermediate 6 with 5-bromoindazol o-3-amine, Replacing i methyl iodide with 1-iodobutane, Replacing Intermediate 3 with 5-bromoindazol o-3- carboxylic acid | II |
| 115 | 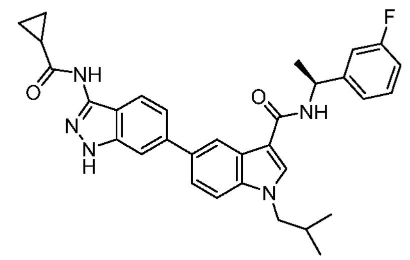 | $^1$H NMR (400 MHz, DMSO-$d_6$) δ 12.60 (s, 1H), 10.67 (s, 1H), 8.45 (d, J = 1.7 Hz, 1H), 8.35 (d, J = 8.0 Hz, 1H), 8.22 (s, 1H), 7.85 (d, J = 8.6 Hz, 2H), 7.66 (d, J = 8.6 Hz, 1H), 7.61 - 7.50 (m, 2H), 7.42 - 7.32 (m, 2H), 7.25 (t, J = 9.7 Hz, 2H), 7.05 (t, J = 8.8 Hz, 1H), 5.27 - 5.08 (m, 1H), 4.11 - 4.06 (m, 2H), 2.22 - 2.09 (m, 1H), 1.50 (d, J = 7.1 Hz, 3H), 0.93 (d, J = 6.6 Hz, 6H), 0.86 (m, 4H). | 538.25 | Replacing Intermediate 6 with 5-bromoindazol o-3-amine, Replacing i methyl iodide with isoamyl iodide | II |
| 116 | 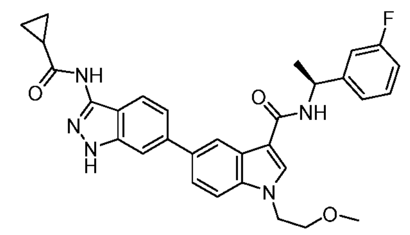 | $^1$H NMR (400 MHz, DMSO-$d_6$) δ 12.60 (s, 1H), 10.66 (s, 1H), 8.45 (s, 1H), 8.38 (d, J = 8.0 Hz, 1H), 8.21 (s, 1H), 7.84 (d, J = 8.4 Hz, 1H), 7.66 (d, J = 8.6 Hz, 1H), 7.62 - 7.52 (m, 2H), 7.43 - 7.30 (m, 2H), 7.25 (t, J = 9.5 Hz, 2H), 7.05 (t, J = 8.5 Hz, 1H), 5.30 - 5.05 (m, 1H), 4.43 (s, 2H), 3.73 (t, J = 5.1 Hz, 2H), 3.26 (s, 3H), 1.49 (d, J = 7.1 Hz, 3H), 0.84 (m, 5H). | 540.23 | Replacing Intermediate 6 with 5-bromoindazol o-3-amine, Replacing i methyl iodide with 1-iodo-2-methox y-ethane | II |

(continued)

| Compound No. | Structure | Structural characterization | ESI-MS | Synthetic raw material | Synthetic method |
|---|---|---|---|---|---|
| 117 | 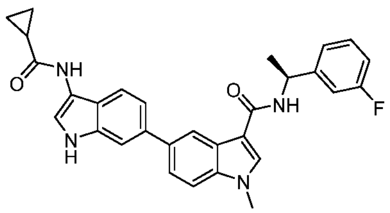 | $^1$H NMR (400 MHz, DMSO-de) δ 12.55 (s, 1H), 10.63 (s, 1H), 7.96 (d, J = 7.5 Hz, 2H), 7.92 (dt, J = | 495.21 | Replacing Intermediate 6 with 5-bromoindolo -3-amine | II |
| | | 1.3, 0.6 Hz, 1H), 7.79 - 7.72 (m, 3H), 7.65 (dd, J = 7.5, 1.6 Hz, 1H), 7.60 (d, J = 1.5 Hz, 1H), 7.52 (s, 1H), 7.36 (td, J = 7.5, 5.0 Hz, 1H), 7.15 (ddq, J = 8.2, 4.1, 1.4 Hz, 2H), 7.06 - 6.97 (m, 1H), 5.16 (qt, J = 6.7, 1.0 Hz, 1H), 3.77 (s, 3H), 2.08 (p, J = 7.0 Hz, 1H), 1.56 (d, J = 6.9 Hz, 3H), 1.01 - 0.89 (m, 4H)o | | | |
| 118 | 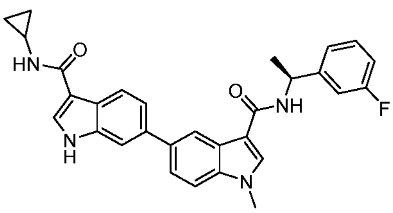 | $^1$H NMR (400 MHz, DMSO-de) δ 11.49 (d, J = 2.9 Hz, 1H), 8.41 (s, 1H), 8.32 (d, J = 8.0 Hz, 1H), 8.17 (d, J = 8.0 Hz, 2H), 7.98 (d, J = 2.9 Hz, 1H), 7.90 (d, J = 3.8 Hz, 1H), 7.63 (d, J = 1.6 Hz, 1H), 7.56 (d, J = 2.1 Hz, 2H), 7.47 - 7.32 (m, 2H), 7.31 - 7.19 (m, 2H), 7.04 (td, J = 8.6, 8.1, 2.5 Hz, 1H), 5.21 (t, J = 7.4 Hz, 1H), 3.88 (s, 3H), 1.99 (s, 1H), 1.48 (d, J = 7.1 Hz, 3H), 0.68 (dt, J = 6.9, 3.3 Hz, 2H), 0.63 - 0.45 (m, 2H). | 495.21 | Replacing Intermediate 6 with 5-bromoindolo-3 -carboxylic acid | II |
| 119 | 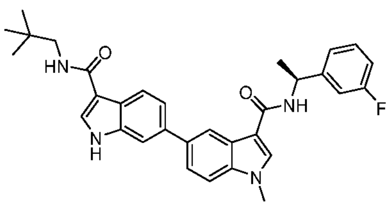 | $^1$H NMR (400 MHz, DMSO-de) δ 11.52 (d, J = 2.9 Hz, 1H), 8.42 (s, 1H), 8.32 (d, J = 8.0 Hz, 1H), 8.22 - 8.13 (m, 2H), 8.10 (d, J = 2.8 Hz, 1H), | 525.26 | Replacing Intermediate 6 with 5-bromoindolo-3 -carboxylic acid | II |

(continued)

| Compound No. | Structure | Structural characterization | ESI-MS | Synthetic raw material | Synthetic method |
|---|---|---|---|---|---|
| | | 7.75 (t, J = 6.4 Hz, 1H), 7.64 (d, J = 1.6 Hz, 1H), 7.56 (d, J = 2.1 Hz, 2H), 7.46 - 7.32 (m, 2H), 7.30 - 7.22 (m, 2H), 7.04 (ddd, J = 10.3, 8.2, 2.7 Hz, 1H), 5.21 (t, J = 7.4 Hz, 1H), 3.12 (d, J = 6.3 Hz, 3H), 1.99 (s, 2H), 1.48 (d, J = 7.1 Hz, 3H), 0.93 (s, 9H). | | | |
| 120 | 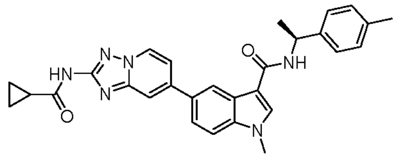 | $^1$H NMR (400 MHz, DMSO-$d_6$) δ 11.15 (s, 1H), 8.92 (d, J = 7.1 Hz, 1H), 8.62 (d, J = 1.9 Hz, 1H), 8.38 (d, J = 8.1 Hz, 1H), 8.29 (s, 1H), 7.95 (d, J = 2.0 Hz, 1H), 7.85 - 7.67 (m, 2H), 7.52 (dd, J = 7.1, 2.0 Hz, 1H), 7.38 (d, J = 7.8 Hz, 2H), 7.21 (d, J = 7.8 Hz, 2H), 5.25 (p, J = 7.2 Hz, 1H), 3.97 (s, 3H), 2.35 (s, 3H), 2.19 - 2.10 (m, 1H), 1.55 (d, J = 7.0 Hz, 3H), 0.92 (h, J = 4.8, 4.0 Hz, 4H). | 493.23 | Replacing iii(S)-1-(3-fluoro phenyl) ethanami ne with (S)-1-(4-methylphenyl)et hylamine | II |
| 121 | 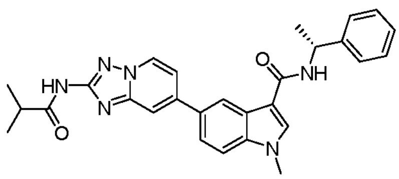 | $^1$H NMR (400 MHz, DMSO-$d_6$) δ 10.73 (s, 1H), 8.84 (d, J = 7.1 Hz, 1H), 8.54 (d, J = 1.8 Hz, 1H), 8.36 (d, J = 8.1 Hz, 1H), 8.22 (s, 1H), 7.92 - 7.84 (m, 1H), 7.72 (dd, J = 8.8, 1.8 Hz, 1H), 7.65 (d, J = 8.6 Hz, 1H), 7.50 - 7.37 (m, 3H), 7.33 (t, J = | 481.23 | Replacing iii(S)-1-(3-fluoro phenyl) ethanami ne with (R)-1-phenylethylamine, Replacing v cyclopropylform yl chloride with isopropylformyl chloride | II |
| | | 7.5 Hz, 2H), 7.22 (t, J = 7.3 Hz, 1H), 5.38 - 5.11 (t, 1H), 3.90 (s, 3H), 2.78 (s, 1H), 1.49 (d, J = 7.0 Hz, 3H), 1.10 (d, J = 5.8 Hz, 6H). | | | |

(continued)

| Compound No. | Structure | Structural characterization | ESI-MS | Synthetic raw material | Synthetic method |
|---|---|---|---|---|---|
| 122 | 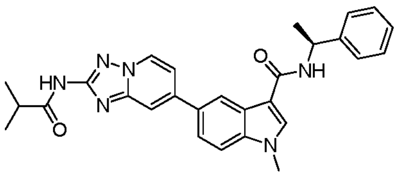 | $^1H$ NMR (400 MHz, DMSO-$d_6$) δ 10.74 (s, 1H), 8.85 (d, J = 7.2 Hz, 1H), 8.55 (s, 1H), 8.36 (d, J = 8.1 Hz, 1H), 8.23 (s, 1H), 7.87 (s, 1H), 7.80 - 7.59 (m, 2H), 7.44 (t, J = 6.6 Hz, 3H), 7.34 (t, J = 7.5 Hz, 2H), 7.22 (t, J = 7.3 Hz, 1H), 5.60 - 5.04 (t, 1H), 3.90 (s, 3H), 2.80 (m, 1H), 1.50 (d, J = 7.1 Hz, 3H), 1.11 (d, J = 6.6 Hz, 6H). | 481.23 | Replacing iii(S)-1-(3-fluoro phenyl) ethanami ne with (S)-1-phenylethy lamine, Replacing v cyclopropylform yl chloride with isopropylformyl chloride | II |
| 123 | 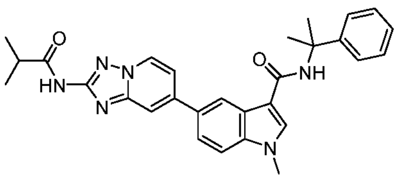 | $^1H$ NMR (400 MHz, DMSO-$d_6$) δ 10.71 (s, 1H), 8.80 (d, J = 7.1 Hz, 1H), 8.45 (d, J = 1.9 Hz, 1H), 8.31 (s, 1H), 7.97 (s, 1H), 7.83 (d, J = 2.0 Hz, 1H), 7.78 - 7.60 (m, 2H), 7.42 (td, J = 6.3, 5.2, 1.7 Hz, 3H), 7.28 (t, J = 7.6 Hz, 2H), 7.16 (t, J = 7.3 Hz, 1H), 3.90 (s, 3H), 2.78 (s, 1H), 1.70 (s, 6H), 1.10 (d, J = 6.8 Hz, 6H). | 495.24 | Replacing iii(S)-1-(3-fluoro phenyl) ethanami ne with A,A-dimethylben zylamine, Replacing v cyclopropylform yl chloride with isopropylformyl chloride | II |
| 124 | 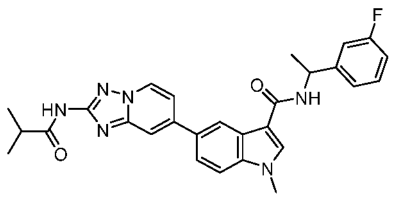 | $^1H$ NMR (400 MHz, DMSO-$d_6$) δ 10.73 (s, 1H), 8.84 (d, J = 7.1 Hz, 1H), 8.53 (d, J = 2.3 Hz, 1H), 8.39 (d, J = 7.9 Hz, 1H), 8.23 (d, J = 2.1 Hz, 1H), 7.86 (s, 1H), 7.80 - 7.58 (m, 2H), 7.48 - 7.33 (m, 2H), 7.25 (t, J = 9.3 Hz, 2H), 7.15 - 6.84 (m, 1H), 5.38 - 5.09 (m, 1H), 3.90 (d, J = 2.1 Hz, 3H), 2.79 (s, 1H), 1.49 (dd, J = 7.1, 2.0 Hz, 3H), 1.11 (dd, J = 6.9, 2.2 Hz, 6H). | 499.22 | Replacing iii(S)-1-(3-fluoro phenyl) ethanami ne with 1-(3-fluoropheny l) ethanamine, Replacing v cyclopropylform yl chloride with isopropylformyl chloride | II |

(continued)

| Compound No. | Structure | Structural characterization | ESI-MS | Synthetic raw material | Synthetic method |
|---|---|---|---|---|---|
| 125 | 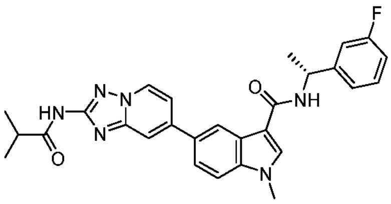 | $^1$H NMR (400 MHz, DMSO-$d_6$) δ 10.73 (s, 1H), 8.85 (d, J = 7.0 Hz, 1H), 8.54 (d, J = 1.9 Hz, 1H), 8.39 (d, J = 8.0 Hz, 1H), 8.23 (s, 1H), 7.95 - 7.79 (m, 1H), 7.79 - 7.57 (m, 2H), 7.51 - 7.33 (m, 2H), 7.25 (t, J = 9.5 Hz, 2H), 7.09 - 6.93 (m, 1H), 5.22 (q, J = 7.1 Hz, 1H), 3.91 (s, 3H), 2.79 (m, 1H), 1.50 (d, J = 7.1 Hz, 3H), 1.12 (d, J = 6.7 Hz, 6H). | 499.56 | Replacing iii(S)-1-(3-fluoro phenyl) ethanami ne with (R)-1-(3-fluorop henyl)ethanamin e, Replacing v cyclopropylform yl chloride with isopropylformyl chloride | II |
| 126 | 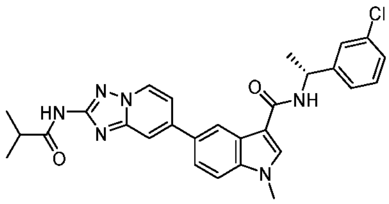 | $^1$H NMR (400 MHz, DMSO-$d_6$) δ 10.74 (s, 1H), 8.85 (d, J = 7.1 Hz, 1H), 8.53 (d, J = 1.8 Hz, 1H), 8.43 (d, J = 7.9 Hz, 1H), 8.24 (s, 1H), 7.87 (d, J = 2.0 Hz, 1H), 7.72 | 515.01 | Replacing iii(S)-1-(3-fluoro phenyl) ethanami ne with (R)-1-(3-chlorop henyl)ethylamin e, Replacing v cyclopropylform | II |
| | | (d, J = 1.9 Hz, 1H), 7.66 (d, J = 8.6 Hz, 1H), 7.51 - 7.42 (m, 2H), 7.41 - 7.32 (m, 2H), 7.29 (dt, J = 6.8, 2.2 Hz, 1H), 5.20 (t, J = 7.3 Hz, 1H), 3.91 (s, 3H), 2.79 (m, 1H), 1.49 (d, J = 7.1 Hz, 3H), 1.12 (d, J = 6.7 Hz, 6H). | | yl chloride with isopropylformyl chloride | |
| 127 | 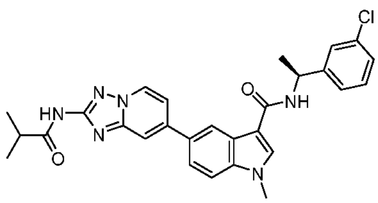 | $^1$H NMR (400 MHz, DMSO-$d_6$) δ 10.73 (s, 1H), 8.85 (d, J = 7.0 Hz, 1H), 8.53 (d, J = 1.8 Hz, 1H), 8.41 (d, J = 7.9 Hz, 1H), 8.23 (s, 1H), 7.97 - 7.80 (m, 1H), 7.73 (dd, J = 8.7, 1.9 Hz, 1H), 7.66 (d, J = 8.6 Hz, 1H), 7.52 - 7.42 (m, 2H), 7.41 - 7.32 (m, 2H), 7.29 (dt, J = 6.6, 2.3 Hz, 1H), 5.20 (q, J = 7.2 Hz, 1H), 3.89 (s, 3H), 2.79 (m, 1H), 1.49 (d, J = 7.1 Hz, 3H), 1.11 (d, J = 6.9, 5.0 Hz, 6H). | 515.01 | Replacing iii(S)-1-(3-fluoro phenyl) ethanami ne with (S)-1-(3-chlorop henyl)ethylamin e, Replacing v cyclopropylform yl chloride with isopropylformyl chloride | II |

(continued)

| Compound No. | Structure | Structural characterization | ESI-MS | Synthetic raw material | Synthetic method |
|---|---|---|---|---|---|
| 128 | 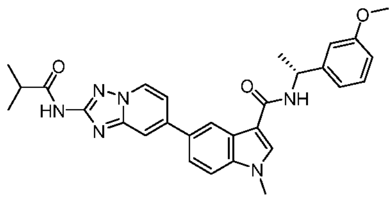 | $^1$H NMR (400 MHz, DMSO-$d_6$) δ 10.73 (s, 1H), 8.85 (d, J = 7.1 Hz, 1H), 8.54 (d, J = 1.9 Hz, 1H), 8.34 (d, J = 8.1 Hz, 1H), 8.22 (s, 1H), 7.87 (d, J = 1.9 Hz, 1H), 7.80 - 7.55 (m, 2H), 7.45 (dd, J = 7.1, 2.0 Hz, 1H), 7.25 (t, J = 8.1 Hz, 1H), 7.10 - 6.92 (m, 2H), 6.91 - 6.73 | 511.24 | Replacing iii(S)-1-(3-fluoro phenyl) ethanami ne with (R)-1-(3-methox yphenyl)ethylam ine , Replacing v cyclopropylform yl chloride with isopropylformyl chloride | II |
| | | (m, 1H), 5.33 - 4.96 (q, 1H), 3.90 (s, 3H), 3.75 (s, 3H), 2.79 (m, 1H), 1.48 (d, J = 7.1 Hz, 3H), 1.12 (d, J = 6.8 Hz, 6H). | | | |
| 129 | 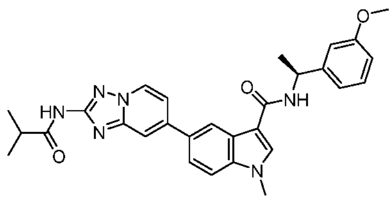 | $^1$H NMR (400 MHz, DMSO-$d_6$) δ 10.70 (s, 1H), 8.84 (t, J = 6.4 Hz, 1H), 8.54 (d, J = 1.9 Hz, 1H), 8.33 (d, J = 8.1 Hz, 1H), 8.22 (s, 1H), 7.86 (d, J = 2.0 Hz, 1H), 7.80 - 7.54 (m, 2H), 7.44 (dd, J = 7.2, 2.0 Hz, 1H), 7.24 (t, J = 8.0 Hz, 1H), 7.01 - 6.92 (m, 2H), 6.88 - 6.70 (m, 1H), 5.19 (q, J = 7.4 Hz, 1H), 3.90 (s, 3H), 3.74 (s, 3H), 2.79 (s, 1H), 1.48 (d, J = 7.0 Hz, 3H), 1.11 (t, J = 5.9 Hz, 6H). | 511.24 | Replacing iii(S)-1-(3-fluoro phenyl) ethanami ne with (S)-1-(3-methox yphenyl)ethylam ine , Replacing v cyclopropylform yl chloride with isopropylformyl chloride | II |
| 130 | 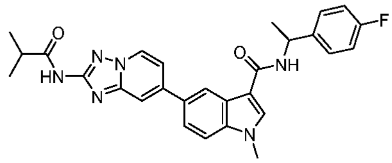 | $^1$H NMR (400 MHz, DMSO-$d_6$) δ 10.73 (s, 1H), 8.85 (d, J = 7.1 Hz, 1H), 8.57 - 8.52 (m, 1H), 8.35 (t, J = 7.6 Hz, 1H), 8.20 (d, J = 7.3 Hz, 1H), 7.86 (s, 1H), 7.77 - 7.69 (m, 1H), 7.65 (d, J = 8.7 Hz, 1H), 7.45 (dd, J = 8.8, 5.7 Hz, 3H), 7.16 (q, J = 8.4 Hz, 2H), 5.21 (t, J = 7.4 Hz, 1H), 3.89 (d, J = 4.7 Hz, 3H), 2.79 (m, 1H), 1.48 (d, J = 7.1 Hz, 3H), 1.11 (d, J = 6.0 Hz, 6H). | 499.22 | Replacing iii(S)-1-(3-fluoro phenyl) ethanami ne with 1-(4-fluoropheny l) ethanamine, Replacing v cyclopropylform yl chloride with isopropylformyl chloride | II |

(continued)

| Compound No. | Structure | Structural characterization | ESI-MS | Synthetic raw material | Synthetic method |
|---|---|---|---|---|---|
| 131 | 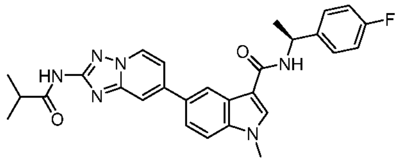 | $^1$H NMR (400 MHz, DMSO-$d_6$) δ 10.73 (s, 1H), 8.85 (d, J = 7.2 Hz, 1H), 8.53 (s, 1H), 8.36 (d, J = 8.1 Hz, 1H), 8.21 (s, 1H), 7.86 (s, 1H), 7.80 - 7.54 (m, 2H), 7.45 (dd, J = 8.7, 5.7 Hz, 3H), 7.15 (t, J = 8.8 Hz, 2H), 5.19 (q, J = 7.3 Hz, 1H), 3.90 (s, 3H), 2.76 (m, 1H), 1.48 (d, J = 7.0 Hz, 3H), 1.11 (d, J = 6.7 Hz, 6H). | 499.22 | Replacing iii(S)-1-(3-fluoro phenyl) ethanami ne with (S)-1-(4-fluorophenyl)ethanamine, Replacing v cyclopropylform yl chloride with isopropylformyl chloride | II |
| 132 | 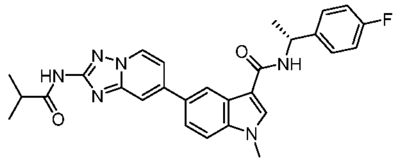 | $^1$H NMR (400 MHz, DMSO-$d_6$) δ 10.74 (s, 1H), 8.85 (d, J = 7.1 Hz, 1H), 8.54 (d, J = 1.9 Hz, 1H), 8.37 (d, J = 8.0 Hz, 1H), 8.21 (s, 1H), 7.86 (d, J = 1.9 Hz, 1H), 7.79 - 7.60 (m, 2H), 7.46 (dd, J = 8.7, 5.8 Hz, 3H), 7.16 (dd, J = 10.1, 7.7 Hz, 2H), 5.21 (q, J = 7.4 Hz, 1H), 3.90 (s, 3H), 2.80 (m, 1H), 1.49 (d, J = 7.0 Hz, 3H), 1.12 (d, J = 6.7 Hz, 6H). | 499.22 | Replacing iii(S)-1-(3-fluoro phenyl) ethanami ne with (R)-1-(4-fluorophenyl)ethanamine, Replacing v cyclopropylform yl chloride with isopropylformyl chloride | II |
| 133 | 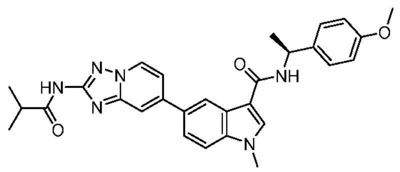 | $^1$H NMR (400 MHz, DMSO-$d_6$) δ 10.74 (s, 1H), 8.85 (d, J = 7.1 Hz, 1H), 8.55 (d, J = 1.8 Hz, 1H), 8.28 (d, J = 8.1 Hz, 1H), 8.20 (s, 1H), 7.87 (d, J = 1.9 Hz, 1H), 7.78 - 7.57 (m, 2H), 7.45 (dd, J = 7.1, 2.0 Hz, | 511.24 | Replacing iii(S)-1-(3-fluoro phenyl) ethanami ne with (S)-1-(4-methoxyphenyl)ethylamine , Replacing v cyclopropylform yl chloride with isopropylformyl | II |
| | | 1H), 7.39 - 7.26 (m, 2H), 6.98 - 6.74 (m, 2H), 5.17 (q, J = 7.2 Hz, 1H), 3.89 (s, 3H), 3.73 (s, 3H), 2.74 (m, 1H), 1.47 (d, J = 7.0 Hz, 3H), 1.12 (d, J = 6.7 Hz, 6H). | | chloride | |

(continued)

| Compound No. | Structure | Structural characterization | ESI-MS | Synthetic raw material | Synthetic method |
|---|---|---|---|---|---|
| 134 | 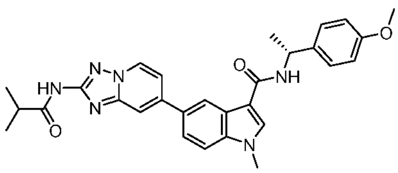 | $^1H$ NMR (400 MHz, DMSO-$d_6$) δ 10.73 (s, 1H), 8.85 (d, J = 7.1 Hz, 1H), 8.54 (s, 1H), 8.36 - 8.07 (m, 2H), 7.86 (s, 1H), 7.78 - 7.60 (m, 2H), 7.44 (d, J = 7.1 Hz, 1H), 7.34 (d, J = 8.3 Hz, 2H), 6.89 (d, J = 8.3 Hz, 2H), 5.32 - 4.99 (m, 1H), 3.89 (s, 3H), 3.72 (s, 3H), 2.79 (m, 1H), 1.47 (d, J = 7.1 Hz, 3H), 1.11 (d, J = 6.7 Hz, 6H). | 511.24 | Replacing iii(S)-1-(3-fluoro phenyl) ethanami ne with (R)-1-(4-methox yphenyl)ethylam ine , Replacing v cyclopropylform yl chloride with isopropylformyl chloride | II |
| 135 | 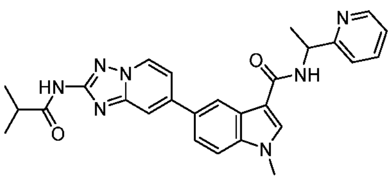 | $^1H$ NMR (400 MHz, DMSO-$d_6$) δ 10.74 (s, 1H), 8.86 (d, J = 7.1 Hz, 1H), 8.65 - 8.50 (m, 2H), 8.38 (d, J = 7.8 Hz, 1H), 8.27 (s, 1H), 7.88 (d, J = 2.0 Hz, 1H), 7.76 (ddd, J = 16.0, 8.0, 1.9 Hz, 2H), 7.67 (d, J = 8.6 Hz, 1H), 7.46 (dd, J = 7.2, 1.9 Hz, 2H), 7.26 (dd, J = 7.5, 4.9 Hz, 1H), 5.25 (p, J = 7.1 Hz, 1H), 3.90 (d, J = 3.9 Hz, 3H), 2.80 (s, 1H), 1.53 (d, J = 7.0 | 482.22 | Replacing iii(S)-1-(3-fluoro phenyl) ethanami ne with 1-(2-pyridine)eth anamine, Replacing v cyclopropylform yl chloride with isopropylformyl chloride | II |
| | | Hz, 3H), 1.12 (d, J = 6.8 Hz, 6H). | | | |

(continued)

| Compound No. | Structure | Structural characterization | ESI-MS | Synthetic raw material | Synthetic method |
|---|---|---|---|---|---|
| 136 | 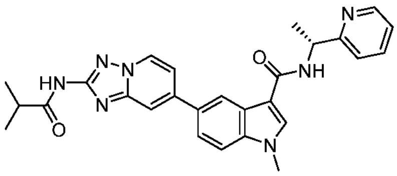 | $^{1}H$ NMR (400 MHz, DMSO-$d_6$) δ 10.74 (s, 1H), 8.86 (d, J = 7.1 Hz, 1H), 8.55 (dd, J = 5.6, 1.9 Hz, 2H), 8.38 (d, J = 7.8 Hz, 1H), 8.27 (s, 1H), 8.00 - 7.84 (m, 1H), 7.82 - 7.63 (m, 3H), 7.51 - 7.39 (m, 2H), 7.35 - 7.23 (m, 1H), 5.25 (t, J = 7.1 Hz, 1H), 3.91 (s, 3H), 2.80 (m, 1H), 1.53 (d, J = 7.1 Hz, 3H), 1.12 (d, J = 6.8 Hz, 6H); $^{13}C$ NMR (101 MHz, DMSO-$d_6$) δ 163.99, 163.85, 149.15, 143.82, 137.54, 137.21, 133.80, 130.41, 129.00, 127.54, 122.43, 121.76, 120.77, 120.24, 113.25, 111.61, 110.69, 110.45, 50.03, 33.62, 21.71, 19.79. | 482.22 | Replacing iii(S)-1-(3-fluoro phenyl) ethanami ne with (R)-1-(2-pyridine ) ethanamine, Replacing v cyclopropylform yl chloride with isopropylformyl chloride | II |
| 137 | 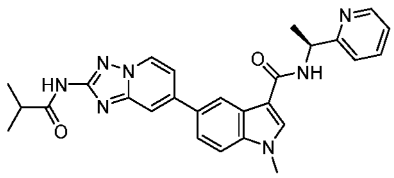 | $^{1}H$ NMR (400 MHz, DMSO-$d_6$) δ 10.73 (s, 1H), 8.86 (d, J = 7.1 Hz, 1H), 8.55 (d, J = 5.3 Hz, 2H), 8.37 (t, J = 7.8 Hz, 1H), 8.26 (d, J = 7.5 Hz, 1H), 7.87 (s, 1H), 7.82 - 7.60 (m, 3H), 7.45 (d, J = 7.7 Hz, 2H), 7.31 - 7.19 (m, 1H), 5.25 (q, J = 7.3 Hz, 1H), 3.91 (s, | 482.22 | Replacing iii(S)-1-(3-fluoro phenyl) ethanami ne with (S)-1-(2-pyridine ) ethanamine, Replacing v cyclopropylform yl chloride with isopropylformyl chloride | II |
| | | 3H), 2.79 (s, 1H), 1.52 (d, J = 7.0 Hz, 3H), 1.11 (d, J = 6.7 Hz, 6H). | | | |
| 138 | 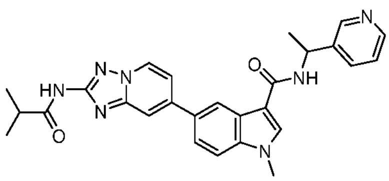 | $^{1}H$ NMR (400 MHz, DMSO-$d_6$) δ 10.81 (s, 1H), 8.86 (d, J = 7.0 Hz, 1H), 8.66 (s, 1H), 8.50 (d, J = 31.7 Hz, 3H), 8.24 (s, 1H), 8.02 - 7.78 (m, 2H), 7.78 - 7.58 (m, 2H), 7.52 - 7.28 (m, 2H), 5.24 (s, 1H), 3.90 (s, 3H), 2.80 (s, 1H), 1.53 (d, J = 7.2 Hz, 3H), 1.12 (d, J = 6.6 Hz, 6H). | 482.22 | Replacing iii(S)-1-(3-fluoro phenyl) ethanami ne with 1-(3-pyridine)eth anamine, Replacing v cyclopropylform yl chloride with isopropylformyl chloride | II |

(continued)

| Compound No. | Structure | Structural characterization | ESI-MS | Synthetic raw material | Synthetic method |
|---|---|---|---|---|---|
| 139 | 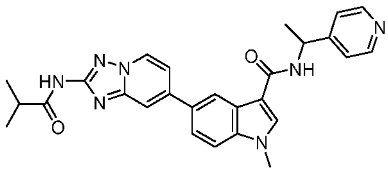 | $^1$H NMR (400 MHz, DMSO-$d_6$) δ 10.82 (s, 1H), 8.86 (d, J = 7.1 Hz, 1H), 8.63 - 8.38 (m, 4H), 8.28 (s, 1H), 7.89 (d, J = 2.0 Hz, 1H), 7.76 - 7.59 (m, 2H), 7.53 - 7.08 (m, 3H), 5.17 (q, J = 7.2 Hz, 1H), 3.91 (s, 3H), 2.80 (s, 1H), 1.50 (d, J = 7.1 Hz, 3H), 1.20 - 1.03 (d, 6H). | 482.22 | Replacing iii(S)-1-(3-fluoro phenyl) ethanami ne with 1-(4-pyridine)eth anamine, Replacing v cyclopropylform yl chloride with isopropylformyl chloride | II |
| 140 | 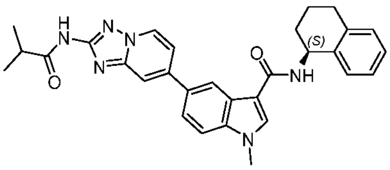 | $^1$H NMR (400 MHz, DMSO-$d_6$) δ 10.74 (s, 1H), 8.87 (d, J = 7.1 Hz, 1H), 8.65 (d, J = 1.9 Hz, 1H), 8.31 (d, J = 8.6 Hz, 1H), 8.17 (s, 1H), 8.00 - 7.82 (m, 1H), 7.82 - 7.59 (m, 2H), 7.48 (dd, J = 7.2, 2.0 Hz, 1H), 7.35 - 7.21 (m, 1H), 7.15 (qd, J = | 507.24 | Replacing iii(S)-1-(3-fluoro phenyl) ethanami ne with (S)-1,2,3,4-tetrahydro-1-nap hthylamine, Replacing v cyclopropylform yl chloride with isopropylformyl chloride | II |
| | | 8.1, 6.9, 3.7 Hz, 3H), 5.43 - 5.20 (m, 1H), 3.87 (s, 3H), 2.88 - 2.73 (m, 3H), 2.00 (d, J = 5.8 Hz, 2H), 1.90 - 1.77 (m, 2H), 1.12 (d, J = 6.8 Hz, 6H). | | | |
| 141 | 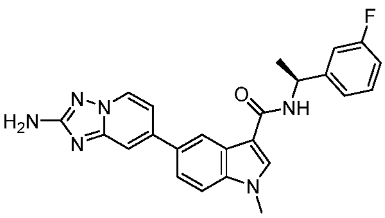 | $^1$H NMR (400 MHz, DMSO-$d_6$) δ 8.55 (d, J = 7.0 Hz, 1H), 8.48 (d, J = 1.8 Hz, 1H), 8.37 (d, J = 8.0 Hz, 1H), 8.21 (s, 1H), 7.73 - 7.60 (m, 2H), 7.55 (d, J = 2.0 Hz, 1H), 7.37 (q, J = 7.4 Hz, 1H), 7.32 - 7.12 (m, 3H), 7.04 (td, J = 8.7, 2.6 Hz, 1H), 5.99 (s, 2H), 5.20 (q, J = 7.3 Hz, 1H), 3.89 (s, 3H), 1.49 (d, J = 7.1 Hz, 3H); | 429.18 | removing v cyclopropylform yl chloride | II |

(continued)

| Compound No. | Structure | Structural characterization | ESI-MS | Synthetic raw material | Synthetic method |
|---|---|---|---|---|---|
| 142 | 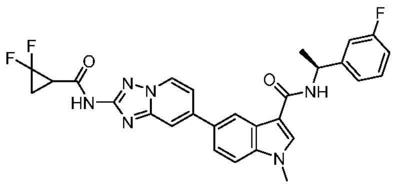 | $^1$H NMR (400 MHz, DMSO-de) δ 11.41 (s, 1H), 8.93 (d, J = 7.0 Hz, 1H), 8.60 (d, J = 1.8 Hz, 1H), 8.45 (d, J = 8.1 Hz, 1H), 8.29 (s, 1H), 7.96 (d, J = 1.9 Hz, 1H), 7.80 (dd, J = 8.6, 1.9 Hz, 1H), 7.72 (d, J = 8.7 Hz, 1H), 7.53 (dd, J = 7.2, 2.0 Hz, 1H), 7.46 - 7.39 (m, 1H), 7.35 - 7.28 (m, 2H), 7.15 - 7.05 (m, 1H), 5.27 (t, J = 7.4 Hz, 1H), 3.95 (d, J = 13.0 Hz, 3H), 2.10 (d, J = 7.1 Hz, 1H), 1.55 (d, J = 7.0 Hz, 3H), 1.31 | 533.18 | Replacing i cyclopropylform yl chloride with 2,2-di fluoro cyclopropylcarb oxylic acid | III |
| | | - 1.18 (m, 2H). | | | |
| 143 | 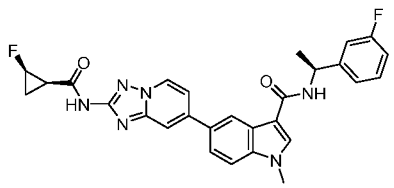 | $^1$H NMR (400 MHz, DMSO-de) δ 11.14 (s, 1H), 8.85 (d, J = 7.1 Hz, 1H), 8.54 (d, J = 1.9 Hz, 1H), 8.40 (d, J = 8.0 Hz, 1H), 8.23 (s, 1H), 7.88 (d, J = 2.0 Hz, 1H), 7.78 - 7.62 (m, 2H), 7.51 - 7.34 (m, 2H), 7.29 - 7.20 (m, 2H), 7.05 (td, J = 8.7, 2.9 Hz, 1H), 5.35 - 5.14 (m, 1H), 4.83 (dq, J = 46.3, 7.0 Hz, 1H), 3.91 (s, 3H), 1.65 (m, 1H), 1.50 (d, J = 7.0 Hz, 3H), 1.29 (d, J = 10.3 Hz, 1H), 1.22 - 1.15 (m, 1H). | 515.19 | Replacing i cyclopropylform yl chloride with (1R, 2R)-2-fluoro -cyclopropylformic acid | III |

(continued)

| Compound No. | Structure | Structural characterization | ESI-MS | Synthetic raw material | Synthetic method |
|---|---|---|---|---|---|
| 144 | 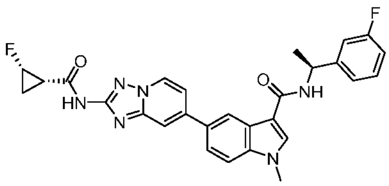 | $^1$H NMR (400 MHz, DMSO-$d_6$) δ 11.17 (s, 1H), 8.72 (dd, J = 7.5, 0.6 Hz, 1H), 7.95 - 7.83 (m, 2H), 7.79 - 7.67 (m, 2H), 7.67 - 7.60 (m, 1H), 7.52 (s, 1H), 7.36 (td, J = 7.6, 5.0 Hz, 1H), 7.15 (ddq, J = 7.6, 4.0, 1.3 Hz, 2H), 7.04 (tt, J = 7.5, 1.6 Hz, 1H), 5.16 (qt, J = 6.7, 1.0 Hz, 1H), 4.83 (dq, J = 46.3, 7.0 Hz, 1H), 3.77 (s, 3H), 2.26 (ddt, J = 25.2, 12.3, 7.0 Hz, 1H), 2.06 (dq, J = 25.2, 7.0 Hz, 1H), 1.65 - 1.51 (m, 4H), 1.22 - 1.15 (m, 1H). | 515.19 | Replacing i cyclopropylform yl chloride with (1S, 2S)-2- fluoro -cyclopropylformic acid | III |
| 145 | 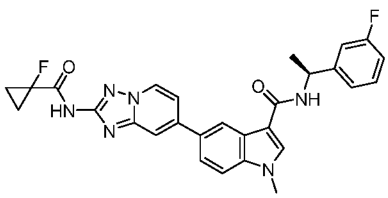 | $^1$H NMR (400 MHz, DMSO-de) δ 11.10 (s, 1H), 8.96 (d, J = 7.1 Hz, 1H), 8.60 (d, J = 1.9 Hz, 1H), 8.45 (d, J = 8.1 Hz, 1H), 8.29 (s, 1H), 8.09 - 7.95 (m, 1H), 7.86 - 7.67 (m, 2H), 7.56 (dd, J = 7.1, 2.0 Hz, 1H), 7.49 - 7.37 (m, 1H), 7.37 - 7.24 (m, 2H), 7.17 - 7.02 (m, 1H), 5.27 (p, J = 7.2 Hz, 1H), 3.96 (s, 3H), 1.55 (d, J = 7.1 Hz, 3H), 1.52 - 1.09 (m, 4H). | 515.19 | Replacing i cyclopropylform yl chloride with 1-fluorocyclopropanecarboxylic acid | III |
| 146 | 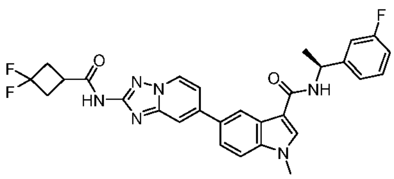 | $^1$H NMR (400 MHz, DMSO-$d_6$) δ 10.95 (s, 1H), 8.87 (d, J = 7.1 Hz, 1H), 8.54 (s, 1H), 8.40 (d, J = 8.0 Hz, 1H), 8.24 (s, 1H), 7.89 (s, 1H), 7.80 - 7.61 (m, 2H), 7.51 - 7.41 (m, 1H), 7.38 (q, J = 7.4 Hz, 1H), 7.25 (t, J = 9.3 Hz, 2H), 7.05 (t, J = 8.2 Hz, 1H), 5.22 (t, J = 7.4 Hz, 1H), 3.91 (s, 3H), 2.82 (dt, J = 17.0, 8.9 Hz, 4H), 2.70 (m, 1H), 1.50 (d, J = 7.0 Hz, 3H). | 547.20 | Replacing i cyclopropylform yl chloride with 3,3-difluorocyclo butanecarboxyli c acid | III |

(continued)

| Compound No. | Structure | Structural characterization | ESI-MS | Synthetic raw material | Synthetic method |
|---|---|---|---|---|---|
| 147 | 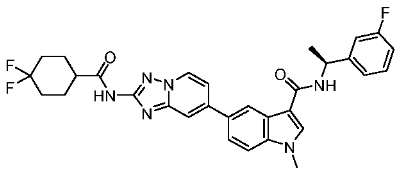 | $^1$H NMR (400 MHz, DMSO-de) δ 10.85 (s, 1H), 8.85 (d, J = 7.0 Hz, 1H), 8.53 (d, J = 1.9 Hz, 1H), 8.38 (t, J = 7.7 Hz, 1H), 8.23 (s, 1H), 7.87 (s, | 575.23 | Replacing i cyclopropylform yl chloride with 4,4-difluorocyclo hexanecarboxyli c acid | III |
| | | 1H), 7.76 - 7.69 (m, 1H), 7.66 (d, J = 8.4 Hz, 1H), 7.45 (dd, J = 7.1, 2.0 Hz, 1H), 7.40 - 7.33 (m, 1H), 7.26 (d, J = 8.1 Hz, 2H), 7.08 - 7.00 (m, 1H), 5.34 - 5.05 (m, 1H), 3.90 (s, 3H), 2.70 (m, 1H), 2.18 - 1.59 (m, 8H), 1.49 (d, J = 7.0 Hz, 3H); | | | |
| 148 | 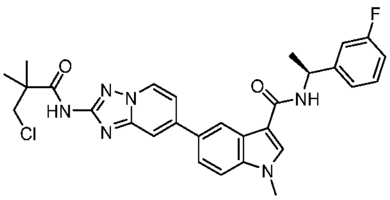 | $^1$H NMR (400 MHz, DMSO-de) δ 11.04 (s, , 1H), 8.87 (d, J = 7.1 Hz, 1H), 8.53 (d, J = 1.9 Hz, 1H), 8.39 (d, J = 8.0 Hz, 1H), 8.23 (s, 1H), 7.92 (s, 1H), 7.76 - 7.59 (m, 2H), 7.49 (dd, J = 7.2, 2.0 Hz, 1H), 7.38 (q, J = 7.4 Hz, 1H), 7.25 (t, J = 9.3 Hz, 2H), 7.05 (td, J = 8.8, 2.4 Hz, 1H), 5.21 (q, J = 7.3 Hz, 1H), 3.91 (s, 3H), 3.66 (s, 2H), 1.50 (d, J = 7.1 Hz, 3H), 1.35 (s, 6H). | 547.03 | Replacing i cyclopropylform yl chloride with 3-chloropivalic Acid | III |
| 149 | 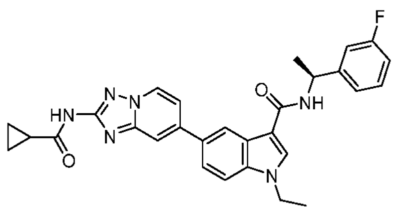 | $^1$H NMR (400 MHz, DMSO-de) δ 11.04 (s, , 1H), 8.83 (t, J = 6.2 Hz, 1H), 8.54 (s, 1H), 8.38 (d, J = 7.9 Hz, 1H), 8.31 (s, 1H), 7.87 (s, 1H), 7.71 (d, J = 1.2 Hz, 2H), 7.51 - 7.32 (m, 2H), 7.31 - 7.17 (m, 2H), 7.09 - 6.93 (m, 1H), 5.22 (q, J = 7.1 Hz, 1H), 4.32 (d, J = | 511.22 | Replacing i methyl iodide with ethyl iodide | II |
| | | 7.2 Hz, 3H), 1.54 - 1.48 (m, 3H), 1.46 (d, J = 7.2 Hz, 1H), 1.10 (t, J = 7.0 Hz, 3H), 0.88 - 0.81 (m, 4H). | | | |

(continued)

| Compound No. | Structure | Structural characterization | ESI-MS | Synthetic raw material | Synthetic method |
|---|---|---|---|---|---|
| 150 | 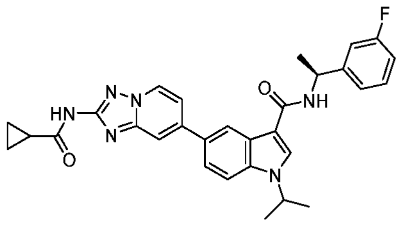 | $^1$H NMR (400 MHz, DMSO-de) δ 11.07 (s, 1H), 8.84 (d, J = 7.1 Hz, 1H), 8.57 (s, 1H), 8.49 - 8.30 (m, 2H), 7.86 (s, 1H), 7.72 (d, J = 8.0 Hz, 2H), 7.41 (dd, J = 17.4, 7.3 Hz, 2H), 7.32 - 7.18 (m, 2H), 7.06 (s, 1H), 5.32 - 5.17 (m, 1H), 4.96 - 4.82 (m, 1H), 2.07 (s, 1H), 1.75 - 1.31 (m, 9H), 0.84 (d, J = 6.3 Hz, 4H). | 525.23 | Replacing i methyl iodide with 2-iodopropane | II |
| 151 | 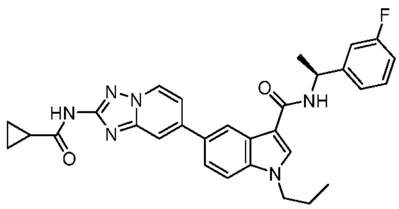 | $^1$H NMR (400 MHz, DMSO-$d_6$) δ 11.05 (s, 1H), 8.84 (d, J = 7.1 Hz, 1H), 8.53 (s, 1H), 8.37 (d, J = 7.9 Hz, 1H), 8.28 (s, 1H), 7.86 (d, J = 1.9 Hz, 1H), 7.70 (d, J = 2.4 Hz, 2H), 7.47 - 7.31 (m, 2H), 7.25 (t, J = 9.8 Hz, 2H), 7.11 - 6.98 (m, 1H), 5.22 (q, J = 7.3 Hz, 1H), 4.25 (t, J = 6.9 Hz, 2H), 2.06 (m, 1H), 1.86 (h, J = 7.5 Hz, 2H), 1.50 (d, J = 7.0 Hz, 3H), 1.24 (d, 3H), 0.89 - 0.69 (m, 4H). | 525.23 | Replacing i methyl iodide with 1-iodopropane | II |
| 152 | 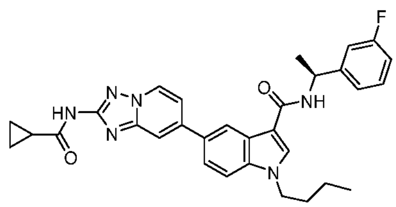 | $^1$H NMR (400 MHz, DMSO-$d_6$) δ 11.06 (s, 1H), 8.84 (d, J = 7.1 Hz, 1H), 8.54 (s, 1H), 8.38 (d, J = 8.0 Hz, 1H), 8.28 (d, J = 1.8 Hz, 1H), 7.87 (s, 1H), 7.71 (s, 2H), 7.49 - 7.33 (m, 2H), 7.26 (t, J = 9.7 Hz, 2H), 7.06 (t, J = 8.7 Hz, 1H), 5.29 - 5.05 (m, 1H), 4.28 (t, J = 7.2 Hz, 2H), 2.06 (m, 1H), 1.83 (p, J = 7.9 Hz, 2H), 1.56 - 1.44 (m, 3H), 1.31 (h, J = 14.0, 6.1 Hz, 2H), 0.94 (t, J = 7.6, 3.8 Hz, 3H), 0.89 - 0.77 (m, 4H). | 539.25 | Replacing i methyl iodide with 1-iodobutane | II |

(continued)

| Compound No. | Structure | Structural characterization | ESI-MS | Synthetic raw material | Synthetic method |
|---|---|---|---|---|---|
| 153 | 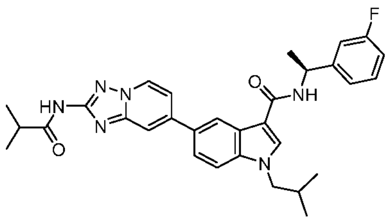 | $^1$H NMR (400 MHz, DMSO-de) δ 10.73 (s, 1H), 8.85 (d, J = 7.1 Hz, 1H), 8.54 (s, 1H), 8.39 (d, J = 7.9 Hz, 1H), 8.26 (s, 1H), 7.86 (s, 1H), 7.78 - 7.59 (m, 2H), 7.51 - 7.33 (m, 2H), 7.33 - 7.16 (m, 2H), 7.06 (t, J = 8.9 Hz, 1H), 5.21 (q, J = 7.3 Hz, 1H), 4.22 - 3.98 (m, 2H), 2.18 (dt, J = 13.6, 6.8 Hz, 1H), 1.50 (d, J = 7.0 Hz, 3H), 1.20 - 1.03 (m, 7H), 0.92 (d, J = 6.6 Hz, 6H). | 541.26 | Replacing i methyl iodide with isobutyl iodide, Replacing v cyclopropylform yl chloride with isopropylformyl chloride | II |
| 154 | 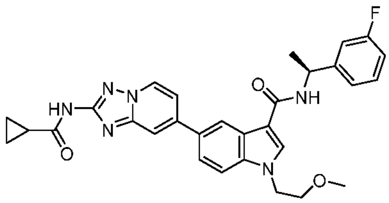 | $^1$H NMR (400 MHz, DMSO-$d_6$) δ 11.06 (s, 1H), 8.84 (d, J = 7.1 Hz, 1H), 8.55 (s, 1H), 8.42 (d, J = 8.0 Hz, 1H), 8.26 (s, 1H), 7.87 (s, 1H), 7.77 - 7.61 (m, 2H), 7.49 - 7.34 (m, 2H), 7.26 (t, J = 9.6 Hz, 2H), 7.13 - 6.95 (m, 1H), 5.23 (m, J = 7.2 Hz, 1H), 4.55 - 4.33 (m, 2H), 3.73 (t, J = 5.1 Hz, 2H), 3.26 (s, 3H), 2.07 (m, 1H), 1.50 (d, J = 7.1 Hz, 3H), 0.84 (d, J = 6.3 Hz, 4H). | 541.23 | Replacing i methyl iodide with 1-iodo-2-methox y-ethane | II |
| 155 | 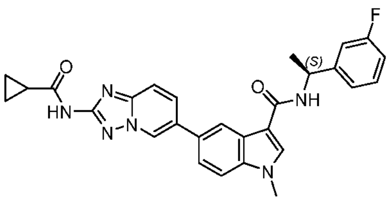 | $^1$H NMR (400 MHz, DMSO-de) δ 11.07 (s, 1H), 9.09 (d, J = 1.9 Hz, 1H), 8.52 - 8.30 (m, 2H), 8.21 (s, 1H), 7.95 (dd, J = 9.3, 1.9 Hz, 1H), 7.74 (d, J = 9.2 Hz, 1H), 7.64 (s, 2H), 7.37 (td, J = 8.0, 6.2 Hz, 1H), 7.31 - 7.19 (m, 2H), 7.04 (td, J = 8.7, 8.2, 2.7 Hz, 1H), 5.21 (p, J = 7.2 Hz, 1H), 3.89 (s, 3H), 2.11 - 1.92 (m, 1H), 1.49 (d, J = 7.1 Hz, 3H), 0.85 - 0.82 (m, 4H). | 497.20 | Replacing Intermediate 6 with 6-bromo-[1,2,4]t riazolo[1,5-a]pyri din- 2-amine | II |

(continued)

| Compound No. | Structure | Structural characterization | ESI-MS | Synthetic raw material | Synthetic method |
|---|---|---|---|---|---|
| 156 | 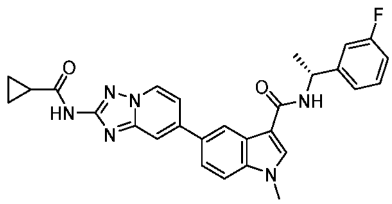 | $^1$H NMR (400 MHz, DMSO-$d_6$) δ 11.08 (s, 1H), 8.84 (d, J = 7.2 Hz, 1H), 8.53 (d, J = 1.9 Hz, 1H), 8.40 (d, J = 8.0 Hz, 1H), | 497.20 | Replacing iii(S)-1-(3-fluoro phenyl) ethanami ne with (R)-1-(3-fluorop henyl)ethanamin | II |
| | | 8.23 (s, 1H), 7.87 (s, 1H), 7.76 - 7.59 (m, 2H), 7.50 - 7.34 (m, 2H), 7.25 (t, J = 9.3 Hz, 2H), 7.12 - 6.99 (m, 1H), 5.29 - 5.18 (m, 1H), 3.90 (s, 3H), 2.06 (s, 1H), 1.49 (d, J = 7.0 Hz, 3H), 0.84 (d, J = 6.4 Hz, 4H). | | e | |
| 157 | 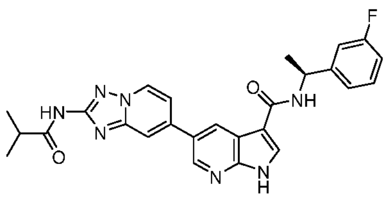 | $^1$H NMR (400 MHz, DMSO-de) δ 12.37 (s, 1H), 10.78 (s, 1H), 8.90 (d, J = 7.2 Hz, 1H), 8.50 (d, J = 7.9 Hz, 1H), 8.42 (s, 1H), 7.99 (s, 1H), 7.53 (dd, J = 20.7, 9.3 Hz, 3H), 7.39 (q, J = 7.5 Hz, 1H), 7.25 (t, J = 9.3 Hz, 2H), 7.06 (t, J = 8.5 Hz, 1H), 5.22 (t, J = 7.3 Hz, 1H), 2.79 (s, 1H), 1.51 (d, J = 7.0 Hz, 3H), 1.12 (d, J = 6.5 Hz, 6H). | 486.20 | Replacing Intermediate 1 with methyl 5-bromo-1H-pyrr olo[2,3-b] pyridin -3-carboxylate, Replacing ii cyclopropylform yl chloride with isopropylformyl chloride | I |
| 158 | 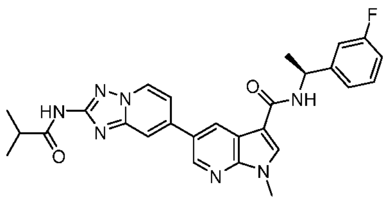 | $^1$H NMR (400 MHz, DMSO-$d_6$) δ 10.76 (s, 1H), 8.91 (d, J = 7.1 Hz, 1H), 8.83 (d, J = 2.2 Hz, 1H), 8.77 (d, J = 2.2 Hz, 1H), 8.52 (d, J = 7.9 Hz, 1H), 8.43 (s, 1H), 8.00 (s, 1H), 7.55 - 7.44 (m, 1H), 7.43 - 7.32 (m, 1H), 7.30 - 7.17 (m, 2H), 7.06 (t, J = 8.9 Hz, 1H), 5.21 (q, J = 7.3 Hz, 1H), 3.94 (s, 3H), 2.80 (s, 1H), 1.51 (d, J = 7.1 Hz, 3H), 1.12 (d, J = | 500.21 | Replacing Intermediate 1 with methyl 5-bromo-1H-pyrr olo[2,3-b] pyridin -3-carboxylate, Replacing ii cyclopropylform yl chloride with isopropylformyl chloride | II |
| | | 6.7 Hz, 6H). | | | |

(continued)

| Compound No. | Structure | Structural characterization | ESI-MS | Synthetic raw material | Synthetic method |
|---|---|---|---|---|---|
| 159 | 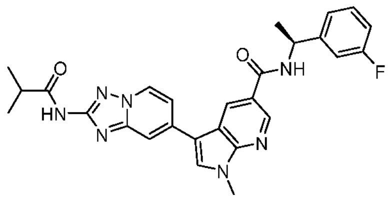 | $^1$H NMR (400 MHz, DMSO-de) δ 11.14 (s, 1H), 8.82 - 8.74 (m, 2H), 10.78 (s, 1H), 8.86 (d, J = 7.9 Hz, 1H), 8.51 (d, J = 2.1 Hz, 1H), 7.61 (d, J = 3.5 Hz, 1H), 7.38 (td, J = 8.0, 6.1 Hz, 1H), 7.31 - 7.19 (m, 3H), 7.05 (td, J = 8.3, 2.4 Hz, 1H), 6.60 (d, J = 3.5 Hz, 1H), 5.22 (p, J = 7.2 Hz, 1H), 3.85 (s, 3H), 1.50 (d, J = 7.1 Hz, 3H), 1.09 (t, J = 6.6 Hz, 6H). | 500.21 | Replacing Intermediate 1 with methyl 3-bromo-1H-pyrr olo[2,3-b] pyridin -5-carboxylate, Replacing ii cyclopropylform yl chloride with isopropylformyl chloride | II |
| 160 | 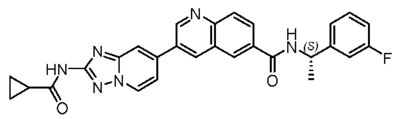 | $^1$H NMR (400 MHz, DMSO-de) δ 11.14 (s, 1H), 9.49 (d, J = 22.9 Hz, 1H), 9.17 (d, J = 7.7 Hz, 1H), 9.10 - 8.92 (m, 2H), 8.65 (s, 1H), 8.40 - 8.24 (m, 2H), 8.17 (d, J = 8.7 Hz, 1H), 7.72 (d, J = 7.3 Hz, 1H), 7.47 - 7.35 (m, 1H), 7.30 (d, J = 9.3 Hz, 2H), 7.10 (d, J = 8.9 Hz, 1H), 5.32 - 5.20 (m, 1H), 2.04 (d, J = 20.6 Hz, 1H), 1.54 (d, J = 7.1 Hz, 3H), 0.86 (d, J = 6.4 Hz, 4H). | 495.19 | Replacing Intermediate 1 with methyl 3-bromoquinolin e-7-carboxylate | II |
| 161 | 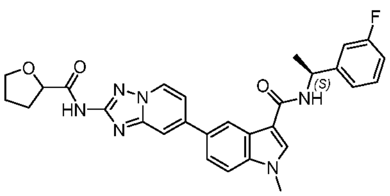 | $^1$H NMR (400 MHz, DMSO-$d_6$) δ 10.45 (s, 1H), 8.87 (d, J = 7.1 Hz, 1H), 8.53 (s, 1H), 8.39 (d, J = 8.1 Hz, 1H), 8.23 (s, 1H), 7.89 (s, 1H), | 527.21 | Replacing i cyclopropylform yl chloride with furoic acid | III |
| | | 7.78 - 7.62 (m, 2H), 7.54 - 7.33 (m, 2H), 7.25 (t, J = 9.4 Hz, 2H), 7.05 (t, J = 8.9 Hz, 1H), 5.22 (t, J = 7.5 Hz, 1H), 4.51 (s, 1H), 4.14 - 3.67 (m, 5H), 2.23 (d, J = 11.9 Hz, 1H), 1.93 (ddd, J = 34.0, 13.1, 6.6 Hz, 3H), 1.49 (d, J = 6.8 Hz, 3H). | | | |

(continued)

| Compound No. | Structure | Structural characterization | ESI-MS | Synthetic raw material | Synthetic method |
|---|---|---|---|---|---|
| 162 | 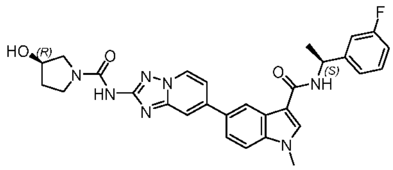 | $^1$H NMR (400 MHz, DMSO-$d_6$) δ 9.23 (s, 1H), 8.80 (d, J = 7.1 Hz, 1H), 8.53 (s, 1H), 8.41 (d, J = 8.0 Hz, 1H), 8.24 (s, 1H), 7.82 (s, 1H), 7.72 (dd, J = 8.6, 1.9 Hz, 1H), 7.66 (d, J = 8.6 Hz, 1H), 7.45 - 7.33 (m, 2H), 7.29 - 7.20 (m, 2H), 7.05 (ddd, J = 10.3, 8.2, 2.7 Hz, 1H), 5.22 (q, J = 7.3 Hz, 1H), 4.98 (d, J = 3.4 Hz, 1H), 4.30 (s, 1H), 3.90 (s, 3H), 3.49 (s, 3H), 3.31 (s, 1H), 1.96 - 1.88 (m, 1H), 1.81 (s, 1H), 1.49 (d, J = 7.0 Hz, 3H). | 542.22 | | IV |
| 163 | 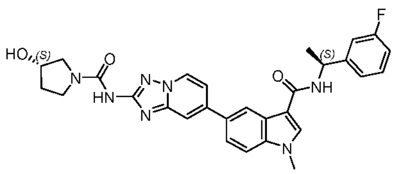 | $^1$H NMR (400 MHz, DMSO-$d_6$) δ 9.21 (s, 1H), 8.80 (d, J = 7.1 Hz, 1H), 8.52 (s, 1H), 8.39 (d, J = 8.0 Hz, 1H), 8.23 (s, 1H), 7.81 (s, 1H), 7.75 - 7.62 (m, 2H), 7.45 - 7.33 (m, 2H), 7.25 (t, J = 9.3 Hz, | 542.22 | Replacing ii(R)-3-pyrrolidin ol with (S)-3-pyrrolidinol | IV |
| | | 2H), 7.05 (t, J = 8.3 Hz, 1H), 5.26 - 5.17 (m, 1H), 4.96 (d, J = 3.5 Hz, 1H), 4.29 (s, 1H), 3.90 (s, 3H), 3.48 (s, 3H), 3.28 (s, 1H), 1.91 (s, 1H), 1.80 (s, 1H), 1.49 (d, J = 7.1 Hz, 3H). | | | |
| 164 | 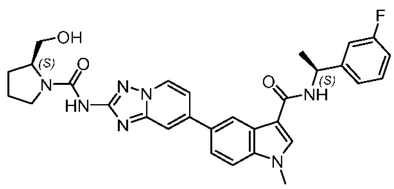 | $^1$H NMR (400 MHz, DMSO-$d_6$) δ 9.63 (s, 1H), 8.79 (d, J = 7.1 Hz, 1H), 8.51 (d, J = 1.9 Hz, 1H), 8.39 (d, J = 8.0 Hz, 1H), 8.23 (s, 1H), 7.80 (s, 1H), 7.74 - 7.62 (m, 2H), 7.43 - 7.34 (m, 2H), 7.31 - 7.21 (m, 2H), 7.10 - 6.99 (m, 1H), 5.21 (t, J = 7.4 Hz, 1H), 3.98 (s, 1H), 3.90 (s, 3H), 3.49 (s, 3H), 3.43 - 3.37 (m, 1H), 1.99 - 1.69 (m, 5H), 1.49 (d, J = 7.1 Hz, 3H). | 556.24 | Replacing ii(R)-3-pyrrolidin ol with L-prolinol | IV |

(continued)

| Compound No. | Structure | Structural characterization | ESI-MS | Synthetic raw material | Synthetic method |
|---|---|---|---|---|---|
| 165 | 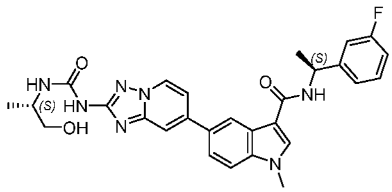 | $^1$H NMR (400 MHz, DMSO-$d_6$) δ 9.84 (s, 1H), 8.79 (d, J = 7.0 Hz, 1H), 8.54 (d, J = 1.9 Hz, 1H), 8.39 (d, J = 8.0 Hz, 1H), 8.31 (d, J = 7.7 Hz, 1H), 8.23 (s, 1H), 7.84 (d, J = 1.9 Hz, 1H), 7.74 (dd, J = 8.7, 1.9 Hz, 1H), 7.66 (d, J = 8.7 Hz, 1H), 7.44 (dd, J =7.1, 2.0 Hz, 1H), 7.38 (td, J = 8.0, 6.2 Hz, 1H), 7.30 - 7.19 (m, 2H), 7.05 (td, J = 8.4, 7.8, 2.5 Hz, 1H), | 530.18 | Replacing ii(R)-3-pyrrolidin ol with L-aminopropano l | IV |
| | | 5.21 (p, J = 7.2 Hz, 1H), 4.85 (t, J = 5.3 Hz, 1H), 3.90 (s, 3H), 3.83 (p, J = 6.1 Hz, 1H), 3.47 (dt, J = 9.9, 4.8 Hz, 1H), 3.40 (dt, J = 10.7, 5.5 Hz, 1H), 1.49 (d, J = 7.0 Hz, 3H), 1.17 (d, J = 6.6 Hz, 3H). | | | |
| 166 | 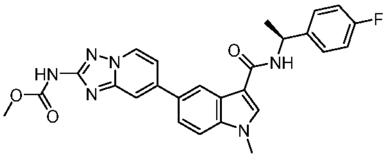 | $^1$H NMR (400 MHz, DMSO-$d_6$) δ 10.55 (s, 1H), 8.89 - 8.80 (m, 1H), 8.53 (s, 1H), 8.39 (d, J = 8.0 Hz, 1H), 8.23 (s, 1H), 7.86 (s, 1H), 7.78 - 7.63 (m, 2H), 7.50 - 7.35 (m, 2H), 7.25 (t, J = 9.7 Hz, 2H), 7.04 (d, J = 10.0 Hz, 1H), 5.22 (s, 1H), 3.91 (s, 3H), 3.70 (s, 3H), 1.50 (d, J = 7.1 Hz, 3H). | 487.18 | Replacing iii(S)-1-(3-fluoro phenyl) ethanami ne with (S)-1-(4-fluoroph enyl)ethanamine | IV |
| 167 | 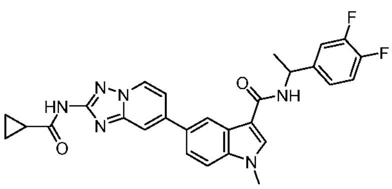 | $^1$H NMR (400 MHz, DMSO-$d_6$) δ 11.11 (s, 1H), 8.85 (d, J = 7.1 Hz, 1H), 8.53 (s, 1H), 8.40 (d, J = 7.9 Hz, 1H), 8.23 (s, 1H), 7.89 (s, 1H), 7.81 - 7.62 (m, 2H), 7.52 - 7.43 (m, 2H), 7.41 - 7.34 (m, 1H), 7.27 (s, 1H), 5.18 (q, J = 7.4 Hz, 1H), 3.91 (s, 3H), 2.07 (s, 1H), 1.49 (d, J = 7.1 Hz, 3H), 0.85 (d, J = 6.5 Hz, 4H). | 515.19 | Replacing iii(S)-1-(3-fluoro phenyl) ethanami ne with 1-(3,4-difluoroph enyl)ethanamine | II |

(continued)

| Compound No. | Structure | Structural characterization | ESI-MS | Synthetic raw material | Synthetic method |
|---|---|---|---|---|---|
| 168 | 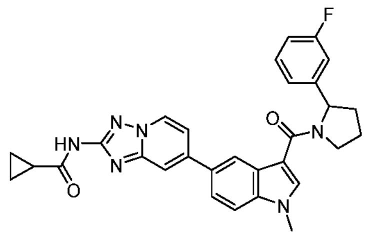 | $^1H$ NMR (400 MHz, DMSO-$d_6$) δ 11.07 (s, 1H), 8.82 (d, J = 7.1 Hz, 1H), 8.48 (d, J = 1.9 Hz, 1H), 7.84 (s, 1H), 7.74 (d, J = 8.5 Hz, 1H), 7.66 (d, J = 8.8 Hz, 1H), 7.57 - 7.45 (m, 1H), 7.42 (dd, J = 7.2, 2.0 Hz, 1H), 7.36 (q, J = 7.4 Hz, 1H), 7.12 (dd, J = 19.4, 9.0 Hz, 2H), 7.03 (t, J = 8.8 Hz, 1H), 5.32 (s, 1H), 4.01 (d, J = 72.6 Hz, 5H), 2.38 (dq, J = 14.4, 7.3 Hz, 1H), 1.96 - 1.79 (m, 4H), 0.84 (d, J = 6.2 Hz, 4H). | 523.22 | Replacing iii(S)-1-(3-fluoro phenyl) ethanami ne with 2-(3-fluoropheny l)-pyrrolidine | II |
| 169 | 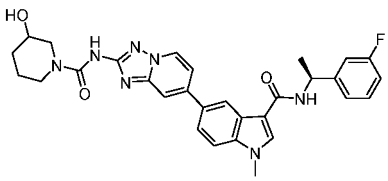 | $^1H$ NMR (400 MHz, DMSO-$d_6$) δ 9.45 (s, 1H), 8.79 (d, J = 7.1 Hz, 1H), 8.53 (d, J = 1.9 Hz, 1H), 8.39 (d, J = 8.0 Hz, 1H), 8.23 (s, 1H), 7.81 (s, 1H), 7.76 - 7.70 (m, 1H), 7.66 (d, J = 8.6 Hz, 1H), 7.43 - 7.35 (m, 2H), 7.25 (t, J = 9.2 Hz, 2H), 7.05 (t, J = 8.5 Hz, 1H), 5.22 (t, J = 7.4 Hz, 1H), 4.86 (d, J = 4.2 Hz, 1H), 3.91 (s, 3H), 3.75 (d, J = 13.6 Hz, 1H), 3.52 - 3.46 (m, 1H), 3.29 - 3.24 (m, 1H), 2.97 (t, J = 11.3 Hz, 1H), 2.81 - 2.75 (m, 1H), 1.87 (s, 1H), 1.68 (s, 1H), 1.50 (d, | 556.24 | Replacing ii(R)-3-pyrrolidin ol with 3-hydroxypiperid ine | IV |
| | | J = 7.0 Hz, 3H), 1.38 (q, J = 10.7, 8.8 Hz, 2H). | | | |

(continued)

| Compound No. | Structure | Structural characterization | ESI-MS | Synthetic raw material | Synthetic method |
|---|---|---|---|---|---|
| 170 | 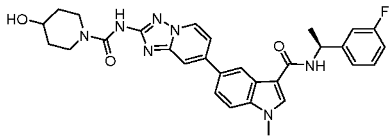 | $^1$H NMR (400 MHz, DMSO-$d_6$) δ 9.49 (s, 1H), 8.79 (d, J = 7.1 Hz, 1H), 8.53 (d, J = 1.9 Hz, 1H), 8.39 (d, J = 8.0 Hz, 1H), 8.23 (s, 1H), 7.81 (d, J = 1.9 Hz, 1H), 7.72 (dd, J = 8.6, 1.9 Hz, 1H), 7.66 (d, J = 8.6 Hz, 1H), 7.43 - 7.34 (m, 2H), 7.30 - 7.20 (m, 2H), 7.05 (td, J = 8.6, 8.2, 2.6 Hz, 1H), 5.22 (p, J = 7.2 Hz, 1H), 4.72 (d, J = 4.3 Hz, 1H), 3.91 (s, 3H), 3.84 (d, J = 13.5 Hz, 2H), 3.68 (q, J = 4.5 Hz, 1H), 3.13 - 3.04 (m, 2H), 1.80 - 1.72 (m, 2H), 1.50 (d, J = 7.1 Hz, 3H), 1.34 (q, J = 9.2 Hz, 2H). | 556.24 | Replacing ii(R)-3-pyrrolidin ol with 4-hydroxypiperid ine | IV |
| 171 | 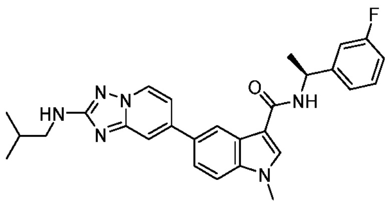 | $^1$H NMR (400 MHz, DMSO-$d_6$) δ 8.58 (d, J = 6.9 Hz, 1H), 8.47 (d, J = 1.4 Hz, 1H), 8.37 (d, J = 8.0 Hz, 1H), 8.21 (s, 1H), 7.68 - 7.61 (m, 2H), 7.58 - 7.55 (m, 1H), 7.41 - 7.35 (m, 1H), 7.24 (t, J = 9.5 Hz, 1H), 7.17 (dd, J = 7.0, 2.0 Hz, 1H), 7.08 - 7.02 (m, 1H), 6.63 (t, J = 6.0 Hz, 1H), 5.22 (q, J = 7.2 Hz, 1H), 3.89 (s, | 485.23 | Replacing v cyclopropylform yl chloride with isopropylformyl chloride | II |
| | | 3H), 3.27 (dd, J = 4.6, 2.3 Hz, 1H), 3.06 (t, J = 6.4 Hz, 2H), 1.90 (dt, J = 13.5, 6.8 Hz, 1H), 1.49 (d, J = 7.0 Hz, 3H), 0.91 (d, J = 6.7 Hz, 6H). | | | |

(continued)

| Compound No. | Structure | Structural characterization | ESI-MS | Synthetic raw material | Synthetic method |
|---|---|---|---|---|---|
| 172 | 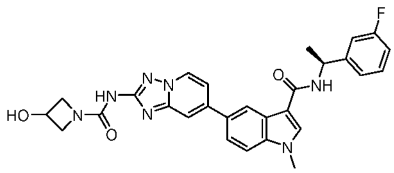 | $^1$H NMR (400 MHz, DMSO-$d_6$) δ 9.53 (s, 1H), 8.81 (d, J = 7.2 Hz, 1H), 8.53 (s, 1H), 8.39 (d, J = 7.9 Hz, 1H), 8.23 (s, 1H), 7.81 (s, 1H), 7.78 - 7.61 (m, 2H), 7.41 (q, J = 12.5, 8.3 Hz, 2H), 7.25 (t, J = 9.2 Hz, 2H), 7.06 (d, J = 8.9 Hz, 1H), 5.64 (d, J = 6.4 Hz, 1H), 5.22 (s, 1H), 4.43 (s, 1H), 4.17 (t, J = 8.0 Hz, 2H), 3.91 (s, 3H), 3.74 (s, 2H), 1.50 (d, J = 7.1 Hz, 3H). | 528.21 | Replacing ii(R)-3-pyrrolidin ol with azetidin-3-ol | IV |
| 173 | 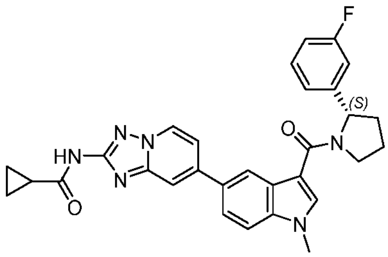 | $^1$H NMR (400 MHz, DMSO-$d_6$) δ 11.06 (s, 1H), 8.82 (d, J = 7.1 Hz, 1H), 8.48 (d, J = 1.9 Hz, 1H), 8.16 (s, 1H), 7.84 (d, J = 2.0 Hz, 1H), 7.74 (d, J = 8.5 Hz, 1H), 7.66 (d, J = 8.6 Hz, 1H), 7.44 - 7.27 (m, 2H), 7.12 (dd, J = 19.5, 9.0 Hz, 2H), 7.05 - 6.95 (m, 1H), 5.32 (s, 1H), 4.01 (d, J = 72.6 Hz, 5H), 2.37 (dt, J = 15.4, 7.7 Hz, 1H), 1.93 (d, J = 16.8 | 523.22 | Replacing iii(S)-1-(3-fluoro phenyl) ethanami ne with (S)-2-(3-fluoroph enyl)-pyrrolidine | II |
| | | Hz, 4H), 0.84 (d, J = 6.2 Hz, 4H). | | | |
| 174 | 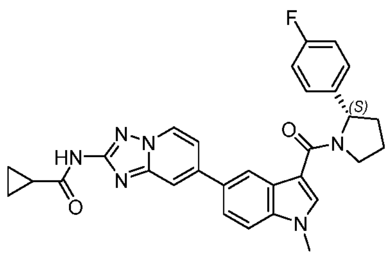 | $^1$H NMR (400 MHz, DMSO-$d_6$) δ 11.07 (s, 1H), 8.82 (d, J = 7.1 Hz, 1H), 8.48 (d, J = 1.8 Hz, 1H), 7.85 (d, J = 1.9 Hz, 1H), 7.73 (d, J = 8.6 Hz, 1H), 7.65 (d, J = 9.0 Hz, 1H), 7.59 - 7.49 (m, 1H), 7.42 (dd, J = 7.1, 2.0 Hz, 1H), 7.32 (dd, J = 8.5, 5.5 Hz, 2H), 7.13 (t, J = 8.7 Hz, 2H), 5.32 (s, 1H), 3.99 (d, J = 67.9 Hz, 5H), 2.35 (dt, J = 14.1, 7.2 Hz, 1H), 1.92 (d, J = 19.7 Hz, 2H), 1.78 (s, 2H), 0.84 (d, J = 6.2 Hz, 4H). | 523.21 | Replacing iii(S)-1-(3-fluoro phenyl) ethanami ne with (S)-2-(4-fluoroph enyl)-pyrrolidine | II |

(continued)

| Compound No. | Structure | Structural characterization | ESI-MS | Synthetic raw material | Synthetic method |
|---|---|---|---|---|---|
| 175 | 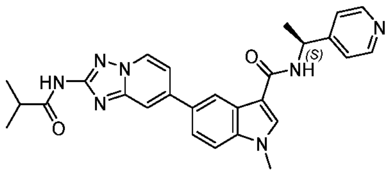 | $^1$H NMR (400 MHz, DMSO-$d_6$) δ 10.82 (s, 1H), 8.86 (d, J = 7.1 Hz, 1H), 8.53 (dd, J = 5.0, 3.1 Hz, 2H), 8.50 (d, J = 3.7 Hz, 1H), 8.49 - 8.46 (m, 1H), 8.28 (s, 1H), 7.89 (d, J = 2.0 Hz, 1H), 7.77 - 7.62 (m, 2H), 7.46 (dd, J = 7.1, 2.0 Hz, 1H), 7.42 (d, J = 5.4 Hz, 2H), 5.17 (p, J = 7.1 Hz, 1H), 3.91 (s, 3H), 2.80 (s, 1H), 1.50 (d, J = 7.4 Hz, 3H), 1.12 (d, J = 6.8 Hz, 6H). | 482.22 | Replacing iii(S)-1-(3-fluoro phenyl) ethanami ne with (S)-1-(4-pyridine ) ethanamine, Replacing v cyclopropylform yl chloride with isopropylformyl chloride | II |
| 176 | 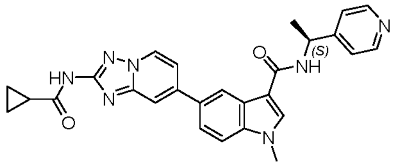 | $^1$H NMR (400 MHz, DMSO-de) δ 11.18 (s, 1H), 8.86 (d, J = 7.0 Hz, 1H), 8.65 - 8.44 (m, 4H), 8.30 (s, 1H), 7.90 (d, J = 1.9 Hz, 1H), 7.73 (dd, J = 8.7, 1.9 Hz, 1H), 7.67 (d, J = 8.7 Hz, 1H), 7.46 (dd, J = 7.2, 2.0 Hz, 1H), 7.44 - 7.37 (m, 2H), 5.17 (p, J = 7.1 Hz, 1H), 3.91 (s, 3H), 2.07 (s, 1H), 1.50 (d, J = 7.1 Hz, 3H), 0.85 (d, J = 6.1 Hz, 4H). | 480.21 | Replacing iii(S)-1-(3-fluoro phenyl) ethanami ne with (S)-1-(4-pyridine ) ethanamine | II |
| 177 | 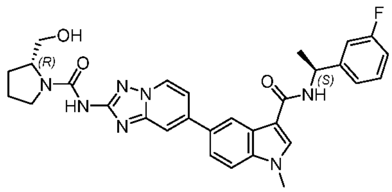 | $^1$H NMR (400 MHz, DMSO-$d_6$) δ 9.63 (s, 1H), 8.79 (d, J = 7.1 Hz, 1H), 8.51 (d, J = 1.9 Hz, 1H), 8.39 (d, J = 8.0 Hz, 1H), 8.23 (s, 1H), 7.80 (s, 1H), 7.74 - 7.62 (m, 2H), 7.43 - 7.34 (m, 2H), 7.31 - 7.21 (m, 2H), 7.10 - 6.99 (m, 1H), 5.21 (t, J = 7.4 Hz, 1H), 3.98 (s, 1H), 3.90 (s, 3H), 3.49 (s, 3H), 3.43 - 3.37 (m, 1H), 1.99 - 1.69 (m, 5H), 1.49 (d, J = 7.1 Hz, 3H). | 556.24 | Replacing ii(R)-3-pyrrolidin ol with D-prolinol | IV |

(continued)

| Compound No. | Structure | Structural characterization | ESI-MS | Synthetic raw material | Synthetic method |
|---|---|---|---|---|---|
| 178 | 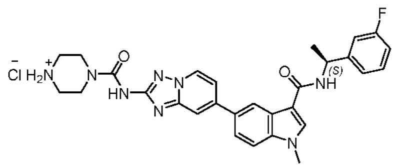 | $^1$H NMR (400 MHz, DMSO-$d_6$) δ 10.10 (s, 1H), 9.17 (s, 2H), 8.87 (d, J = 7.1 Hz, 1H), 8.55 (d, J = 1.8 Hz, 1H), 8.44 (d, J = 8.0 Hz, 1H), 8.27 (s, 1H), 7.87 (s, 1H), 7.76 - 7.66 (m, 2H), | 541.24 | Replacing ii(R)-3-pyrrolidin ol with piperazine | IV |
| | | 7.51 (dd, J = 7.1, 2.0 Hz, 1H), 7.41 - 7.35 (m, 1H), 7.25 (dd, J = 12.9, 4.9 Hz, 2H), 7.05 (td, J = 8.6, 8.0, 2.6 Hz, 1H), 5.21 (t, J = 7.3 Hz, 1H), 3.91 (s, 3H), 3.72 (d, J = 10.7 Hz, 4H), 3.15 (s, 4H), 1.50 (d, J = 7.0 Hz, 3H). | | | |
| 179 | 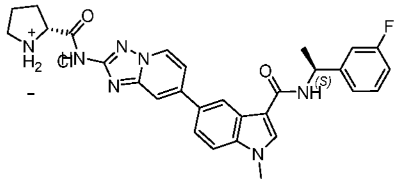 | $^1$H NMR (400 MHz, DMSO-$d_6$) δ 11.56 (s, 1H), 9.59 (s, 1H), 8.91 (d, J = 7.2 Hz, 1H), 8.73 (s, 1H), 8.56 (d, J = 1.8 Hz, 1H), 8.43 (d, J = 8.0 Hz, 1H), 8.26 (s, 1H), 7.94 (d, J = 2.0 Hz, 1H), 7.75 (dd, J = 8.6, 1.9 Hz, 1H), 7.68 (d, J = 8.7 Hz, 1H), 7.55 - 7.48 (m, 1H), 7.41 - 7.35 (m, 1H), 7.29 - 7.20 (m, 2H), 7.05 (td, J = 8.5, 7.9, 2.6 Hz, 1H), 5.24 - 5.18 (m, 1H), 4.42 (s, 1H), 3.91 (s, 3H), 3.29 (dd, J = 12.1, 5.8 Hz, 2H), 2.03 - 1.91 (m, 3H), 1.50 (d, J = 7.0 Hz, 3H). | 526.23 | Replacing i cyclopropylform yl chloride with L-proline | III |
| 180 | 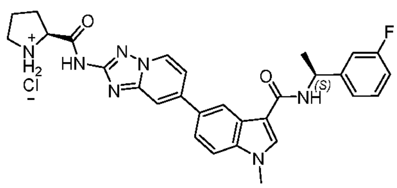 | $^1$H NMR (400 MHz, DMSO-$d_6$) δ 11.56 (s, 1H), 9.59 (s, 1H), 8.91 (d, J = 7.2 Hz, 1H), 8.73 (s, 1H), 8.56 (d, J = 1.8 Hz, 1H), 8.43 (d, J = 8.0 Hz, 1H), 8.26 (s, 1H), 7.94 (d, J = 2.0 | 526.23 | Replacing i cyclopropylform yl chloride with D-proline | III |

(continued)

| Compound No. | Structure | Structural characterization | ESI-MS | Synthetic raw material | Synthetic method |
|---|---|---|---|---|---|
| | | Hz, 1H), 7.75 (dd, J = 8.6, 1.9 Hz, 1H), 7.68 (d, J = 8.7 Hz, 1H), 7.55 - 7.48 (m, 1H), 7.41 - 7.35 (m, 1H), 7.29 - 7.20 (m, 2H), 7.05 (td, J = 8.5, 7.9, 2.6 Hz, 1H), 5.24 - 5.18 (m, 1H), 4.42 (s, 1H), 3.91 (s, 3H), 3.29 (dd, J = 12.1, 5.8 Hz, 2H), 2.03 - 1.91 (m, 3H), 1.50 (d, J = 7.0 Hz, 3H). | | | |
| 181 | 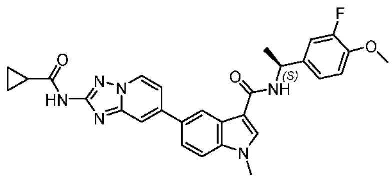 | $^1$H NMR (400 MHz, DMSO-$d_6$) δ 11.07 (s, 1H), 8.85 (s, 1H), 8.54 (s, 1H), 8.31 (d, J = 8.0 Hz, 1H), 8.20 (s, 1H), 7.87 (s, 1H), 7.78 - 7.60 (m, 2H), 7.60 - 7.41 (m, 1H), 7.26 (d, J = 12.7 Hz, 1H), 7.15 (dd, J = 21.7, 8.6 Hz, 2H), 5.23 - 5.04 (m, 1H), 3.90 (s, 3H), 3.81 (s, 3H), 2.07 (s, 1H), 1.47 (d, J = 7.0 Hz, 3H), 0.84 (d, J = 6.5 Hz, 4H). | 527.21 | Replacing iii(S)-1-(3-fluoro phenyl) ethanami ne with (S)-1-(3-fluoro-4 -methoxyphenyl) ethylamine | II |
| 182 | 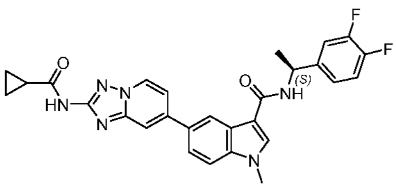 | $^1$H NMR (400 MHz, DMSO-$d_6$) δ 11.07 (s, 1H), 8.84 (d, J = 7.1 Hz, 1H), 8.53 (d, J = 1.9 Hz, 1H), 8.45 - 8.32 (m, 1H), 8.22 (s, 1H), 7.87 (s, 1H), 7.78 - 7.61 (m, 2H), 7.59 - 7.33 (m, 3H), 7.27 (s, 1H), 5.19 (t, J = 7.3 Hz, 1H), 3.91 (s, 3H), 2.14 - 1.86 | 515.19 | Replacing iii(S)-1-(3-fluoro phenyl) ethanami ne with (S)-1-(3,4-difluor omethoxyphenyl ) ethylamine | II |
| | | (m, 1H), 1.49 (d, J = 6.9 Hz, 3H), 0.84 (d, J = 6.2 Hz, 4H). | | | |

(continued)

| Compound No. | Structure | Structural characterization | ESI-MS | Synthetic raw material | Synthetic method |
|---|---|---|---|---|---|
| 183 | 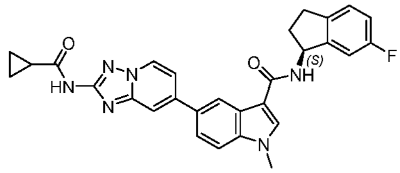 | $^1$H NMR (400 MHz, DMSO-$d_6$) δ 11.17 (s, 1H), 8.88 (d, J = 7.1 Hz, 1H), 8.64 (d, J = 1.9 Hz, 1H), 8.38 (d, J = 8.1 Hz, 1H), 8.18 (s, 1H), 7.94 (s, 1H), 7.78 - 7.65 (m, 2H), 7.54 - 7.49 (m, 1H), 7.35 - 7.27 (m, 1H), 7.05 (t, J = 9.6 Hz, 2H), 5.68 - 5.37 (m, 1H), 3.89 (s, 3H), 3.07 - 2.74 (m, 2H), 2.04 (dd, J = 12.5, 8.7 Hz, 1H), 1.10 (t, J = 7.0 Hz, 2H), 0.86 (d, J = 6.1 Hz, 4H). | 509.20 | Replacing iii(S)-1-(3-fluoro phenyl) ethanami ne with S-6-fluoro-1-ind amine | II |
| 184 | 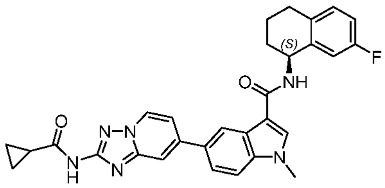 | $^1$H NMR (400 MHz, DMSO-de) δ 11.08 (s, 1H), 8.87 (d, J = 7.1 Hz, 1H), 8.75 - 8.46 (m, 1H), 8.40 - 8.23 (m, 1H), 8.16 (s, 1H), 7.91 (s, 1H), 7.81 - 7.59 (m, 2H), 7.48 (dd, J = 7.2, 2.0 Hz, 1H), 7.38 - 7.22 (m, 1H), 6.99 (t, J = 8.9 Hz, 2H), 5.30 (d, J = 26.9 Hz, 1H), 3.87 (s, 3H), 2.81 (s, 2H), 1.98 (d, J = 6.2 Hz, 3H), 1.85 (dd, J = 18.5, 9.6 Hz, 1H), 1.50 (d, J = 7.9 Hz, 1H), 0.85 (d, J = 6.3 Hz, 4H). | 523.22 | Replacing iii(S)-1-(3-fluoro phenyl) ethanami ne with S-7-fluoro-tetrah ydronaphthalen e -1-amine | II |
| 185 | 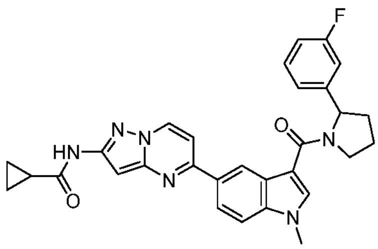 | $^1$H NMR (400 MHz, DMSO-$d_6$) δ 11.16 (s, 1H), 9.01 - 8.72 (m, 2H), 8.19 - 8.02 (m, 2H), 7.64 (d, J = 8.6 Hz, 1H), 7.49 (d, J = 7.3 Hz, 1H), 7.39 - 7.25 (m, 1H), 7.13 (dd, J = 19.5, 9.0 Hz, 2H), 7.03 (t, J = 8.9 Hz, 1H), 6.85 (s, 1H), 5.34 (s, 1H), 4.01 (d, J = 71.2 Hz, 5H), 2.38 (dt, J = 14.4, 7.1 Hz, 1H), 1.97 (d, J = 16.0 Hz, 4H), 0.89 - 0.72 (m, 4H). | 523.22 | Replacing iii(S)-1-(3-fluoro phenyl) ethanami ne with (S)-2-(4-fluoroph enyl)-pyrrolidine, Replacing Intermediate 6 with 2-amino-5-chlor opyrazolo[1,5-a] pyrimidine | II |

(continued)

| Compound No. | Structure | Structural characterization | ESI-MS | Synthetic raw material | Synthetic method |
|---|---|---|---|---|---|
| 186 | 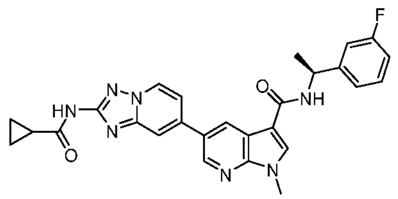 | 1H NMR (400 MHz, DMSO-$d_6$) δ 11.15 (s, 1H), 8.95 (d, J = 7.1 Hz, 1H), 8.85 (dd, J = 23.5, 2.2 Hz, 2H), 8.58 (d, J = 7.9 Hz, 1H), 8.48 (s, 1H), 8.06 (s, 1H), 7.63 - 7.51 (m, 1H), 7.44 (q, J = 7.4 Hz, 1H), 7.31 (t, J = 8.8 Hz, 2H), 7.14 - 7.04 (m, 1H), 5.33 - 5.20 (m, 1H), 3.99 (s, 3H), 2.12 (s, 1H), 1.56 (d, J = 7.1 Hz, 3H), 0.90 (d, J = 6.2 Hz, 4H). | 498.20 | Replacing Intermediate 1 with methyl 5-bromo-1H-pyrr olo[2,3-b] pyridin - 3-carboxylate | II |
| 187 | 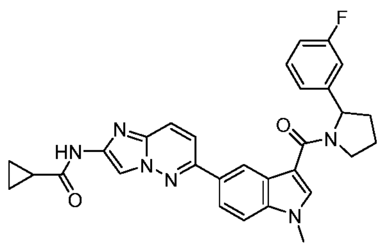 | 1H NMR (400 MHz, DMSO-$d_6$) δ 11.16 (s, 1H), 8.71 (d, J = 1.8 Hz, 1H), 8.25 (s, 1H), 8.16 (s, 1H), 8.00 (d, J = 9.5 Hz, 1H), 7.97 - 7.88 (m, 1H), 7.72 (d, J = 9.5 Hz, 1H), 7.66 (d, J = | 523.22 | Replacing iii(S)-1-(3-fluoro phenyl) ethanami ne with (S)-2-(4-fluoroph enyl)-pyrrolidine, Replacing Intermediate 6 with | II |
| | | 8.7 Hz, 1H), 7.41 - 7.28 (m, 1H), 7.13 (dd, J = 19.1, 9.1 Hz, 2H), 7.03 (t, J = 8.5 Hz, 1H), 5.33 (s, 1H), 4.01 (d, J = 72.5 Hz, 5H), 2.38 (dt, J = 14.8, 7.5 Hz, 1H), 1.88 (d, J = 64.6 Hz, 4H), 0.85 (dt, J = 10.5, 5.6 Hz, 4H). | | 2-amino-6-chlor oimidazo[1,2-b]p yridazine | |
| 188 | 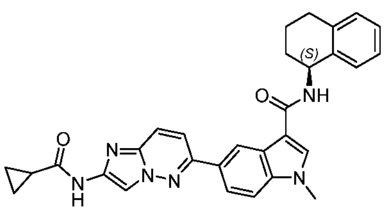 | 1H NMR (400 MHz, DMSO-$d_6$) δ 11.18 (s, 1H), 8.89 (d, J = 1.8 Hz, 1H), 8.37 - 8.24 (m, 2H), 8.17 (s, 1H), 8.05 (d, J = 9.4 Hz, 1H), 7.94 (dd, J = 8.7, 1.9 Hz, 1H), 7.78 (d, J = 9.5 Hz, 1H), 7.67 (d, J = 8.7 Hz, 1H), 7.38 - 7.25 (m, 1H), 7.25 - 7.06 (m, 3H), 5.40 - 5.19 (m, 1H), 3.87 (s, 3H), 2.80 (d, J = 6.2 Hz, 2H), 2.06 - 1.93 (m, 3H), 1.88 - 1.75 (m, 2H), 0.86 (dq, J = 7.7, 4.4 Hz, 4H). | 505.23 | Replacing iii(S)-1-(3-fluoro phenyl) ethanami ne with S-tetrahydronapht halene-1-amine, Replacing Intermediate 6 with 2-amino-6-chlor oimidazo [1,2-b] pyridazine | II |

(continued)

| Compound No. | Structure | Structural characterization | ESI-MS | Synthetic raw material | Synthetic method |
|---|---|---|---|---|---|
| 189 | 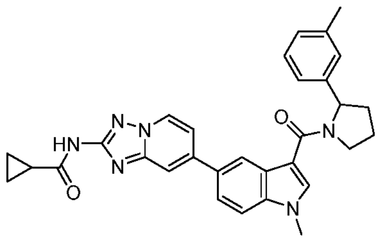 | $^1$H NMR (400 MHz, DMSO-de) δ 11.06 (s, 1H), 8.82 (d, J = 7.1 Hz, 1H), 8.48 (d, J = 1.9 Hz, 1H), 8.14 (s, 1H), 7.87 - 7.81 (m, 1H), 7.74 (d, J = 8.5 Hz, 1H), 7.42 (dd, J = 7.2, 2.0 Hz, 1H), 7.07 (d, J = 9.7 Hz, 2H), 7.20 (t, J = 7.5 Hz, 1H), 7.02 (d, J = 7.5 Hz, 1H), 5.30 | 519.24 | Replacing iii(S)-1-(3-fluoro phenyl) ethanami ne with 2-(3-methylphen yl)-pyrrolidine | II |
| | | (s, 1H), 3.92 (m, 5H), 2.35 (dd, J = 12.3, 7.2 Hz, 1H), 2.29 (s, 3H), 2.06 (m, 1H), 1.94 (m, 2H), 1.78 (m, 2H), 0.84 (d, J = 6.2 Hz, 4H). | | | |
| 190 | 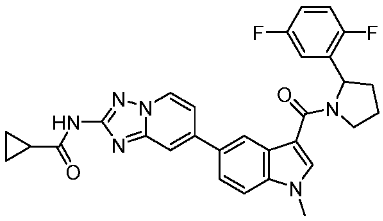 | $^1$H NMR (400 MHz, DMSO-de) δ 11.06 (s, 1H), 8.82 (d, J = 7.1 Hz, 1H), 8.47 (s, 1H), 8.18 (s, 1H), 7.85 (d, J = 2.0 Hz, 1H), 7.80 - 7.71 (m, 1H), 7.67 (d, J = 8.6 Hz, 1H), 7.42 (dd, J = 7.1, 2.0 Hz, 1H), 7.32 - 7.18 (m, 1H), 7.17 - 6.99 (m, 2H), 5.44 (t, J = 6.8 Hz, 1H), 3.93 (s, 5H), 2.41 (dq, J = 13.8, 7.1 Hz, 1H), 2.02 - 1.99 (m, 4H), 0.84 (d, J = 6.2 Hz, 4H). | 541.21 | Replacing iii(S)-1-(3-fluoro phenyl) ethanami ne with 2-(2,5-difluoroph enyl)-pyrrolidine | II |
| 191 | 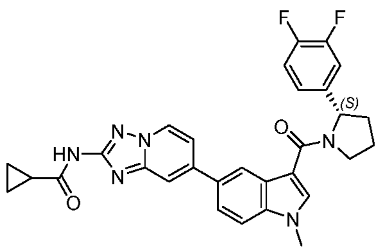 | $^1$H NMR (400 MHz, DMSO-$d_6$) δ 11.18 - 10.89 (m, 1H), 8.82 (d, J = 7.1 Hz, 1H), 8.47 (d, J = 1.9 Hz, 1H), 8.15 (s, 1H), 7.84 (d, J = 2.0 Hz, 1H), 7.76 - 7.55 (m, 2H), 7.47 - 7.27 (m, 3H), 7.15 (s, 1H), 5.28 (s, 1H), 4.01 (d, J = 75.7 Hz, 5H), 2.36 (dt, J = 14.2, 7.1 Hz, 1H), 2.06 (s, 1H), 2.01 - 1.88 (m, 1H), 1.87 - 1.71 (m, 2H), 0.84 (d, J = 6.2 Hz, 4H). | 541.21 | Replacing iii(S)-1-(3-fluoro phenyl) ethanami ne with (S)-2-(3,4-difluor ophenyl)-pyrroli dine | II |

(continued)

| Compound No. | Structure | Structural characterization | ESI-MS | Synthetic raw material | Synthetic method |
|---|---|---|---|---|---|
| 192 | 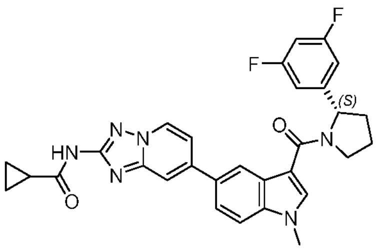 | $^1$H NMR (400 MHz, DMSO-$d_6$) δ 11.06 (s, 1H), 8.82 (d, J = 7.1 Hz, 1H), 8.47 (d, J = 1.9 Hz, 1H), 8.17 (s, 1H), 7.88 - 7.81 (m, 1H), 7.74 (dd, J = 8.7, 1.9 Hz, 1H), 7.66 (d, J = 8.6 Hz, 1H), 7.42 (dd, J = 7.2, 2.0 Hz, 1H), 7.11 - 6.94 (m, 3H), 5.29 (s, 1H), 4.02 (d, J = 80.3 Hz, 5H), 2.38 (dq, J = 14.0, 7.2 Hz, 1H), 2.18 - 1.59 (m, 4H), 0.84 (d, J = 6.2 Hz, 4H). | 541.21 | Replacing iii(S)-1-(3-fluoro phenyl) ethanami ne with (S)-2-(3,5-difluor ophenyl)-pyrroli dine | II |
| 193 | 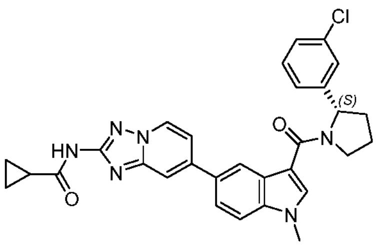 | $^1$H NMR (400 MHz, DMSO-$d_6$) δ 11.06 (s, 1H), 8.82 (d, J = 7.1 Hz, 1H), 8.47 (d, J = 1.8 Hz, 1H), 8.16 (s, 1H), 7.84 (d, J = 1.9 Hz, 1H), 7.78 - 7.70 (m, 1H), 7.66 (d, J = 8.7 Hz, 1H), 7.41 (dd, J = 7.2, 2.0 Hz, 1H), 7.39 - 7.30 (m, 2H), 7.29 - 7.22 (m, 2H), 5.29 (s, 1H), 4.01 (d, J = 72.4 Hz, 5H), 2.39 (dq, J = 14.0, 7.1 Hz, 1H), 1.93 (t, J = 55.2 Hz, 4H), 0.84 (d, J = 6.2 Hz, 4H). | 539.19 | Replacing iii(S)-1-(3-fluoro phenyl) ethanami ne with (S)-2-(3-chlorph enyl)-pyrrolidine | II |
| 194 | 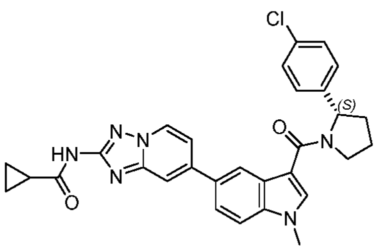 | $^1$H NMR (400 MHz, DMSO-$d_6$) δ 11.06 (s, 1H), 8.82 (d, J = 7.1 Hz, 1H), 8.48 (d, J = 1.9 Hz, 1H), 8.14 (s, 1H), 7.84 (d, J = 1.9 Hz, 1H), 7.74 (d, | 539.19 | Replacing iii(S)-1-(3-fluoro phenyl) ethanami ne with (S)-2-(4-chlorph enyl)-pyrrolidine | II |
| | | J = 8.5 Hz, 1H), 7.65 (d, J = 8.9 Hz, 1H), 7.41 (dd, J = 7.2, 2.0 Hz, 1H), 7.37 (d, J = 8.4 Hz, 2H), 7.31 (d, J = 8.3 Hz, 2H), 5.31 (d, J = 12.7 Hz, 1H), 4.00 (d, J = 66.8 Hz, 5H), 2.37 (dt, J = 14.1, 6.9 Hz, 1H), 1.95 (s, 3H), 1.77 (s, 1H), 0.84 (d, J = 6.1 Hz, 4H). | | | |

(continued)

| Compound No. | Structure | Structural characterization | ESI-MS | Synthetic raw material | Synthetic method |
|---|---|---|---|---|---|
| 195 | 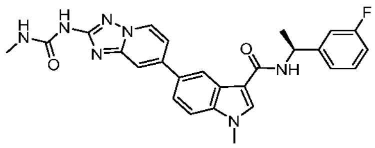 | $^1$H NMR (400 MHz, DMSO-de) δ 9.92 (s, 1H), 8.79 (d, J = 7.1 Hz, 1H), 8.54 (d, J = 1.8 Hz, 1H), 8.39 (d, J = 8.0 Hz, 1H), 8.23 (s, 2H), 7.87 (d, J = 1.9 Hz, 1H), 7.73 (dd, J = 8.7, 1.9 Hz, 1H), 7.67 (d, J = 8.7 Hz, 1H), 7.45 (dd, J = 7.1, 2.0 Hz, 1H), 7.42 - 7.35 (m, 1H), 7.25 (dd, J = 10.0, 8.2 Hz, 2H), 7.05 (td, J = 8.6, 8.0, 2.6 Hz, 1H), 5.22 (q, J = 7.2 Hz, 1H), 3.91 (s, 3H), 2.80 (d, J = 4.5 Hz, 3H), 1.50 (d, J = 7.0 Hz, 3H). | 486.20 | Replacing ii(R)-3-pyrrolidin ol with methylamine | IV |
| 196 | 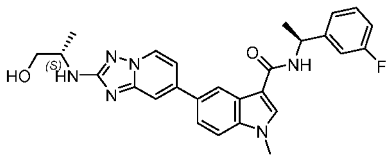 | $^1$H NMR (400 MHz, DMSO-de) δ 8.59 (d, J = 6.9 Hz, 1H), 8.48 (s, 1H), 8.37 (d, J = 8.0 Hz, 1H), 8.21 (s, 1H), 7.71 - 7.61 (m, 2H), 7.58 (s, 1H), 7.38 (q, J = 7.5 Hz, 1H), 7.25 (t, J = 9.4 Hz, 2H), 7.21 - 7.16 | 487.22 | Replacing v cyclopropylform yl chloride with L-aminopropano l | II |
| | | (m, 1H), 7.05 (t, J = 8.7 Hz, 1H), 6.25 (d, J = 8.0 Hz, 1H), 5.25 - 5.19 (m, 1H), 4.67 (t, J = 5.7 Hz, 1H), 3.90 (s, 3H), 3.80 - 3.71 (m, 1H), 3.54 (dd, J = 10.5, 5.3 Hz, 1H), 3.38 - 3.35 (m, 1H), 1.49 (d, J = 7.0 Hz, 3H), 1.17 (d, J = 6.5 Hz, 3H). | | | |

(continued)

| Compound No. | Structure | Structural characterization | ESI-MS | Synthetic raw material | Synthetic method |
|---|---|---|---|---|---|
| 197 | 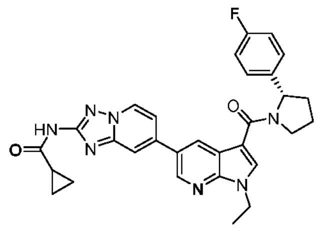 | $^1$H NMR (400 MHz, DMSO-$d_6$) δ 11.09 (s, 1H), 8.87 (d, *J* = 7.1 Hz, 1H), 8.81 (s, 1H), 8.73 (s, 1H), 8.38 (s, 1H), 7.97 (d, *J* = 1.9 Hz, 1H), 7.47 (dd, *J* = 7.1, 2.0 Hz, 1H), 7.32 (s, 2H), 7.13 (t, *J* = 8.8 Hz, 2H), 5.31 (s, 2H), 4.41 (s, 3H), 2.36 (s, 1H), 1.99 (d, *J*= 19.6 Hz, 3H), 1.79 (s, 1H), 1.47 (s, 2H), 1.24 (s, 2H), 0.84 (d, *J* = 6.2 Hz, 3H). | 538.22 | Replacing iii(S)-1-(3-fluoro phenyl) ethanami ne with (S)-2-(4-fluoroph enyl)-pyrrolidine | II |
| 198 | 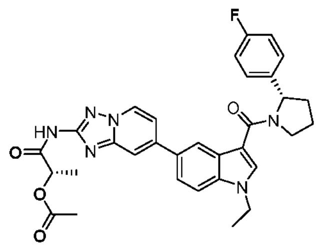 | $^1$H NMR (400 MHz, DMSO-$d_6$) δ 11.07 (s, 1H), 8.84 (d, *J* = 7.1 Hz, 1H), 8.48 (s, 1H), 8.17 (s, 1H), 7.86 (d, *J* = 1.9 Hz, 1H), 7.71 (s, 2H), 7.44 (dd, *J* = 7.2, 2.0 Hz, 1H), 7.37 - 7.30 (m, 2H), 7.13 (t, *J* = 8.8 Hz, 2H), 5.33 (s, 2H), 5.15 (s, 1H), 4.32 (s, 2H), 3.98-3.86 (m, 2H), 2.36 (dd, *J* = 12.4, | 583.24 | Replacing iii(S)-1-(3-fluoro phenyl) ethanami ne with (S)-2-(4-fluoroph enyl)-pyrrolidine, Replacing v cyclopropylform yl chloride with L-acetolactic acid | II |
| | | 7.2 Hz, 1H), 2.09 (s, 3H), 2.04 (d, *J* = 12.5 Hz, 1H), 1.93 (d, *J* = 11.2 Hz, 2H), 1.82 - 1.75 (m, 1H), 1.45 (d, *J* = 6.8 Hz, 3H), 1.24 (s, 1H). | | | |
| 199 | 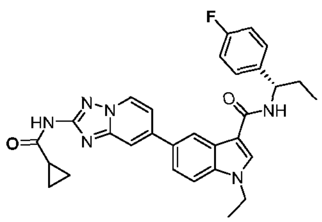 | $^1$H NMR (400 MHz, DMSO-$d_6$) δ 11.07 (s, 1H), 8.84 (d, *J* = 7.1 Hz, 1H), 8.53 (s, 1H), 8.30 (d, *J* = 5.4 Hz, 2H), 7.86 (s, 1H), 7.70 (s, 2H), 7.45 (dd, *J* = 8.2, 5.8 Hz, 3H), 7.16 (t, *J* = 8.8 Hz, 2H), 4.95 (q, *J* = 7.9 Hz, 1H), 4.32 (t, *J* = 7.2 Hz, 2H), 2.09 - 1.98 (m, 1H), 1.89 - 1.77 (m, 2H), 1.46 (t, *J* = 7.2 Hz, 3H), 0.94 (t, *J* = 7.3 Hz, 3H), 0.84 (d, *J* = 6.2 Hz, 4H). | 525.24 | Replacing iii(S)-1-(3-fluoro phenyl) ethanami ne with (S)-1-(4-fluoroph enyl) propane-1-amin e | II |

(continued)

| Compound No. | Structure | Structural characterization | ESI-MS | Synthetic raw material | Synthetic method |
|---|---|---|---|---|---|
| 200 | 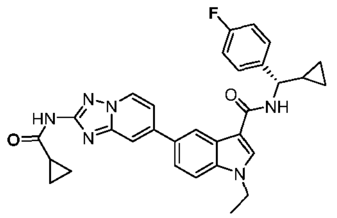 | $^1$H NMR (400 MHz, DMSO-$d_6$) δ 11.07 (s, 1H), 8.84 (d, *J* = 7.1 Hz, 1H), 8.53 (d, *J* = 8.1 Hz, 2H), 8.31 (s, 1H), 7.87 (d, *J* = 1.9 Hz, 1H), 7.71 (s, 2H), 7.52 (dd, *J* = 8.5, 5.6 Hz, 2H), 7.44 (dd, *J* = 7.1, 1.9 Hz, 1H), 7.17 (t, *J* = 8.8 Hz, 2H), 4.45 (t, *J* = 8.7 Hz, 1H), 4.31 (q, *J* = 7.1 Hz, 2H), 2.02 (t, *J* = 7.7 Hz, 1H), 1.46 (t, *J* = 7.2 Hz, 3H), 1.35 - 1.30 (m, 1H), 0.84 (d, *J* = 6.2 Hz, 4H), 0.58 (d, | 537.23 | Replacing iii(S)-1-(3-fluoro phenyl) ethanami ne with (S)-1-(4-fluorophenyl) cyclopropylmeth yl-1-amine | II |
| | | *J* = 8.0 Hz, 2H), 0.43 (s, 2H). | | | |
| 201 | 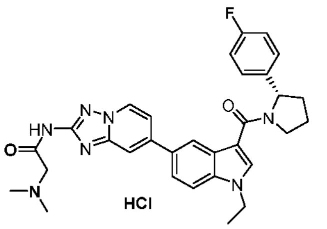 | $^1$H NMR (400 MHz, DMSO-$d_6$) δ 11.59 (s, 1H), 8.88 (d, *J* = 7.0 Hz, 1H), 8.50 (s, 1H), 7.90 (d, *J* = 1.9 Hz, 1H), 7.72 (s, 2H), 7.56 - 7.47 (m, 2H), 7.32 (dd, *J* = 8.5, 5.6 Hz, 2H), 7.13 (t, *J* = 8.8 Hz, 2H), 5.32 (s, 2H), 4.35 - 4.27 (m, 3H), 3.94 (s, 2H), 2.90 (d, *J* = 3.9 Hz, 6H), 2.40 - 2.33 (m, 1H), 2.03 - 1.91 (m, 3H), 1.78 (s, 1H), 0.94 (t, *J* = 6.7 Hz, 1H), 0.85 (d, *J* = 7.0 Hz, 1H). | 554.26 | Replacing iii(S)-1-(3-fluoro phenyl) ethanami ne with (S)-2-(4-fluoroph enyl)-pyrrolidine, Replacing v cyclopropylform yl chloride with N,N-dimethylgly cine | II |
| 202 | 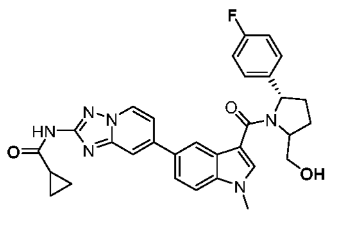 | $^1$H NMR (400 MHz, DMSO-$d_6$) δ 11.07 (s, 1H), 8.83 (d, *J* = 7.1 Hz, 1H), 8.45 (s, 1H), 7.86 (s, 1H), 7.73 (d, *J* = 8.7 Hz, 1H), 7.62 (d, *J* = 8.8 Hz, 1H), 7.54 (s, 1H), 7.45 - 7.35 (m, 3H), 7.13 (t, *J* = 8.7 Hz, 2H), 5.33 (s, 1H), 5.18 (s, 1H), 4.40 (s, 1H), 3.89 (d, *J* = 14.0 Hz, 2H), 3.80 (s, 3H), 2.35 (d, *J* = 15.3 Hz, 1H), 1.93 (d, *J* = 53.0 Hz, 4H), 0.84 (d, *J* = 6.3 Hz, 4H). | 553.23 | Replacing iii(S)-1-(3-fluoro phenyl) ethanami ne with (S)-2-(4-fluoroph enyl)-pyrrolidine -5-methanol | II |

(continued)

| Compound No. | Structure | Structural characterization | ESI-MS | Synthetic raw material | Synthetic method |
|---|---|---|---|---|---|
| 203 | 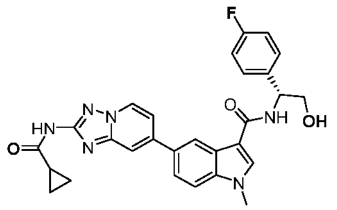 | $^1$H NMR (400 MHz, DMSO-$d_6$) δ 11.07 (s, 1H), 8.84 (d, *J* = 7.1 Hz, 1H), 8.53 (s, 1H), 8.30 - 8.21 (m, 2H), 7.87 (s, 1H), 7.75 - 7.65 (m, 2H), 7.48 - 7.43 (m, 3H), 7.16 (t, *J* = 8.7 Hz, 2H), 5.11 (d, *J* = 6.5 Hz, 1H), 4.98 (s, 1H), 3.91 (s, 3H), 3.69 (s, 2H), 2.05 (s, 1H), 0.84 (d, *J* = 6.2 Hz, 4H). | 513.20 | Replacing iii(S)-1-(3-fluoro phenyl) ethanami ne with (S)-1-(4-fluorophenyl)ethanol-1-amine | II |
| 204 | 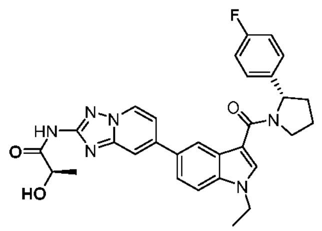 | $^1$H NMR (400 MHz, DMSO-$d_6$) δ 10.25 (s, 1H), 8.86 (d, *J* = 7.1 Hz, 1H), 8.47 (s, 1H), 8.16 (s, 1H), 7.86 (d, *J* = 1.9 Hz, 1H), 7.70 (s, 2H), 7.44 (dd, *J* = 7.2, 2.0 Hz, 1H), 7.32 (dd, *J* = 8.4, 5.4 Hz, 2H), 7.13 (t, *J* = 8.8 Hz, 2H), 5.71 (s, 1H), 5.32 (s, 1H), 4.28 (d, *J* = 10.9 Hz, 3H), 4.08 (s, 1H), 3.93 (s, 1H), 2.36 (dq, *J* = 14.2, 7.3 Hz, 1H), 1.94 (s, 2H), 1.82 - 1.74 (m, 1H), 1.41 (s, 3H), 1.33 (d, *J* = 6.7 Hz, 3H). | 541.23 | Replacing iii(S)-1-(3-fluoro phenyl) ethanami ne with (S)-2-(4-fluorophenyl)-pyrrolidine, Replacing v cyclopropylform yl chloride with D-lactic acid | II |
| 205 | 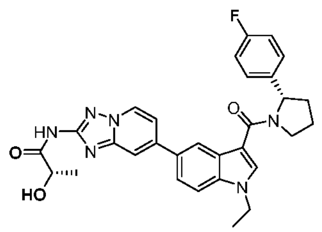 | $^1$H NMR (400 MHz, DMSO-$d_6$) δ 10.25 (s, 1H), 8.86 (d, *J* = 7.2 Hz, 1H), 8.47 (s, 1H), 8.16 (s, 1H), 7.86 (s, 1H), 7.71 (s, 2H), 7.47 - 7.42 (m, 1H), 7.32 (dd, *J* = 8.3, 5.3 Hz, 2H), 7.13 (t, *J* = 8.7 Hz, 2H), 5.70 (s, 1H), 5.33 (s, 1H), 4.28 (s, 3H), 4.08 (s, 1H), 3.93 (s, 1H), 2.36 (dd, *J* = 12.8, 7.1 Hz, 1H), 1.94 (s, 2H), 1.79 (s, 1H), 1.41 (s, 3H), 1.32 (d, *J* = 6.7 Hz, 3H). | 541.23 | Replacing iii(S)-1-(3-fluoro phenyl) ethanami ne with (S)-2-(4-fluorophenyl)-pyrrolidine, Replacing v cyclopropylform yl chloride with L-lactic acid | II |

(continued)

| Compound No. | Structure | Structural characterization | ESI-MS | Synthetic raw material | Synthetic method |
|---|---|---|---|---|---|
| 206 | 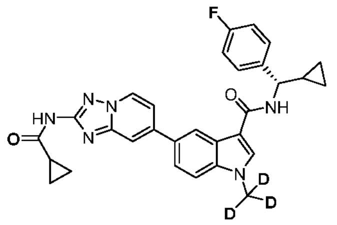 | $^1$H NMR (400 MHz, DMSO-$d_6$) δ 11.07 (s, 1H), 8.84 (d, $J$ = 7.1 Hz, 1H), 8.54 (d, $J$ = 6.9 Hz, 1H), 8.22 (s, 1H), 7.87 (s, 1H), 7.73 (d, $J$ = 8.4 Hz, 1H), 7.66 (d, $J$ = 8.6 Hz, 1H), 7.52 (dd, $J$ = 8.6, 5.6 Hz, 1H), 7.48 - 7.43 (m, 1H), 7.25 - 7.20 (m, 1H), 7.16 (t, $J$ = 8.8 Hz, 1H), 6.66 (s, 1H), 5.32 (d, $J$ = 5.2 Hz, 1H), 4.44 (t, $J$ = 8.7 Hz, 1H), 2.01 (t, $J$ = 7.5 Hz, 2H), 1.46 (s, 2H), 1.33 - 1.29 (m, 1H), 0.88 - 0.81 (m, 3H), 0.57 (d, $J$ = 7.7 Hz, 1H), 0.42 (d, $J$ = 4.8 Hz, 1H). | 526.24 | Replacing i methyl iodide with deuterated methyl iodide | II |
| 207 | 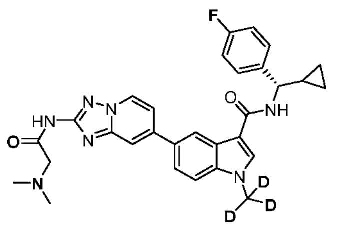 | $^1$H NMR (400 MHz, DMSO-$d_6$) δ 11.07 (s, 1H), 8.87 (d, $J$ = 7.1 Hz, 1H), 8.60 - 8.50 (m, 2H), 8.23 (s, 1H), 7.89 (s, 1H), 7.73 (d, $J$ = 8.3 Hz, 1H), 7.66 (d, $J$ = 8.7 Hz, 1H), 7.60 - 7.44 (m, 2H), 7.16 (t, $J$ = | 543.26 | Replacing i methyl iodide with deuterated methyl iodide, Replacing v cyclopropylform yl chloride with N,N-dimethylgly cine | II |
| | | 8.9 Hz, 2H), 4.45 (d, $J$ = 8.9 Hz, 1H), 4.23 (t, $J$ = 6.6 Hz, 1H), 3.41 (s, 2H), 2.31 (m, 1H), 1.34 - 1.13 (m, 6H), 0.94 - 0.83 (m, 2H), 0.57 (d, $J$ = 8.0 Hz, 1H), 0.42 (d, $J$ = 4.7 Hz, 1H). | | | |

(continued)

| Compound No. | Structure | Structural characterization | ESI-MS | Synthetic raw material | Synthetic method |
|---|---|---|---|---|---|
| 208 | 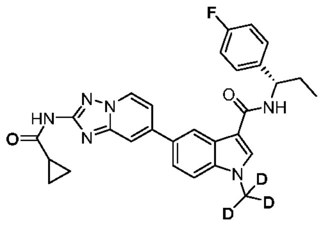 | $^1$H NMR (400 MHz, DMSO-$d_6$) δ 11.07 (s, 1H), 8.84 (d, $J$ = 7.1 Hz, 1H), 8.52 (s, 1H), 8.30 (d, $J$ = 8.3 Hz, 1H), 8.21 (s, 1H), 7.87 (s, 1H), 7.72 (d, $J$ = 8.7 Hz, 1H), 7.65 (d, $J$ = 8.6 Hz, 1H), 7.45 (dd, $J$ = 8.2, 4.3 Hz, 3H), 7.15 (t, $J$ = 8.6 Hz, 2H), 4.95 (d, $J$ = 7.6 Hz, 1H), 2.06 (s, 1H), 1.81 (td, $J$ = 15.4, 8.3 Hz, 2H), 0.93 (t, $J$ = 7.3 Hz, 3H), 0.84 (d, $J$ = 6.3 Hz, 4H). | 514.24 | Replacing iii(S)-1-(3-fluoro phenyl) ethanami ne with (S)-1-(4-fluoroph enyl) propane-1-amin e, Replacing i methyl iodide with deuterated methyl iodide, Replacing v cyclopropylform yl chloride with N,N-dimethylgly cine | II |
| 209 | 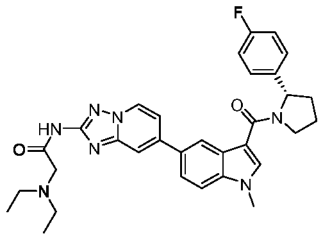 | $^1$H NMR (400 MHz, DMSO-$d_6$) δ 11.07 (s, 1H), 8.85 (d, $J$ = 7.1 Hz, 1H), 8.47 (s, 1H), 8.15 (s, 1H), 7.86 (s, 1H), 7.73 (d, $J$ = 8.2 Hz, 1H), 7.66 (s, 1H), 7.52 - 7.42 (m, 2H), 7.32 (t, $J$ = 7.0 Hz, 2H), 7.13 (t, $J$ = 8.7 Hz, 1H), 5.32 (s, 1H), 4.22 (t, $J$ = 6.4 Hz, 2H), 2.67 (m, 6H), 2.04 - 1.88 (m, 3H), 1.78 (s, 2H), 1.47 (s, 2H), 1.05 | 568.28 | Replacing v cyclopropylform yl chloride with N,N-diethylglyci ne, Replacing iii(S)-1-(3-fluoro phenyl) ethanami ne with (S)-2-(4-fluoroph enyl)-pyrrolidine, Replacing v cyclopropylform yl chloride with L-lactic acid | II |
| | | (m, 4H), 0.91 (t, $J$ = 7.4 Hz, 1H), 0.85 (d, J=7.3 Hz, 1H). | | | |
| 210 | 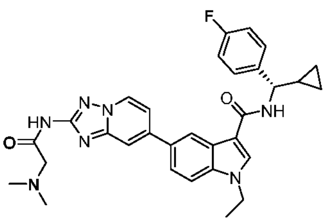 | $^1$H NMR (400 MHz, DMSO-$d_6$) δ 10.45 (s, 1H), 8.86 (d, $J$ = 7.1 Hz, 1H), 8.58 - 8.52 (m, 1H), 8.31 (s, 1H), 7.88 (d, $J$ = 1.8 Hz, 1H), 7.85 - 7.65 (m, 3H), 7.61 - 7.43 (m, 2H), 7.17 (t, $J$ = 8.9 Hz, 2H), 4.44 (t, $J$ = 8.7 Hz, 1H), 4.31 (q, $J$ = 7.2 Hz, 2H), 4.23 (t, $J$ = 6.5 Hz, 1H), 2.31 (s, 3H), 2.08 - 1.93 (m, 1H), 1.46 (t, $J$ = 7.2 Hz, 3H), 1.23 (d, $J$ = 3.6 Hz, 3H), 0.88 (dt, $J$ = 23.1, 7.2 Hz, 2H), 0.58 (d, $J$ = 8.1 Hz, 2H), 0.42 (s, 2H). | 554.26 | Replacing v cyclopropylform yl chloride with N,N-dimethylgly cine, Replacing iii(S)-1-(3-fluoro phenyl) ethanami ne with (S)-1-(4-fluoroph eny)cyclopropyl methyl-1-amine | II |

(continued)

| Compound No. | Structure | Structural characterization | ESI-MS | Synthetic raw material | Synthetic method |
|---|---|---|---|---|---|
| 211 | 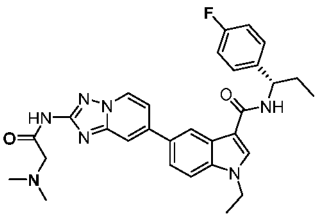 | $^1$H NMR (400 MHz, DMSO-$d_6$) δ 10.45 (s, 1H), 8.86 (d, $J$ = 7.3 Hz, 1H), 8.53 (s, 1H), 8.30 (d, $J$ = 8.9 Hz, 1H), 7.88 (s, 1H), 7.77 - 7.65 (m, 2H), 7.55 - 7.40 (m, 2H), 7.16 (t, $J$ = 8.8 Hz, 2H), 6.84 (s, 1H), 6.70 - 6.63 (m, 1H), 5.75 (s, 1H), 5.33 (s, 1H), 4.33 - 4.28 (m, 1H), 4.22 (d, $J$ = 6.6 Hz, 1H), 2.34 (s, 3H), 2.07 - 1.95 (m, 2H), 1.65 (d, $J$ = 7.3 Hz, 1H), 1.46 (t, $J$ = 7.2 Hz, | 542.26 | Replacing v cyclopropylform yl chloride with N,N-dimethylgly cine, Replacing iii(S)-1-(3-fluoro phenyl) ethanami ne with (S)-1-(4-fluoroph enyl) propane-1-amin e | II |
| | | 3H), 1.42 - 1.30 (m, 3H), 0.96 - 0.80 (m, 3H). | | | |
| 212 | 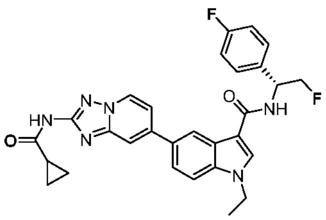 | $^1$H NMR (400 MHz, DMSO-$d_6$) δ 11.08 (s, 1H), 8.84 (d, $J$ = 7.2 Hz, 1H), 8.64 (d, $J$ = 8.0 Hz, 1H), 8.54 (s, 1H), 8.35 (s, 1H), 7.87 (s, 1H), 7.72 (s, 2H), 7.58 (d, $J$ = 23.8 Hz, 2H), 7.45 (d, $J$ = 7.0 Hz, 1H), 7.35 (d, $J$ = 8.6 Hz, 1H), 7.14 (s, 1H), 5.50 (s, 1H), 4.77 (d, $J$ = 5.7 Hz, 1H), 4.66 (s, 1H), 4.33 (d, $J$ = 6.9 Hz, 2H), 2.03 (s, 1H), 1.47 (t, $J$ = 7.2 Hz, 2H), 1.24 (s, 2H), 0.84 (d, $J$ = 6.2 Hz, 3H). | 529.21 | Replacing iii(S)-1-(3-fluoro phenyl) ethanami ne with (S)-1-(4-fluorophenyl)fluo roethane -1-amine | II |

(continued)

| Compound No. | Structure | Structural characterization | ESI-MS | Synthetic raw material | Synthetic method |
|---|---|---|---|---|---|
| 213 | 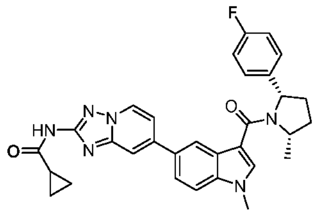 | $^1$H NMR (400 MHz, DMSO-$d_6$) δ 11.07 (s, 1H), 8.83 (d, $J$ = 7.1 Hz, 1H), 8.46 (d, $J$ = 1.9 Hz, 1H), 7.85 (d, $J$ = 2.0 Hz, 1H), 7.72 (dd, $J$ = 8.7, 1.8 Hz, 1H), 7.61 (d, $J$ = 8.6 Hz, 1H), 7.46 - 7.34 (m, 4H), 7.17 (t, $J$ = 8.7 Hz, 2H), 5.34 (d, $J$ = 7.0 Hz, 1H), 4.47 (d, $J$ = 6.2 Hz, 2H), 3.78 (s, 3H), 2.35 (d, $J$ = 11.4 Hz, 1H), 2.10 - 2.01 (m, 2H), 1.89 (dd, $J$ = 12.8, 6.3 Hz, 1H), 1.49 (d, $J$ = 6.3 Hz, 3H), 0.84 (d, $J$ = 6.2 Hz, 4H). | 537.23 | Replacing iii(S)-1-(3-fluoro phenyl) ethanami ne with (S)-2-(4-fluoroph enyl)-pyrrolidine -5-methyl | II |
| 214 | 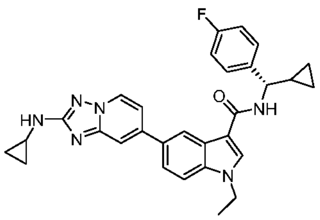 | $^1$H NMR (400 MHz, DMSO-$d_6$) δ 11.07 (s, 1H), 8.96 (s, 1H), 8.55 (dd, $J$ = 12.1, 7.8 Hz, 2H), 8.30 (s, 1H), 7.97 (d, $J$ = 8.7 Hz, 1H), 7.68 (d, $J$ = 8.6 Hz, 1H), 7.52 (t, $J$ = 7.1 Hz, 3H), 7.18 (d, $J$ = 8.7 Hz, 2H), 6.70 - 6.63 (m, 2H), 4.44 (s, 1H), 4.30 (d, $J$ = 7.3 Hz, 2H), 2.01 (d, $J$ = 8.5 Hz, 1H), 1.46 (t, $J$ = 7.2 Hz, 3H), 0.87 - 0.82 (m, 2H), 0.58 (d, $J$ = 8.2 Hz, 2H), 0.49 (d, $J$ = 3.9 Hz, 2H), 0.43 (s, 2H). | 509.24 | Replacing iii(S)-1-(3-fluoro phenyl) ethanami ne with (S)-1-(4-fluoroph enyl)fluoropheny lmethyl-1-amine | II |

**Example 5 In vitro kinase assay and protection against programmed necrosis assay with compounds of the present invention**

In vitro kinase assays were conducted using Kinase Profiler services provided by Eurofins. The experimental method was as follows: the small molecule compound to be tested (0.001-10 μM), the protein kinase to be tested were incubated with a buffer containing substrate, 10 mM magnesium acetate, and [γ-$^{33}$P-ATP], the reaction was started by adding Mg\ATPmix, after incubation for a period of time at room temperature, a 3% phosphate solution was added to the buffer to stop the reaction. Subsequently, 10 μL of the reaction mixture was quantitatively pipetted onto P30 filter paper, which was washed three times with 75 mM phosphate solution and then once with methanol. The P30 filter paper was air-dried and scintillation fluid was added for scintillation counting. The inhibitory activity of the compounds was expressed as the half maximalinhibitory concentration, $IC_{50}$, which was fitted to the inhibition ratio corresponding to each concentration gradient.

In vitro protection against programmed necrosis of HT-29 cells was conducted by ourselves.

**1) Experimental materials**

DMEM medium was purchased from Gibco Inc., penicillin and streptomycin were purchased from HyClone Inc., TNF $\alpha$ was purchased from PeproTech Inc., Smac mimetic and Z-VAD-FMK were purchased from Selleck Inc., CCK8 was purchased from Medchem Express Inc..

**2) Experimental method**

HT-29 cells (colon cancer cells) were cultured in DMEM+10% FBS + Penicillin/Streptomycin medium. During the experiments, HT-29 cells in logarithmic growth phase were harvested, seeded in a 96-well plate at specific numbers per well (typically $8\times10^3$ cells/well of adherent cells) and cultured overnight in a 37°C, 5% $CO_2$ cell culture incubator. The next day, the compounds to be tested were diluted with culture medium to the corresponding concentrations and added into the corresponding wells of a 96-well plate, with 3 replicate wells for each sample, and a Vehicle control group and a blank Control group containing only culture medium were set at the same time. The medicated cells were jointly induced with TNF $\alpha$/Smac mimetic/Z-VAD-FMK for 24h, and then 10 $\mu$l of CCK8 solution was added into each well, and the mixture was incubated in a cell culture incubator for 1-3 h. The absorbance was measured at 495 nm wavelength using a microplate reader, and the protection rate of the drug on the cells necrosis was calculated according to the following formula:

$$\text{Protection rate of cell necrosis} = [(X-C_0)/(C-C_0)]\times100\%$$

Wherein, C, $C_0$, and X represent the mean absorbance values of the Vehicle control group, Blank control group and Drug treatment group, respectively. Finally, cell viability curves were fitted using Graphpad Prism 5.0 software and $EC_{50}$ values of compounds to be tested for inhibition against programmed necrosis were calculated. The results are shown in Table 2, wherein +++++ represents $IC_{50}$ or $EC_{50}$ <0.001 $\mu$M, ++++ represents 0.01 $\mu$M> $IC_{50}$ or $EC_{50}\geqq$ 0.001 $\mu$M, +++ represents 0.1 $\mu$M> $IC_{50}$ or $EC_{50}$ $\geqq$ 0.01 $\mu$M, ++ represents 1 $\mu$M> $IC_{50}$ or $EC_{50}$ $\geqq$ 0.1 $\mu$M, and + represents $IC_{50}$ or $EC_{50}$ $\geqq$ 1 $\mu$M.

Table 2 The activity of the compounds of the present invention against inhibiting RIPK1 kinase and protecting against cell programmed necrosis

| **Compound No.** | **RIPK1 ($IC_{50}$)** | **HT-29 ($EC_{50}$)** | **Compound No.** | **RIPK1 ($IC_{50}$)** | **HT-29 ($EC_{50}$)** |
|---|---|---|---|---|---|
| 1 | +++ | +++ | 108 | ++++ | ++++ |
| 2 | +++ | ++ | 109 | ++++ | +++++ |
| 3 | +++ | +++ | 110 | +++++ | +++++ |
| 4 | +++ | +++ | 111 | ++++ | +++++ |
| 5 | ++ | + | 112 | +++ | ++++ |
| 6 | ++ | +++ | 113 | ++++ | ++++ |
| 7 | +++ | +++ | 114 | +++ | ++++ |
| 8 | +++ | ++++ | 115 | ++++ | +++ |
| 9 | +++ | ++++ | 116 | ++++ | +++ |
| 10 | ++ | ++ | 117 | +++ | +++ |
| 11 | ++ | + | 118 | +++ | ++++ |
| 12 | ++ | + | 119 | ++++ | ++++ |
| 13 | +++ | ++++ | 120 | ++++ | +++++ |
| 14 | + | + | 121 | +++ | ++++ |
| 15 | +++ | +++ | 122 | ++++ | +++++ |
| 16 | +++ | +++ | 123 | +++ | ++++ |
| 17 | +++ | +++ | 124 | +++ | ++++ |

(continued)

| Compound No. | RIPK1 ($IC_{50}$) | HT-29 ($EC_{50}$) | Compound No. | RIPK1 ($IC_{50}$) | HT-29 ($EC_{50}$) |
|---|---|---|---|---|---|
| 18 | +++ | ++++ | 125 | + | + |
| 19 | +++ | ++++ | 126 | +++ | +++ |
| 20 | + | + | 127 | +++ | +++++ |
| 21 | +++ | ++++ | 128 | ++ | ++ |
| 22 | +++ | ++++ | 129 | +++ | ++++ |
| 23 | ++ | ++++ | 130 | +++ | ++++ |
| 24 | ++ | +++ | 131 | +++ | +++++ |
| 25 | +++ | +++++ | 132 | ++ | ++ |
| 26 | ++ | + | 133 | +++ | +++++ |
| 27 | +++ | +++ | 134 | + | ++ |
| 28 | ++ | + | 135 | +++ | ++++ |
| 29 | ++ | ++ | 136 | ++ | +++ |
| 30 | +++ | +++++ | 137 | +++ | +++++ |
| 31 | ++ | ++ | 138 | + | + |
| 32 | +++ | ++++ | 139 | +++ | +++ |
| 33 | +++ | +++++ | 140 | ++ | +++ |
| 34 | +++ | +++++ | 141 | +++ | ++++ |
| 35 | ++ | +++ | 142 | +++ | +++++ |
| 36 | ++ | ++ | 143 | +++ | +++++ |
| 37 | +++ | ++++ | 144 | +++ | +++++ |
| 38 | +++ | ++++ | 145 | ++++ | +++++ |
| 39 | ++ | +++ | 146 | ++++ | +++++ |
| 40 | ++ | +++ | 147 | +++ | ++++ |
| 41 | ++ | ++ | 148 | + | + |
| 42 | ++ | + | 149 | +++ | +++++ |
| 43 | + | + | 150 | +++ | ++++ |
| 44 | ++ | +++ | 151 | ++++ | +++++ |
| 45 | +++ | +++ | 152 | +++ | +++ |
| 46 | +++ | +++++ | 153 | ++ | ++ |
| 47 | +++ | ++++ | 154 | +++ | ++++ |
| 48 | +++ | ++++ | 155 | +++ | ++++ |
| 49 | +++ | +++ | 156 | +++ | +++++ |
| 50 | +++ | +++ | 157 | +++ | ++++ |
| 51 | +++ | +++ | 158 | ++++ | +++++ |
| 52 | ++ | +++ | 159 | ++ | ++ |
| 53 | ++ | ++ | 160 | +++ | +++ |
| 54 | +++ | +++++ | 161 | ++++ | +++++ |
| 55 | +++ | ++++ | 162 | +++ | +++ |

(continued)

| Compound No. | RIPK1 ($IC_{50}$) | HT-29 ($EC_{50}$) | Compound No. | RIPK1 ($IC_{50}$) | HT-29 ($EC_{50}$) |
|---|---|---|---|---|---|
| 56 | +++ | ++++ | 163 | ++ | +++ |
| 57 | ++ | +++ | 164 | ++ | ++++ |
| 58 | ++ | ++ | 165 | +++ | +++++ |
| 59 | +++ | ++++ | 166 | +++ | +++++ |
| 60 | + | + | 167 | +++ | +++ |
| 61 | + | + | 168 | +++ | +++++ |
| 62 | ++ | ++++ | 169 | ++++ | +++++ |
| 63 | +++ | +++ | 170 | ++++ | +++++ |
| 64 | ++ | +++ | 171 | ++++ | +++++ |
| 65 | ++ | +++ | 172 | + | ++ |
| 66 | + | + | 173 | ++++ | +++++ |
| 67 | + | + | 174 | ++++ | +++++ |
| 68 | + | + | 175 | +++ | +++ |
| 69 | + | + | 176 | ++ | ++ |
| 70 | + | ++ | 177 | +++ | +++ |
| 71 | + | + | 178 | ++++ | ++++ |
| 72 | + | + | 179 | +++++ | +++++ |
| 73 | + | + | 180 | ++++ | +++++ |
| 74 | ++ | ++ | 181 | ++ | ++ |
| 75 | + | ++ | 182 | ++++ | +++++ |
| 76 | ++ | ++ | 183 | ++ | ++ |
| 77 | ++ | ++ | 184 | ++ | ++ |
| 78 | + | ++ | 185 | ++++ | +++++ |
| 79 | ++ | +++ | 186 | ++ | ++ |
| 80 | ++ | +++ | 187 | +++++ | +++++ |
| 81 | +++ | ++ | 188 | ++ | ++ |
| 82 | +++ | ++ | 189 | ++ | ++ |
| 83 | ++ | ++ | 190 | ++++ | ++++ |
| 84 | ++ | ++ | 191 | +++++ | +++++ |
| 85 | + | + | 192 | +++++ | +++++ |
| 86 | + | + | 193 | +++ | +++ |
| 87 | + | + | 194 | +++++ | +++++ |
| 88 | +++ | ++++ | 195 | +++++ | +++++ |
| 89 | +++ | ++++ | 196 | +++ | +++++ |
| 90 | ++ | ++++ | 197 | ++++ | +++++ |
| 91 | ++ | ++ | 198 | +++ | ++++ |
| 92 | ++ | ++ | 199 | ++++ | +++++ |
| 93 | + | + | 200 | +++ | +++++ |

(continued)

| Compound No. | RIPK1 ($IC_{50}$) | HT-29 ($EC_{50}$) | Compound No. | RIPK1 ($IC_{50}$) | HT-29 ($EC_{50}$) |
|---|---|---|---|---|---|
| 94 | ++++ | +++++ | 201 | +++ | ++++ |
| 95 | ++ | ++ | 202 | + | +++ |
| 96 | +++ | +++ | 203 | +++ | +++++ |
| 97 | + | + | 204 | +++ | +++++ |
| 98 | + | + | 205 | +++ | +++++ |
| 99 | + | + | 206 | ++++ | +++++ |
| 100 | + | + | 207 | ++ | ++++ |
| 101 | +++ | ++ | 208 | +++ | +++++ |
| 102 | ++ | ++ | 209 | ++++ | +++++ |
| 103 | +++ | ++ | 210 | +++ | +++++ |
| 104 | ++++ | +++++ | 211 | ++++ | +++++ |
| 105 | +++ | ++++ | 212 | ++++ | +++++ |
| 106 | ++++ | +++++ | 213 | ++++ | +++++ |
| 107 | +++ | ++++ | 214 | + | + |

Preferred compounds 34 and 94 were tested for single concentration inhibition ratio against 422 kinases (including mutants) available from Eurofins at a concentration of 10 μM, and $IC_{50}$ testing against kinases with higher inhibitory activity. The results (tested by Eurofins and providing activity data) are shown in Tables 3 and 4 below:

Table 3 Single concentration inhibitory activity (10 μM) of Compound 34 against 422 Kinases

| Nomenclature of kinases | Inhibition ratio/% | Nomenclatureof kinases | Inhibition ratio/% | Nomenclature of kinases | Inhibition ratio/% |
|---|---|---|---|---|---|
| AAK1(h) | 24 | FGFR1(V561M) (h) | 80 | PI3 Kinase (p110a (E545K)/p85a)(h) | 32 |
| Abl (H396P) (h) | 98 | Fgr(h) | 42 | PI3 Kinase (p110a (E545K)/p85a)(m) | 24 |
| Abl (M351T)(h) | 97 | Flt1 (h) | 100 | PI3 Kinase (p110a (H1047R)/p85a)(h) | 34 |
| Abl (Q252H) (h) | 97 | Flt3(D835Y)(h) | 82 | PI3 Kinase (p110a (H1047R)/p85a)(m) | 17 |
| Abl(h) | 102 | Flt3(h) | 79 | PI3 Kinase (p110a/p65a)(h) | 55 |
| Abl(m) | 99 | Flt4(h) | 93 | PI3 Kinase (p110a/p65a)(m) | 30 |
| Abl(T315I)(h) | 101 | Fms(h) | 21 | PI3 Kinase (p110a/p85a)(h) | 23 |
| Abl(Y253F)(h) | 95 | Fms(Y969C)(h) | 18 | PI3 Kinase (p110a/p85a)(m) | 43 |
| ACK1(h) | 35 | Fyn(h) | 43 | PI3 Kinase (p110b/p85a)(h) | 16 |
| ACTR2(h) | 6 | GCK(h) | 62 | PI3 Kinase (p110b/p85a)(m) | 15 |

(continued)

| Nomenclature of kinases | Inhibition ratio/% | Nomenclatureof kinases | Inhibition ratio/% | Nomenclature of kinases | Inhibition ratio/% |
|---|---|---|---|---|---|
| ALK(h) | 48 | GCN2(h) | 14 | PI3 Kinase (p110b/p85b)(m) | 45 |
| ALK1(h) | 50 | GRK1(h) | -24 | PI3 Kinase (p110d/p85a)(h) | 54 |
| ALK2(h) | 64 | GRK2(h) | -1 | PI3 Kinase (p110d/p85a)(m) | 31 |
| ALK4(h) | 3 | GRK3(h) | -1 | PI3 Kinase (p120g)(h) | 35 |
| ALK6(h) | -4 | GRK5(h) | 0 | PI3KC2a(h) | 16 |
| AMPKα1(h) | 4 | GRK6(h) | 0 | PI3KC2g(h) | 76 |
| AMPKα2(h) | 13 | GRK7(h) | 4 | Pim-1(h) | 3 |
| A-Raf(h) | -1 | GSK3α(h) | 10 | Pim-2(h) | -5 |
| Arg(h) | 89 | GSK3β(h) | 13 | Pim-3(h) | 3 |
| Arg(m) | 87 | Haspin(h) | 39 | PIP4K2a(h) | 17 |
| ARK5(h) | 43 | Hck(h) | 37 | PIP5K1a(h) | 21 |
| ASK1(h) | 12 | Hck(h) activated | 26 | PIP5K1g(h) | 18 |
| ATM(h) | 3 | HIPK1(h) | 68 | PKA(h) | -12 |
| Aurora-A(h) | 37 | HIPK2(h) | 66 | PKAcβ(h) | -4 |
| Aurora-B(h) | 21 | HIPK3(h) | 79 | PKBα(h) | 11 |
| Aurora-C(h) | 26 | HIPK4(h) | 95 | PKBβ(h) | 12 |
| Axl(h) | 86 | HPK1(h) | 75 | PKBγ(h) | 11 |
| BlKe(h) | 8 | HRI(h) | -3 | PKCα(h) | 6 |
| Blk(h) | 65 | ICK(h) | 7 | PKCβI(h) | 27 |
| Blk(m) | 65 | IGF-1R(h) | 28 | PKCβII(h) | 7 |
| BMPR2(h) | 30 | IGF-1R(h), activated | 21 | PKCγ(h) | 7 |
| Bmx(h) | 54 | IKKα(h) | 5 | PKCδ(h) | 7 |
| B-Raf(h) | 20 | IKKβ(h) | 12 | PKCε(h) | -8 |
| B-Raf(V599E)(h) | 14 | IKKε(h) | 19 | PKCζ(h) | 10 |
| BRK(h) | 4 | IR(h) | 36 | PKCη(h) | -3 |
| BrSK1(h) | -28 | IR(h), activated | 13 | PKCθ(h) | -24 |
| BrSK2(h) | 8 | IRAK1(h) | 32 | PKCi(h) | -4 |
| BTK(h) | 15 | IRAK4(h) | 15 | PKCμ(h) | 6 |
| BTK(R28H)(h) | 1 | IRE1(h) | 16 | PKD2(h) | 46 |
| CaMKI(h) | 5 | IRR(h) | 50 | PKD3(h) | -5 |
| CaMKIIα(h) | 5 | Itk(h) | 99 | PKG1α(h) | -12 |
| CaMKIIβ(h) | 21 | JAK1(h) | 26 | PKG1β(h) | 7 |
| CaMKIIγ(h) | 2 | JAK2(h) | 11 | PKR(h) | 11 |
| CaMKIIδ(h) | 22 | JAK3(h) | 23 | Plk1(h) | 8 |

(continued)

| Nomenclature of kinases | Inhibition ratio/% | Nomenclatureof kinases | Inhibition ratio/% | Nomenclature of kinases | Inhibition ratio/% |
|---|---|---|---|---|---|
| CaMKIβ(h) | 11 | JNK1α1(h) | 15 | Plk3(h) | 9 |
| CaMKIV(h) | 20 | JNK2α2(h) | 25 | Plk4(h) | 14 |
| CaMKIγ(h) | 7 | JNK3(h) | 63 | PRAK(h) | 27 |
| CaMKIδ(h) | 22 | KDR(h) | 83 | PRK1(h) | 26 |
| CaMKK1(h) | 74 | Lck(h) | 65 | PRK2(h) | 0 |
| CaMKK2(h) | 34 | Lck(h) activated | 76 | PRKG2(h) | 3 |
| Cdc7/cyclinB1 (h ) | 19 | LIMK1(h) | 14 | PrKX(h) | 6 |
| CDK1/cyclinB(h) | 13 | LIMK2(h) | 44 | PRP4(h) | 4 |
| CDK12/cyclinK ( h) | 21 | LKB1(h) | 6 | PTK5(h) | 51 |
| CDK13/cyclinK ( h) | 19 | LOK(h) | 85 | Pyk2(h) | 45 |
| CDK14/cyclinY ( h) | 47 | LRRK2(h) | 30 | Ret (V804L)(h) | 99 |
| CDK16/cyclinY ( h) | -30 | LTK(h) | 23 | Ret(h) | 96 |
| CDK17/cyclinY ( h) | -11 | Lyn(h) | 52 | Ret(V804M)(h) | 98 |
| CDK18/cyclinY ( h) | 7 | Lyn(m) | 72 | RIPK1(h) | 100 |
| CDK2/cyclinA(h) | 11 | MAK(h) | 2 | RIPK2(h) | 67 |
| CDK2/cyclinE(h) | 9 | MAP4K3(h) | 95 | ROCK-I(h) | 15 |
| CDK3/cyclinE(h) | -13 | MAP4K4(h) | 98 | ROCK-II(h) | 11 |
| CDK4/cyclinD3 ( h) | -5 | MAP4K5(h) | 64 | ROCK-II(r) | 12 |
| CDK5/p25(h) | 16 | MAPK1(h) | 25 | Ron(h) | 44 |
| CDK5/p35(h) | -7 | MAPK2(h) | 11 | Ros(h) | 31 |
| CDK6/cyclinD3 ( h) | 6 | MAPK2(m) | 12 | Rse(h) | 69 |
| CDK7/cyclinH/M | 3 | MAPKAP-K2(h) | -7 | Rsk1(h) | 37 |
| AT1(h) | | | | | |
| CDK9/cyclin T1 (h) | 41 | MAPKAP-K3(h) | -1 | Rsk1(r) | 45 |
| CDKL1(h) | 50 | MARK1(h) | 6 | Rsk2(h) | 1 |
| CDKL2(h) | 68 | MARK3(h) | 0 | Rsk3(h) | 49 |
| CDKL3(h) | 51 | MARK4(h) | 3 | Rsk4(h) | -15 |
| CDKL4(h) | 56 | MEK1(h) | 12 | SAPK2a(h) | 29 |
| ChaK1(h) | 5 | MEK2(h) | 8 | SAPK2a(T106M)(h) | 63 |

(continued)

| Nomenclature of kinases | Inhibition ratio/% | Nomenclatureof kinases | Inhibition ratio/% | Nomenclature of kinases | Inhibition ratio/ % |
|---|---|---|---|---|---|
| CHK1(h) | 2 | MEKK2(h) | 3 | SAPK2b(h) | 12 |
| CHK2(h) | 22 | MEKK3(h) | 2 | SAPK3(h) | 84 |
| CHK2(I157T)(h) | 24 | MELK(h) | 36 | SAPK4(h) | 94 |
| CHK2(R145W)( h) | 37 | Mer(h) | 97 | SBK1(h) | -12 |
| CK1(y) | 25 | Met(D1246H)(h ) | 95 | SGK(h) | 2 |
| CK1γ1(h) | 5 | Met(D1246N)(h ) | 86 | SGK2(h) | 2 |
| CK1γ2(h) | 9 | Met(h) | 95 | SGK3(h) | -1 |
| CK1y3(h) | -15 | Met(M1268T)(h ) | 93 | SIK(h) | 21 |
| CK1δ(h) | 33 | Met(Y1248C)(h ) | 99 | SIK2(h) | 39 |
| CK1ε(h) | 35 | Met(Y1248D)(h ) | 99 | SIK3(h) | 23 |
| CK2(h) | -9 | Met(Y1248H)(h ) | 107 | SLK(h) | 50 |
| CK2α1(h) | 7 | MINK(h) | 93 | Snk(h) | 0 |
| CK2α2(h) | 23 | MKK3(h) | 35 | SNRK(h) | -24 |
| cKit(D816H)(h) | 44 | MKK4(m) | 9 | Src(1-530)(h) | 54 |
| cKit(D816V)(h) | 0 | MKK6(h) | 7 | Src(T341M)(h) | 36 |
| cKit(h) | 4 | MLCK(h) | 14 | SRMS(h) | 12 |
| cKit(V560G)(h) | 88 | MLK1(h) | 98 | SRPK1(h) | 29 |
| cKit(V654A)(h) | -1 | MLK2(h) | 99 | SRPK2(h) | 17 |
| CLIK1(h) | 22 | MLK3(h) | 101 | STK16(h) | 17 |
| CLK1(h) | 80 | Mnk2(h) | 38 | STK25(h) | 2 |
| CLK2(h) | 77 | MOK(h) | -15 | STK32A(h) | 16 |
| CLK3(h) | 35 | MRCKα(h) | 12 | STK32B(h) | -2 |
| CLK4(h) | 79 | MRCKβ(h) | -5 | STK32C(h) | 3 |
| c-RAF(h) | 15 | MRCKγ(h) | -1 | STK33(h) | 4 |
| CRIK(h) | -5 | MSK1(h) | 53 | Syk(h) | 98 |
| CSK(h) | 13 | MSK2(h) | 23 | TAF1L(h) | 58 |
| cSRC(h) | 43 | MSSK1(h) | 24 | TAK1(h) | 45 |
| DAPK1(h) | 12 | MST1(h) | 5 | TAO1(h) | 24 |
| DAPK2(h) | 13 | MST2(h) | -9 | TAO2(h) | 58 |
| DCAMKL2(h) | 6 | MST3(h) | 7 | TAO3(h) | 62 |
| DCAMKL3(h) | 8 | MST4(h) | 11 | TBK1(h) | 53 |
| DDR1(h) | 51 | mTOR(h) | -7 | Tec(h) activated | 17 |
| DDR2(h) | 76 | mTOR/FKBP12 (h) | 10 | TGFBR1(h) | 7 |
| DMPK(h) | 10 | MuSK(h) | 88 | TGFBR2(h) | 25 |
| DNA-PK(h) | 24 | MYLK2(h) | 55 | Tie2 (h) | 96 |
| DRAK1(h) | 38 | MYO3B(h) | 16 | Tie2(R849W)(h) | 86 |

(continued)

| Nomenclature of kinases | Inhibition ratio/% | Nomenclatureof kinases | Inhibition ratio/% | Nomenclature of kinases | Inhibition ratio/% |
|---|---|---|---|---|---|
| DRAK2(h) | 18 | NDR2(h) | 23 | Tie2(Y897S)(h) | 88 |
| DYRK1A(h) | 47 | NEK1(h) | 18 | TLK1(h) | 19 |
| DYRK1B(h) | 12 | NEK11(h) | 35 | TLK2(h) | 5 |
| DYRK2(h) | 5 | NEK2(h) | 21 | TNIK(h) | 99 |
| DYRK3(h) | 48 | NEK3(h) | 10 | TRB2(h) | 65 |
| eEF-2K(h) | -41 | NEK4(h) | 30 | TrkA(h) | 102 |
| EGFR(h) | 19 | NEK6(h) | 4 | TrkB(h) | 100 |
| EGFR(L858R)(h ) | 65 | NEK7(h) | 4 | TrkC(h) | 104 |
| EGFR(L861Q)(h ) | 49 | NEK9(h) | 13 | TSSK1(h) | 12 |
| EGFR(T790M)(h ) | 40 | NIM1(h) | -19 | TSSK2(h) | -6 |
| EGFR(T790M,L858R)(h) | 84 | NLK(h) | 25 | TSSK3(h) | -6 |
| EphA1(h) | 3 | NUAK2(h) | 9 | TSSK4(h) | 13 |
| EphA2(h) | 38 | p70S6K(h) | 25 | TTBK1(h) | 4 |
| EphA3(h) | -8 | PAK1(h) | 4 | TTBK2(h) | 8 |
| EphA4(h) | 14 | PAK2(h) | 19 | TTK(h) | 55 |
| EphA5(h) | 40 | PAK3(h) | -1 | Txk(h) | 47 |
| EphA7(h) | 96 | PAK4(h) | 10 | TYK2(h) | 12 |
| EphA8(h) | 85 | PAK5(h) | -2 | ULK1(h) | 11 |
| EphB1(h) | 45 | PAK6(h) | -14 | ULK2(h) | 13 |
| EphB2(h) | 42 | PAR-1Bα(h) | 2 | ULK3(h) | 30 |
| EphB3(h) | 3 | PASK(h) | -25 | VRK1(h) | 2 |
| EphB4(h) | 11 | PDGFRα(D842 V)(h) | 56 | VRK2(h) | 21 |
| ErbB2(h) | 10 | PDGFRα(h) | 32 | Wee1(h) | -1 |
| ErbB4(h) | 22 | PDGFRα(V561 D)(h) | 99 | Wee1B(h) | -2 |
| FAK(h) | 33 | PDGFRβ(h) | 9 | WNK1(h) | 10 |
| Fer(h) | 62 | PDHK2(h) | 3 | WNK2(h) | 38 |
| Fes(h) | 26 | PDHK4(h) | 72 | WNK3(h) | 18 |
| FGFR1(h) | 72 | PDK1(h) | 19 | WNK4(h) | 17 |
| FGFR1(V561M)( h) | 80 | PEK(h) | 1 | Yes(h) | 85 |
| FGFR2(h) | 63 | PhKγ1(h) | 16 | ZAK(h) | 67 |
| FGFR2(N549H)( h) | 86 | PhKγ2(h) | 5 | ZAP-70(h) | 8 |

(continued)

| Nomenclature of kinases | Inhibition ratio/% | Nomenclatureof kinases | Inhibition ratio/% | Nomenclature of kinases | Inhibition ratio/% |
|---|---|---|---|---|---|
| FGFR3(h) | 35 | PI3 Kinase (p110a(E542K) / p85a)(h) | 38 | ZIPK(h) | -4 |
| FGFR4(h) | 5 | PI3 Kinase (p110a(E542K) / p85a)(m) | 33 | | |

Table 4 Single concentration inhibitory activity (10 μM) of Compound 94 against 422 Kinases

| Nomenclature of kinases | Inhibition ratio/% | Nomenclature of kinases | Inhibition ratio/% | Nomenclature of kinases | Inhibition ratio/% |
|---|---|---|---|---|---|
| AAK1(h) | 2 | FGFR1(V561 M)(h) | 30 | PI3 Kinase (p110a(E545K)/ p85a)(h) | 33 |
| Abl (H396P) (h) | 34 | Fgr(h) | 8 | PI3 Kinase (p110a(E545K)/ p85a)(m) | 32 |
| Abl (M351T) (h) | 29 | Flt1(h) | 42 | PI3Kinase(p110a(H1047R) /p85a)(h) | 24 |
| Abl (Q252H) (h) | 29 | Flt3(D835Y)(h ) | 12 | PI3Kinase(p110a(H1047R) /p85a)(m) | 47 |
| Abl(h) | 32 | Flt3(h) | 27 | PI3 Kinase (p110a/p65a)(h) | 4 |
| Abl(m) | 61 | Flt4(h) | 34 | PI3 Kinase (p110a/p65a)(m) | 10 |
| Abl(T3151)(h ) | 33 | Fms(h) | 22 | PI3 Kinase (p110a/p85a)(h) | 3 |
| Abl(Y253F) ( h) | 23 | Fms(Y969C)( h) | 13 | PI3 Kinase (p110a/p85a)(m) | -16 |
| ACK1(h) | 33 | Fyn(h) | -43 | PI3 Kinase (p110b/p85a)(h) | -7 |
| ACTR2(h) | -3 | GCK(h) | -6 | PI3 Kinase (p110b/p85a)(m) | 10 |
| ALK(h) | -7 | GCN2(h) | 23 | PI3 Kinase (p110b/p85b)(m) | 44 |
| ALK1(h) | 0 | GRK1(h) | 52 | PI3 Kinase (p110d/p85a)(h) | -1 |
| ALK2(h) | -17 | GRK2(h) | 53 | PI3 Kinase (p110d/p85a)(m) | 44 |
| ALK4(h) | -18 | GRK3(h) | -12 | PI3 Kinase (p120g)(h) | -12 |
| ALK6(h) | -5 | GRK5(h) | 21 | PI3KC2a(h) | 40 |
| AMPKα1(h) | 57 | GRK6(h) | 2 | PI3KC2g(h) | 24 |
| AMPKα2(h) | 16 | GRK7(h) | -16 | Pim-1(h) | 9 |
| A-Raf(h) | 10 | GSK3α(h) | 48 | Pim-2(h) | -14 |
| Arg(h) | 42 | GSK3β(h) | 1 | Pim-3(h) | 32 |
| Arg(m) | 11 | Haspin(h) | 48 | PIP4K2a(h) | 7 |
| ARK5(h) | 8 | Hck(h) | -8 | PIP5K1a(h) | 16 |
| ASK1(h) | 38 | Hck(h) activated | -12 | PIP5K1g(h) | -16 |
| ATM(h) | -15 | HIPK1(h) | 48 | PKA(h) | 55 |
| Aurora-A(h) | -7 | HIPK2(h) | 36 | PKAcβ(h) | 40 |
| Aurora-B(h) | 25 | HIPK3(h) | 62 | PKBα(h) | -11 |

(continued)

| Nomenclature of kinases | Inhibition ratio/% | Nomenclature of kinases | Inhibition ratio/% | Nomenclature of kinases | Inhibition ratio/% |
|---|---|---|---|---|---|
| Aurora-C(h) | 15 | HIPK4(h) | 68 | PKBβ(h) | -8 |
| Axl(h) | 31 | HPK1(h) | -3 | PKBγ(h) | 31 |
| BIKe(h) | -2 | HRI(h) | -4 | PKCα(h) | 16 |
| Blk(h) | 65 | ICK(h) | 52 | PKCβI(h) | 6 |
| Blk(m) | 77 | IGF-1R(h) | -4 | PKCβII(h) | 10 |
| BMPR2(h) | -6 | IGF-1 R(h), activated | -17 | PKCγ(h) | 47 |
| Bmx(h) | 34 | IKKα(h) | 29 | PKCδ(h) | 48 |
| B-Raf(h) | -5 | IKKβ(h) | -3 | PKCε(h) | 35 |
| B-Raf(V599 E)(h) | -10 | IKKε(h) | -15 | PKCζ(h) | -2 |
| BRK(h) | 47 | IR(h) | 27 | PKCη(h) | 27 |
| BrSK1(h) | 31 | IR(h), activated | 35 | PKCθ(h) | 7 |
| BrSK2(h) | -2 | IRAK1(h) | 23 | PKCi(h) | 54 |
| BTK(h) | 65 | IRAK4(h) | -11 | PKCμ(h) | 50 |
| BTK(R28H)( h) | 77 | IRE1(h) | 22 | PKD2(h) | 9 |
| CaMKI(h) | 49 | IRR(h) | 33 | PKD3(h) | -13 |
| CaMKIIα(h) | 76 | Itk(h) | 92 | PKG1α(h) | 12 |
| CaMKIIβ(h) | 78 | JAK1(h) | 52 | PKG1β(h) | 0 |
| CaMKIIγ(h) | -15 | JAK2(h) | 45 | PKR(h) | 19 |
| CaMKIIδ(h) | -11 | JAK3(h) | 38 | Plk1(h) | 26 |
| CaMKIß(h) | 31 | JNK1α1(h) | 53 | Plk3(h) | 12 |
| CaMKIV(h) | 21 | JNK2α2(h) | 40 | Plk4(h) | 15 |
| CaMKIγ(h) | -13 | JNK3(h) | 40 | PRAK(h) | 30 |
| CaMKIδ(h) | 26 | KDR(h) | -12 | PRK1(h) | -12 |
| CaMKK1(h) | 35 | Lck(h) | 44 | PRK2(h) | -4 |
| CaMKK2(h) | 63 | Lck(h) activated | 46 | PRKG2(h) | -9 |
| Cdc7/cyclinB 1(h) | 38 | LIMK1(h) | -1 | PrKX(h) | -10 |
| CDK1/cyclin B (h) | 78 | LIMK2(h) | 19 | PRP4(h) | 5 |
| CDK12/cyclinK(h) | -8 | LKB1(h) | 55 | PTK5(h) | 48 |
| CDK13/cyclinK(h) | 66 | LOK(h) | 25 | Pyk2(h) | -5 |
| CDK14/cyclinY(h) | 55 | LRRK2(h) | 10 | Ret (V804L)(h) | 50 |
| CDK16/cyclinY(h) | 51 | LTK(h) | 5 | Ret(h) | 50 |

(continued)

| Nomenclature of kinases | Inhibition ratio/% | Nomenclature of kinases | Inhibition ratio/% | Nomenclature of kinases | Inhibition ratio/% |
|---|---|---|---|---|---|
| CDK17/cyclinY(h) | 70 | Lyn(h) | 55 | Ret(V804M)(h) | 45 |
| CDK18/cyclinY(h) | 74 | Lyn(m) | 19 | RIPK1(h) | 100 |
| CDK2/cyclin A (h) | 24 | MAK(h) | 45 | RIPK2(h) | -7 |
| CDK2/cyclin E (h) | 36 | MAP4K3(h) | 28 | ROCK-I(h) | -10 |
| CDK3/cyclin E (h) | 65 | MAP4K4(h) | -3 | ROCK-II(h) | 7 |
| CDK4/cyclin D3(h) | 4 | MAP4K5(h) | 6 | ROCK-II(r) | 52 |
| CDK5/p25(h ) | 46 | MAPK1(h) | 33 | Ron(h) | 17 |
| CDK5/p35(h ) | -8 | MAPK2(h) | 40 | Ros(h) | -18 |
| CDK6/cyclin D3(h) | 80 | MAPK2(m) | 33 | Rse(h) | 53 |
| CDK7/cyclin H/MAT1(h) | 53 | MAPKAP-K2( h) | -1 | Rsk1(h) | 41 |
| CDK9/cyclin T1(h) | 61 | MAPKAP-K3( h) | -12 | Rsk1(r) | 20 |
| CDKL1(h) | 35 | MARK1(h) | -13 | Rsk2(h) | 4 |
| CDKL2(h) | 62 | MARK3(h) | -3 | Rsk3(h) | 27 |
| CDKL3(h) | -5 | MARK4(h) | 38 | Rsk4(h) | 3 |
| CDKL4(h) | 76 | MEK1(h) | 43 | SAPK2a(h) | 17 |
| ChaK1(h) | 66 | MEK2(h) | 13 | SAPK2a(T106M) (h) | -20 |
| CHK1(h) | -2 | MEKK2(h) | -10 | SAPK2b(h) | 36 |
| CHK2(h) | 8 | MEKK3(h) | 14 | SAPK3(h) | 46 |
| CHK2(I157T ) (h) | 61 | MELK(h) | 1 | SAPK4(h) | 12 |
| CHK2(R145W)(h) | 69 | Mer(h) | -6 | SBK1(h) | -19 |
| CK1(y) | 30 | Met(D1246H) (h) | 34 | SGK(h) | 51 |
| CK1γ1(h) | 19 | Met(D1246N) (h) | -11 | SGK2(h) | -18 |
| CK1γ2(h) | 50 | Met(h) | -29 | SGK3(h) | 41 |
| CK1γ3(h) | -10 | Met(M1268T) (h) | -10 | SIK(h) | 54 |
| CK1δ(h) | -12 | Met(Y1248C)( h) | 11 | SIK2(h) | -4 |
| CK1ε(h) | 59 | Met(Y1248D)( h) | 13 | SIK3(h) | 47 |
| CK2(h) | 44 | Met(Y1248H)( h) | -11 | SLK(h) | 15 |
| CK2α1(h) | 58 | MINK(h) | 53 | Snk(h) | 1 |
| CK2α2(h) | 25 | MKK3(h) | 38 | SNRK(h) | 25 |

(continued)

| Nomenclature of kinases | Inhibition ratio/% | Nomenclature of kinases | Inhibition ratio/% | Nomenclature of kinases | Inhibition ratio/% |
|---|---|---|---|---|---|
| cKit(D816H)(h) | 42 | MKK4(m) | 27 | Src(1-530)(h) | 20 |
| cKit(D816V)(h) | 75 | MKK6(h) | 30 | Src(T341M)(h) | -6 |
| cKit(h) | 21 | MLCK(h) | 14 | SRMS(h) | 43 |
| cKit(V560G)(h) | 65 | MLK1(h) | 94 | SRPK1(h) | 50 |
| cKit(V654A)(h) | 68 | MLK2(h) | 99 | SRPK2(h) | -16 |
| CLIK1(h) | 22 | MLK3(h) | 102 | STK16(h) | 20 |
| CLK1(h) | 80 | Mnk2(h) | 51 | STK25(h) | 26 |
| CLK2(h) | 77 | MOK(h) | -15 | STK32A(h) | 55 |
| CLK3(h) | 35 | MRCKα(h) | -19 | STK32B(h) | 27 |
| CLK4(h) | 79 | MRCKβ(h) | -4 | STK32C(h) | 22 |
| c-RAF(h) | -13 | MRCKγ(h) | 55 | STK33(h) | 23 |
| CRIK(h) | -7 | MSK1(h) | -20 | Syk(h) | 69 |
| CSK(h) | 60 | MSK2(h) | 9 | TAF1L(h) | 48 |
| cSRC(h) | -17 | MSSK1(h) | -12 | TAK1(h) | 9 |
| DAPK1(h) | 40 | MST1(h) | 20 | TAO1(h) | 50 |
| DAPK2(h) | 3 | MST2(h) | -14 | TAO2(h) | -14 |
| DCAMKL2(h) | 74 | MST3(h) | 33 | TAO3(h) | 6 |
| DCAMKL3(h) | 33 | MST4(h) | 12 | TBK1(h) | 13 |
| DDR1(h) | 21 | mTOR(h) | -11 | Tec(h) activated | -20 |
| DDR2(h) | 20 | mTOR/FKBP 12(h) | 36 | TGFBR1(h) | -3 |
| DMPK(h) | -2 | MuSK(h) | 13 | TGFBR2(h) | -6 |
| DNA-PK(h) | 47 | MYLK2(h) | -8 | Tie2 (h) | 30 |
| DRAK1(h) | 30 | MYO3B(h) | -18 | Tie2(R849W)(h) | 16 |
| DRAK2(h) | 37 | NDR2(h) | 46 | Tie2(Y897S)(h) | 18 |
| DYRK1A(h) | 2 | NEK1(h) | 50 | TLK1(h) | 11 |
| DYRK1B(h) | -1 | NEK11(h) | 44 | TLK2(h) | 25 |
| DYRK2(h) | 2 | NEK2(h) | 24 | TNIK(h) | 59 |
| DYRK3(h) | 10 | NEK3(h) | 14 | TRB2(h) | -5 |
| eEF-2K(h) | 26 | NEK4(h) | 27 | TrkA(h) | -12 |
| EGFR(h) | 10 | NEK6(h) | 23 | TrkB(h) | 0 |
| EGFR(L858R)(h) | -5 | NEK7(h) | 43 | TrkC(h) | 28 |
| EGFR(L861Q)(h) | 75 | NEK9(h) | -10 | TSSK1(h) | -8 |

(continued)

| Nomenclature of kinases | Inhibition ratio/% | Nomenclature of kinases | Inhibition ratio/% | Nomenclature of kinases | Inhibition ratio/% |
|---|---|---|---|---|---|
| EGFR(T790 M)(h) | -10 | NIM1(h) | 10 | TSSK2(h) | 52 |
| EGFR(T790 M,L858R)(h) | 52 | NLK(h) | 16 | TSSK3(h) | 0 |
| EphA1(h) | -12 | NUAK2(h) | 30 | TSSK4(h) | 49 |
| EphA2(h) | 15 | p70S6K(h) | -18 | TTBK1(h) | 13 |
| EphA3(h) | 6 | PAK1(h) | 18 | TTBK2(h) | -16 |
| EphA4(h) | 11 | PAK2(h) | -14 | TTK(h) | 13 |
| EphA5(h) | 4 | PAK3(h) | -19 | Txk(h) | -12 |
| EphA7(h) | 20 | PAK4(h) | 5 | TYK2(h) | 50 |
| EphA8(h) | 4 | PAK5(h) | 47 | ULK1(h) | 45 |
| EphB1(h) | 1 | PAK6(h) | 49 | ULK2(h) | 55 |
| EphB2(h) | 48 | PAR-1Bα(h) | 33 | ULK3(h) | 3 |
| EphB3(h) | 26 | PASK(h) | 53 | VRK1(h) | 46 |
| EphB4(h) | 56 | PDGFRα(D84 2V)(h) | 37 | VRK2(h) | -18 |
| ErbB2(h) | 47 | PDGFRα(h) | 51 | Wee1(h) | -1 |
| ErbB4(h) | 63 | PDGFRα(V56 1D)(h) | 38 | Wee1B(h) | -2 |
| FAK(h) | -3 | PDGFRβ(h) | 49 | WNK1(h) | 45 |
| Fer(h) | 10 | PDHK2(h) | 32 | WNK2(h) | 6 |
| Fes(h) | 54 | PDHK4(h) | -18 | WNK3(h) | -20 |
| FGFR1(h) | 70 | PDK1(h) | 18 | WNK4(h) | 48 |
| FGFR1(V56 1M)(h) | 45 | PEK(h) | 36 | Yes(h) | 29 |
| FGFR2(h) | -13 | PhKγ1(h) | -12 | ZAK(h) | 59 |
| FGFR2(N54 9H)(h) | 12 | PhKγ2(h) | 48 | ZAP-70(h) | 1 |
| FGFR3(h) | 40 | PI3 Kinase (p110a(E542 K) /p85a)(h) | -4 | ZIPK(h) | 6 |
| FGFR4(h) | 37 | PI3 Kinase (p110a(E542 K) /p85a)(m) | -1 | | |

The selectivity profiles for Compounds 34 and 94 were plotted according to the data in Table 3 and Table 4. (Reference http://bcb.med.usherbrooke.ca/kinomerenderLig.php for plotting schemes and procedures), as shown in FIG. 1 and FIG. 2. Wherein the red circular spot indicated the kinase were inhibited obviously , and the larger the radius of the red spot, the higher the inhibition against the kinase, and vice versa. It can be seen from Tables 3 and 4 and FIGs. 1 and 2 that Compounds 34 and 94 have good kinase selectivity.

As shown in FIG. 3, Compound 94 inhibited the programmed necrosis model of HT-29 with an $EC_{50}$ value of 0.012 nM, while its series of Compounds 34 and 46 inhibited the programmed necrosis model of HT-29 with $EC_{50}$ values of 0.117 nM and 0.070 nM, respectively. The above experimental results demonstrated that Compound 94 of the present invention and series of compounds thereof are able to protect HT-29 cells from TSZ-induced necrosis-like apoptosis.

**Example 6 Regulation of Compounds 94 and 46 of the present invention on RIPK1 signaling pathway**

**1) Experimental materials**

TNF α was purchased from PeproTech Inc., Smac mimetic and Z-VAD-FMK was purchased from Selleck Inc., RIPA lysis buffer was purchased from Beyotime Institute of Biotechnology, cocktail was purchased from MedChemExpress (MCE) Limited, PMSF protease inhibitors, sodium dodecylsulfonate SDS, and glycine were purchased from Sigma Inc., acrylamide, tris(hydroxymethyl)aminomethane Tris, ammonium persulfate APS and N,N,N',N'-tetramethyl ethylenediamine TEMED were purchased from Wuhan Servicebio Technology Co., Ltd.

**2) Experimental method**

Extraction of total cellular protein: after the cells were treated by adding the corresponding concentration of Compounds 94 and 46 or blank vehicle to the cell supernatant and induced with TNF α/Smac mimetic/Z-VAD-FMK combination for a certain period of time, the supernatant was discarded, followed by washing three times with pre-cooled PBS or normal saline, then an appropriate volume of RIPA lysis buffer (containing 1% cocktail and 1% PMSF protease inhibitor) was added, the mixture was immediately placed on ice horizontally for lysis of 15 min, then the cell lysis buffer was scraped with a scraper and transferred to a 1.5 ml EP tube, and the cells were disrupted with a sonicator. The tubes were then centrifuged in a low temperature high speed centrifuge (12000 rpm, 15 min) to remove cell debris. Protein quantification was then conducted with a BCA method, a standard curve was plotted with protein standards, a concentration of each protein sample was calculated according to the standard curve, and the concentration of each group protein sample was leveled by calculation, 5x protein loading buffer was added, and the sample was placed in a 100°C dry thermostat to maintain 10 min, followed by directly loading for electrophoresis or aliquoting for storing at -20°C for use. Protein samples were protected from repeated freezing and thawing. After the protein samples were prepared, the proteins were separated by polyacrylamide gel electrophoresis(SDS-PAGE). The polyacrylamide gel preparation formula was shown in Table 5. Generally, 10% separation gel was used for separation. After electrophoretic separation, the protein was transferred onto the PVDF membrane sufficiently by using a trough-type wet-transfer method, then the PVDF membrane was placed in 5% skim milk powder (prepared in TBS/T) for blocking for more than 2 h at room temperature, the PVDF membrane band containing the corresponding protein was obtained according to the molecular weight of the desired protein, the primary antibody was diluted according to the dilution ratio recommended in the instruction for use of the antibody, and the protein band was incubated at 4°C overnight. The next day, each band was fetched and rinsed with TBS/T buffer (5 min, 3 times), and HRP-labeled secondary antibody diluted 1: 5000 was added, the mixture was incubated with shaking at 37°C for 1 h, then eluted with TBS/T to remove excess antibody, then the HRP substrate was evenly added dropwise on the PVDF membrane, then developed and photographed in a rapid gel imaging system.

Table 5 Formulation of separation gel and spacer gel in SDS-PAGE

| Reagents | 6% Spacer gel (mL) | 10% Separation gel (mL) |
|---|---|---|
| Ultrapure water | 1.4 | 1.9 |
| 30% Acrylamide | 0.33 | 1.7 |
| 1.5 mol/L Tris-HCl | - | 1.3 |
| 1.0 mol/L Tris-HCl | 0.25 | - |
| 10% SDS | 0.02 | 0.05 |
| 10% Ammonium persulfate | 0.02 | 0.05 |
| TEMED | 0.002 | 0.002 |
| Total | 2 | 5 |

**3) Experimental results**

Results were as shown in FIG. 4, Compounds 94 and 46 affected the phosphorylation of RIPK3 and MLKL downstream of RIPK1 by inhibiting its autophosphorylation, and this inhibitory activity had a concentration-dependent effect, consistent with the protective activity at the cellular level, indicating that Compounds 94 and 46 inhibited programmed necrosis by blocking the programmed necrosis signaling pathway.

**Example 7 Protective effect of Compounds 94 and 46 of the present invention on TNF α-induced mouse SIRS model**

**1) Experimental materials**

TNF α was purchased from PeproTech Inc., castor oil was purchased from MCE Limited, and sterile normal saline was purchased from Chengdu Baisheng Kechuang Biotechnology Co., Ltd.

**2) Experimental method**

TNF α formulated in sterile normal saline was administered at a dose of 500 μg/kg by tail vein injection for modeling. The body temperature of the mice was measured by an infrared electric thermometer. The orally-administrated vehicle was 25% castor oil in ethanol and 75% normal saline. Compounds 94 and 46 and RIPK1 positive compound GSK2982772 were administered orally before modeling with a dose of 40 mg/kg. Body temperature and the survival of mice were recorded every hour and recorded for 24 h and 48 h after 11 h. Survival curves were fitted using Graphpad Prism 5.0 software.

**3) Experimental results**

A classical inflammation model SIRS was selected for this experiment to verify whether Compounds 94 and 46 also had activity against RIPK1 and inhibition against programmed necrosis in vivo. Systemic inflammatory response syndrome (SIRS) is a systemic inflammatory response which is caused by the fact that infection or non-infection factors act on the body to cause the body to be out of control and self-destructed. Critical patients are prone to SIRS due to the decreased ability of compensatory anti-inflammatory response and metabolic dysfunction. The TNF α-induced SIRS disease model was shown to be highly correlated with RIPK1-dependent apoptosis and programmed necrosis in long-term studies. This experiment established a TNF α-induced SIRS mouse model to evaluate the in vivo activity of compounds of the present invention. TNF α formulated in sterile normal saline was administered to mice at a dose of 500 μg/kg by tail vein injection for modeling. Since the measurement of rectal temperature would cause mechanical injury to mice and may interfere with the experimental results, an infrared electric thermometer was used to measure the body temperature of mice and the survival condition was observed. Compounds 94 and 46 were administered at a dose of 40 mg/kg once prior to modeling. The Vehicle group was given the corresponding vehicle control.

Experimental results as shown in FIG. 5, after the tail vein injection of TNF α, the Vehicle group mice developed symptoms such as continuous decreases in body temperature, decrease in activity within a short period of time, and began to die at 24 h. However, in the groups administered with Compounds 94 and 46, except for individual mice, the body temperature of the rest mice was relatively normal within 11 h after TNF α injection, the viability of mice was significantly better than that of the Vehicle group, the overall mortality was significantly reduced compared with the Vehicle group, and Compound 94 showed better protective effect than positive drug GSK2982772. It was suggested that Compounds 94 and 46 exerted a significant anti-inflammatory effect by inhibiting RIPK1 in vivo, counteracting the death induced by high doses of TNF α in mice.

**Example 8 Protective effect of Compound 94 of the present invention on DSS-induced mouse IBD model**

**1) Experimental materials**

Dextran sulfate sodium salt (DSS, 36000-50000 KD) was purchased from Shanghai Yeasen Biotech Co., Ltd., 0.C.T tissue fixtures was purchased from Servicebio Technology Co., Ltd. PEG300 and Tween-80 were purchased from MCE Limited, and DMSO was purchased from Sigma Inc.

**2) Experimental method**

Female C57BU6 mice weighing 17-20 g were used. Mice in experimental groups were given DSS (2.5% wt/vol) dissolved in drinking water ad libitum for 7 days (from day 0 to day 7). Fresh DSS solution was changed on Days 2, 4, and 6, respectively. All water was changed to normal drinking water on Day 7 and the mice were randomized (7 per group) for oral administration: Vehicle group (10% DMSO, 40% PEG300, 5% Tween-80, 45% normal saline), GSK3145095 group (40 mg/kg), and Compound 94 group (40 mg/kg). The Control group was given normal drinking water daily and corresponding vehicles orally starting on day 7. Body weights and survival rates of mice were recorded daily. Survival curves were fitted using Graphpad Prism 5.0 software. Mice (2/group) were sacrificed on day 12 and observed for changes in colon length.

### 3) Experimental results

A classical inflammatory bowel disease (IBD) was selected for this experiment to verify whether Compound 94 also had activity against RIPK1 and inhibition against programmed necrosis in an in vivo autoimmune disease model. Inflammatory bowel disease (IBD) refers to abnormal immune-mediated intestinal inflammation caused by environmental, genetic, infection, immune, and other reasons, which is a chronic, non-specific inflammatory bowel disease. The major pathological types of IBD are ulcerative colitis (UC) and Crohn's disease (CD). The pathogenesis of IBD is not clear yet, but excessive apoptosis of intestinal epithelial cells, impaired intestinal mucosal barrier, and increased permeability of intestinal epithelial cells were one of the causes of IBD. Some studies had shown that programmed necrosis plays an important role in the pathogenesis of IBD.

The mice in the experimental group had bloody stool and weight loss 7 days after modeling, indicating that a successful model was created. As shown in FIG. 6, after 7 days of modeling, the mice were randomly divided into groups for drug administration, the Vehicle group and GSK3145095 group had a continuous declination in body weight, and occurrence of murine death, while the mice treated with Compound 94 had a gradual increase in body weight, and there was no death by 14 days, and the mice vitality was significantly better than that of Vehicle group. As shown in FIG. 7, when mice (2/group) were sacrificed on day 12, the colon length of mice in the Vehicle group was significantly shorter than that of mice in the Control group, while the colon length of mice in Compound 94 group was longer than that of mice in Vehicle group, which was basically equivalent to that of mice in Control group, indicating that Compound 94 could alleviate the shortening of mice colon induced by DSS and showed better therapeutic effect than positive drug GSK3145095. It was suggested that Compound 94 exerted a significant anti-inflammatory effect by inhibiting RIPK1 in vivo, alleviating DSS-induced colitis in mice.

## Example 9 In vivo pharmacokinetic studies of representative compounds of the present invention

### 1) Experimental method

An appropriate amount of a representative compound was weighed accurately, dissolved with a final volume of 5% DMSO, 40% PEG400, and 55% saline, and the mixture was blended thoroughly via a vortex shaker or an ultrasonic instrument to obtain a clear dosing solution of 1 mg/mL, the dose to rats (10 mg/kg) based on body weight was calculated, and oral medication was conducted intravenously or intragastrically. Blood was collected from rats at different times after administration at the following time points: 0.083 h, 0.25 h, 0.5 h, 1 h, 2 h, 4 h, 8 h, and 24 h after intravenous administration; 0.25 h, 0.5 h, 1 h, 2 h, 4 h, 6 h, 8 h, and 24 h after oral administration. About 0.20 mL of blood was collected via jugular vein or other suitable means for each sample at the corresponding time, with heparin sodium for anticoagulation; the blood sample was placed on ice after collection and centrifuged within 1 h to separate the plasma (centrifugation conditions: centrifugal force 6800g, 6 min, 2-8°C). The collected plasma samples were stored in a refrigerator at -80°C before analysis, and the remaining plasma samples continued to be temporarily stored in the refrigerator at -80°C for one month after analysis.

Pharmacokinetic parameters such as AUC (0-t), $T_{1/2}$, $C_{max}$, $T_{max}$, and MRT were calculated using WinNonlin based on plasma concentration data at different time points. The plasma drug concentration-time curve was plotted with BLQ recorded as 0. For the calculation of pharmacokinetic parameters, the concentration before administration was calculated as 0; BLQ before $C_{max}$ (including "No peak") was calculated as 0; no BLQ (including "No peak") appeared after $C_{max}$ participated in the calculation. The experiment was completed by Shanghai Medicilon Inc.

### 2) Experimental results

As can be seen from the results in Table 6, rats had a good oral absorption of six representative compounds after a single oral administration of 10 mg/kg of the representative compound, with a maximum oral bioavailability of 60.85%. It can be seen from preliminary pharmacokinetic experiments that the accumulated drug concentration in the body after a single administration of the compound could reach the half maximal inhibitory concentration value of inhibiting RIPK1 and necrosis signaling pathway, indicating that this series of compounds was a promising RIPK1 inhibitor.

Table 6 Pharmacokinetic parameters in rats following oral administration of representative compounds

| Pharmacokinetic parameters | 34 | 94 | 168 | 174 | 191 | 193 |
|---|---|---|---|---|---|---|
| $T_{1/2}$ (h) | 2.06 | 7.02 | 3.10 | 1.99 | 4.67 | 2.74 |
| $C_{max}$ (ng/mL) | 852.42 | 330.39 | 6361.34 | 3838.25 | 2131.15 | 2876.88 |
| $T_{max}$ (h) | 4.00 | 1.67 | 0.33 | 0.42 | 0.33 | 2.25 |

(continued)

| | | | | | | |
|---|---|---|---|---|---|---|
| AUC(0-t) h*ng/mL | 4445.10 | 1852.52 | 15253.55 | 11510.55 | 4556.24 | 14817.49 |
| Bioavailability (%) | 7.89 | 12.05 | 60.85 | 34.96 | 20.40 | 31.67 |

**Example 10 Effect of Compound 94 of the present invention on hERG potassium ion channels**

**1) Experimental method**

Stable HEK-hERG cells were washed with DPBS, digested with Trypsin or Tryple solution, resuspended in culture medium, and stored in centrifuge tubes for use. Prior to be recorded by patch clamp, the cells were added dropwise into small petri dishes to ensure that the cells had a certain density and that the cells were in a single detached state.

hERG currents were recorded with a whole-cell patch clamp technique. The cell suspension was placed in a small petri dish and placed on an objective table of an inverted microscope. After attachment, the cells were perfused with extracellular fluid at a recommended flow rate of 1-2 mL/min. The glass microelectrode was pulled in two steps by a microelectrode puller, and the resistance in electrode fill solution was 2-5 MΩ.

After establishing the whole-cell recording mode, the clamping potential was kept at -80 mV. Depolarization voltage was given to +60 mV for 850 ms, then repolarization was maintained to -50 mV for 1275 ms to elicit the hERG tail current. This set of pulses was repeated every 15 seconds throughout the experiment.

After the current was stable, continuous extracellular perfusion administration was conducted from low concentration to high concentration. The perfusion was continued from a low concentration until the pharmaceutical effect was stable, and then the perfusion of the next concentration was conducted. Compound 94 (0.3, 1, 3, 10, 30 μM) and the positive compound terfenadine were tested on the blocking effect of the hERG tail current ($N \geq 2$) in this experiment, respectively.

Pharmaceutical effect is stable was defined as: the last 5 stimulation bar current values for each concentration dosing phase varied by less than 10% of the mean value (when the current was greater than or equal to 200 pA) or less than 30% of the mean value (when the current was less than 200 pA), it could be considered as stable; if it was unstable, this concentration data would not be taken.

Stimulation distribution and signal acquisition were conducted by PatchMaster software; the signal was amplified with a patch-clamp amplifier, with a filter of 10 KHz.

Further data analysis and curve fitting were conducted using FitMaster, EXCEL, Graphpad Prism, and SPSS 21.0. Data were expressed as mean and standard deviation.

In data processing, the peak value of the tail current and its baseline were corrected during judging the blocking effect on hERG. The effect of each compound at different concentrations was expressed as inhibition against the tail current. % Inhibition = 100 × (peak tail current before dosing - peak tail current after dosing) / peak tail current before dosing. SD≤ 15 of inhibition of all cells at each concentration was taken as acceptance criteria (except for abnormal data).

$IC_{50}$ values were obtained by fitting the Hill equation (if applicable):

$$y = \left[ \frac{\max - \min}{1 + \left( \frac{[\text{drug}]}{IC_{50}} \right)^{n_H}} \right] + \min$$

The experiment was completed by Shanghai Medicilon Inc.

**2) Experimental results**

The concentration-response curve for the inhibition against hERG current by the positive compound terfenadine was shown in FIG. 8. According to the results, the effect of terfenadine on hERG potassium channel was concentration-dependent, and the inhibition against terfenadine on hERG current had an $IC_{50}$ value of 0.045 μM (N = 3) by fitting the Hill equation, which was consistent with the literature report.

Final concentrations of Compound 94 were 0.3, 1, 3, 10, and 30 μM, and final concentration of DMSO in extracellular fluid was 0.3%. The inhibitory concentration-response curve of Compound 94 on hERG current was shown in FIG. 8. Under the experimental conditions, Compound 94 had no significant inhibitory effect on the hERG potassium channel current at various concentrations (0.3, 1, 3, 10, and 30 μM). The average inhibitory ratio of Compound 94 on

hERG potassium channel current at 30 μM was 3.40% (N = 3). Therefore, the inhibition of Compound 94 on hERG current with an $IC_{50}$ value of greater than 30 μM. This indicated that compound 94 did not cause side effects due to significant inhibition against the hERG potassium channel current.

In summary, the series of compounds provided herein may protect HT-29 cells from TSZ-induced necrosis-like apoptosis with good kinase selectivity. According to the mechanism of action, the phosphorylation of RIPK3 and MLKL downstream of RIPK1 was influenced by inhibiting the autophosphorylation of RIPK1, thus inhibiting programmed necrosis by blocking the programmed necrosis signaling pathway. The series of compounds provided by the present invention exert a significant anti-inflammatory effect by inhibiting RIPK1 in vivo, and have no significant inhibitory effect on hERG potassium channel current at each concentration, without causing side effects due to significant inhibitory effect on hERG potassium channel current. Thus, the series of compounds provided herein have the potential for use as active ingredients of RIPK1 inhibitors and anti-inflammatory drugs.

## Claims

1. A compound represented by Formula I, or a stereisomer, or a pharmaceutically acceptable salt thereof:

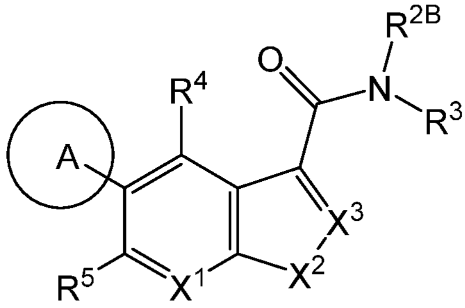

Formula I,

wherein,

$X^1$ and $X^3$ are independently selected from $-CR^6-$ or N;
$X^2$ is selected from $-NR^1-$ or -CH = CH-;
wherein $R^1$ is selected from hydrogen, substituted or unsubstituted $C_1$-$C_{10}$ alkyl, substituted or unsubstituted $C_1$-$C_{10}$ ether group, substituted or unsubstituted $C_3$-$C_{10}$ cycloalkyl, substituted or unsubstituted 3-10-membered heterocycloalkyl, substituted or unsubstituted $C_6$-$C_{20}$ aryl, substituted or unsubstituted 3-20-membered heteroaryl; wherein the substituent is deuterium, $C_1$-$C_{10}$ alkyl, cyano, hydroxyl, carboxyl, halogen, or nitro;
$R^{2B}$ is selected from

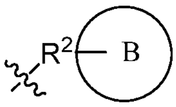

;

wherein $R^2$ is selected from $C_0$-$C_6$ alkylene substituted by one or two $R^{21}$ or unsubstituted; wherein $R^{21}$ is selected from substituted or unsubstituted $C_1$-$C_{10}$ alkyl, substituted or unsubstituted $C_3$-$C_{10}$ cycloalkyl, cyano, hydroxyl, carboxyl, halogen, or nitro; wherein the substituent is cyano, hydroxyl, carboxyl, halogen, or nitro;
ring B is selected from $C_4$-$C_{10}$ aryl substituted by one, two, or three $R^{22}$ or unsubstituted, 4-10-membered heteroaryl substituted by one, two, or three $R^{22}$ or unsubstituted, or $C_3$-$C_{10}$ cycloalkyl substituted by one, two, or three $R^{22}$ or unsubstituted; wherein each $R^{22}$ is independently selected from substituted or unsubstituted $C_1$-$C_{10}$ alkyl, substituted or unsubstituted $C_1$-$C_6$ alkoxy, substituted or unsubstituted 4-10-membered heterocycloalkyl, cyano, hydroxy, carboxyl, halogen, or nitro; or two $R^{22}$ are joined to form a substituted or unsubstituted 4-10-membered heterocycloalkyl; wherein the substituent is $C_1$-$C_{10}$ alkyl, cyano, hydroxyl, carboxyl, halogen, or nitro;
$R^3$ is selected from hydrogen, substituted or unsubstituted $C_1$-$C_{10}$ alkyl; wherein the substituent is cyano, hydroxyl, carboxyl, halogen, or nitro;
or $R^{2B}$ and $R^3$ are joined to form substituted or unsubstituted 3-10-membered heterocycloalkyl; wherein the

substituent is $C_1$-$C_{10}$ alkyl, $C_4$-$C_{10}$ aryl, $C_4$-$C_{10}$ aryl substituted by one or two $R^{31}$, cyano, hydroxyl, carboxyl, halogen, or nitro; wherein the $R^{31}$ is selected from $C_1$-$C_{10}$ alkyl or halogen;

$R^4$, $R^5$, and $R^6$ are each independently selected from hydrogen, $C_1$-$C_{10}$ alkyl, cyano, hydroxyl, carboxyl, halogen, or nitro;

ring A is selected from $C_4$-$C_{10}$ aryl substituted by one or two $R^A$ or unsubstituted, 4-10-membered heteroaryl substituted by one or two $R^A$ or unsubstituted; wherein $R^A$ is selected from substituted or unsubstituted amino,

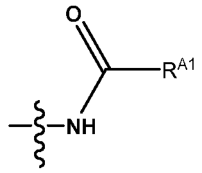 , 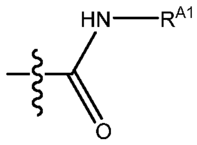 ,

substituted or unsubstituted $C_1$-$C_{10}$ alkyl, substituted or unsubstituted $C_4$-$C_{10}$ aryl, substituted or unsubstituted 4-10-membered heteroaryl, substituted or unsubstituted 3-10-membered heterocycloalkyl; wherein the substituent is -$R^{A2}$-$R^{A3}$, $R^{A4}$, $C_1$-$C_{10}$ alkyl, halogen-substituted $C_1$-$C_{10}$ alkyl, cyano, hydroxyl, carboxyl, halogen, or nitro; wherein $R^{A4}$ is selected from 3-10-membered heterocycloalkyl substituted by -$R^{A2}$-$R^{A3}$ or unsubstituted;

$R^{A1}$ is selected from substituted or unsubstituted $C_1$-$C_{10}$ alkyl, substituted or unsubstituted $C_1$-$C_{10}$ alkenyl, substituted or unsubstituted $C_3$-$C_{10}$ cycloalkyl, substituted or unsubstituted 3-10-membered heterocycloalkyl, substituted or unsubstituted $C_1$-$C_6$ alkoxy, substituted or unsubstituted $C_2$-$C_6$ ether group, or substituted or unsubstituted $C_2$-$C_6$ amine; wherein the substituent is $C_1$-$C_{10}$ alkyl, hydroxyl-substituted $C_1$-$C_{10}$ alkyl, a $C_1$-$C_{10}$ ester group, 3-10-membered heterocycloalkyl, amino, amine, cyano, hydroxyl, carboxyl, halogen, or nitro;

$R^{A2}$ is selected from substituted or unsubstituted $C_0$-$C_6$ alkylene, carbonyl; wherein the substituent is $C_1$-$C_{10}$ alkyl, cyano, hydroxyl, carboxyl, halogen, or nitro;

$R^{A3}$ is selected from substituted or unsubstituted 3-10-membered heterocycloalkyl; wherein the substituent is $C_1$-$C_{10}$ alkyl, cyano, hydroxyl, carboxyl, halogen, or nitro.

**2.** The compound according to claim 1, or a stereoisomer, or a pharmaceutically acceptable salt thereof, **characterized in that** the compound of Formula I is represented by Formula II:

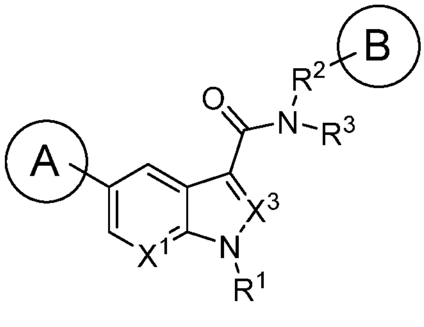

Formula II

wherein,

$R^1$ is selected from hydrogen, substituted or unsubstituted $C_1$-$C_{10}$ alkyl, substituted or unsubstituted $C_3$-$C_{10}$ cycloalkyl, substituted or unsubstituted 3-10-membered heterocycloalkyl, substituted or unsubstituted $C_6$-$C_{20}$ aryl, substituted or unsubstituted 3-20-membered heteroaryl; wherein the substituent is $C_1$-$C_{10}$ alkyl, cyano, hydroxyl, carboxyl, halogen, or nitro;

$R^2$ is selected from $C_0$-$C_6$ alkylene substituted by one $R^{21}$ or unsubstituted; wherein $R^{21}$ is selected from substituted or unsubstituted $C_1$-$C_{10}$ alkyl, cyano, hydroxy, carboxyl, halogen, or nitro; wherein the substituent is cyano, hydroxyl, carboxyl, halogen, or nitro;

ring B is selected from $C_4$-$C_{10}$ aryl substituted by one, two, or three $R^{22}$ or unsubstituted, or 4-10-membered heteroaryl substituted by one, two, or three $R^{22}$ or unsubstituted; wherein each $R^{22}$ is independently selected from substituted or unsubstituted $C_1$-$C_{10}$ alkyl, substituted or unsubstituted $C_1$-$C_6$ alkoxy, substituted or unsubstituted 4-10-membered heterocycloalkyl, cyano, hydroxy, carboxyl, halogen, or nitro; or two $R^{22}$ are joined to form a substituted or unsubstituted 4-10-membered heterocycloalkyl; wherein the substituent is $C_1$-$C_{10}$ alkyl,

cyano, hydroxyl, carboxyl, halogen, or nitro;

$R^3$ is selected from hydrogen, substituted or unsubstituted $C_1$-$C_{10}$ alkyl; wherein the substituent is cyano, hydroxyl, carboxyl, halogen, or nitro;

or $R^2$ and $R^3$ are joined to form a substituted or unsubstituted 3-10-membered heterocycloalkyl; wherein the substituent is $C_1$-$C_{10}$ alkyl, cyano, hydroxyl, carboxyl, halogen, or nitro;

$X^1$ and $X^3$ are independently selected from CH or N;

ring A is selected from $C_4$-$C_{10}$ aryl substituted by one or two $R^A$ or unsubstituted, 4-10-membered heteroaryl substituted by one or two $R^A$ or unsubstituted; wherein $R^A$ is selected from amino,

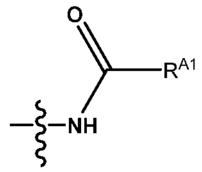 , 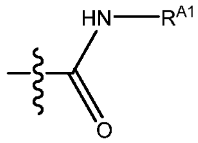 ,

substituted or unsubstituted $C_1$-$C_{10}$ alkyl, substituted or unsubstituted $C_4$-$C_{10}$ aryl, substituted or unsubstituted 4-10-membered heteroaryl, substituted or unsubstituted 3-10-membered heterocycloalkyl; wherein the substituent is -$R^{A2}$-$R^{A3}$, $R^{A4}$, $C_1$-$C_{10}$ alkyl, cyano, hydroxyl, carboxyl, halogen, or nitro; wherein $R^{A4}$ is selected from 3-10-membered heterocycloalkyl substituted by -$R^{A2}$-$R^{A3}$ or unsubstituted;

$R^{A1}$ is selected from substituted or unsubstituted $C_1$-$C_{10}$ alkyl, substituted or unsubstituted $C_1$-$C_{10}$ alkenyl, substituted or unsubstituted $C_3$-$C_{10}$ cycloalkyl, substituted or unsubstituted 3-10-membered heterocycloalkyl, substituted or unsubstituted $C_1$-$C_6$ alkoxy, or substituted or unsubstituted $C_2$-$C_6$ ether group; wherein, the substituent is $C_1$-$C_{10}$ alkyl, 3-10-membered heterocycloalkyl, amino, amine, cyano, hydroxyl, carboxyl, halogen, or nitro;

$R^{A2}$ is selected from substituted or unsubstituted $C_0$-$C_6$ alkylene or carbonyl; wherein the substituent is $C_1$-$C_{10}$ alkyl, cyano, hydroxyl, carboxyl, halogen, or nitro;

$R^{A3}$ is selected from substituted or unsubstituted 3-10-membered heterocycloalkyl; wherein the substituent is $C_1$-$C_{10}$ alkyl, cyano, hydroxyl, carboxyl, halogen, or nitro.

3. The compound according to claim 1 or 2, or a stereoisomer, or a pharmaceutically acceptable salt thereof, **characterized in that** the compound of Formula I is represented by Formula III or IV:

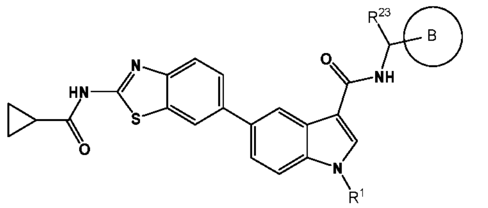

Formula III

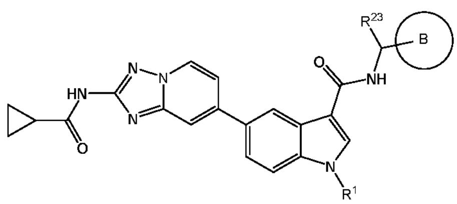

Formula IV

wherein $R^1$ is selected from hydrogen or $C_1$-$C_4$ alkyl; $R^{23}$ is selected from hydrogen or methyl; ring B is selected from phenyl substituted by one or two $R^{22}$ or unsubstituted; wherein each $R^{22}$ is independently selected from F, Cl, cyano, methyl, trifluoromethyl, methoxy, or trifluoromethoxy.

4. The compound according to claim 1, or a stereoisomer, or a pharmaceutically acceptable salt thereof, **characterized in that** the compound of Formula I is represented by Formula V:

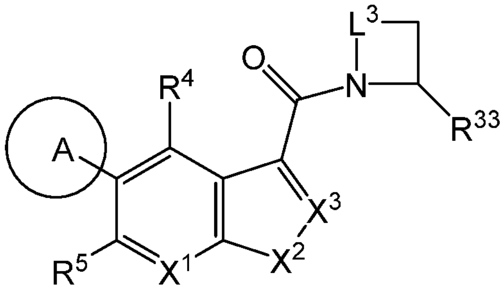

Formula V

wherein,

$X^1$ and $X^3$ are independently selected from $-CR^6-$ or N;
$X^2$ is selected from $-NR^1-$ or -CH = CH-;
the $R^1$ is selected from hydrogen, substituted or unsubstituted $C_1$-$C_{10}$ alkyl, substituted or unsubstituted $C_3$-$C_{10}$ cycloalkyl, substituted or unsubstituted 3-10-membered heterocycloalkyl, substituted or unsubstituted $C_6$-$C_{20}$ aryl, substituted or unsubstituted 3-20-membered heteroaryl; wherein the substituent is $C_1$-$C_{10}$ alkyl, cyano, hydroxyl, carboxyl, halogen, or nitro;
$L^3$ is selected from substituted or unsubstituted $C_1$-$C_4$ alkylene; wherein the substituent is $C_1$-$C_{10}$ alkyl, cyano, hydroxyl, carboxyl, halogen, or nitro;
$R^{33}$ is selected from hydrogen, substituted or unsubstituted $C_1$-$C_{10}$ alkyl, substituted or unsubstituted $C_3$-$C_{10}$ cycloalkyl, substituted or unsubstituted 3-10-membered heterocycloalkyl, substituted or unsubstituted $C_6$-$C_{10}$ aryl; wherein the substituent is $C_1$-$C_{10}$ alkyl, cyano, hydroxyl, carboxyl, halogen, or nitro;
$R^4$, $R^5$, and $R^6$ are each independently selected from hydrogen, $C_1$-$C_{10}$ alkyl, cyano, hydroxyl, carboxyl, halogen, or nitro;
ring A is selected from $C_4$-$C_{10}$ aryl substituted by one or two $R^A$ or unsubstituted, 4-10-membered heteroaryl substituted by one or two $R^A$ or unsubstituted; wherein $R^A$ is selected from amino,

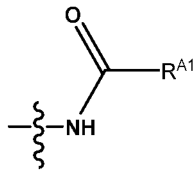
,
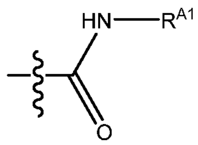
,

substituted or unsubstituted $C_1$-$C_{10}$ alkyl, substituted or unsubstituted $C_4$-$C_{10}$ aryl, substituted or unsubstituted 4-10-membered heteroaryl, substituted or unsubstituted 3-10-membered heterocycloalkyl; wherein the substituent is $-R^{A2}-R^{A3}$, $R^{A4}$, $C_1$-$C_{10}$ alkyl, cyano, hydroxyl, carboxyl, halogen, or nitro; wherein $R^{A4}$ is selected from 3-10-membered heterocycloalkyl substituted by $-R^{A2}-R^{A3}$ or unsubstituted;
$R^{A1}$ is selected from substituted or unsubstituted $C_1$-$C_{10}$ alkyl, substituted or unsubstituted $C_1$-$C_{10}$ alkenyl, substituted or unsubstituted $C_3$-$C_{10}$ cycloalkyl, substituted or unsubstituted 3-10-membered heterocycloalkyl, substituted or unsubstituted $C_1$-$C_6$ alkoxy, or substituted or unsubstituted $C_2$-$C_6$ ether group; wherein the substituent is $C_1$-$C_{10}$ alkyl, a $C_1$-$C_{10}$ ester group, 3-10-membered heterocycloalkyl, amino, amine, cyano, hydroxyl, carboxyl, halogen, or nitro;
$R^{A2}$ is selected from substituted or unsubstituted $C_0$-$C_6$ alkylene or carbonyl; wherein the substituent is $C_1$-$C_{10}$ alkyl, cyano, hydroxyl, carboxyl, halogen, or nitro;
$R^{A3}$ is selected from substituted or unsubstituted 3-10-membered heterocycloalkyl; wherein the substituent is $C_1$-$C_{10}$ alkyl, cyano, hydroxyl, carboxyl, halogen, or nitro.

5. The compound according to claim 4, **characterized in that** $R^{33}$ is selected from phenyl, phenyl substituted by one or two substituents; wherein the substituents are selected from F, Cl, or methyl.

6. The compound according to claim 1, or a stereoisomer, or a pharmaceutically acceptable salt thereof, **characterized in that** the compound of Formula I is represented by Formula VI:

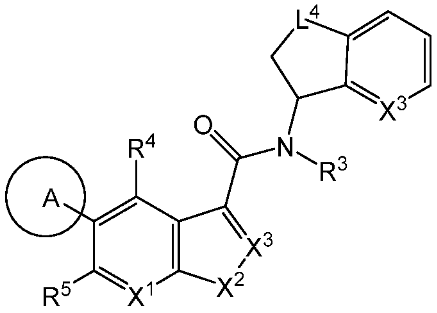

Formula VI

wherein,

$X^1$ and $X^3$ are independently selected from $-CR^6-$ or N;
$X^2$ is selected from $-NR^1-$ or $-CH = CH-$;
$X^3$ is selected from CH or N;
the $R^1$ is selected from hydrogen, substituted or unsubstituted $C_1$-$C_{10}$ alkyl, substituted or unsubstituted $C_3$-$C_{10}$ cycloalkyl, substituted or unsubstituted 3-10-membered heterocycloalkyl, substituted or unsubstituted $C_6$-$C_{20}$ aryl, substituted or unsubstituted 3-20-membered heteroaryl; wherein the substituent is $C_1$-$C_{10}$ alkyl, cyano, hydroxyl, carboxyl, halogen, or nitro;
$L^4$ is selected from substituted or unsubstituted $C_1$-$C_3$ alkylene; wherein the substituent is $C_1$-$C_{10}$ alkyl, cyano, hydroxyl, carboxyl, halogen, or nitro;
$R^3$ is selected from hydrogen, substituted or unsubstituted $C_1$-$C_{10}$ alkyl; wherein the substituent is cyano, hydroxyl, carboxyl, halogen, or nitro;
$R^4$, $R^5$, and $R^6$ are each independently selected from hydrogen, $C_1$-$C_{10}$ alkyl, cyano, hydroxyl, carboxyl, halogen, or nitro;
ring A is selected from $C_4$-$C_{10}$ aryl substituted by one or two $R^A$ or unsubstituted, 4-10-membered heteroaryl substituted by one or two $R^A$ or unsubstituted; wherein $R^A$ is selected from amino,

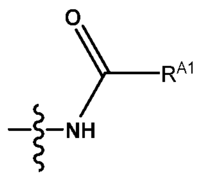 , 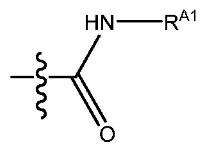 ,

substituted or unsubstituted $C_1$-$C_{10}$ alkyl, substituted or unsubstituted $C_4$-$C_{10}$ aryl, substituted or unsubstituted 4-10-membered heteroaryl, substituted or unsubstituted 3-10-membered heterocycloalkyl; wherein the substituent is $-R^{A2}-R^{A3}$, $R^{A4}$, $C_1$-$C_{10}$ alkyl, cyano, hydroxyl, carboxyl, halogen, or nitro; wherein $R^{A4}$ is selected from 3-10-membered heterocycloalkyl substituted by $-R^{A2}-R^{A3}$ or unsubstituted;
$R^{A1}$ is selected from substituted or unsubstituted $C_1$-$C_{10}$ alkyl, substituted or unsubstituted $C_1$-$C_{10}$ alkenyl, substituted or unsubstituted $C_3$-$C_{10}$ cycloalkyl, substituted or unsubstituted 3-10-membered heterocycloalkyl, substituted or unsubstituted $C_1$-$C_6$ alkoxy, or substituted or unsubstituted $C_2$-$C_6$ ether group; wherein, the substituent is $C_1$-$C_{10}$ alkyl, 3-10-membered heterocycloalkyl, amino, amine, cyano, hydroxyl, carboxyl, halogen, or nitro;
$R^{A2}$ is selected from substituted or unsubstituted $C_0$-$C_6$ alkylene or carbonyl; wherein the substituent is $C_1$-$C_{10}$ alkyl, cyano, hydroxyl, carboxyl, halogen, or nitro;
$R^{A3}$ is selected from substituted or unsubstituted 3-10-membered heterocycloalkyl; wherein the substituent is $C_1$-$C_{10}$ alkyl, cyano, hydroxyl, carboxyl, halogen, or nitro.

7. The compound according to claim 6, or a stereoisomer, or a pharmaceutically acceptable salt thereof, **characterized in that** the $L^4$ is selected from $C_1$- $C_2$ alkylene.

8. The compound according to claim 6, or a stereoisomer, or a pharmaceutically acceptable salt thereof, **characterized in that** the $X^3$ is selected from CH or N.

**9.** The compound according to any one of claims 1-4 or 6, or a stereoisomer, or a pharmaceutically acceptable salt thereof, **characterized in that** $R^1$ is selected from hydrogen, $C_1$-$C_4$ alkyl, or $C_3$ ether group.

**10.** The compound according to any one of claims 1, 4, or 6, or a stereoisomer, or a pharmaceutically acceptable salt thereof, **characterized in that** the $R^4$, $R^5$, and $R^6$ are each independently selected from hydrogen or F.

**11.** The compound according to any one of claims 1-4 or 6, or a stereoisomer, or a pharmaceutically acceptable salt thereof, **characterized in that**:

ring A is selected from 5-9-membered heteroaryl substituted by one or two $R^A$ or unsubstituted, 6-membered aryl substituted by one or two $R^A$ or unsubstituted; wherein $R^A$ is selected from amino, $C_1$-$C_4$ alkyl-substituted amino, $-CF_3$-substituted amino, $-CHF_2$-substituted amino,

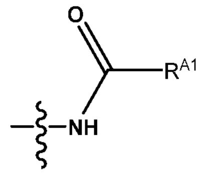
,

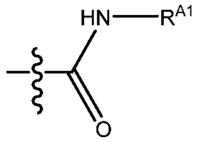
,

methyl, 5-6-membered heteroaryl substituted by $-R^{A2}$-$R^{A3}$ or unsubstituted, $R^{A4}$-substituted methyl, phenyl substituted by $-R^{A2}$-$R^{A3}$ or unsubstituted; wherein $R^{A4}$ is selected from 6-membered heterocycloalkyl substituted by $-R^{A2}$-$R^{A3}$ or unsubstituted; $R^{A1}$ is selected from $C_2$-$C_5$ alkyl, chlorine-substituted $C_4$ alkyl, 5-membered heterocycloalkyl-substituted methyl, vinyl, N,N-dimethylamino-substituted vinyl, $C_3$-$C_6$ cycloalkyl, $C_3$-$C_6$ cycloalkyl substituted by one or two fluorine, 4-6-membered heterocycloalkyl, hydroxyl-substituted 4-6-membered heterocycloalkyl, $-CH_2OH$-substituted 4-5-membered heterocycloalkyl, methyl-substituted 6-membered heterocycloalkyl, methoxy, fluorine-substituted methoxy, $C_2$-$C_3$ ether group, or hydroxyl-substituted propylamine;
$R^{A2}$ is selected from $C_0$-$C_1$ alkylene or carbonyl;
$R^{A3}$ is selected from methyl-substituted or unsubstituted 6-membered heterocycloalkyl.

**12.** The compound according to claim 11, or a stereoisomer, or a pharmaceutically acceptable salt thereof, **characterized in that**:
ring A is selected from the group consisting of:

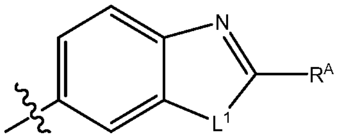
,
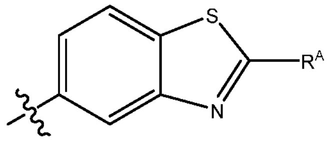
,

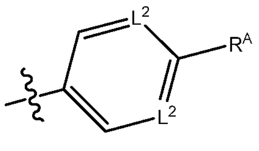
,
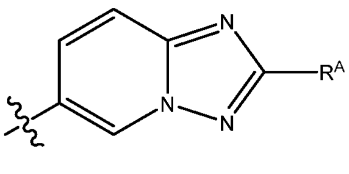
,
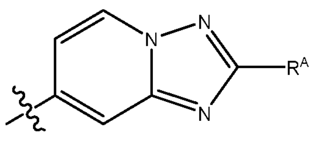
,

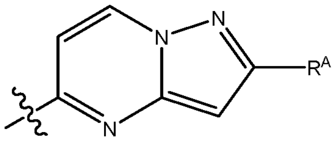, 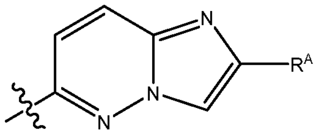, 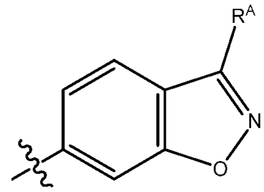, 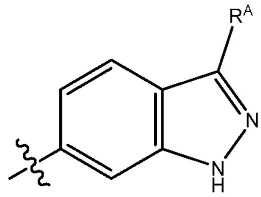,

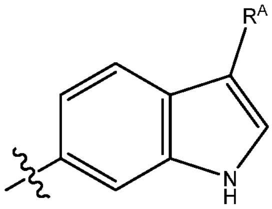, 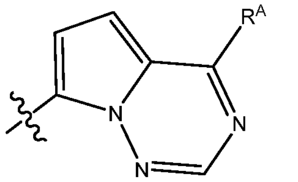, 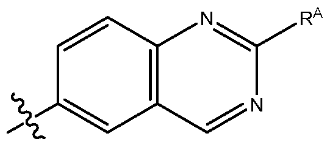, 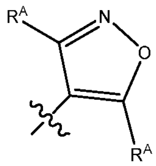,

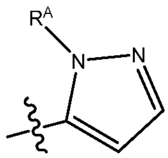, 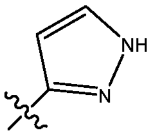;

wherein $L^1$ is selected from S or NH; and each $L^2$ is independently selected from CH or N;

**13.** The compound according to claim 12, or a stereoisomer, or a pharmaceutically acceptable salt thereof, **characterized in that**:
ring A is selected from:

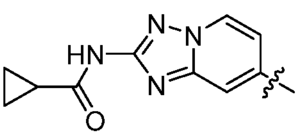, 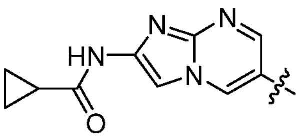, or 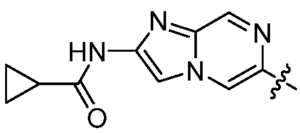.

**14.** The compound according to claim 1 or 2, or a stereoisomer, or a pharmaceutically acceptable salt thereof, **characterized in that**:

$R^2$ is selected from $C_0$-$C_2$ alkylene substituted by one or two $R^{21}$ or unsubstituted; wherein $R^{21}$ is selected from methyl;
ring B is selected from $C_6$-$C_{10}$ aryl substituted by one, two, or three $R^{22}$ or unsubstituted, 5-9-membered heteroaryl substituted by one, two, or three $R^{22}$ or unsubstituted, or $C_9$-$C_{10}$ cycloalkyl substituted by one, two, or three $R^{22}$ or unsubstituted; wherein each $R^{22}$ is independently selected from $C_1$-$C_4$ alkyl, methoxy, halogen, halogen-substituted methyl, halogen-substituted methoxy, cyano, nitro, or

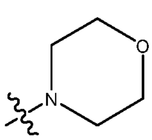;

halogen is selected from F, Cl, or Br.

**15.** The compound according to claim 1 or 2, or a stereoisomer, or a pharmaceutically acceptable salt thereof, **characterized in that** $R^3$ is selected from hydrogen.

**16.** The compound according to claim 1 or 2, or a stereoisomer, or a pharmaceutically acceptable salt thereof, **characterized in that** the compound of Formula I is represented by Formula VII:

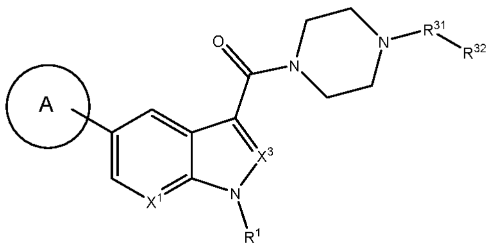

Formula Ⅶ

wherein,

$R^1$ is selected from hydrogen, substituted or unsubstituted $C_1$-$C_{10}$ alkyl, substituted or unsubstituted $C_3$-$C_{10}$ cycloalkyl, substituted or unsubstituted 3-10-membered heterocycloalkyl, substituted or unsubstituted $C_6$-$C_{20}$ aryl, substituted or unsubstituted 3-20-membered heteroaryl; wherein the substituent is $C_1$-$C_{10}$ alkyl, cyano, hydroxyl, carboxyl, halogen, or nitro;
$R^{31}$ is selected from $C_1$-$C_6$ alkylene;
$R^{32}$ is selected from $C_4$-$C_{10}$ aryl substituted by one, two, or three $R^{311}$ or unsubstituted, wherein each $R^{311}$ is independently selected from $C_1$-$C_6$ alkoxy, or two $R^{311}$ are joined to form a 4-10-membered heterocycloalkyl;
$X^1$ and $X^3$ are independently selected from CH or N;
ring A is selected from $C_4$-$C_{10}$ aryl substituted by one or two $R^A$ or unsubstituted, 4-10-membered heteroaryl substituted by one or two $R^A$ or unsubstituted; wherein $R^A$ is selected from amino,

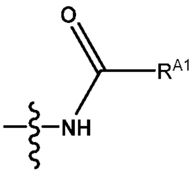 , 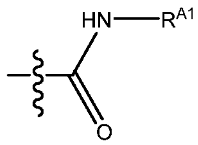 ,

substituted or unsubstituted $C_1$-$C_{10}$ alkyl, substituted or unsubstituted $C_4$-$C_{10}$ aryl, substituted or unsubstituted 4-10-membered heteroaryl, substituted or unsubstituted 3-10-membered heterocycloalkyl; wherein the substituent is -$R^{A2}$-$R^{A3}$, $R^{A4}$, $C_1$-$C_{10}$ alkyl, cyano, hydroxyl, carboxyl, halogen, or nitro; wherein $R^{A4}$ is selected from 3-10-membered heterocycloalkyl substituted by -$R^{A2}$-$R^{A3}$ or unsubstituted;
$R^{A1}$ is selected from substituted or unsubstituted $C_1$-$C_{10}$ alkyl, substituted or unsubstituted $C_1$-$C_{10}$ alkenyl, substituted or unsubstituted $C_3$-$C_{10}$ cycloalkyl, substituted or unsubstituted 3-10-membered heterocycloalkyl, substituted or unsubstituted $C_1$-$C_6$ alkoxy, or substituted or unsubstituted $C_2$-$C_6$ ether group; wherein the substituent is $C_1$-$C_{10}$ alkyl, 3-10-membered heterocycloalkyl, amino, amine, cyano, hydroxyl, carboxyl, halogen, or nitro;
$R^{A2}$ is selected from substituted or unsubstituted $C_0$-$C_6$ alkylene or carbonyl; wherein the substituent is $C_1$-$C_{10}$ alkyl, cyano, hydroxyl, carboxyl, halogen, or nitro;
$R^{A3}$ is selected from substituted or unsubstituted 3-10-membered heterocycloalkyl; wherein the substituent is $C_1$-$C_{10}$ alkyl, cyano, hydroxyl, carboxyl, halogen, or nitro.

**17.** The compound according to claim 16, or a stereoisomer, or a pharmaceutically acceptable salt thereof, **characterized in that**:
-$R^{31}$-$R^{32}$ is selected from

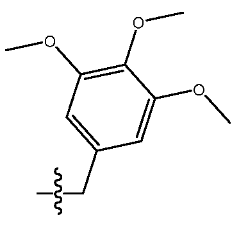 or 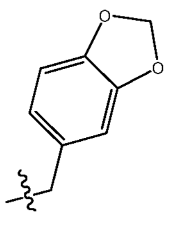 .

**18.** The compound according to any one of claims 1-17, or a stereoisomer, or a pharmaceutically acceptable salt thereof,

**characterized in that** the compounds of Formulas I-VII are as follows:

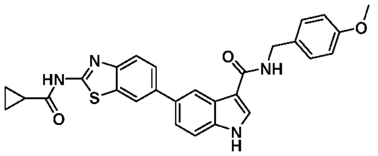,
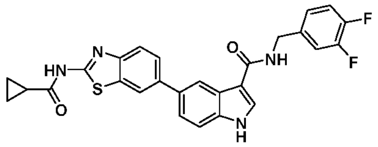,
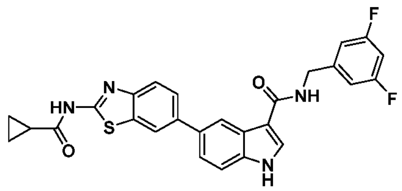,

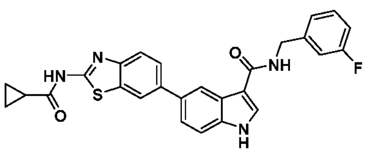,
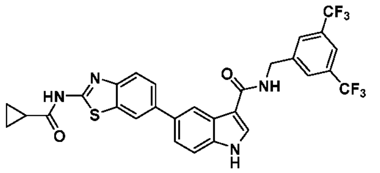,
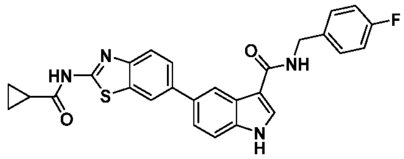,

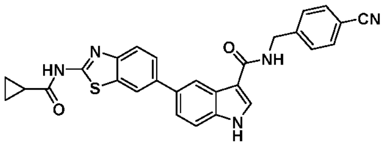,
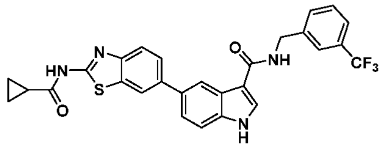,
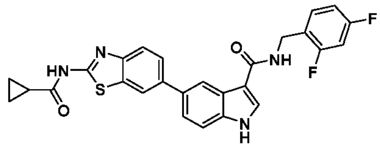,

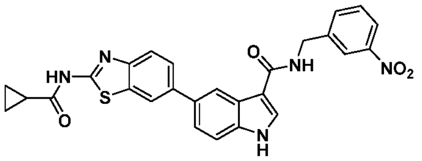,
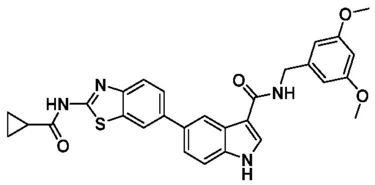,
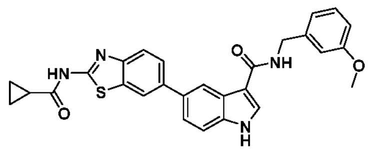,

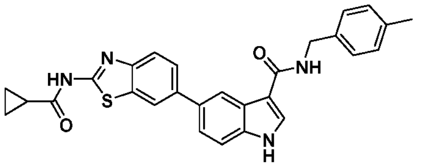,
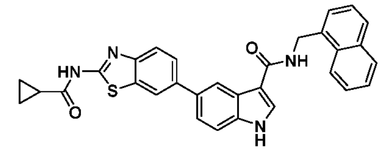,
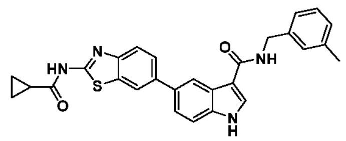,

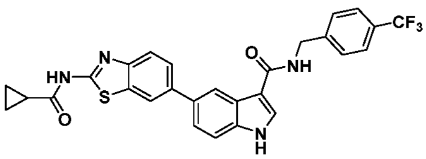,
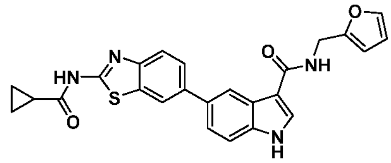,
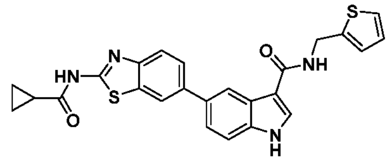,

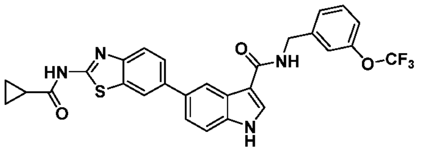,
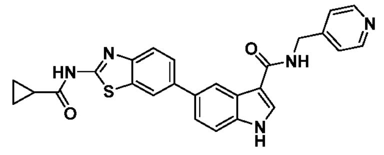,
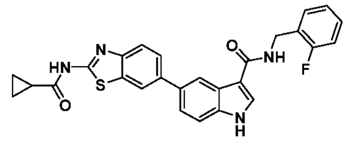,

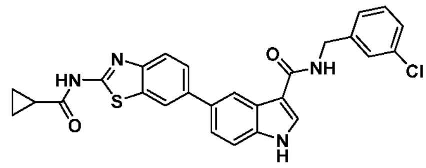 , 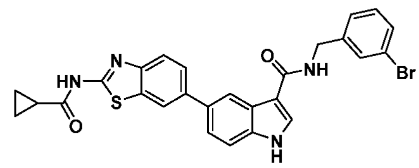 , 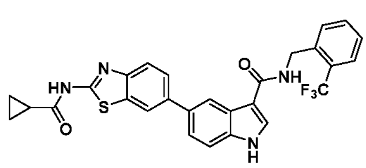 ,

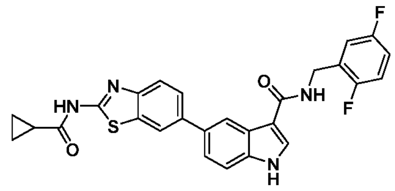 , 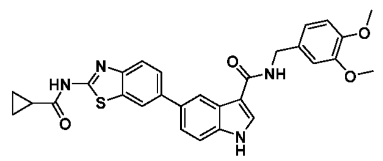 , 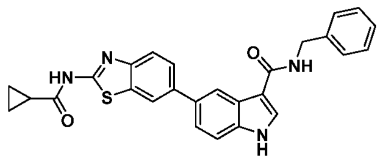 ,

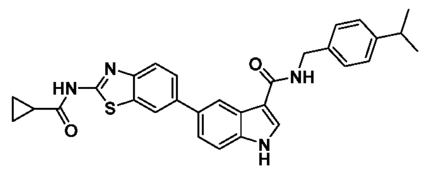 , 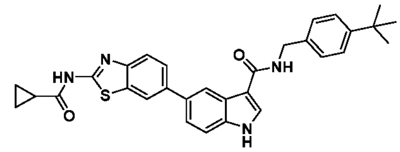 , 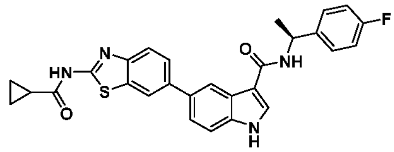 ,

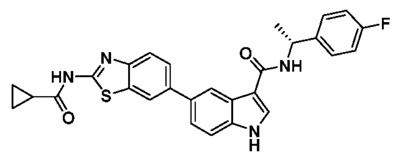 , 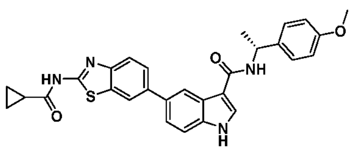 , 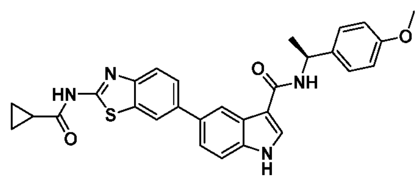 ,

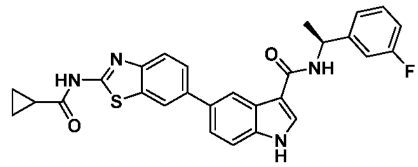 , 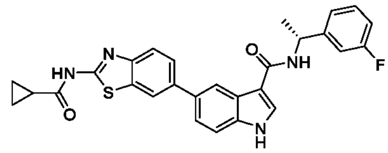 , 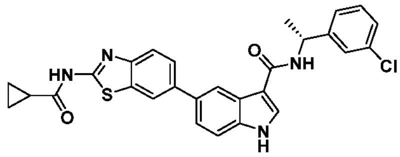 ,

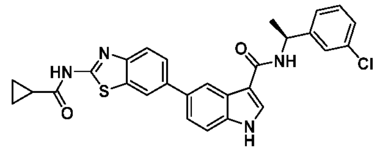 , 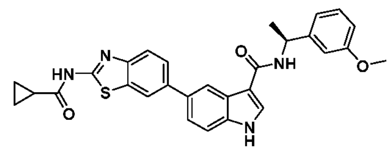 , 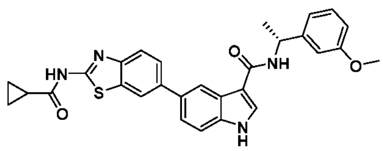 ,

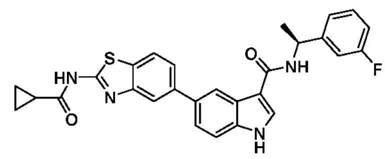 , 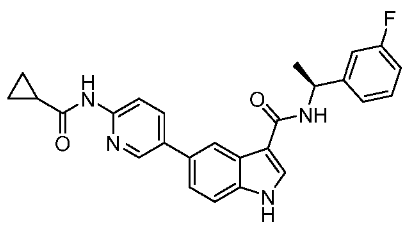 , 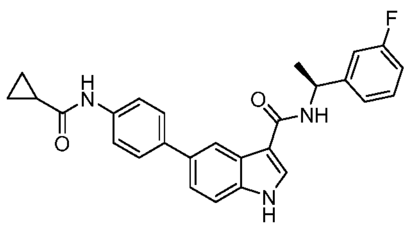 ,

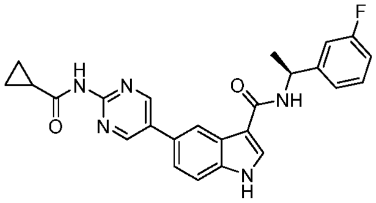 , 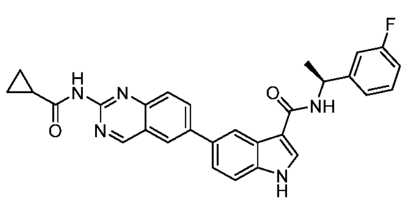 , 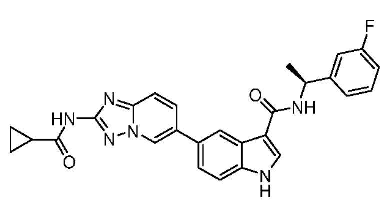 ,

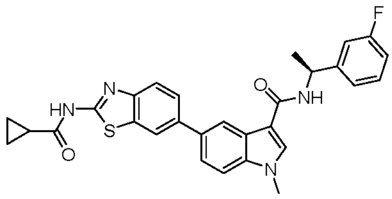 , 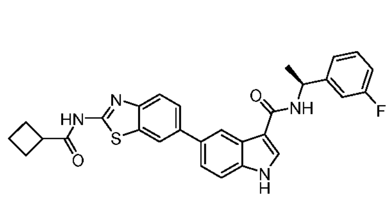 , 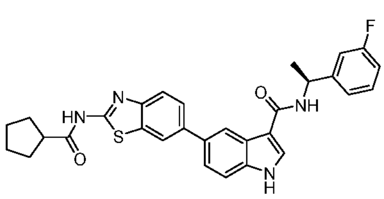 ,

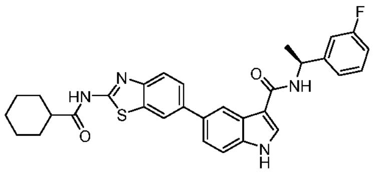 , 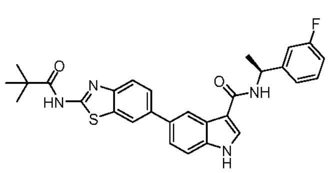 , 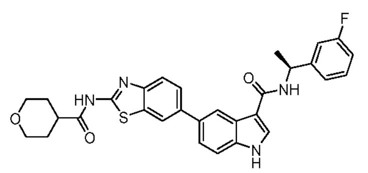 ,

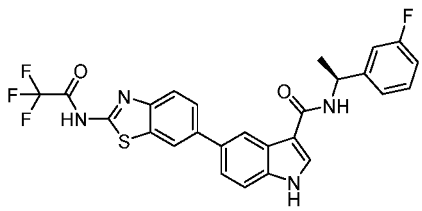 , 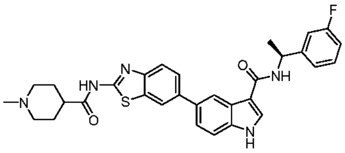 , 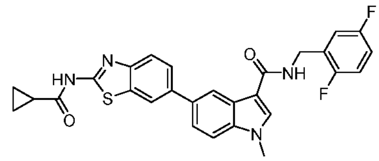 ,

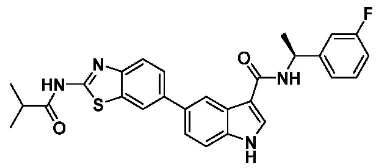 , 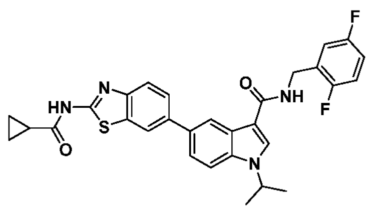 , 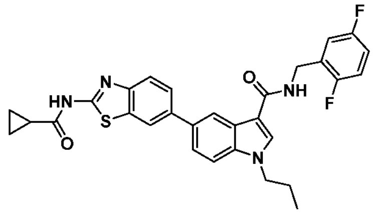 ,

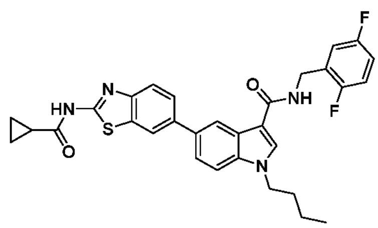 , 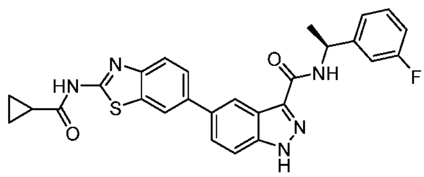 , 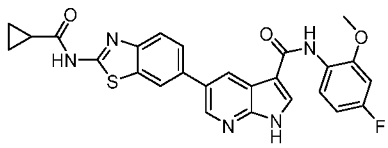 ,

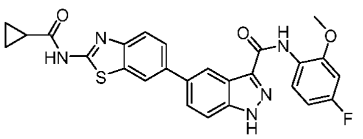 , 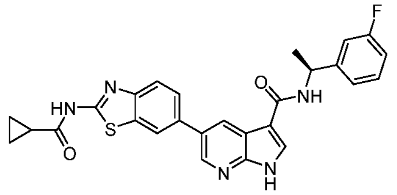 , 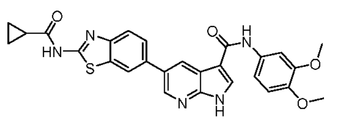 ,

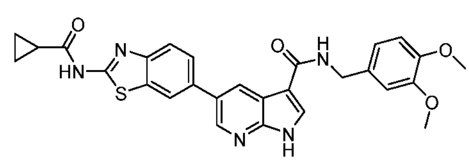 , 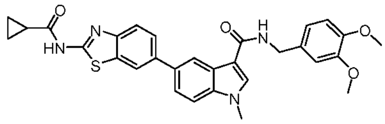 , 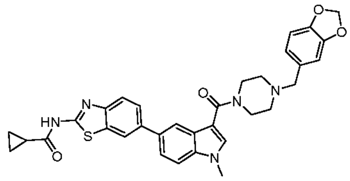 ,

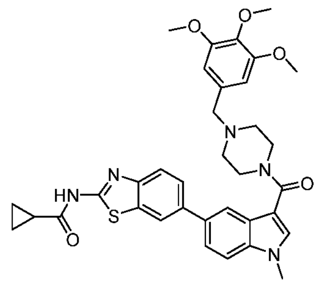 , 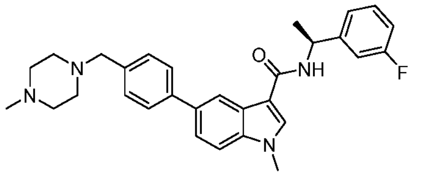 , 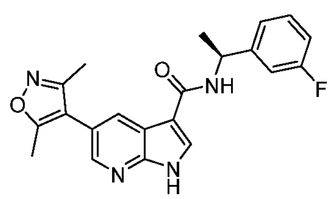 ,

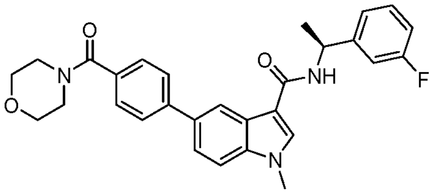 , 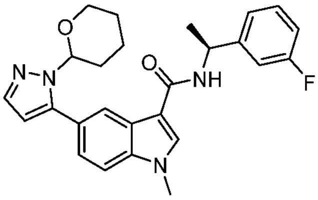 , 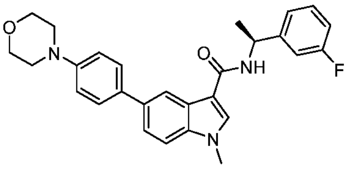 ,

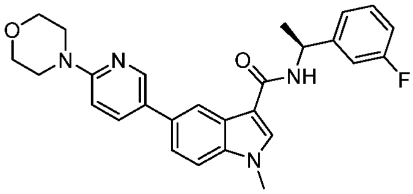 , 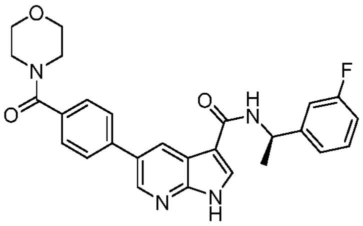 , 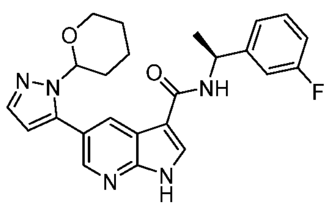 ,

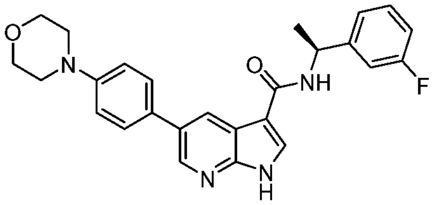 , 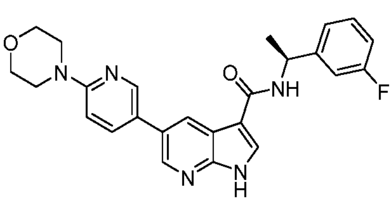 , 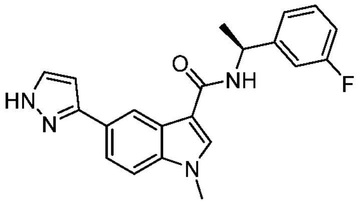 ,

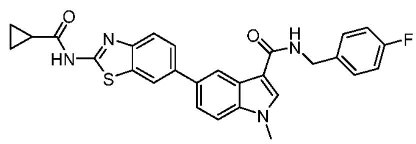 , 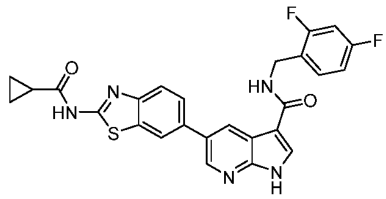 , 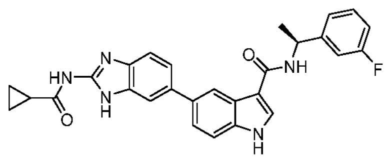 ,

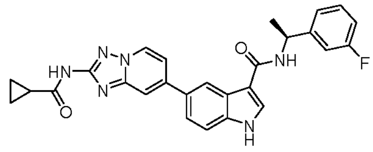 , 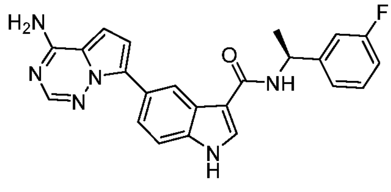 , 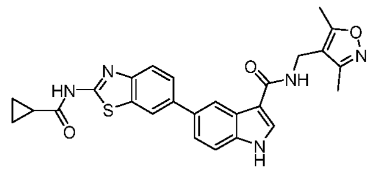 ,

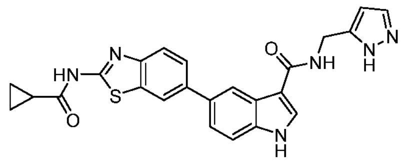 , 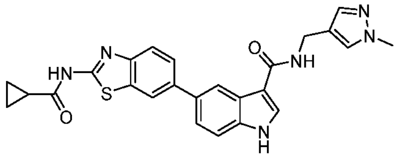 , 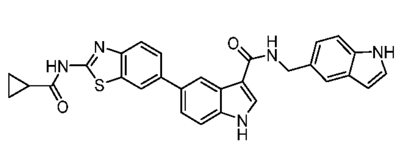 ,

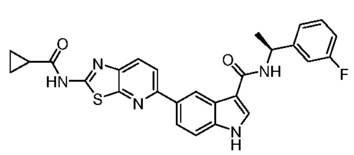 , 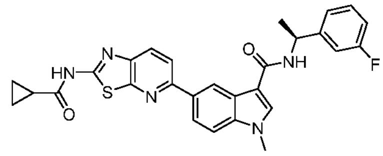 , 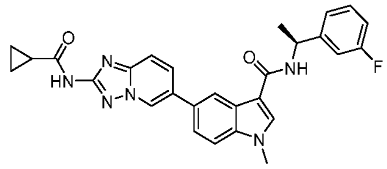 ,

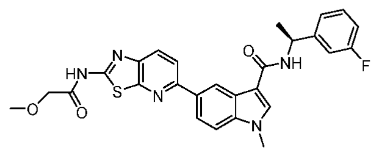 , 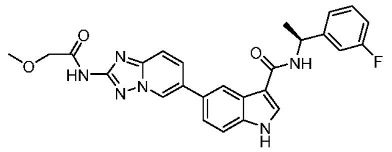 , 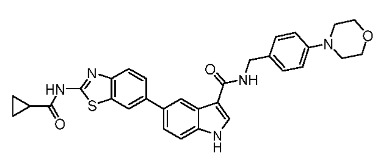 ,

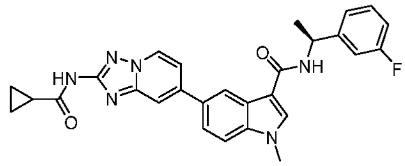 , 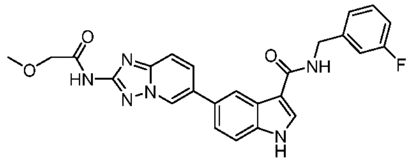 , 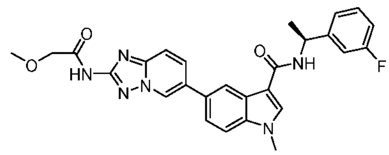 ,

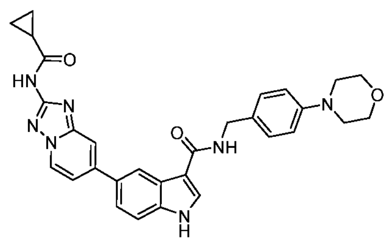 , 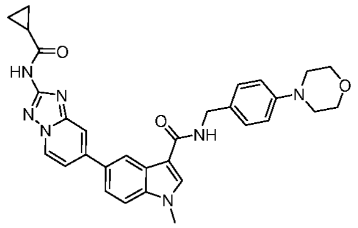 , 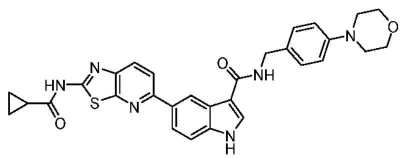 ,

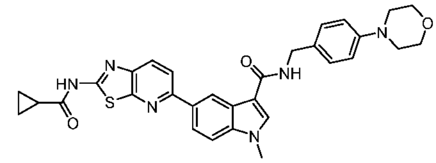 , 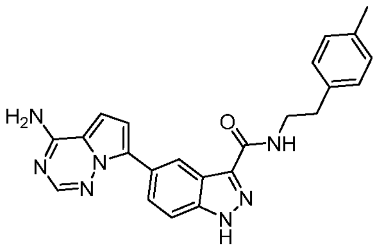 , 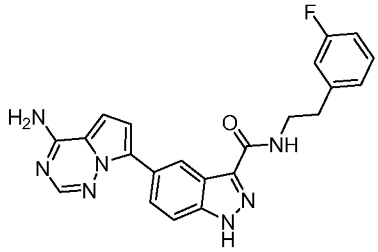 ,

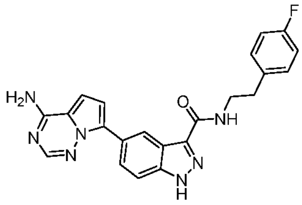 , 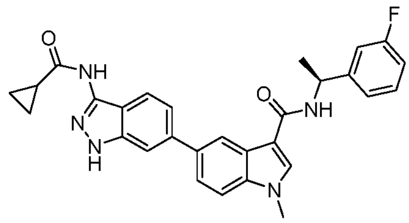 , 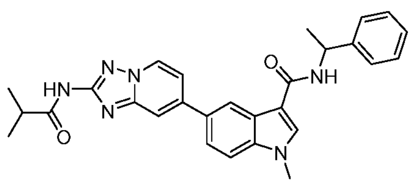 ,

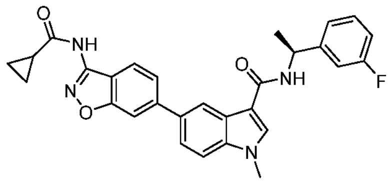 , 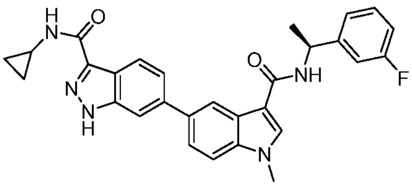 , 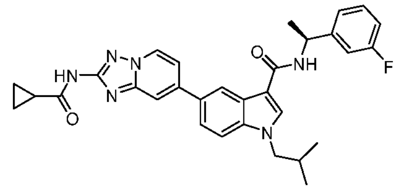 ,

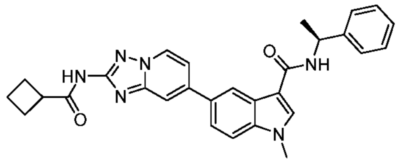 , 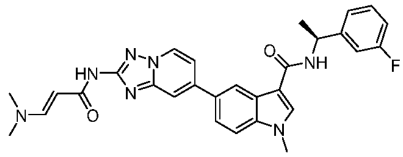 , 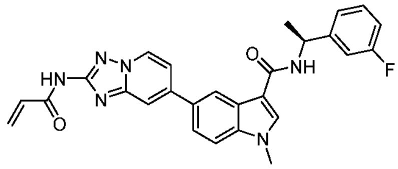 ,

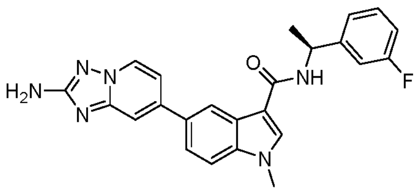 , 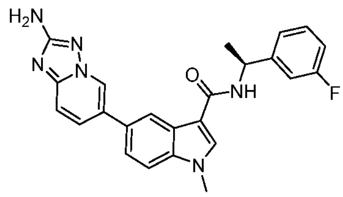 , 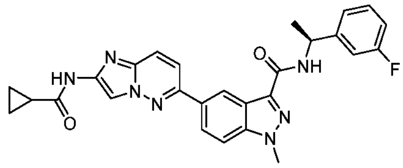 ,

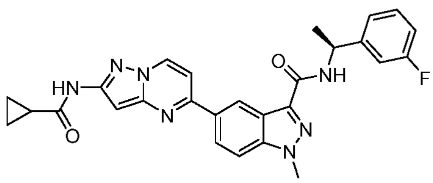 , 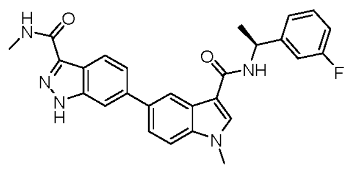 , 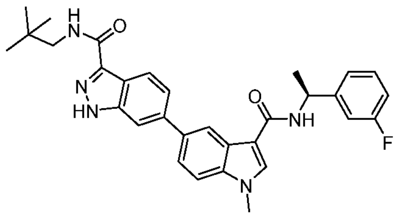 ,

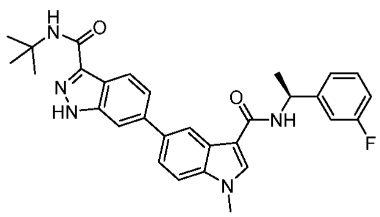 , 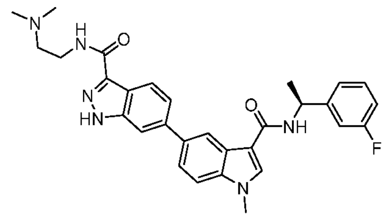 , 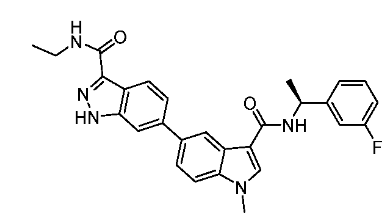 ,

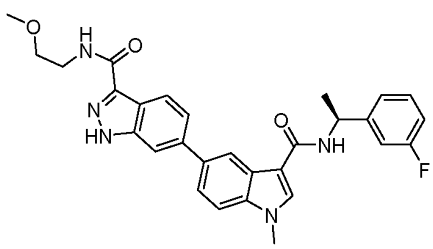 , 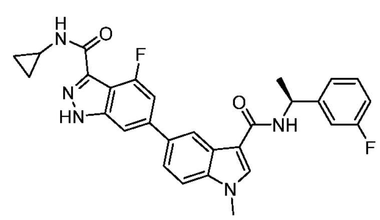 , 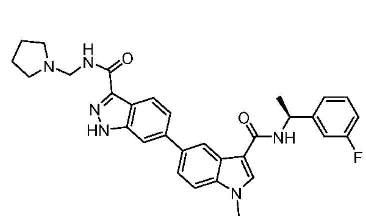 ,

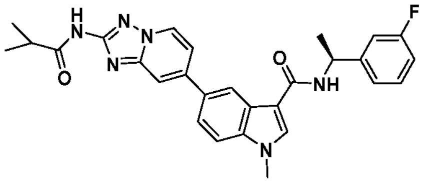,
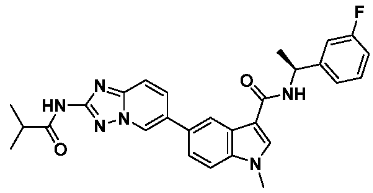,
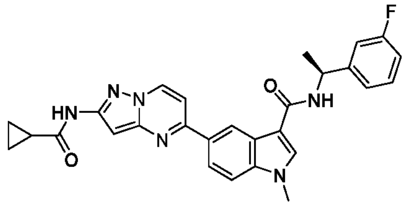,

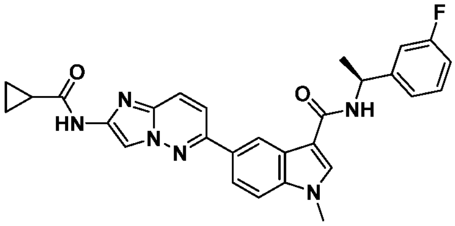,
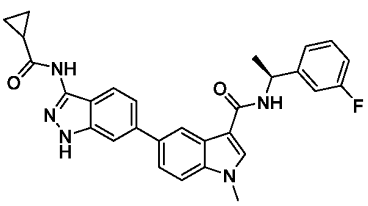,
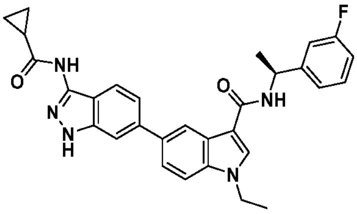,

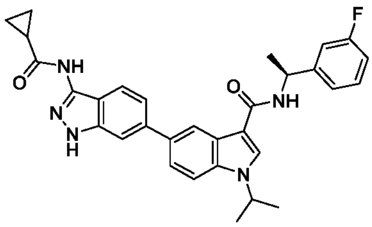,
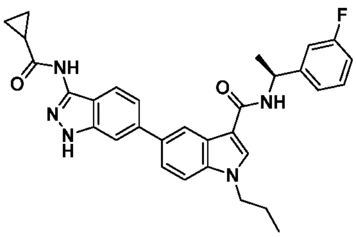,
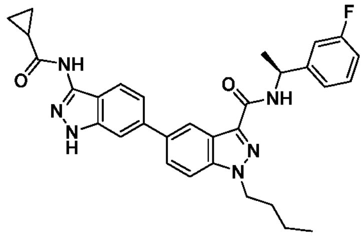,

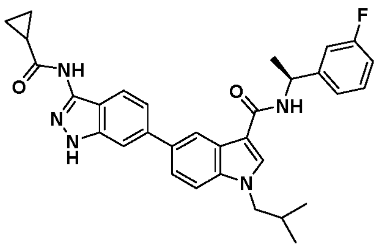,
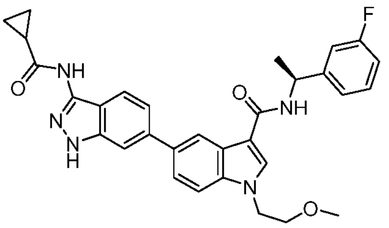,
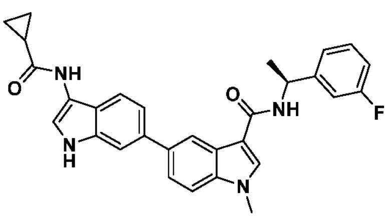,

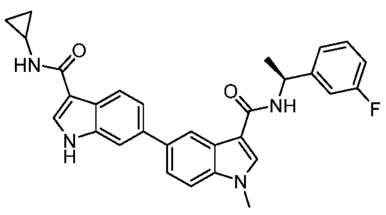,
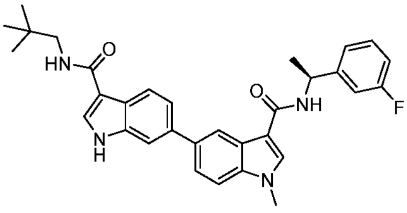,
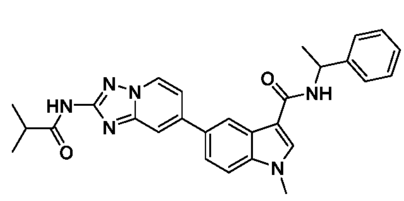,

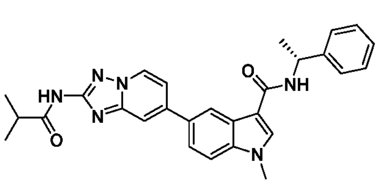,
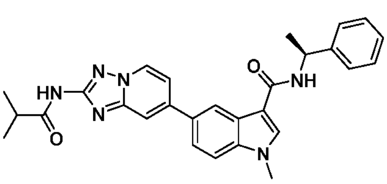,
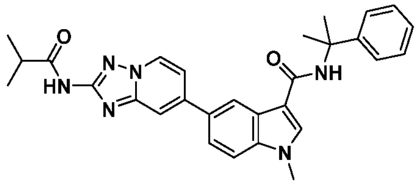,

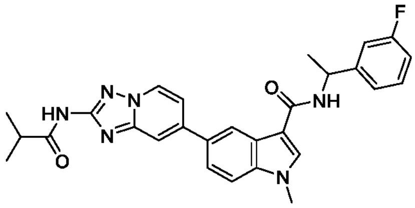

,

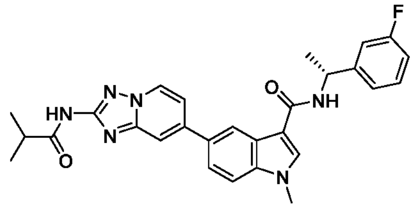

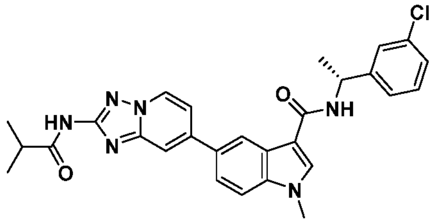

,

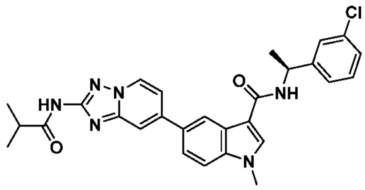

,

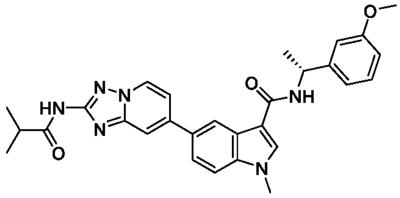

,

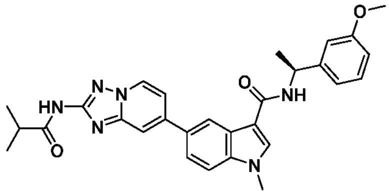

,

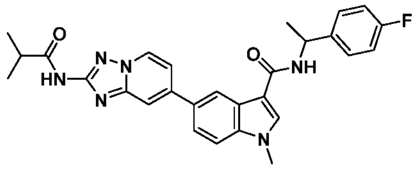

,

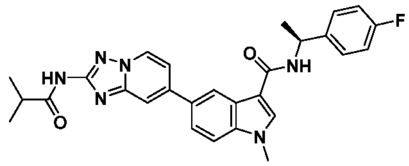

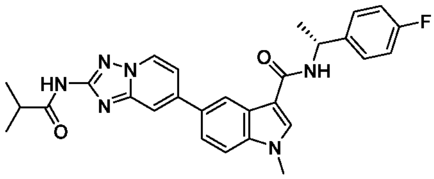

,

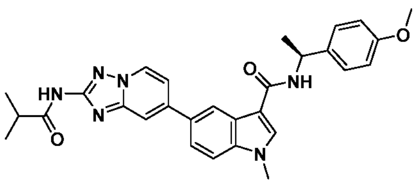

,

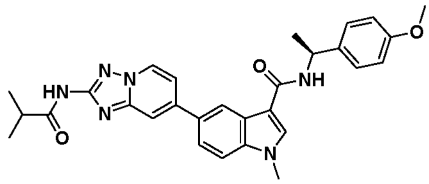

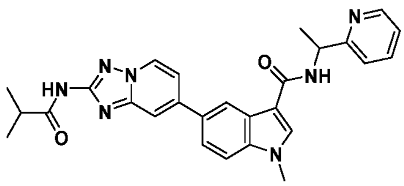

,

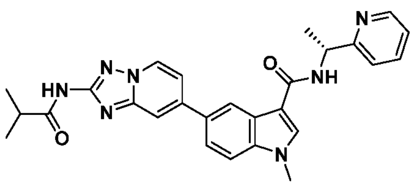

,

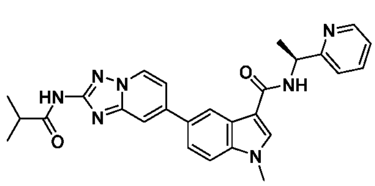

,

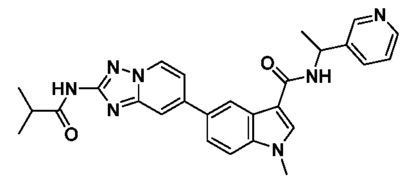,

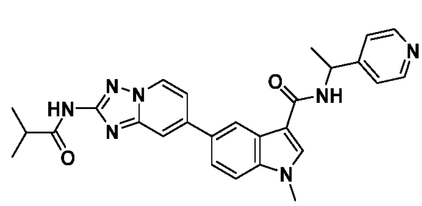, 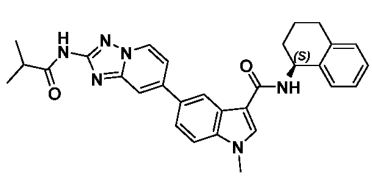, 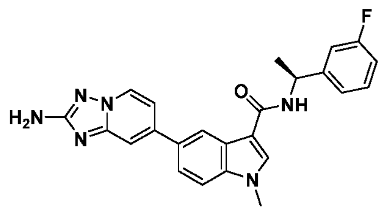,

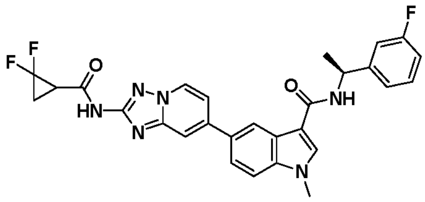, 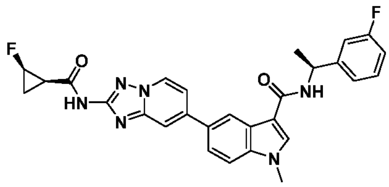, 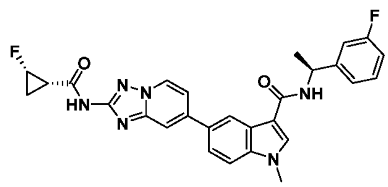,

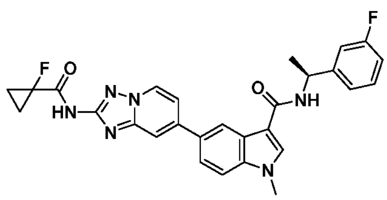, 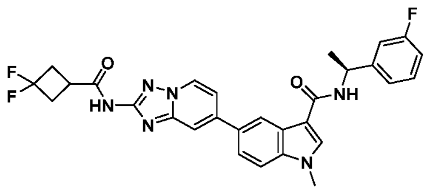, 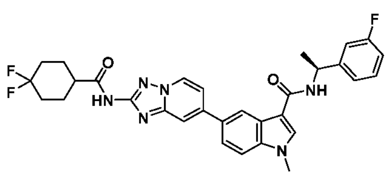,

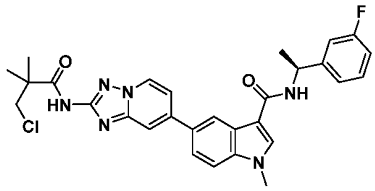, 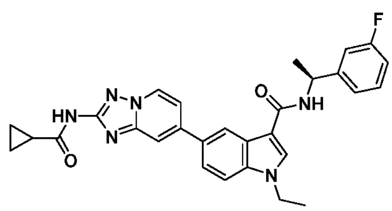, 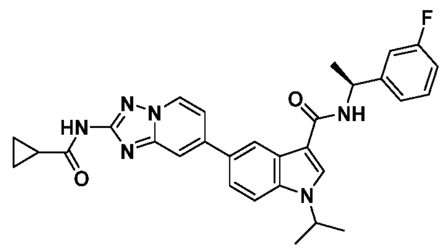,

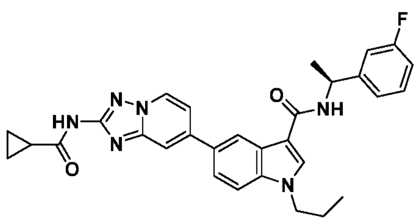, 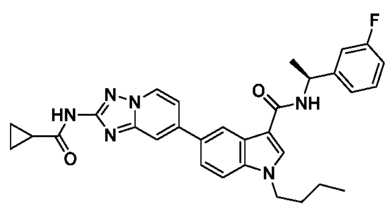, 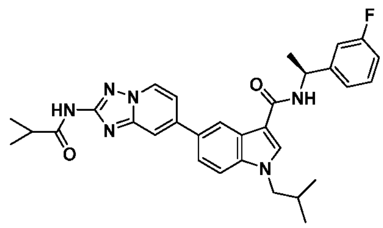,

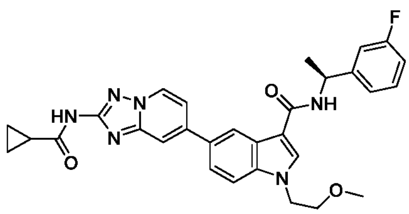, 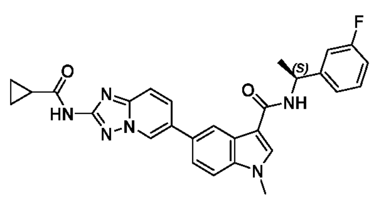, 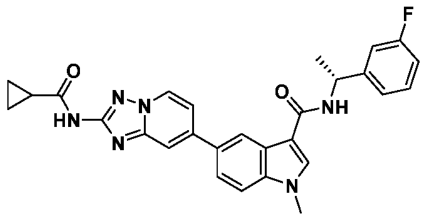,

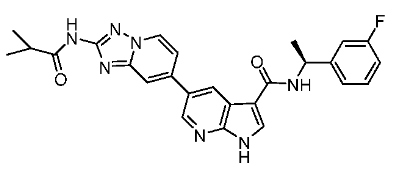,
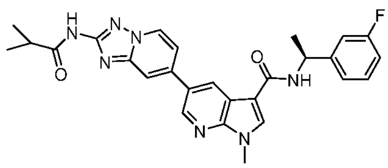,
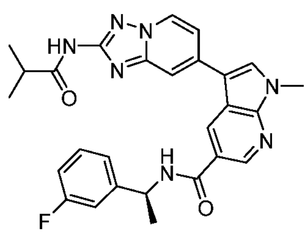,
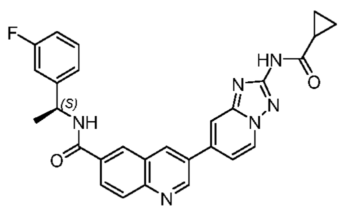,
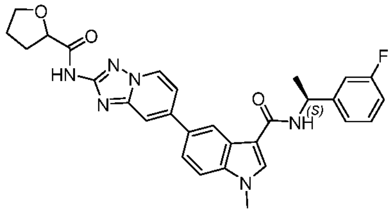,
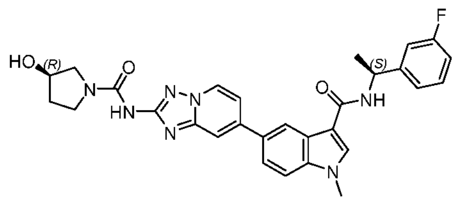,
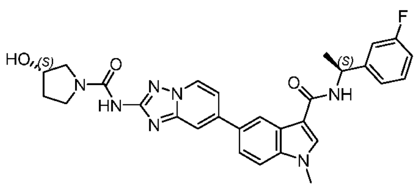,
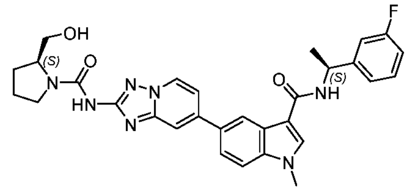,
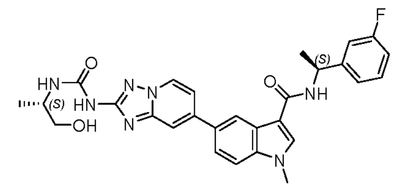,
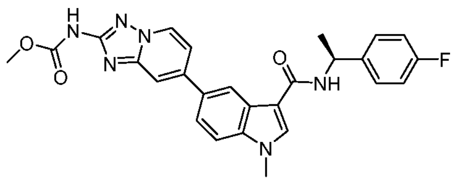,
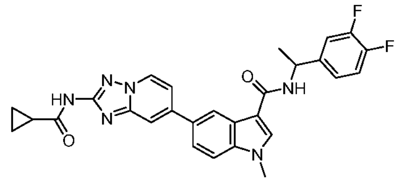,
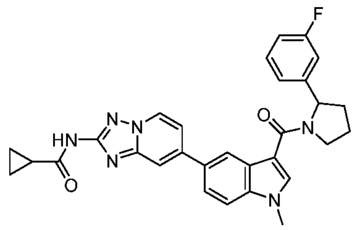,
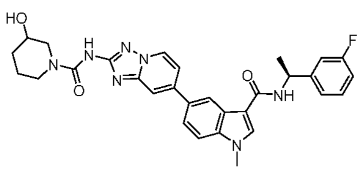,
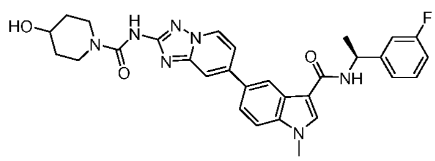,
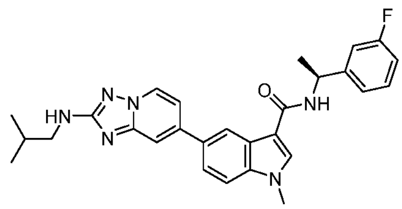,
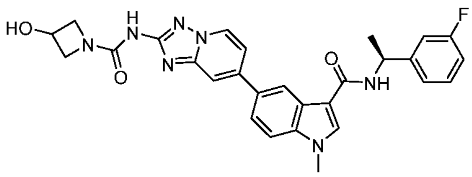,
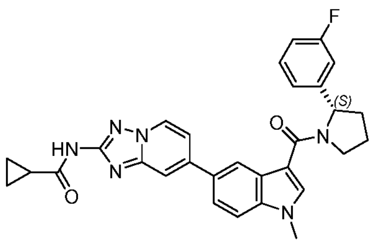,
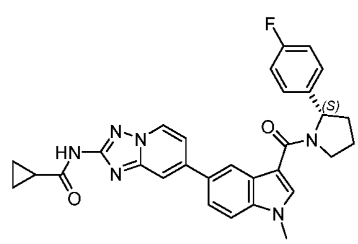,

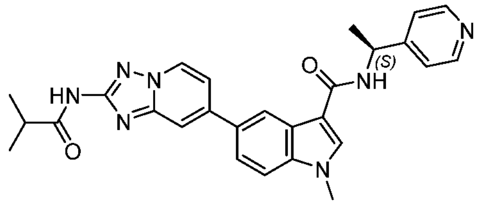
,
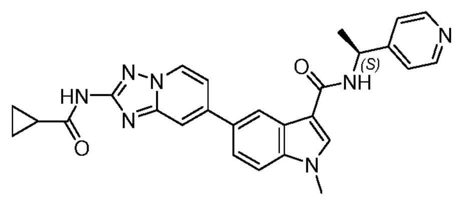
,
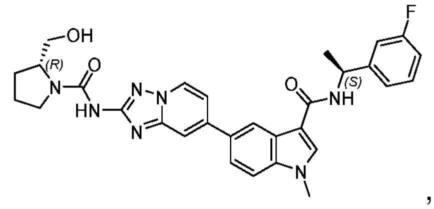
,
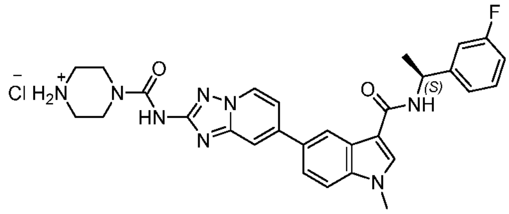
,
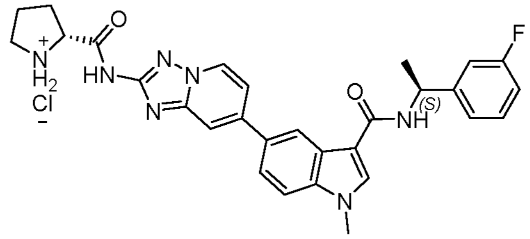
,
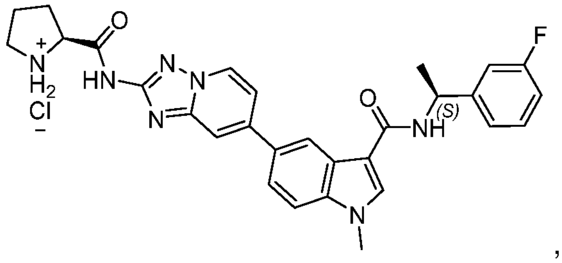
,
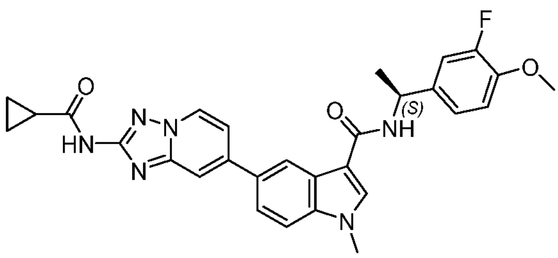
,
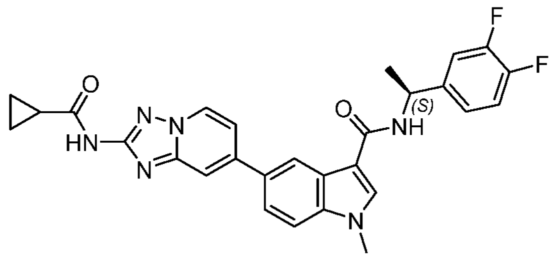
,
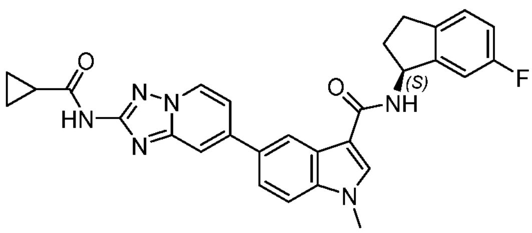
,
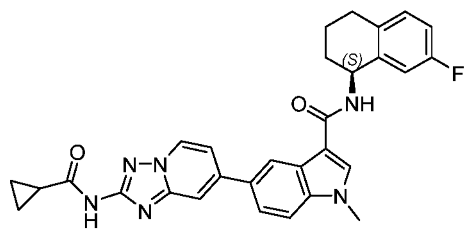
,
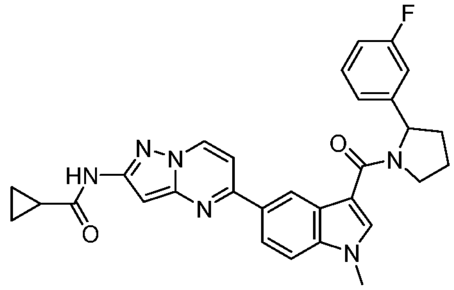
,
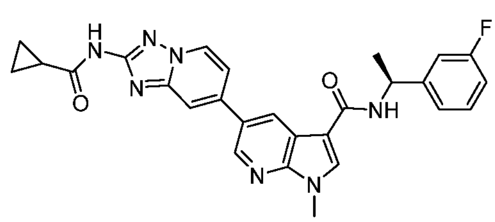
,

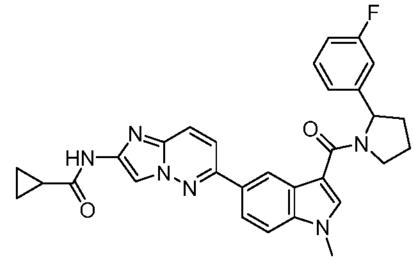, 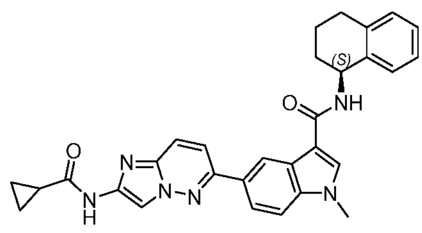,

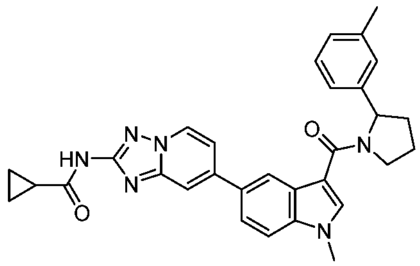, 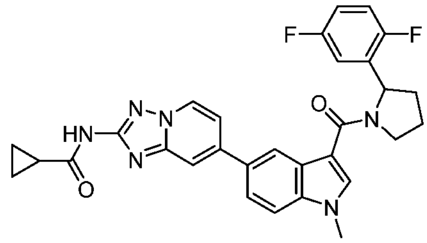,

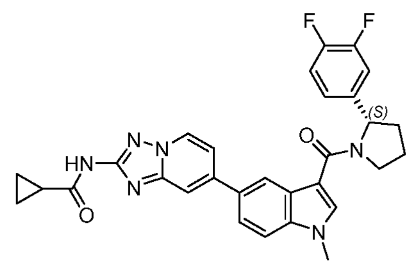, 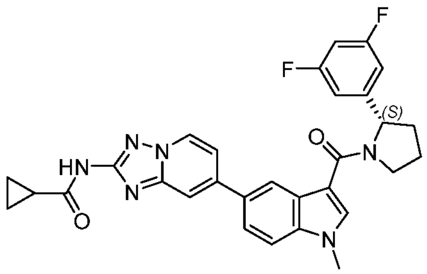,

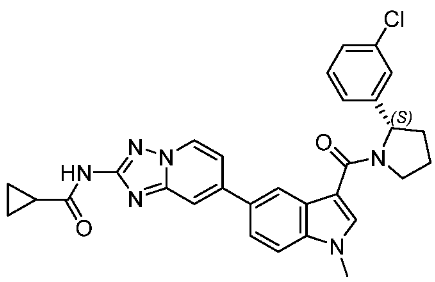, 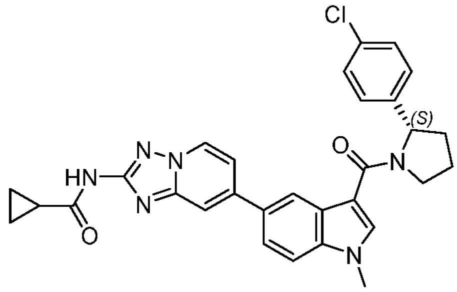,

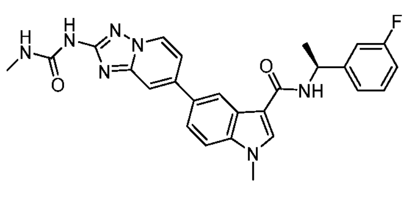, 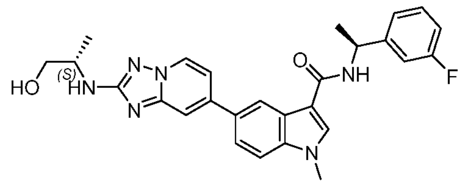, 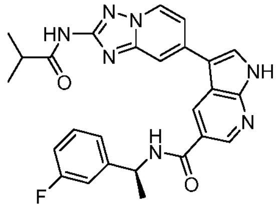,

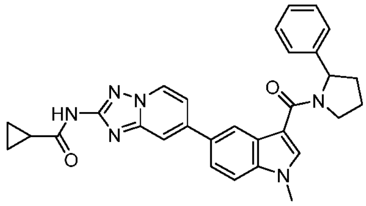, 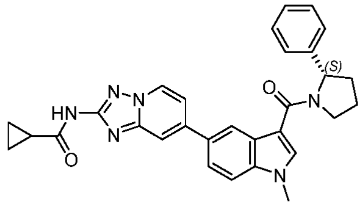, 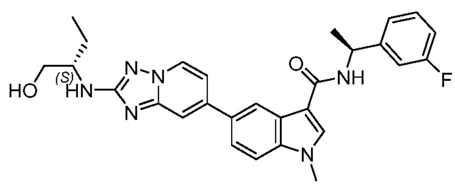,

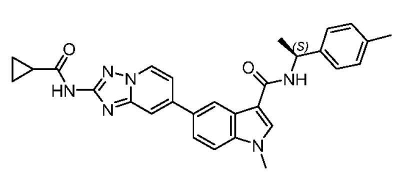,

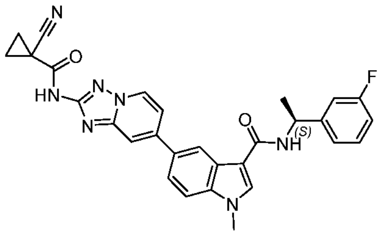,

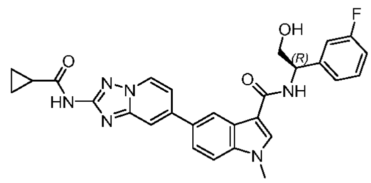,

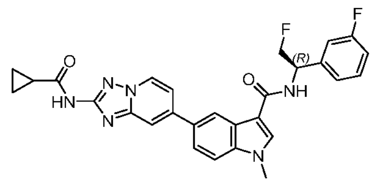,

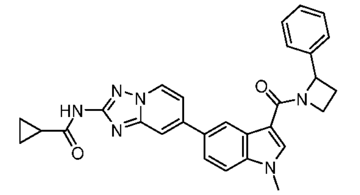,

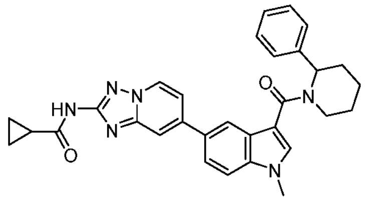,

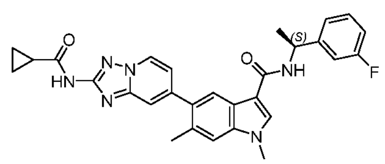,

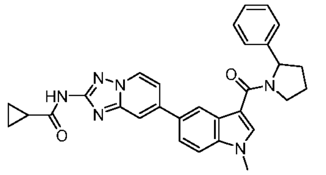,

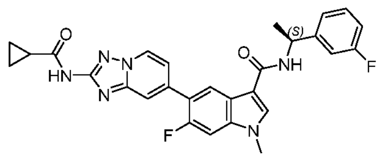,

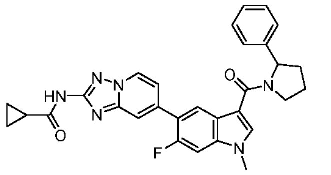,

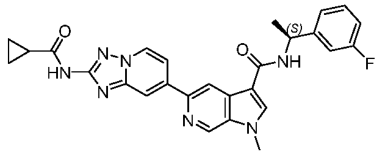,

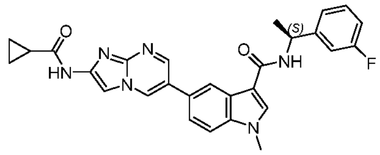,

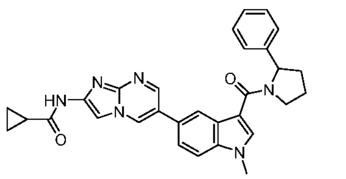,

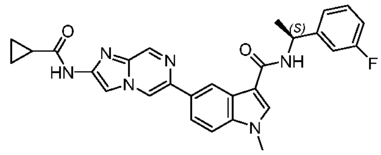,

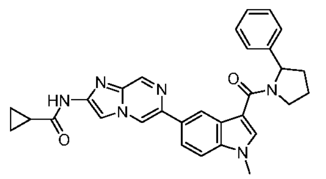,

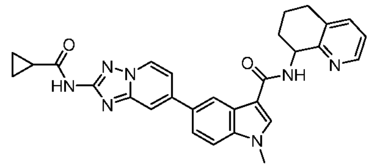,

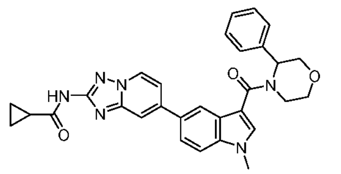,

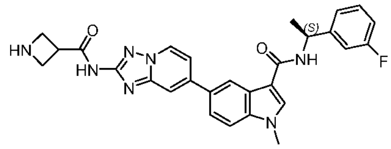,

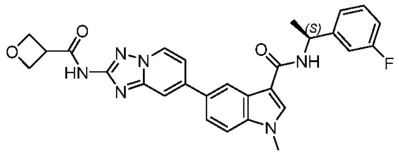,

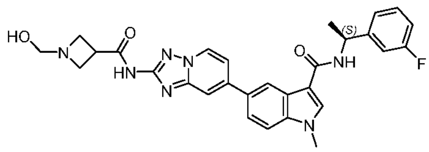,

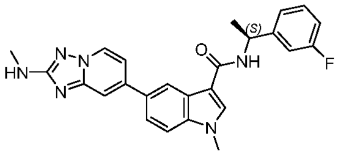,

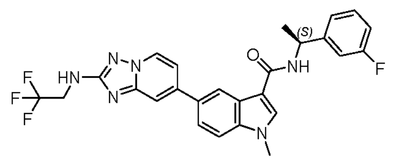 ,

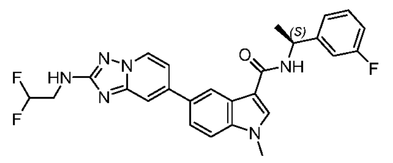 ,

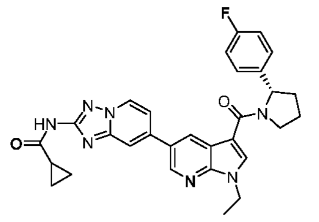 ,

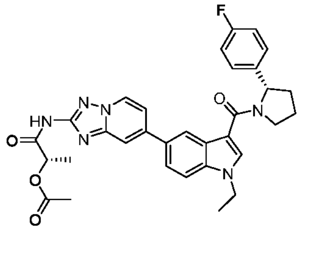 ,

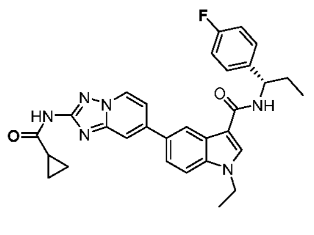 ,

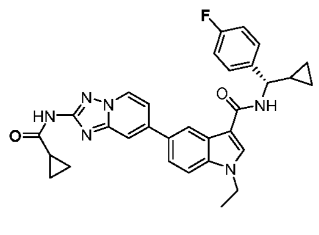 ,

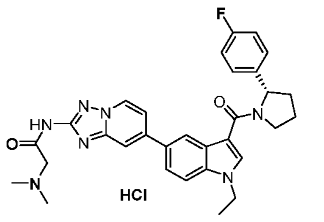 ,

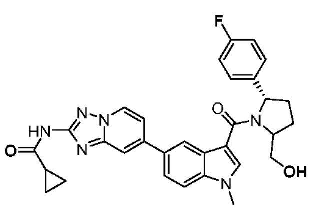 ,

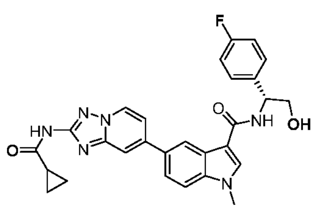 ,

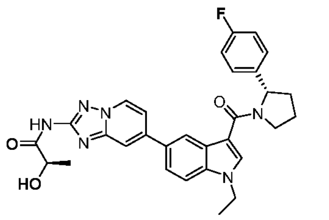 ,

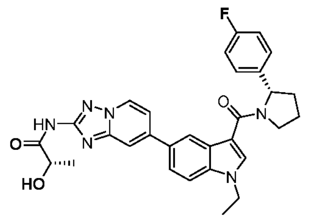 ,

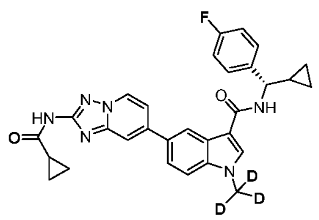 ,

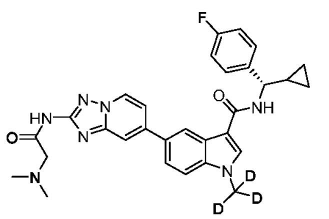 ,

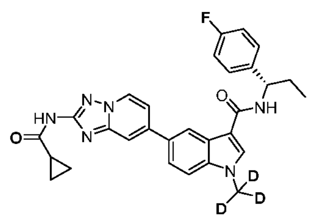 ,

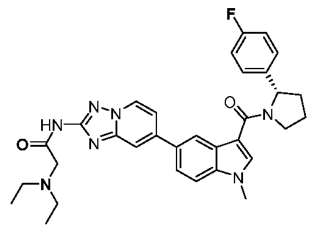 ,

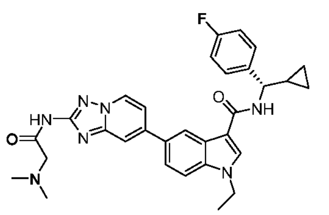 ,

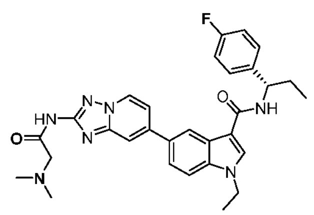 ,

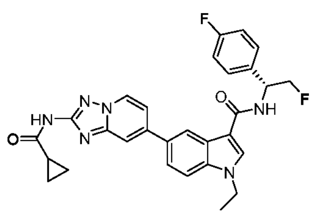 ,

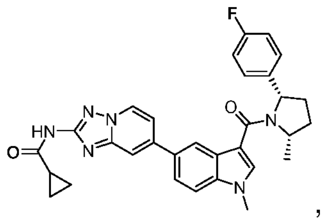,
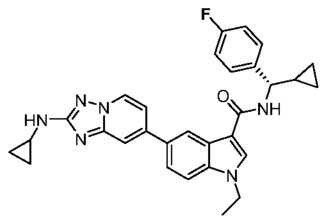.

**19.** A method for the preparation of the compound according to any one of claims 1-18, **characterized in that** the method is conducted by the following reaction steps:

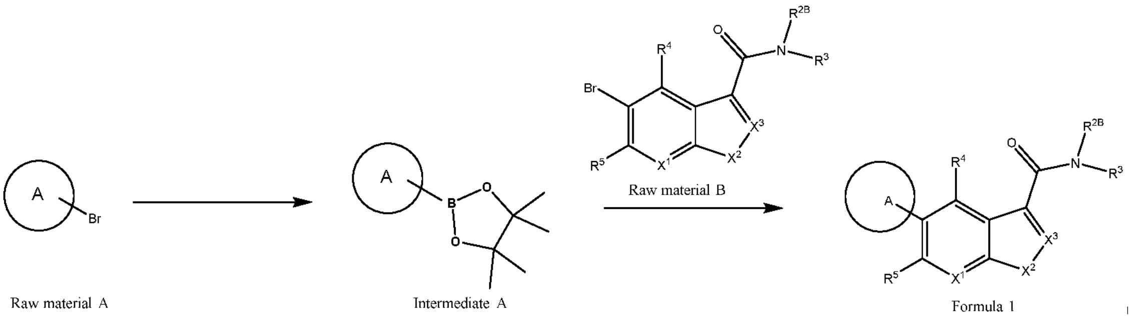


or

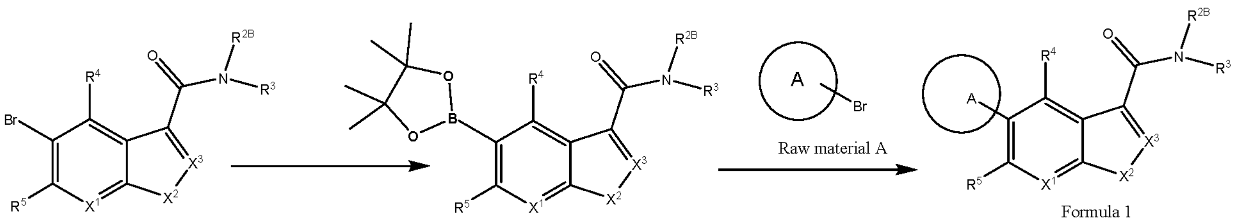


,

wherein $R^{2B}$, $R^4$, $R^5$, $X^1$, $X^2$, $X^3$, and ring A are as defined in any one of claims 1-17.

**20.** The preparation method according to claim 19, **characterized in that**:

a process for the synthesis of Intermediate A is as follows:

dissolving raw material A, potassium acetate, bis(pinacolato)diboron, and [1,1'-bis(diphenylphosphino)ferrocene]palladium(II) dichloride dichloromethane complex in anhydrous dioxane, replacing the reaction system with inert gas, then conducting the reaction, after the reaction is finished, concentrating the reaction solution, mixing the sample, and conducting column chromatography to obtain a product;
a process for the synthesis of the compound of Formula I from Intermediate A and raw material B is as follows:

dissolving Intermediate A, raw material B, [1,1'-bis(diphenylphosphino)ferrocene]palladium(II) dichloride dichloromethane complex, tricyclohexylphosphane, and cesium carbonate in a dioxane/water mixture, replacing the reaction system with inert gas, then conducting the reaction, after the reaction is finished, concentrating the reaction solution, mixing the sample, and conducting column chromatography to obtain aproduct;
a process for the synthesis of Intermediate B is as follows:

dissolving raw material B, potassium acetate, bis(pinacolato)diboron, and [1,1'-bis(diphenylphosphino)ferrocene]palladium(II) dichloride dichloromethane complex in anhydrous dioxane, replacing the reaction system with an inert gas, then conducting the reaction, after the reaction is finished, concentrating and stirring the reaction solution after the reaction is finished, mixing the sample,

and conducting column chromatography to obtain a product;
a process for the synthesis of the compound of Formula I from Intermediate B and raw material A is as follows:
dissolving Intermediate B, raw material A, [1,1'-bis(diphenylphosphino)ferrocene]palladium(II) dichloride dichloromethane complex, tricyclohexylphosphane, and cesium carbonate in a dioxane/water mixture, replacing the reaction system with inert gas, then conducting the reaction, after the reaction is finished, concentrating the reaction solution, mixing the sample, and conducting column chromatography to obtain a product.

**21.** Use of the compound according to any one of claims 1-18, or a stereoisomer, or a pharmaceutically acceptable salt thereof, in the preparation of an RIPK1 inhibitor.

**22.** Use of the compound according to any one of claims 1-18, or a stereoisomer, or a pharmaceutically acceptable salt thereof, in the preparation of a medicament for treating inflammation, immunological diseases, neurodegenerative diseases, or tumors.

**23.** The use according to claim 22, **characterized in that** the medicament is for treating inflammatory responses associated with programmed necrosis, immunological diseases, neurodegenerative diseases, or tumors.

**24.** The use according to claim 22 or 23, **characterized in that** the inflammation is colitis.

**25.** A pharmaceutical composition, **characterized in that** the composition is a preparation prepared by a compound of any one of claims 1-18, or a stereoisomer, or a pharmaceutically acceptable salt thereof, and a pharmaceutically acceptable excipient.

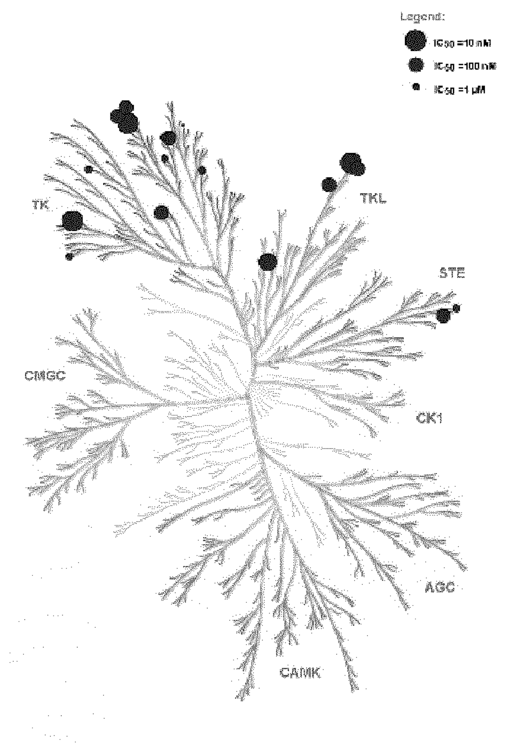
Legend:
IC50 =10 nM
IC50 =100 nM
IC50 =1 μM
TK
TKL
STE
CMGC
CK1
AGC
CAMK

FIG. 1

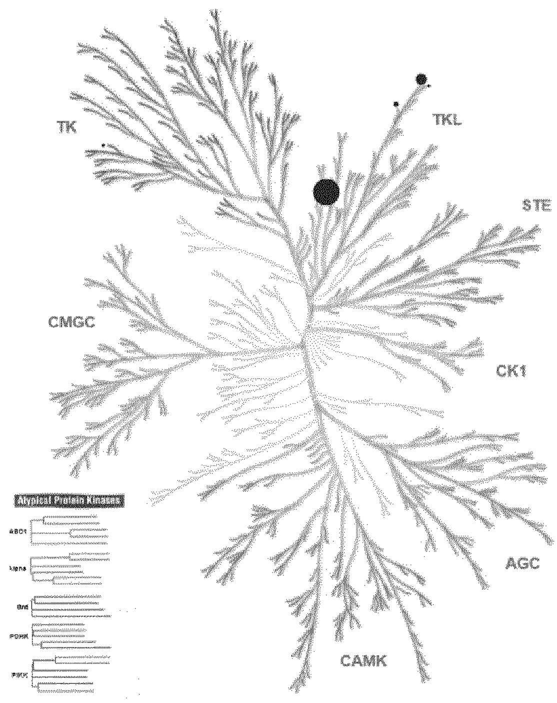
TK
TKL
STE
CMGC
CK1
AGC
CAMK
Atypical Protein Kinases

FIG. 2

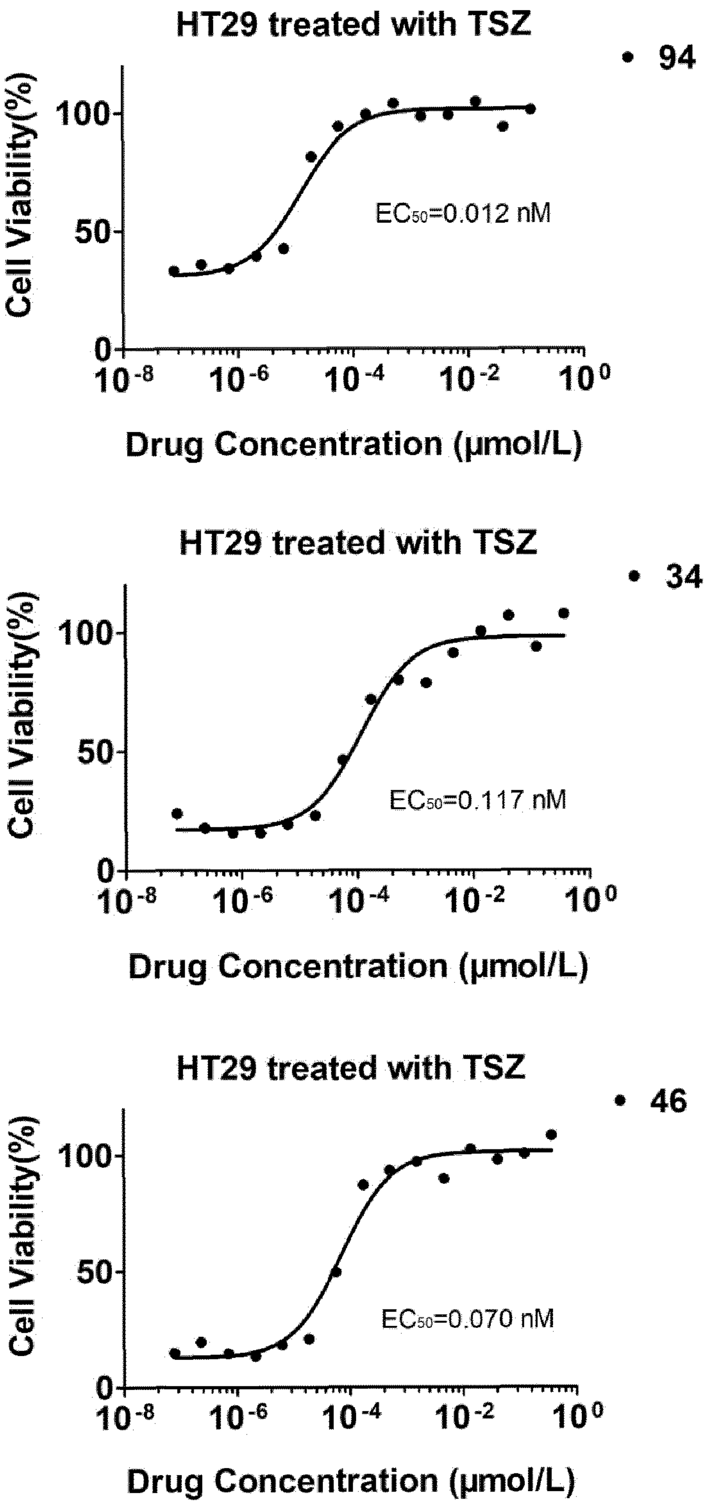
HT29 treated with TSZ
• 94
Cell Viability(%)
EC50=0.012 nM
Drug Concentration (μmol/L)
HT29 treated with TSZ
• 34
Cell Viability(%)
EC50=0.117 nM
Drug Concentration (μmol/L)
HT29 treated with TSZ
• 46
Cell Viability(%)
EC50=0.070 nM
Drug Concentration (μmol/L)

FIG. 3

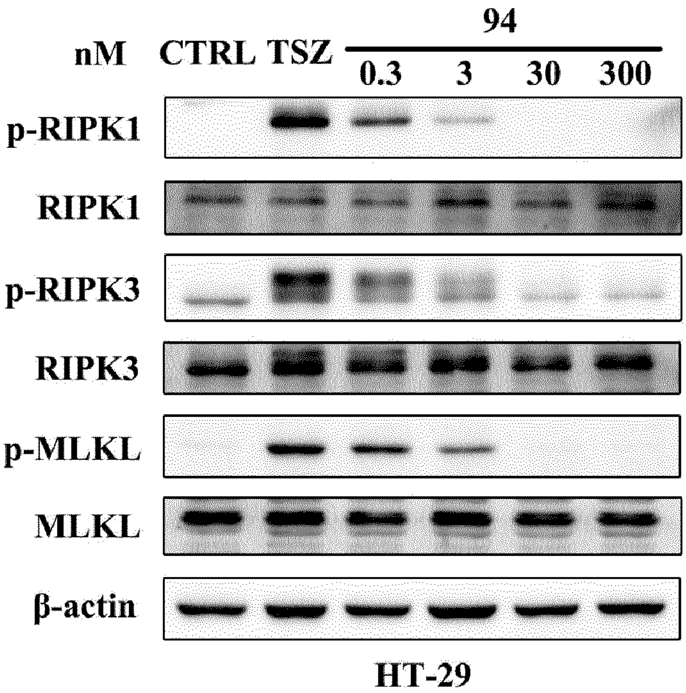
nM
CTRL
TSZ
94
0.3
3
30
300
p-RIPK1
RIPK1
p-RIPK3
RIPK3
p-MLKL
MLKL
β-actin
HT-29

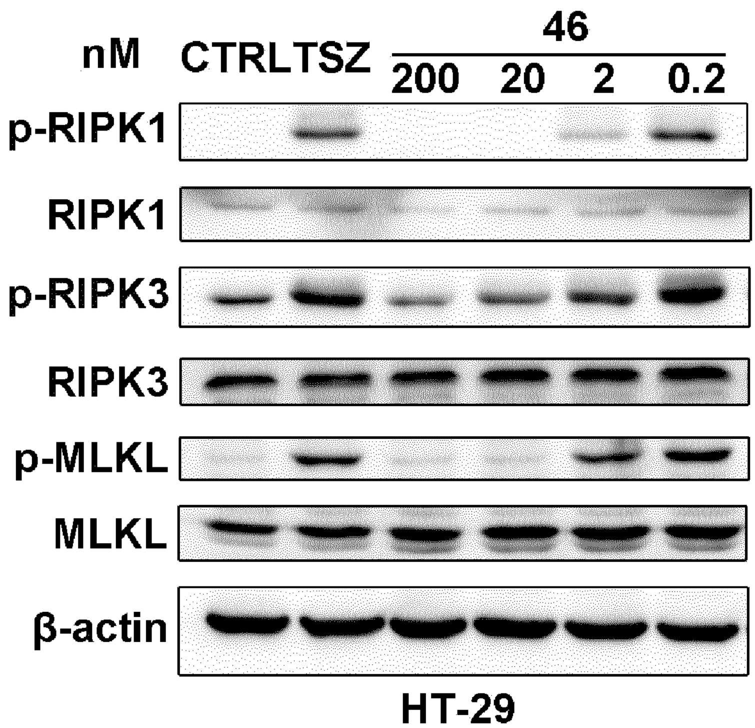
nM
CTRL
TSZ
46
200
20
2
0.2
p-RIPK1
RIPK1
p-RIPK3
RIPK3
p-MLKL
MLKL
β-actin
HT-29

FIG. 4

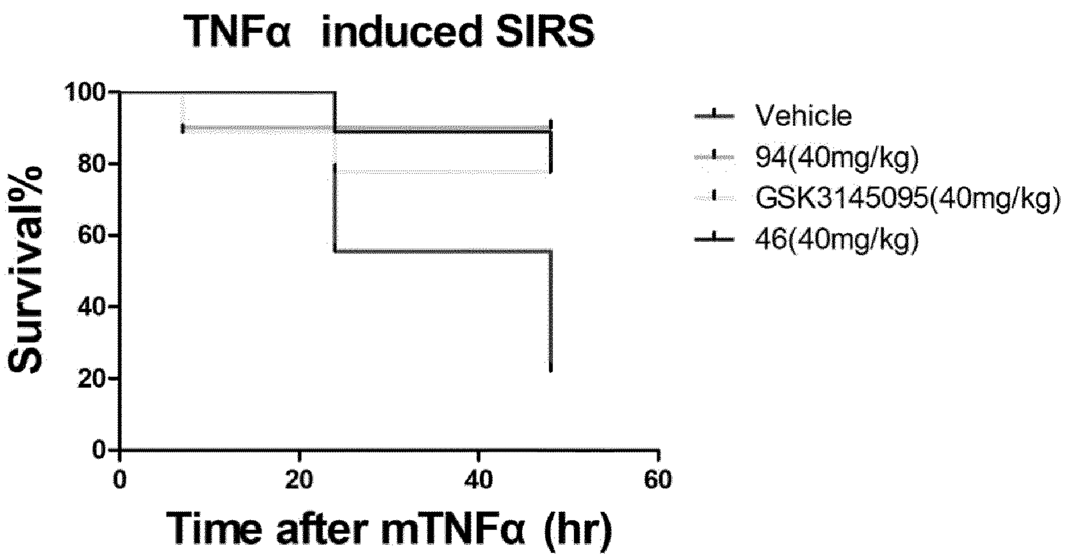
TNFα induced SIRS
Survival%
100
80
60
40
20
0
0
20
40
60
Time after mTNFα (hr)
Vehicle
94(40mg/kg)
GSK3145095(40mg/kg)
46(40mg/kg)

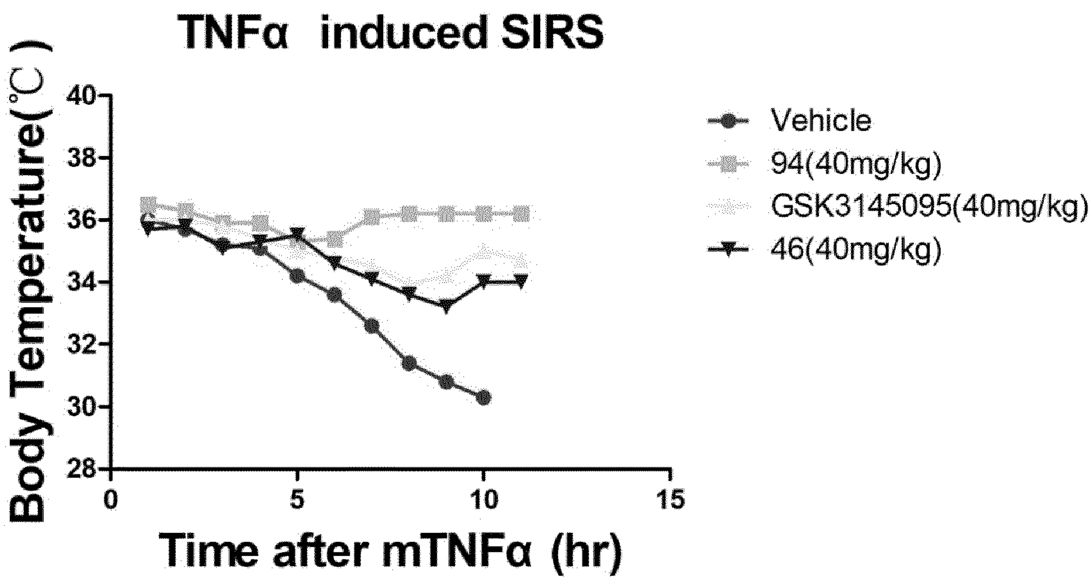
TNFα induced SIRS
Body Temperature(℃)
40
38
36
34
32
30
28
0
5
10
15
Time after mTNFα (hr)
Vehicle
94(40mg/kg)
GSK3145095(40mg/kg)
46(40mg/kg)

FIG. 5

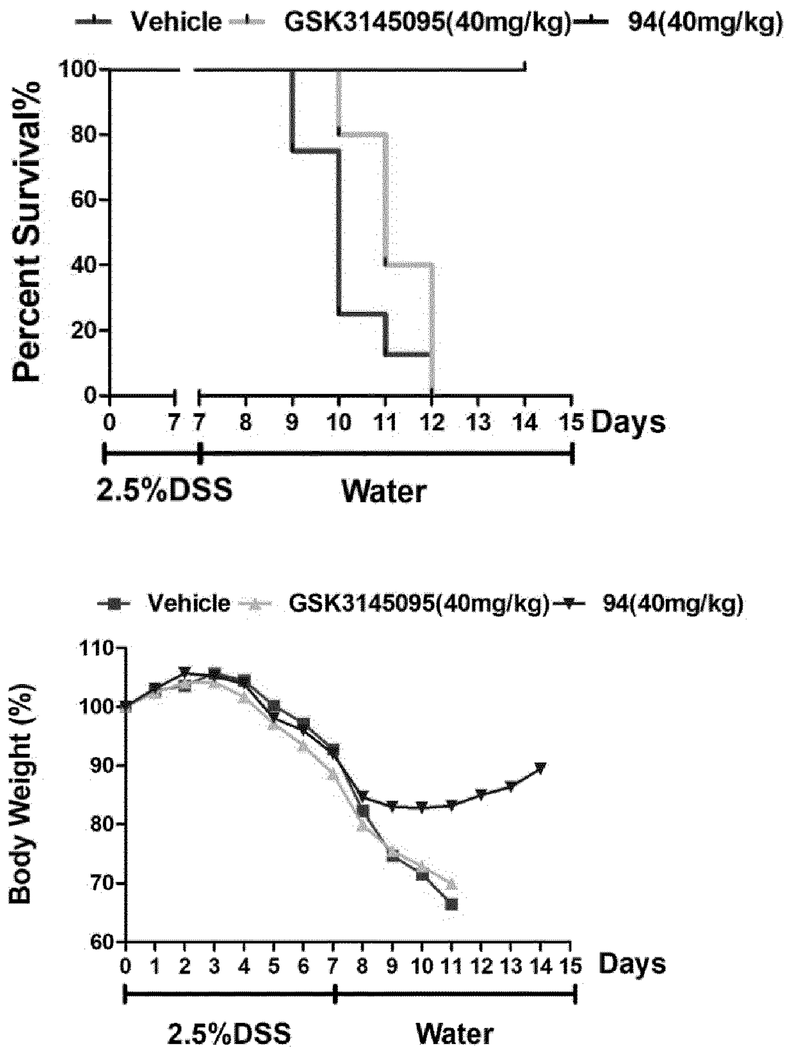
Vehicle
GSK3145095(40mg/kg)
94(40mg/kg)
Percent Survival%
100
80
60
40
20
0
0 7 7 8 9 10 11 12 13 14 15 Days
2.5%DSS
Water
Vehicle
GSK3145095(40mg/kg)
94(40mg/kg)
Body Weight (%)
110
100
90
80
70
60
0 1 2 3 4 5 6 7 8 9 10 11 12 13 14 15 Days
2.5%DSS
Water

FIG. 6

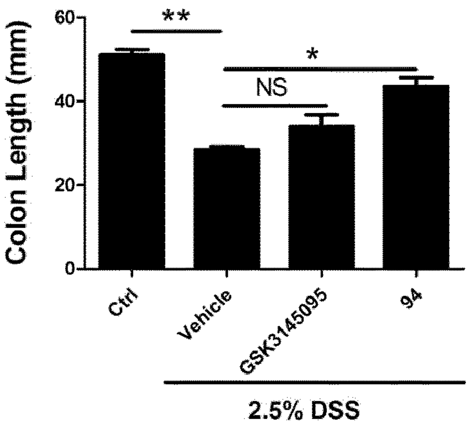
Colon Length (mm)
60
40
20
0
**
*
NS
Ctrl
Vehicle
GSK3145095
94
2.5% DSS

FIG. 7

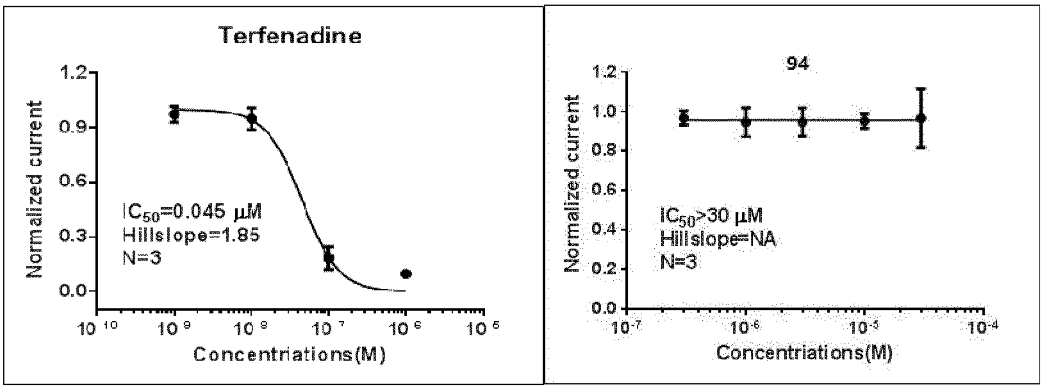
Terfenadine
Normalized current
1.2
0.9
0.6
0.3
0.0
IC50=0.045 µM
Hillslope=1.85
N=3
10 10
10 9
10 8
10 7
10 6
10 5
Concentriations(M)
94
Normalized current
1.2
1.0
0.8
0.6
0.4
0.2
0.0
IC50>30 µM
Hillslope=NA
N=3
10-7
10-6
10-5
10-4
Concentriations(M)

FIG. 8

**INTERNATIONAL SEARCH REPORT**

International application No. **PCT/CN2021/116256**

**A. CLASSIFICATION OF SUBJECT MATTER**

C07D 403/06(2006.01)i; A61K 31/404(2006.01)i; A61P 29/00(2006.01)i

According to International Patent Classification (IPC) or to both national classification and IPC

**B. FIELDS SEARCHED**

Minimum documentation searched (classification system followed by classification symbols)

C07D403/-; A61K31/-; A61P29/-

Documentation searched other than minimum documentation to the extent that such documents are included in the fields searched

Electronic data base consulted during the international search (name of data base and, where practicable, search terms used)

WPI, EPODOC, CNPAT, STN: structural formula, 酰胺, 吲哚, 炎症, amide, indole, inflammation

**C. DOCUMENTS CONSIDERED TO BE RELEVANT**

| Category* | Citation of document, with indication, where appropriate, of the relevant passages | Relevant to claim No. |
|---|---|---|
| X | WO 2011050245 A1 (FENG YANGBO et al.) 28 April 2011 (2011-04-28) claims 1-17, 38-39, 45-48, description page 69, embodiment 42, description pages 152-153 | 1-25 |
| X | WO 9806715 A1 (SMITHKLINE BEECHAM CORPORATION et al.) 19 February 1998 (1998-02-19) claims 1, 6, 12, description page 6, embodiment 24 | 16, 21-23, 25 |
| A | WO 2019215075 A1 (AZIENDE CHIMICHE RIUNITE ANGELINI FRANCESCO) 14 November 2019 (2019-11-14) description table A | 1-25 |
| A | CN 108368110 A (PLEXXIKON, INC.) 03 August 2018 (2018-08-03) claims 1-33 | 1-25 |
| A | WO 2020150552 A2 (SAMUMED LLC et al.) 23 July 2020 (2020-07-23) description, table 3 | 1-25 |
| A | CN 111434661 A (IKONO BIOMEDICAL CO., LTD.) 21 July 2020 (2020-07-21) description, pages 1-46 | 1-25 |
| A | CN 106535890 A (SAMUMED, LLC) 22 March 2017 (2017-03-22) claims 1-56 | 1-25 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 November 2021** | **25 November 2021** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/116256**

**C. DOCUMENTS CONSIDERED TO BE RELEVANT**

| Category* | Citation of document, with indication, where appropriate, of the relevant passages | Relevant to claim No. |
|---|---|---|
| A | WO 2018075858 A1 (SAMUMED LLC et al.) 26 April 2018 (2018-04-26) description, table 1 | 1-25 |
| A | WO 03082868 A1 (EISAI LONDON RESEARCH LABORATORIES LIMITED et al.) 09 October 2003 (2003-10-09) claims 1-63 | 1-25 |

Form PCT/ISA/210 (second sheet) (January 2015)

**INTERNATIONAL SEARCH REPORT**
**Information on patent family members**

International application No.
**PCT/CN2021/116256**

| Patent document cited in search report | Publication date (day/month/year) | Patent family member(s) | Publication date (day/month/year) |
|---|---|---|---|
| WO 2011050245 A1 | 28 April 2011 | None | |
| WO 9806715 A1 | 19 February 1998 | JP 2001506230 A | 15 May 2001 |
| | | EP 0922042 A1 | 16 June 1999 |
| WO 2019215075 A1 | 14 November 2019 | SG 11202009230 W A | 27 November 2020 |
| | | CN 112135821 A | 25 December 2020 |
| | | MA 52557 A | 17 March 2021 |
| | | KR 20210005863 A | 15 January 2021 |
| | | BR 112020021922 A2 | 26 January 2021 |
| | | CA 3094896 A1 | 14 November 2019 |
| | | AU 2019265606 A1 | 15 October 2020 |
| | | US 2021053956 A1 | 25 February 2021 |
| | | EA 202092422 A1 | 12 February 2021 |
| | | JP 2021523134 A | 02 September 2021 |
| | | EP 3790873 A1 | 17 March 2021 |
| CN 108368110 A | 03 August 2018 | RU 2018123825 A | 15 January 2020 |
| | | IL 259803 D0 | 31 July 2018 |
| | | JP 2018537533 A | 20 December 2018 |
| | | JP 6862468 B2 | 21 April 2021 |
| | | US 2017158690 A1 | 08 June 2017 |
| | | US 9938273 B2 | 10 April 2018 |
| | | CO 2018007052 A2 | 19 July 2018 |
| | | EP 3386980 A1 | 17 October 2018 |
| | | EP 3386980 B1 | 13 October 2021 |
| | | BR 112018011475 A2 | 04 December 2018 |
| | | MX 2018006856 A | 01 August 2018 |
| | | PH 12018501179 A1 | 04 February 2019 |
| | | WO 2017100201 A1 | 15 June 2017 |
| | | KR 20180086247 A | 30 July 2018 |
| | | AU 2016367147 A1 | 05 July 2018 |
| | | AU 2016367147 B2 | 08 April 2021 |
| | | SG 11201804711 R A | 30 July 2018 |
| | | CA 3007462 A1 | 15 June 2017 |
| WO 2020150552 A2 | 23 July 2020 | WO 2020150552 A3 | 27 August 2020 |
| CN 111434661 A | 21 July 2020 | WO 2020146858 A1 | 16 July 2020 |
| CN 106535890 A | 22 March 2017 | PE 20170127 A1 | 30 March 2017 |
| | | BR 112016021626 A2 | 15 May 2018 |
| | | US 2018127377 A1 | 10 May 2018 |
| | | US 10669240 B2 | 02 June 2020 |
| | | RU 2016141088 A | 25 April 2018 |
| | | RU 2016141088 A3 | 20 November 2018 |
| | | US 2015266825 A1 | 24 September 2015 |
| | | US 9745271 B2 | 29 August 2017 |
| | | MX 2016012208 A | 26 January 2017 |
| | | SG CN 11201607816 U A | 28 October 2016 |
| | | SG 10201914111 X A | 30 March 2020 |
| | | JP 2017508763 A | 30 March 2017 |
| | | JP 6586104 B2 | 02 October 2019 |
| | | MA 39763 A | 25 January 2017 |
| | | PH 12016501835 A1 | 09 January 2017 |

Form PCT/ISA/210 (patent family annex) (January 2015)

**INTERNATIONAL SEARCH REPORT**
**Information on patent family members**

International application No.
**PCT/CN2021/116256**

| Patent document cited in search report | Publication date (day/month/year) | Patent family member(s) | Publication date (day/month/year) |
|---|---|---|---|
| | | EP 3119393 A1 | 25 January 2017 |
| | | EP 3119393 A4 | 28 February 2018 |
| | | KR 20160135283 A | 25 November 2016 |
| | | CA 2942687 A1 | 24 September 2015 |
| | | WO 2015143380 A1 | 24 September 2015 |
| | | CL 2016002364 A1 | 23 June 2017 |
| | | IL 247713 D0 | 30 November 2016 |
| WO 2018075858 A1 | 26 April 2018 | CN 110709082 A | 17 January 2020 |
| | | RU 2019114954 A | 24 November 2020 |
| | | RU 2019114954 A3 | 28 January 2021 |
| | | MX 2019004616 A | 21 November 2019 |
| | | US 2021121448 A1 | 29 April 2021 |
| | | BR 112019008061 A2 | 17 September 2019 |
| | | KR 20190071782 A | 24 June 2019 |
| | | AU 2017345699 A1 | 16 May 2019 |
| | | IL 266145 D0 | 30 June 2019 |
| | | US 2018185343 A1 | 05 July 2018 |
| | | US 10806726 B2 | 20 October 2020 |
| | | JP 2019535672 A | 12 December 2019 |
| | | CL 2019001079 A1 | 15 November 2019 |
| | | CA 3041291 A1 | 26 April 2018 |
| | | EP 3528808 A1 | 28 August 2019 |
| | | EP 3528808 B1 | 06 October 2021 |
| WO 03082868 A1 | 09 October 2003 | CN 1656094 A | 17 August 2005 |
| | | CN 100368411 C | 13 February 2008 |
| | | AU 2003214412 A1 | 13 October 2003 |
| | | CA 2480317 A1 | 09 October 2003 |
| | | KR 20040111445 A | 31 December 2004 |
| | | EP 1490364 A1 | 29 December 2004 |
| | | EP 1490364 B1 | 26 September 2007 |
| | | JP 2005534618 A | 17 November 2005 |
| | | US 2005272761 A1 | 08 December 2005 |
| | | US 7534800 B2 | 19 May 2009 |
| | | AT 374200 T | 15 October 2007 |
| | | DE 60316542 D1 | 08 November 2007 |
| | | DE 60316542 T2 | 03 July 2008 |
| | | IL 164187 D0 | 18 December 2005 |

Form PCT/ISA/210 (patent family annex) (January 2015)